# EUROPÄISCHE PATENTSCHRIFT

(11) **EP 2 602 313 B1**
(45) Veröffentlichungstag und Bekanntmachung des Hinweises auf die Patenterteilung: **12.04.2017**
(21) Anmeldenummer: 13158312.2
(22) Anmeldetag: 27.10.2010
(51) Int. Cl.: C12N 1/16, C12N 9/02, C12P 7/42, C12P 7/62, C12R 1/74

(54) **Candida tropicalis Zellen und deren Verwendung**
Candida tropicalis cells and their use
Cellules de Candida tropicalis et leur utilisation

(30) Priorität: 11.11.2009 DE 102009046626
(43) Veröffentlichungstag der Anmeldung: 12.06.2013
(62) Teilanmeldung aus: 10188993.9
(73) Patentinhaber: Evonik Degussa GmbH, 45128 Essen (DE)
(72) Erfinder: Pötter, Markus, 48149 Münster (DE); Hennemann, Hans-Georg, 45770 Marl (DE); Schaffer, Steffen, 45699 Herten (DE); Haas, Thomas, 48161 Münster (DE)

(56) Entgegenhaltungen:
- WO-A1-91/06660
- WO-A2-03/100013
- DE-A1-102007 060 705
- DATABASE EMBL [Online] 22. Oktober 2009 (2009-10-22), Ness J.E.: "Candida tropicalis strain H5343 clone A10 alcohol dehydrogenase (ADH) gene, partial cds.", XP002694319, gefunden im EBI Database accession no. GU056283
- FABRITIUS D ET AL: "Identification and production of 3-hydroxy-[Delta]<9>-cis-1,18-octadecenedi oic acid by mutants of Candida tropicalis", APPLIED MICROBIOLOGY AND BIOTECHNOLOGY 1996 DE, Bd. 45, Nr. 3, 1996, Seiten 342-348, XP002694301, ISSN: 0175-7598
- CHENG QI ET AL: "Candida yeast long chain fatty alcohol oxidase is a c-type haemoprotein and plays an important role in long chain fatty acid metabolism.", BIOCHIMICA ET BIOPHYSICA ACTA 15 AUG 2005, Bd. 1735, Nr. 3, 15. August 2005 (2005-08-15), Seiten 192-203, XP002694302, ISSN: 0006-3002

### SEQUENCE LISTING

<110> Evonik Degussa GmbH
<120> Candida tropicalis Zellen und deren Verwendung
<130> 200900334
<160> 68
<170> PatentIn version 3.4
<210> 1
   <211> 1575
   <212> DNA
   <213> Candida tropicalis
<220>
   <221> CDS
   <222> (1)..(1575)
<400> 1
   atg gcc aca caa gaa att att gat tct gca ctt ccg tac ttg aca aag      48
   Met Ala Thr Gln Glu Ile Ile Asp Ser Ala Leu Pro Tyr Leu Thr Lys
   1               5                   10                  15
   tgg tat act gtt atc act tta gca gct ttg gtt ttc tta att tca tct      96
   Trp Tyr Thr Val Ile Thr Leu Ala Ala Leu Val Phe Leu Ile Ser Ser
               20                  25                  30
   aat att aaa aat tac gtc aag gct aag aag ttg aaa tgc aga gat cct     144
   Asn Ile Lys Asn Tyr Val Lys Ala Lys Lys Leu Lys Cys Arg Asp Pro
           35                  40                  45
   cca tat ttc aaa gga gcc ggt tgg aca ggt att agt cca tta att gaa     192
   Pro Tyr Phe Lys Gly Ala Gly Trp Thr Gly Ile Ser Pro Leu Ile Glu
       50                  55                  60
   att att aaa gtt aaa ggt aat ggt aga ttg gca aga ttt tgg ccg ata     240
   Ile Ile Lys Val Lys Gly Asn Gly Arg Leu Ala Arg Phe Trp Pro Ile
   65                  70                  75                  80
   aaa aca ttc gac gac tat cca aac cat act ttt tac atg tct att att     288
   Lys Thr Phe Asp Asp Tyr Pro Asn His Thr Phe Tyr Met Ser Ile Ile
                   85                  90                  95
   ggt gct ttg aaa atc gtc ttg act gtg atc caa gaa aat att aaa gct     336
   Gly Ala Leu Lys Ile Val Leu Thr Val Ile Gln Glu Asn Ile Lys Ala
               100                 105                 110
   gtt ttg gct act caa ttt act gat ttc tcc tta ggt act aga cat gcc     384
   Val Leu Ala Thr Gln Phe Thr Asp Phe Ser Leu Gly Thr Arg His Ala
           115                 120                 125
   cat ttc tat cca tta tta ggt gat ggt att ttt act ttg gat ggt gaa     432
   His Phe Tyr Pro Leu Leu Gly Asp Gly Ile Phe Thr Leu Asp Gly Glu
       130                 135                 140
   ggt tgg aaa cat agt aga gct atg ttg aga cca caa ttt gct aga gat     480
   Gly Trp Lys His Ser Arg Ala Met Leu Arg Pro Gln Phe Ala Arg Asp
   145                 150                 155                 160
   caa att ggt cat gtt aaa gct ttg gaa cca cat att caa atc ttg gct      528
   Gln Ile Gly His Val Lys Ala Leu Glu Pro His Ile Gln Ile Leu Ala
                   165                 170                 175
   aaa caa atc aaa ttg aat aaa ggt aaa act ttt gat att caa gaa ttg      576
   Lys Gln Ile Lys Leu Asn Lys Gly Lys Thr Phe Asp Ile Gln Glu Leu
               180                 185                 190
   ttt ttc aga ttt act gtt gat act gct act gaa ttc ttg ttt ggt gaa      624
   Phe Phe Arg Phe Thr Val Asp Thr Ala Thr Glu Phe Leu Phe Gly Glu
           195                 200                 205
   tct gtt cac tct ttg tat gat gaa aaa tta ggt att cct act cca aat      672
   Ser Val His Ser Leu Tyr Asp Glu Lys Leu Gly Ile Pro Thr Pro Asn
       210                 215                 220
   gaa att cca ggt aga gat aat ttt gca act gct ttt aac act tct caa      720
   Glu Ile Pro Gly Arg Asp Asn Phe Ala Thr Ala Phe Asn Thr Ser Gln
   225                 230                 235                 240
   cat tat ttg gct acc aga aca tac tcc caa act ttc tac ttt tta act      768
   His Tyr Leu Ala Thr Arg Thr Tyr Ser Gln Thr Phe Tyr Phe Leu Thr
                   245                 250                 255
   aac cct aag gaa ttt aga gac tgt aat gct aaa gtt cat tac ttg gct      816
   Asn Pro Lys Glu Phe Arg Asp Cys Asn Ala Lys Val His Tyr Leu Ala
               260                 265                 270
   aaa tat ttt gtc aat aaa gct ttg aat ttc act ccg gaa gaa att gaa      864
   Lys Tyr Phe Val Asn Lys Ala Leu Asn Phe Thr Pro Glu Glu Ile Glu
           275                 280                 285
   gaa aag tcc aaa tct ggt tat gtt ttc ttg tat gaa ttg gtt aaa caa      912
   Glu Lys Ser Lys Ser Gly Tyr Val Phe Leu Tyr Glu Leu Val Lys Gln
       290                 295                 300
   acc aga gat cca aaa gtt tta caa gat caa tta ttg aac att atg gtt      960
   Thr Arg Asp Pro Lys Val Leu Gln Asp Gln Leu Leu Asn Ile Met Val
   305                 310                 315                 320
   gcc ggt aga gat acc act gct ggt tta tta tca ttt gca atg ttt gaa     1008
   Ala Gly Arg Asp Thr Thr Ala Gly Leu Leu Ser Phe Ala Met Phe Glu
                   325                 330                 335
   tta gct aga cat cca gaa att tgg tct aaa tta aga gaa gaa att gaa     1056
   Leu Ala Arg His Pro Glu Ile Trp Ser Lys Leu Arg Glu Glu Ile Glu
               340                 345                 350
   gtt aac ttt ggt gtt ggt gaa gaa tct cgt gtt gaa gaa att act ttt     1104
   Val Asn Phe Gly Val Gly Glu Glu Ser Arg Val Glu Glu Ile Thr Phe
           355                 360                 365
   gaa tct ttg aag aga tgt gaa tac ttg aaa gct att ctt aat gaa act     1152
   Glu Ser Leu Lys Arg Cys Glu Tyr Leu Lys Ala Ile Leu Asn Glu Thr
       370                 375                 380
   ttg cgt atg tat cct tct gtt cca gtt aat tcc aga aca gcc act aga     1200
   Leu Arg Met Tyr Pro Ser Val Pro Val Asn Ser Arg Thr Ala Thr Arg
   385                 390                 395                 400
   gat acc aca tta cca aga ggt ggt ggt cca aat ggt act gat cca att     1248
   Asp Thr Thr Leu Pro Arg Gly Gly Gly Pro Asn Gly Thr Asp Pro Ile
                   405                 410                 415
   ttt att cca aag ggt tcc act gtt gct tat att gtt tac aaa act cat      1296
   Phe Ile Pro Lys Gly Ser Thr Val Ala Tyr Ile Val Tyr Lys Thr His
               420                 425                 430
   cgt tta gaa gaa tat tat ggt aaa gat gct gat gat ttc aga cca gaa      1344
   Arg Leu Glu Glu Tyr Tyr Gly Lys Asp Ala Asp Asp Phe Arg Pro Glu
           435                 440                 445
   aga tgg ttt gaa cca tca act aaa aag tta ggt tgg gct tat gtt cca      1392
   Arg Trp Phe Glu Pro Ser Thr Lys Lys Leu Gly Trp Ala Tyr Val Pro
       450                 455                 460
   ttt aat ggt ggt cca aga att tgt tta ggc caa caa ttt gct tta act      1440
   Phe Asn Gly Gly Pro Arg Ile Cys Leu Gly Gln Gln Phe Ala Leu Thr
   465                 470                 475                 480
   gaa gct tct tat gtt att acc aga ttg gta caa atg ttt gaa act gtt      1488
   Glu Ala Ser Tyr Val Ile Thr Arg Leu Val Gln Met Phe Glu Thr Val
                   485                 490                 495
   tct tct ccc cca gat gtt gaa tac cct cca cca aaa tgt att cat ttg      1536
   Ser Ser Pro Pro Asp Val Glu Tyr Pro Pro Pro Lys Cys Ile His Leu
               500                 505                 510
   act atg agt cat gat gat ggt gtt ttc gtt aaa atg taa                  1575
   Thr Met Ser His Asp Asp Gly Val Phe Val Lys Met
           515                 520
<210> 2
   <211> 524
   <212> PRT
   <213> Candida tropicalis
<400> 2
   Met Ala Thr Gln Glu Ile Ile Asp Ser Ala Leu Pro Tyr Leu Thr Lys
   1               5                   10                  15
   Trp Tyr Thr Val Ile Thr Leu Ala Ala Leu Val Phe Leu Ile Ser Ser
               20                  25                  30
   Asn Ile Lys Asn Tyr Val Lys Ala Lys Lys Leu Lys Cys Arg Asp Pro
           35                  40                  45
   Pro Tyr Phe Lys Gly Ala Gly Trp Thr Gly Ile Ser Pro Leu Ile Glu
       50                  55                  60
   Ile Ile Lys Val Lys Gly Asn Gly Arg Leu Ala Arg Phe Trp Pro Ile
   65                  70                  75                  80
   Lys Thr Phe Asp Asp Tyr Pro Asn His Thr Phe Tyr Met Ser Ile Ile
                   85                  90                  95
   Gly Ala Leu Lys Ile Val Leu Thr Val Ile Gln Glu Asn Ile Lys Ala
               100                 105                 110
   Val Leu Ala Thr Gln Phe Thr Asp Phe Ser Leu Gly Thr Arg His Ala
           115                 120                 125
   His Phe Tyr Pro Leu Leu Gly Asp Gly Ile Phe Thr Leu Asp Gly Glu
       130                 135                 140
   Gly Trp Lys His Ser Arg Ala Met Leu Arg Pro Gln Phe Ala Arg Asp
   145                 150                 155                 160
   Gln Ile Gly His Val Lys Ala Leu Glu Pro His Ile Gln Ile Leu Ala
                   165                 170                 175
   Lys Gln Ile Lys Leu Asn Lys Gly Lys Thr Phe Asp Ile Gln Glu Leu
               180                 185                 190
   Phe Phe Arg Phe Thr Val Asp Thr Ala Thr Glu Phe Leu Phe Gly Glu
           195                 200                 205
   Ser Val His Ser Leu Tyr Asp Glu Lys Leu Gly Ile Pro Thr Pro Asn
       210                 215                 220
   Glu Ile Pro Gly Arg Asp Asn Phe Ala Thr Ala Phe Asn Thr Ser Gln
   225                 230                 235                 240
   His Tyr Leu Ala Thr Arg Thr Tyr Ser Gln Thr Phe Tyr Phe Leu Thr
                   245                 250                 255
   Asn Pro Lys Glu Phe Arg Asp Cys Asn Ala Lys Val His Tyr Leu Ala
               260                 265                 270
   Lys Tyr Phe Val Asn Lys Ala Leu Asn Phe Thr Pro Glu Glu Ile Glu
           275                 280                 285
   Glu Lys Ser Lys Ser Gly Tyr Val Phe Leu Tyr Glu Leu Val Lys Gln
       290                 295                 300
   Thr Arg Asp Pro Lys Val Leu Gln Asp Gln Leu Leu Asn Ile Met Val
   305                 310                 315                 320
   Ala Gly Arg Asp Thr Thr Ala Gly Leu Leu Ser Phe Ala Met Phe Glu
                   325                 330                 335
   Leu Ala Arg His Pro Glu Ile Trp Ser Lys Leu Arg Glu Glu Ile Glu
               340                 345                 350
   Val Asn Phe Gly Val Gly Glu Glu Ser Arg Val Glu Glu Ile Thr Phe
               355                 360                 365
   Glu Ser Leu Lys Arg Cys Glu Tyr Leu Lys Ala Ile Leu Asn Glu Thr
       370                 375                 380
   Leu Arg Met Tyr Pro Ser Val Pro Val Asn Ser Arg Thr Ala Thr Arg
   385                 390                 395                 400
   Asp Thr Thr Leu Pro Arg Gly Gly Gly Pro Asn Gly Thr Asp Pro Ile
                   405                 410                 415
   Phe Ile Pro Lys Gly Ser Thr Val Ala Tyr Ile Val Tyr Lys Thr His
               420                 425                 430
   Arg Leu Glu Glu Tyr Tyr Gly Lys Asp Ala Asp Asp Phe Arg Pro Glu
           435                 440                 445
   Arg Trp Phe Glu Pro Ser Thr Lys Lys Leu Gly Trp Ala Tyr Val Pro
       450                 455                 460
   Phe Asn Gly Gly Pro Arg Ile Cys Leu Gly Gln Gln Phe Ala Leu Thr
   465                 470                 475                 480
   Glu Ala Ser Tyr Val Ile Thr Arg Leu Val Gln Met Phe Glu Thr Val
                   485                 490                 495
   Ser Ser Pro Pro Asp Val Glu Tyr Pro Pro Pro Lys Cys Ile His Leu
               500                 505                 510
   Thr Met Ser His Asp Asp Gly Val Phe Val Lys Met
           515                 520
<210> 3
   <211> 1572
   <212> DNA
   <213> Candida tropicalis
<220>
   <221> CDS
   <222> (1)..(1572)
<400> 3
   atg gcc aca caa gaa atc atc gat tct gta ctt ccg tac ttg acc aaa       48
   Met Ala Thr Gln Glu Ile Ile Asp Ser Val Leu Pro Tyr Leu Thr Lys
   1               5                   10                  15
   tgg tac act gtg att act gca gca gta tta gtc ttc ctt atc tcc aca       96
   Trp Tyr Thr Val Ile Thr Ala Ala Val Leu Val Phe Leu Ile Ser Thr
               20                  25                  30
   aac atc aag aac tac gtc aag gca aag aaa ttg aaa tgt gtc gat cca      144
   Asn Ile Lys Asn Tyr Val Lys Ala Lys Lys Leu Lys Cys Val Asp Pro
           35                  40                  45
   cca tac ttg aag gat gcc ggt ctc act ggt att ctg tct ttg atc gcc      192
   Pro Tyr Leu Lys Asp Ala Gly Leu Thr Gly Ile Leu Ser Leu Ile Ala
       50                  55                  60
   gcc atc aag gcc aag aac gac ggt aga ttg gct aac ttt gcc gat gaa      240
   Ala Ile Lys Ala Lys Asn Asp Gly Arg Leu Ala Asn Phe Ala Asp Glu
   65                  70                  75                  80
   gtt ttc gac gag tac cca aac cac acc ttc tac ttg tct gtt gcc ggt      288
   Val Phe Asp Glu Tyr Pro Asn His Thr Phe Tyr Leu Ser Val Ala Gly
                   85                  90                  95
   gct ttg aag att gtc atg act gtt gac cca gaa aac atc aag gct gtc      336
   Ala Leu Lys Ile Val Met Thr Val Asp Pro Glu Asn Ile Lys Ala Val
               100                 105                 110
   ttg gcc acc caa ttc act gac ttc tcc ttg ggt acc aga cac gcc cac      384
   Leu Ala Thr Gln Phe Thr Asp Phe Ser Leu Gly Thr Arg His Ala His
           115                 120                 125
   ttt gct cct ttg ttg ggt gac ggt atc ttc acc ttg gac gga gaa ggt      432
   Phe Ala Pro Leu Leu Gly Asp Gly Ile Phe Thr Leu Asp Gly Glu Gly
       130                 135                 140
   tgg aag cac tcc aga gct atg ttg aga cca cag ttt gct aga gac cag      480
   Trp Lys His Ser Arg Ala Met Leu Arg Pro Gln Phe Ala Arg Asp Gln
   145                 150                 155                 160
   att gga cac gtt aaa gcc ttg gaa cca cac atc caa atc atg gct aag      528
   Ile Gly His Val Lys Ala Leu Glu Pro His Ile Gln Ile Met Ala Lys
                   165                 170                 175
   cag atc aag ttg aac cag gga aag act ttc gat atc caa gaa ttg ttc      576
   Gln Ile Lys Leu Asn Gln Gly Lys Thr Phe Asp Ile Gln Glu Leu Phe
               180                 185                 190
   ttt aga ttt acc gtc gac acc gct act gag ttc ttg ttt ggt gaa tcc      624
   Phe Arg Phe Thr Val Asp Thr Ala Thr Glu Phe Leu Phe Gly Glu Ser
           195                 200                 205
   gtt cac tcc ttg tac gat gaa aaa ttg ggc atc cca act cca aac gaa      672
   Val His Ser Leu Tyr Asp Glu Lys Leu Gly Ile Pro Thr Pro Asn Glu
       210                 215                 220
   atc cca gga aga gaa aac ttt gcc gct gct ttc aac gtt tcc caa cac      720
   Ile Pro Gly Arg Glu Asn Phe Ala Ala Ala Phe Asn Val Ser Gln His
   225                 230                 235                 240
   tac ttg gcc acc aga agt tac tcc cag act ttt tac ttt ttg acc aac      768
   Tyr Leu Ala Thr Arg Ser Tyr Ser Gln Thr Phe Tyr Phe Leu Thr Asn
                   245                 250                 255
   cct aag gaa ttc aga gac tgt aac gcc aag gtc cac cac ttg gcc aag      816
   Pro Lys Glu Phe Arg Asp Cys Asn Ala Lys Val His His Leu Ala Lys
               260                 265                 270
   tac ttt gtc aac aag gcc ttg aac ttt act cct gaa gaa ctc gaa gag      864
   Tyr Phe Val Asn Lys Ala Leu Asn Phe Thr Pro Glu Glu Leu Glu Glu
           275                 280                 285
   aaa tcc aag tcc ggt tac gtt ttc ttg tac gaa ttg gtt aag caa acc      912
   Lys Ser Lys Ser Gly Tyr Val Phe Leu Tyr Glu Leu Val Lys Gln Thr
       290                 295                 300
   aga gat cca aag gtc ttg caa gat caa ttg ttg aac att atg gtt gcc      960
   Arg Asp Pro Lys Val Leu Gln Asp Gln Leu Leu Asn Ile Met Val Ala
   305                 310                 315                 320
   gga aga gac acc act gcc ggt ttg ttg tcc ttt gct ttg ttt gaa ttg     1008
   Gly Arg Asp Thr Thr Ala Gly Leu Leu Ser Phe Ala Leu Phe Glu Leu
                   325                 330                 335
   gct aga cac cca gag atg tgg tcc aag ttg aga gaa gaa atc gaa gtt     1056
   Ala Arg His Pro Glu Met Trp Ser Lys Leu Arg Glu Glu Ile Glu Val
               340                 345                 350
   aac ttt ggt gtt ggt gaa gac tcc cgc gtt gaa gaa att acc ttc gaa     1104
   Asn Phe Gly Val Gly Glu Asp Ser Arg Val Glu Glu Ile Thr Phe Glu
           355                 360                 365
   gcc ttg aag aga tgt gaa tac ttg aag gct atc ctt aac gaa acc ttg     1152
   Ala Leu Lys Arg Cys Glu Tyr Leu Lys Ala Ile Leu Asn Glu Thr Leu
       370                 375                 380
   cgt atg tac cca tct gtt cct gtc aac ttt aga acc gcc acc aga gac     1200
   Arg Met Tyr Pro Ser Val Pro Val Asn Phe Arg Thr Ala Thr Arg Asp
   385                 390                 395                 400
   acc act ttg cca aga ggt ggt ggt gct aac ggt acc gac cca atc tac     1248
   Thr Thr Leu Pro Arg Gly Gly Gly Ala Asn Gly Thr Asp Pro Ile Tyr
                   405                 410                 415
   att cct aaa ggc tcc act gtt gct tac gtt gtc tac aag acc cac cgt     1296
   Ile Pro Lys Gly Ser Thr Val Ala Tyr Val Val Tyr Lys Thr His Arg
               420                 425                 430
   ttg gaa gaa tac tac ggt aag gac gct aac gac ttc aga cca gaa aga     1344
   Leu Glu Glu Tyr Tyr Gly Lys Asp Ala Asn Asp Phe Arg Pro Glu Arg
           435                 440                 445
   tgg ttt gaa cca tct act aag aag ttg ggc tgg gct tat gtt cca ttc     1392
   Trp Phe Glu Pro Ser Thr Lys Lys Leu Gly Trp Ala Tyr Val Pro Phe
       450                 455                 460
   aac ggt ggt cca aga gtc tgc ttg ggt caa caa ttc gcc ttg act gaa     1440
   Asn Gly Gly Pro Arg Val Cys Leu Gly Gln Gln Phe Ala Leu Thr Glu
   465                 470                 475                 480
   gct tct tat gtg atc act aga ttg gcc cag atg ttt gaa act gtc tca     1488
   Ala Ser Tyr Val Ile Thr Arg Leu Ala Gln Met Phe Glu Thr Val Ser
                   485                 490                 495
   tct gat cca ggt ctc gaa tac cct cca cca aag tgt att cac ttg acc     1536
   Ser Asp Pro Gly Leu Glu Tyr Pro Pro Pro Lys Cys Ile His Leu Thr
               500                 505                 510
   atg agt cac aac gat ggt gtc ttt gtc aag atg taa                     1572
   Met Ser His Asn Asp Gly Val Phe Val Lys Met
           515                 520
<210> 4
   <211> 523
   <212> PRT
   <213> Candida tropicalis
<400> 4
   Met Ala Thr Gln Glu Ile Ile Asp Ser Val Leu Pro Tyr Leu Thr Lys
   1               5                   10                  15
   Trp Tyr Thr Val Ile Thr Ala Ala Val Leu Val Phe Leu Ile Ser Thr
               20                  25                  30
   Asn Ile Lys Asn Tyr Val Lys Ala Lys Lys Leu Lys Cys Val Asp Pro
           35                  40                  45
   Pro Tyr Leu Lys Asp Ala Gly Leu Thr Gly Ile Leu Ser Leu Ile Ala
       50                  55                  60
   Ala Ile Lys Ala Lys Asn Asp Gly Arg Leu Ala Asn Phe Ala Asp Glu
   65                  70                  75                  80
   Val Phe Asp Glu Tyr Pro Asn His Thr Phe Tyr Leu Ser Val Ala Gly
                   85                  90                  95
   Ala Leu Lys Ile Val Met Thr Val Asp Pro Glu Asn Ile Lys Ala Val
               100                 105                 110
   Leu Ala Thr Gln Phe Thr Asp Phe Ser Leu Gly Thr Arg His Ala His
           115                 120                 125
   Phe Ala Pro Leu Leu Gly Asp Gly Ile Phe Thr Leu Asp Gly Glu Gly
       130                 135                 140
   Trp Lys His Ser Arg Ala Met Leu Arg Pro Gln Phe Ala Arg Asp Gln
   145                 150                 155                 160
   Ile Gly His Val Lys Ala Leu Glu Pro His Ile Gln Ile Met Ala Lys
                   165                 170                 175
   Gln Ile Lys Leu Asn Gln Gly Lys Thr Phe Asp Ile Gln Glu Leu Phe
               180                 185                 190
   Phe Arg Phe Thr Val Asp Thr Ala Thr Glu Phe Leu Phe Gly Glu Ser
           195                 200                 205
   Val His Ser Leu Tyr Asp Glu Lys Leu Gly Ile Pro Thr Pro Asn Glu
       210                 215                 220
   Ile Pro Gly Arg Glu Asn Phe Ala Ala Ala Phe Asn Val Ser Gln His
   225                 230                 235                 240
   Tyr Leu Ala Thr Arg Ser Tyr Ser Gln Thr Phe Tyr Phe Leu Thr Asn
   245 250 255
   Pro Lys Glu Phe Arg Asp Cys Asn Ala Lys Val His His Leu Ala Lys
   260 265 270
   Tyr Phe Val Asn Lys Ala Leu Asn Phe Thr Pro Glu Glu Leu Glu Glu
   275 280 285
   Lys Ser Lys Ser Gly Tyr Val Phe Leu Tyr Glu Leu Val Lys Gln Thr
   290 295 300
   Arg Asp Pro Lys Val Leu Gln Asp Gln Leu Leu Asn Ile Met Val Ala
   305 310 315 320
   Gly Arg Asp Thr Thr Ala Gly Leu Leu Ser Phe Ala Leu Phe Glu Leu
   325 330 335
   Ala Arg His Pro Glu Met Trp Ser Lys Leu Arg Glu Glu Ile Glu Val
   340 345 350
   Asn Phe Gly Val Gly Glu Asp Ser Arg Val Glu Glu Ile Thr Phe Glu
   355 360 365
   Ala Leu Lys Arg Cys Glu Tyr Leu Lys Ala Ile Leu Asn Glu Thr Leu
   370 375 380
   Arg Met Tyr Pro Ser Val Pro Val Asn Phe Arg Thr Ala Thr Arg Asp
   385 390 395 400
   Thr Thr Leu Pro Arg Gly Gly Gly Ala Asn Gly Thr Asp Pro Ile Tyr
   405 410 415
   Ile Pro Lys Gly Ser Thr Val Ala Tyr Val Val Tyr Lys Thr His Arg
   420 425 430
   Leu Glu Glu Tyr Tyr Gly Lys Asp Ala Asn Asp Phe Arg Pro Glu Arg
   435 440 445
   Trp Phe Glu Pro Ser Thr Lys Lys Leu Gly Trp Ala Tyr Val Pro Phe
   450 455 460
   Asn Gly Gly Pro Arg Val Cys Leu Gly Gln Gln Phe Ala Leu Thr Glu
   465 470 475 480
   Ala Ser Tyr Val Ile Thr Arg Leu Ala Gln Met Phe Glu Thr Val Ser
   485 490 495
   Ser Asp Pro Gly Leu Glu Tyr Pro Pro Pro Lys Cys Ile His Leu Thr
   500 505 510
   Met Ser His Asn Asp Gly Val Phe Val Lys Met
   515 520
<210> 5
   <211> 1569
   <212> DNA
   <213> Candida tropicalis
<220>
   <221> CDS
   <222> (1)..(1569)
<400> 5
   atg act gta cac gat att atc gcc aca tac ttc acc aaa tgg tac gtg      48
   Met Thr Val His Asp Ile Ile Ala Thr Tyr Phe Thr Lys Trp Tyr Val
   1               5                   10                  15
   ata gta cca ctc gct ttg att gct tat aga gtc ctc gac tac ttc tat      96
   Ile Val Pro Leu Ala Leu Ile Ala Tyr Arg Val Leu Asp Tyr Phe Tyr
               20                  25                  30
   ggc aga tac ttg atg tac aag ctt ggt gct aaa cca ttt ttc cag aaa     144
   Gly Arg Tyr Leu Met Tyr Lys Leu Gly Ala Lys Pro Phe Phe Gln Lys
           35                  40                  45
   cag aca gac ggc tgt ttc gga ttc aaa gct ccg ctt gaa ttg ttg aag     192
   Gln Thr Asp Gly Cys Phe Gly Phe Lys Ala Pro Leu Glu Leu Leu Lys
       50                  55                  60
   aag aag agc gac ggt acc ctc ata gac ttc aca ctc cag cgt atc cac     240
   Lys Lys Ser Asp Gly Thr Leu Ile Asp Phe Thr Leu Gln Arg Ile His
   65                  70                  75                  80
   gat ctc gat cgt ccc gat atc cca act ttc aca ttc ccg gtc ttt tcc     288
   Asp Leu Asp Arg Pro Asp Ile Pro Thr Phe Thr Phe Pro Val Phe Ser
                   85                  90                  95
   atc aac ctt gtc aat acc ctt gag ccg gag aac atc aag gcc atc ttg     336
   Ile Asn Leu Val Asn Thr Leu Glu Pro Glu Asn Ile Lys Ala Ile Leu
               100                 105                 110
   gcc act cag ttc aac gat ttc tcc ttg ggt acc aga cac tcg cac ttt     384
   Ala Thr Gln Phe Asn Asp Phe Ser Leu Gly Thr Arg His Ser His Phe
           115                 120                 125
   gct cct ttg ttg ggt gat ggt atc ttt acg ttg gat ggc gcc ggc tgg     432
   Ala Pro Leu Leu Gly Asp Gly Ile Phe Thr Leu Asp Gly Ala Gly Trp
       130                 135                 140
   aag cac agc aga tct atg ttg aga cca cag ttt gcc aga gaa cag att     480
   Lys His Ser Arg Ser Met Leu Arg Pro Gln Phe Ala Arg Glu Gln Ile
   145                 150                 155                 160
   tcc cac gtc aag ttg ttg gag cca cac gtt cag gtg ttc ttc aaa cac     528
   Ser His Val Lys Leu Leu Glu Pro His Val Gln Val Phe Phe Lys His
                   165                 170                 175
   gtc aga aag gca cag ggc aag act ttt gac atc cag gaa ttg ttt ttc      576
   Val Arg Lys Ala Gln Gly Lys Thr Phe Asp Ile Gln Glu Leu Phe Phe
               180                 185                 190
   aga ttg acc gtc gac tcc gcc acc gag ttt ttg ttt ggt gaa tcc gtt      624
   Arg Leu Thr Val Asp Ser Ala Thr Glu Phe Leu Phe Gly Glu Ser Val
           195                 200                 205
   gag tcc ttg aga gat gaa tct atc ggc atg tcc atc aat gcg ctt gac      672
   Glu Ser Leu Arg Asp Glu Ser Ile Gly Met Ser Ile Asn Ala Leu Asp
       210                 215                 220
   ttt gac ggc aag gct ggc ttt gct gat gct ttt aac tat tcg cag aat      720
   Phe Asp Gly Lys Ala Gly Phe Ala Asp Ala Phe Asn Tyr Ser Gln Asn
   225                 230                 235                 240
   tat ttg gct tcg aga gcg gtt atg caa caa ttg tac tgg gtg ttg aac      768
   Tyr Leu Ala Ser Arg Ala Val Met Gln Gln Leu Tyr Trp Val Leu Asn
                   245                 250                 255
   ggg aaa aag ttt aag gag tgc aac gct aaa gtg cac aag ttt gct gac      816
   Gly Lys Lys Phe Lys Glu Cys Asn Ala Lys Val His Lys Phe Ala Asp
               260                 265                 270
   tac tac gtc aac aag gct ttg gac ttg acg cct gaa caa ttg gaa aag      864
   Tyr Tyr Val Asn Lys Ala Leu Asp Leu Thr Pro Glu Gln Leu Glu Lys
           275                 280                 285
   cag gat ggt tat gtg ttt ttg tac gaa ttg gtc aag caa acc aga gac      912
   Gln Asp Gly Tyr Val Phe Leu Tyr Glu Leu Val Lys Gln Thr Arg Asp
       290                 295                 300
   aag caa gtg ttg aga gac caa ttg ttg aac atc atg gtt gct ggt aga      960
   Lys Gln Val Leu Arg Asp Gln Leu Leu Asn Ile Met Val Ala Gly Arg
   305                 310                 315                 320
   gac acc acc gcc ggt ttg ttg tcg ttt gtt ttc ttt gaa ttg gcc aga     1008
   Asp Thr Thr Ala Gly Leu Leu Ser Phe Val Phe Phe Glu Leu Ala Arg
                   325                 330                 335
   aac cca gaa gtt acc aac aag ttg aga gaa gaa att gag gac aag ttt     1056
   Asn Pro Glu Val Thr Asn Lys Leu Arg Glu Glu Ile Glu Asp Lys Phe
               340                 345                 350
   gga ctc ggt gag aat gct agt gtt gaa gac att tcc ttt gag tcg ttg     1104
   Gly Leu Gly Glu Asn Ala Ser Val Glu Asp Ile Ser Phe Glu Ser Leu
           355                 360                 365
   aag tcc tgt gaa tac ttg aag gct gtt ctc aac gaa acc ttg aga ttg     1152
   Lys Ser Cys Glu Tyr Leu Lys Ala Val Leu Asn Glu Thr Leu Arg Leu
       370                 375                 380
   tac cca tcc gtg cca cag aat ttc aga gtt gcc acc aag aac act acc     1200
   Tyr Pro Ser Val Pro Gln Asn Phe Arg Val Ala Thr Lys Asn Thr Thr
   385                 390                 395                 400
   ctc cca aga ggt ggt ggt aag gac ggg ttg tct cct gtt ttg gtg aga     1248
   Leu Pro Arg Gly Gly Gly Lys Asp Gly Leu Ser Pro Val Leu Val Arg
                   405                 410                 415
   aag ggt cag acc gtt att tac ggt gtc tac gca gcc cac aga aac cca      1296
   Lys Gly Gln Thr Val Ile Tyr Gly Val Tyr Ala Ala His Arg Asn Pro
               420                 425                 430
   gct gtt tac ggt aag gac gct ctt gag ttt aga cca gag aga tgg ttt      1344
   Ala Val Tyr Gly Lys Asp Ala Leu Glu Phe Arg Pro Glu Arg Trp Phe
           435                 440                 445
   gag cca gag aca aag aag ctt ggc tgg gcc ttc ctc cca ttc aac ggt      1392
   Glu Pro Glu Thr Lys Lys Leu Gly Trp Ala Phe Leu Pro Phe Asn Gly
       450                 455                 460
   ggt cca aga atc tgt ttg gga cag cag ttt gcc ttg aca gaa gct tcg      1440
   Gly Pro Arg Ile Cys Leu Gly Gln Gln Phe Ala Leu Thr Glu Ala Ser
   465                 470                 475                 480
   tat gtc act gtc agg ttg ctc cag gag ttt gca cac ttg tct atg gac      1488
   Tyr Val Thr Val Arg Leu Leu Gln Glu Phe Ala His Leu Ser Met Asp
                   485                 490                 495
   cca gac acc gaa tat cca cct aag aaa atg tcg cat ttg acc atg tcg      1536
   Pro Asp Thr Glu Tyr Pro Pro Lys Lys Met Ser His Leu Thr Met Ser
               500                 505                 510
   ctt ttc gac ggt gcc aat att gag atg tat tag                          1569
   Leu Phe Asp Gly Ala Asn Ile Glu Met Tyr
           515                 520
<210> 6
   <211> 522
   <212> PRT
   <213> Candida tropicalis
<400> 6
   Met Thr Val His Asp Ile Ile Ala Thr Tyr Phe Thr Lys Trp Tyr Val
   1               5                   10                  15
   Ile Val Pro Leu Ala Leu Ile Ala Tyr Arg Val Leu Asp Tyr Phe Tyr
               20                  25                  30
   Gly Arg Tyr Leu Met Tyr Lys Leu Gly Ala Lys Pro Phe Phe Gln Lys
           35                  40                  45
   Gln Thr Asp Gly Cys Phe Gly Phe Lys Ala Pro Leu Glu Leu Leu Lys
       50                  55                  60
   Lys Lys Ser Asp Gly Thr Leu Ile Asp Phe Thr Leu Gln Arg Ile His
   65                  70                  75                  80
   Asp Leu Asp Arg Pro Asp Ile Pro Thr Phe Thr Phe Pro Val Phe Ser
                   85                  90                  95
   Ile Asn Leu Val Asn Thr Leu Glu Pro Glu Asn Ile Lys Ala Ile Leu
               100                 105                 110
   Ala Thr Gln Phe Asn Asp Phe Ser Leu Gly Thr Arg His Ser His Phe
   115 120 125
   Ala Pro Leu Leu Gly Asp Gly Ile Phe Thr Leu Asp Gly Ala Gly Trp
   130 135 140
   Lys His Ser Arg Ser Met Leu Arg Pro Gln Phe Ala Arg Glu Gln Ile
   145 150 155 160
   Ser His Val Lys Leu Leu Glu Pro His Val Gln Val Phe Phe Lys His
   165 170 175
   Val Arg Lys Ala Gln Gly Lys Thr Phe Asp Ile Gln Glu Leu Phe Phe
   180 185 190
   Arg Leu Thr Val Asp Ser Ala Thr Glu Phe Leu Phe Gly Glu Ser Val
   195 200 205
   Glu Ser Leu Arg Asp Glu Ser Ile Gly Met Ser Ile Asn Ala Leu Asp
   210 215 220
   Phe Asp Gly Lys Ala Gly Phe Ala Asp Ala Phe Asn Tyr Ser Gln Asn
   225 230 235 240
   Tyr Leu Ala Ser Arg Ala Val Met Gln Gln Leu Tyr Trp Val Leu Asn
   245 250 255
   Gly Lys Lys Phe Lys Glu Cys Asn Ala Lys Val His Lys Phe Ala Asp
   260 265 270
   Tyr Tyr Val Asn Lys Ala Leu Asp Leu Thr Pro Glu Gln Leu Glu Lys
   275 280 285
   Gln Asp Gly Tyr Val Phe Leu Tyr Glu Leu Val Lys Gln Thr Arg Asp
   290 295 300
   Lys Gln Val Leu Arg Asp Gln Leu Leu Asn Ile Met Val Ala Gly Arg
   305 310 315 320
   Asp Thr Thr Ala Gly Leu Leu Ser Phe Val Phe Phe Glu Leu Ala Arg
   325 330 335
   Asn Pro Glu Val Thr Asn Lys Leu Arg Glu Glu Ile Glu Asp Lys Phe
   340 345 350
   Gly Leu Gly Glu Asn Ala Ser Val Glu Asp Ile Ser Phe Glu Ser Leu
   355 360 365
   Lys Ser Cys Glu Tyr Leu Lys Ala Val Leu Asn Glu Thr Leu Arg Leu
       370                 375                 380
   Tyr Pro Ser Val Pro Gln Asn Phe Arg Val Ala Thr Lys Asn Thr Thr
   385                 390                 395                 400
   Leu Pro Arg Gly Gly Gly Lys Asp Gly Leu Ser Pro Val Leu Val Arg
                   405                 410                 415
   Lys Gly Gln Thr Val Ile Tyr Gly Val Tyr Ala Ala His Arg Asn Pro
               420                 425                 430
   Ala Val Tyr Gly Lys Asp Ala Leu Glu Phe Arg Pro Glu Arg Trp Phe
           435                 440                 445
   Glu Pro Glu Thr Lys Lys Leu Gly Trp Ala Phe Leu Pro Phe Asn Gly
       450                 455                 460
   Gly Pro Arg Ile Cys Leu Gly Gln Gln Phe Ala Leu Thr Glu Ala Ser
   465                 470                 475                 480
   Tyr Val Thr Val Arg Leu Leu Gln Glu Phe Ala His Leu Ser Met Asp
                   485                 490                 495
   Pro Asp Thr Glu Tyr Pro Pro Lys Lys Met Ser His Leu Thr Met Ser
               500                 505                 510
   Leu Phe Asp Gly Ala Asn Ile Glu Met Tyr
           515                 520
<210> 7
   <211> 1569
   <212> DNA
   <213> Candida tropicalis
<220>
   <221> CDS
   <222> (1)..(1569)
<400> 7
   atg act gca cag gat att atc gcc aca tac atc acc aaa tgg tac gtg       48
   Met Thr Ala Gln Asp Ile Ile Ala Thr Tyr Ile Thr Lys Trp Tyr Val
   1               5                   10                  15
   ata gta cca ctc gct ttg att gct tat agg gtc ctc gac tac ttt tac       96
   Ile Val Pro Leu Ala Leu Ile Ala Tyr Arg Val Leu Asp Tyr Phe Tyr
               20                  25                  30
   ggc aga tac ttg atg tac aag ctt ggt gct aaa ccg ttt ttc cag aaa      144
   Gly Arg Tyr Leu Met Tyr Lys Leu Gly Ala Lys Pro Phe Phe Gln Lys
           35                  40                  45
   caa aca gac ggt tat ttc gga ttc aaa gct cca ctt gaa ttg tta aaa       192
   Gln Thr Asp Gly Tyr Phe Gly Phe Lys Ala Pro Leu Glu Leu Leu Lys
       50                  55                  60
   aag aag agt gac ggt acc ctc ata gac ttc act ctc gag cgt atc caa       240
   Lys Lys Ser Asp Gly Thr Leu Ile Asp Phe Thr Leu Glu Arg Ile Gln
   65                  70                  75                  80
   gcg ctc aat cgt cca gat atc cca act ttt aca ttc cca atc ttt tcc       288
   Ala Leu Asn Arg Pro Asp Ile Pro Thr Phe Thr Phe Pro Ile Phe Ser
                   85                  90                  95
   atc aac ctt atc agc acc ctt gag ccg gag aac atc aag gct atc ttg       336
   Ile Asn Leu Ile Ser Thr Leu Glu Pro Glu Asn Ile Lys Ala Ile Leu
               100                 105                 110
   gcc acc cag ttc aac gat ttc tcc ttg ggc acc aga cac tcg cac ttt       384
   Ala Thr Gln Phe Asn Asp Phe Ser Leu Gly Thr Arg His Ser His Phe
           115                 120                 125
   gct cct ttg ttg ggc gat ggt atc ttt acc ttg gac ggt gcc ggc tgg       432
   Ala Pro Leu Leu Gly Asp Gly Ile Phe Thr Leu Asp Gly Ala Gly Trp
       130                 135                 140
   aag cac agc aga tct atg ttg aga cca cag ttt gcc aga gaa cag att       480
   Lys His Ser Arg Ser Met Leu Arg Pro Gln Phe Ala Arg Glu Gln Ile
   145                 150                 155                 160
   tcc cac gtc aag ttg ttg gag cca cac atg cag gtg ttc ttc aag cac       528
   Ser His Val Lys Leu Leu Glu Pro His Met Gln Val Phe Phe Lys His
                   165                 170                 175
   gtc aga aag gca cag ggc aag act ttt gac atc caa gaa ttg ttt ttc       576
   Val Arg Lys Ala Gln Gly Lys Thr Phe Asp Ile Gln Glu Leu Phe Phe
               180                 185                 190
   aga ttg acc gtc gac tcc gcc act gag ttt ttg ttt ggt gaa tcc gtt       624
   Arg Leu Thr Val Asp Ser Ala Thr Glu Phe Leu Phe Gly Glu Ser Val
           195                 200                 205
   gag tcc ttg aga gat gaa tct att ggg atg tcc atc aat gca ctt gac       672
   Glu Ser Leu Arg Asp Glu Ser Ile Gly Met Ser Ile Asn Ala Leu Asp
       210                 215                 220
   ttt gac ggc aag gct ggc ttt gct gat gct ttt aac tac tcg cag aac       720
   Phe Asp Gly Lys Ala Gly Phe Ala Asp Ala Phe Asn Tyr Ser Gln Asn
   225                 230                 235                 240
   tat ttg gct tcg aga gcg gtt atg caa caa ttg tac tgg gtg ttg aac       768
   Tyr Leu Ala Ser Arg Ala Val Met Gln Gln Leu Tyr Trp Val Leu Asn
                   245                 250                 255
   ggg aaa aag ttt aag gag tgc aac gct aaa gtg cac aag ttt gct gac       816
   Gly Lys Lys Phe Lys Glu Cys Asn Ala Lys Val His Lys Phe Ala Asp
               260                 265                 270
   tat tac gtc agc aag gct ttg gac ttg aca cct gaa caa ttg gaa aag       864
   Tyr Tyr Val Ser Lys Ala Leu Asp Leu Thr Pro Glu Gln Leu Glu Lys
           275                 280                 285
   cag gat ggt tat gtg ttc ttg tac gag ttg gtc aag caa acc aga gac       912
   Gln Asp Gly Tyr Val Phe Leu Tyr Glu Leu Val Lys Gln Thr Arg Asp
       290                 295                 300
   agg caa gtg ttg aga gac cag ttg ttg aac atc atg gtt gcc ggt aga      960
   Arg Gln Val Leu Arg Asp Gln Leu Leu Asn Ile Met Val Ala Gly Arg
   305                 310                 315                 320
   gac acc acc gcc ggt ttg ttg tcg ttt gtt ttc ttt gaa ttg gcc aga     1008
   Asp Thr Thr Ala Gly Leu Leu Ser Phe Val Phe Phe Glu Leu Ala Arg
                   325                 330                 335
   aac cca gag gtg acc aac aag ttg aga gaa gaa atc gag gac aag ttt     1056
   Asn Pro Glu Val Thr Asn Lys Leu Arg Glu Glu Ile Glu Asp Lys Phe
               340                 345                 350
   ggt ctt ggt gag aat gct cgt gtt gaa gac att tcc ttt gag tcg ttg     1104
   Gly Leu Gly Glu Asn Ala Arg Val Glu Asp Ile Ser Phe Glu Ser Leu
           355                 360                 365
   aag tca tgt gaa tac ttg aag gct gtt ctc aac gaa act ttg aga ttg     1152
   Lys Ser Cys Glu Tyr Leu Lys Ala Val Leu Asn Glu Thr Leu Arg Leu
       370                 375                 380
   tac cca tcc gtg cca cag aat ttc aga gtt gcc acc aaa aac act acc     1200
   Tyr Pro Ser Val Pro Gln Asn Phe Arg Val Ala Thr Lys Asn Thr Thr
   385                 390                 395                 400
   ctt cca agg gga ggt ggt aag gac ggg tta tct cct gtt ttg gtc aga     1248
   Leu Pro Arg Gly Gly Gly Lys Asp Gly Leu Ser Pro Val Leu Val Arg
                   405                 410                 415
   aag ggt caa acc gtt atg tac ggt gtc tac gct gcc cac aga aac cca     1296
   Lys Gly Gln Thr Val Met Tyr Gly Val Tyr Ala Ala His Arg Asn Pro
               420                 425                 430
   gct gtc tac ggt aag gac gcc ctt gag ttt aga cca gag agg tgg ttt     1344
   Ala Val Tyr Gly Lys Asp Ala Leu Glu Phe Arg Pro Glu Arg Trp Phe
           435                 440                 445
   gag cca gag aca aag aag ctt ggc tgg gcc ttc ctt cca ttc aac ggt     1392
   Glu Pro Glu Thr Lys Lys Leu Gly Trp Ala Phe Leu Pro Phe Asn Gly
       450                 455                 460
   ggt cca aga att tgc ttg gga cag cag ttt gcc ttg aca gaa gct tcg     1440
   Gly Pro Arg Ile Cys Leu Gly Gln Gln Phe Ala Leu Thr Glu Ala Ser
   465                 470                 475                 480
   tat gtc act gtc aga ttg ctc caa gag ttt gga cac ttg tct atg gac     1488
   Tyr Val Thr Val Arg Leu Leu Gln Glu Phe Gly His Leu Ser Met Asp
                   485                 490                 495
   ccc aac acc gaa tat cca cct agg aaa atg tcg cat ttg acc atg tcc     1536
   Pro Asn Thr Glu Tyr Pro Pro Arg Lys Met Ser His Leu Thr Met Ser
               500                 505                 510
   ctt ttc gac ggt gcc aac att gag atg tat tag                         1569
   Leu Phe Asp Gly Ala Asn Ile Glu Met Tyr
           515                 520
<210> 8
   <211> 522
   <212> PRT
   <213> Candida tropicalis
<400> 8
   Met Thr Ala Gln Asp Ile Ile Ala Thr Tyr Ile Thr Lys Trp Tyr Val
   1               5                   10                  15
   Ile Val Pro Leu Ala Leu Ile Ala Tyr Arg Val Leu Asp Tyr Phe Tyr
               20                  25                  30
   Gly Arg Tyr Leu Met Tyr Lys Leu Gly Ala Lys Pro Phe Phe Gln Lys
           35                  40                  45
   Gln Thr Asp Gly Tyr Phe Gly Phe Lys Ala Pro Leu Glu Leu Leu Lys
       50                  55                  60
   Lys Lys Ser Asp Gly Thr Leu Ile Asp Phe Thr Leu Glu Arg Ile Gln
   65                  70                  75                  80
   Ala Leu Asn Arg Pro Asp Ile Pro Thr Phe Thr Phe Pro Ile Phe Ser
                   85                  90                  95
   Ile Asn Leu Ile Ser Thr Leu Glu Pro Glu Asn Ile Lys Ala Ile Leu
               100                 105                 110
   Ala Thr Gln Phe Asn Asp Phe Ser Leu Gly Thr Arg His Ser His Phe
           115                 120                 125
   Ala Pro Leu Leu Gly Asp Gly Ile Phe Thr Leu Asp Gly Ala Gly Trp
       130                 135                 140
   Lys His Ser Arg Ser Met Leu Arg Pro Gln Phe Ala Arg Glu Gln Ile
   145                 150                 155                 160
   Ser His Val Lys Leu Leu Glu Pro His Met Gln Val Phe Phe Lys His
                   165                 170                 175
   Val Arg Lys Ala Gln Gly Lys Thr Phe Asp Ile Gln Glu Leu Phe Phe
               180                 185                 190
   Arg Leu Thr Val Asp Ser Ala Thr Glu Phe Leu Phe Gly Glu Ser Val
           195                 200                 205
   Glu Ser Leu Arg Asp Glu Ser Ile Gly Met Ser Ile Asn Ala Leu Asp
       210                 215                 220
   Phe Asp Gly Lys Ala Gly Phe Ala Asp Ala Phe Asn Tyr Ser Gln Asn
   225                 230                 235                 240
   Tyr Leu Ala Ser Arg Ala Val Met Gln Gln Leu Tyr Trp Val Leu Asn
                   245                 250                 255
   Gly Lys Lys Phe Lys Glu Cys Asn Ala Lys Val His Lys Phe Ala Asp
               260                 265                 270
   Tyr Tyr Val Ser Lys Ala Leu Asp Leu Thr Pro Glu Gln Leu Glu Lys
           275                 280                 285
   Gln Asp Gly Tyr Val Phe Leu Tyr Glu Leu Val Lys Gln Thr Arg Asp
       290                 295                 300
   Arg Gln Val Leu Arg Asp Gln Leu Leu Asn Ile Met Val Ala Gly Arg
   305                 310                 315                 320
   Asp Thr Thr Ala Gly Leu Leu Ser Phe Val Phe Phe Glu Leu Ala Arg
                   325                 330                 335
   Asn Pro Glu Val Thr Asn Lys Leu Arg Glu Glu Ile Glu Asp Lys Phe
               340                 345                 350
   Gly Leu Gly Glu Asn Ala Arg Val Glu Asp Ile Ser Phe Glu Ser Leu
           355                 360                 365
   Lys Ser Cys Glu Tyr Leu Lys Ala Val Leu Asn Glu Thr Leu Arg Leu
       370                 375                 380
   Tyr Pro Ser Val Pro Gln Asn Phe Arg Val Ala Thr Lys Asn Thr Thr
   385                 390                 395                 400
   Leu Pro Arg Gly Gly Gly Lys Asp Gly Leu Ser Pro Val Leu Val Arg
                   405                 410                 415
   Lys Gly Gln Thr Val Met Tyr Gly Val Tyr Ala Ala His Arg Asn Pro
               420                 425                 430
   Ala Val Tyr Gly Lys Asp Ala Leu Glu Phe Arg Pro Glu Arg Trp Phe
           435                 440                 445
   Glu Pro Glu Thr Lys Lys Leu Gly Trp Ala Phe Leu Pro Phe Asn Gly
       450                 455                 460
   Gly Pro Arg Ile Cys Leu Gly Gln Gln Phe Ala Leu Thr Glu Ala Ser
   465                 470                 475                 480
   Tyr Val Thr Val Arg Leu Leu Gln Glu Phe Gly His Leu Ser Met Asp
                   485                 490                 495
   Pro Asn Thr Glu Tyr Pro Pro Arg Lys Met Ser His Leu Thr Met Ser
               500                 505                 510
   Leu Phe Asp Gly Ala Asn Ile Glu Met Tyr
           515                 520
<210> 9
   <211> 1623
   <212> DNA
   <213> Candida tropicalis
<220>
   <221> CDS
   <222> (1)..(1623)
<400> 9
   atg tcg tct tct cca tcg ttt gcc caa gag gtt ctc gct acc act agt      48
   Met Ser Ser Ser Pro Ser Phe Ala Gln Glu Val Leu Ala Thr Thr Ser
   1               5                   10                  15
   cct tac atc gag tac ttt ctt gac aac tac acc aga tgg tac tac ttc      96
   Pro Tyr Ile Glu Tyr Phe Leu Asp Asn Tyr Thr Arg Trp Tyr Tyr Phe
               20                  25                  30
   ata cct ttg gtg ctt ctt tcg ttg aac ttt ata agt ttg ctc cac aca      144
   Ile Pro Leu Val Leu Leu Ser Leu Asn Phe Ile Ser Leu Leu His Thr
           35                  40                  45
   agg tac ttg gaa cgc agg ttc cac gcc aag cca ctc ggt aac ttt gtc      192
   Arg Tyr Leu Glu Arg Arg Phe His Ala Lys Pro Leu Gly Asn Phe Val
       50                  55                  60
   agg gac cct acg ttt ggt atc gct act ccg ttg ctt ttg atc tac ttg      240
   Arg Asp Pro Thr Phe Gly Ile Ala Thr Pro Leu Leu Leu Ile Tyr Leu
   65                  70                  75                  80
   aag tcg aaa ggt acg gtc atg aag ttt gct tgg ggc ctc tgg aac aac      288
   Lys Ser Lys Gly Thr Val Met Lys Phe Ala Trp Gly Leu Trp Asn Asn
                   85                  90                  95
   aag tac atc gtc aga gac cca aag tac aag aca act ggg ctc agg att      336
   Lys Tyr Ile Val Arg Asp Pro Lys Tyr Lys Thr Thr Gly Leu Arg Ile
               100                 105                 110
   gtt ggc ctc cca ttg att gaa acc atg gac cca gag aac atc aag gct      384
   Val Gly Leu Pro Leu Ile Glu Thr Met Asp Pro Glu Asn Ile Lys Ala
           115                 120                 125
   gtt ttg gct act cag ttc aat gat ttc tct ttg gga acc aga cac gat      432
   Val Leu Ala Thr Gln Phe Asn Asp Phe Ser Leu Gly Thr Arg His Asp
       130                 135                 140
   ttc ttg tac tcc ttg ttg ggt gac ggt att ttc acc ttg gac ggt gct      480
   Phe Leu Tyr Ser Leu Leu Gly Asp Gly Ile Phe Thr Leu Asp Gly Ala
   145                 150                 155                 160
   ggc tgg aaa cat agt aga act atg ttg aga cca cag ttt gct aga gaa      528
   Gly Trp Lys His Ser Arg Thr Met Leu Arg Pro Gln Phe Ala Arg Glu
               165                 170                 175
   cag gtt tct cac gtc aag ttg ttg gag cca cac gtt cag gtg ttc ttc      576
   Gln Val Ser His Val Lys Leu Leu Glu Pro His Val Gln Val Phe Phe
           180                 185                 190
   aag cac gtt aga aag cac cgc ggt caa acg ttc gac atc caa gaa ttg      624
   Lys His Val Arg Lys His Arg Gly Gln Thr Phe Asp Ile Gln Glu Leu
       195                 200                 205
   ttc ttc agg ttg acc gtc gac tcc gcc acc gag ttc ttg ttt ggt gag      672
   Phe Phe Arg Leu Thr Val Asp Ser Ala Thr Glu Phe Leu Phe Gly Glu
       210                 215                 220
   tct gct gaa tcc ttg agg gac gaa tct att gga ttg acc cca acc acc      720
   Ser Ala Glu Ser Leu Arg Asp Glu Ser Ile Gly Leu Thr Pro Thr Thr
   225                 230                 235                 240
   aag gat ttc gat ggc aga aga gat ttc gct gac gct ttc aac tat tcg      768
   Lys Asp Phe Asp Gly Arg Arg Asp Phe Ala Asp Ala Phe Asn Tyr Ser
               245                 250                 255
   cag act tac cag gcc tac aga ttt ttg ttg caa caa atg tac tgg atc      816
   Gln Thr Tyr Gln Ala Tyr Arg Phe Leu Leu Gln Gln Met Tyr Trp Ile
           260                 265                 270
   ttg aat ggc tcg gaa ttc aga aag tcg att gct gtc gtg cac aag ttt      864
   Leu Asn Gly Ser Glu Phe Arg Lys Ser Ile Ala Val Val His Lys Phe
       275                 280                 285
   gct gac cac tat gtg caa aag gct ttg gag ttg acc gac gat gac ttg      912
   Ala Asp His Tyr Val Gln Lys Ala Leu Glu Leu Thr Asp Asp Asp Leu
       290                 295                 300
   cag aaa caa gac ggc tat gtg ttc ttg tac gag ttg gct aag caa acc      960
   Gln Lys Gln Asp Gly Tyr Val Phe Leu Tyr Glu Leu Ala Lys Gln Thr
   305                 310                 315                 320
   aga gac cca aag gtc ttg aga gac cag tta ttg aac att ttg gtt gcc     1008
   Arg Asp Pro Lys Val Leu Arg Asp Gln Leu Leu Asn Ile Leu Val Ala
               325                 330                 335
   ggt aga gac acg acc gcc ggt ttg ttg tca ttt gtt ttc tac gag ttg     1056
   Gly Arg Asp Thr Thr Ala Gly Leu Leu Ser Phe Val Phe Tyr Glu Leu
           340                 345                 350
   tca aga aac cct gag gtg ttt gct aag ttg aga gag gag gtg gaa aac     1104
   Ser Arg Asn Pro Glu Val Phe Ala Lys Leu Arg Glu Glu Val Glu Asn
       355                 360                 365
   aga ttt gga ctc ggt gaa gaa gct cgt gtt gaa gag atc tcg ttt gag     1152
   Arg Phe Gly Leu Gly Glu Glu Ala Arg Val Glu Glu Ile Ser Phe Glu
       370                 375                 380
   tcc ttg aag tct tgt gag tac ttg aag gct gtc atc aat gaa acc ttg     1200
   Ser Leu Lys Ser Cys Glu Tyr Leu Lys Ala Val Ile Asn Glu Thr Leu
   385                 390                 395                 400
   aga ttg tac cca tcg gtt cca cac aac ttt aga gtt gct acc aga aac     1248
   Arg Leu Tyr Pro Ser Val Pro His Asn Phe Arg Val Ala Thr Arg Asn
               405                 410                 415
   act acc ctc cca aga ggt ggt ggt gaa gat gga tac tcg cca att gtc     1296
   Thr Thr Leu Pro Arg Gly Gly Gly Glu Asp Gly Tyr Ser Pro Ile Val
               420                 425                 430
   gtc aag aag ggt caa gtt gtc atg tac act gtt att gct acc cac aga      1344
   Val Lys Lys Gly Gln Val Val Met Tyr Thr Val Ile Ala Thr His Arg
           435                 440                 445
   gac cca agt atc tac ggt gcc gac gct gac gtc ttc aga cca gaa aga      1392
   Asp Pro Ser Ile Tyr Gly Ala Asp Ala Asp Val Phe Arg Pro Glu Arg
       450                 455                 460
   tgg ttt gaa cca gaa act aga aag ttg ggc tgg gca tac gtt cca ttc      1440
   Trp Phe Glu Pro Glu Thr Arg Lys Leu Gly Trp Ala Tyr Val Pro Phe
   465                 470                 475                 480
   aat ggt ggt cca aga atc tgt ttg ggt caa cag ttt gcc ttg acc gaa      1488
   Asn Gly Gly Pro Arg Ile Cys Leu Gly Gln Gln Phe Ala Leu Thr Glu
                   485                 490                 495
   gct tca tac gtc act gtc aga ttg ctc cag gag ttt gca cac ttg tct      1536
   Ala Ser Tyr Val Thr Val Arg Leu Leu Gln Glu Phe Ala His Leu Ser
               500                 505                 510
   atg gac cca gac acc gaa tat cca cca aaa ttg cag aac acc ttg acc      1584
   Met Asp Pro Asp Thr Glu Tyr Pro Pro Lys Leu Gln Asn Thr Leu Thr
           515                 520                 525
   ttg tcg ctc ttt gat ggt gct gat gtt aga atg tac taa                  1623
   Leu Ser Leu Phe Asp Gly Ala Asp Val Arg Met Tyr
       530                 535                 540
<210> 10
   <211> 540
   <212> PRT
   <213> Candida tropicalis
<400> 10
   Met Ser Ser Ser Pro Ser Phe Ala Gln Glu Val Leu Ala Thr Thr Ser
   1               5                   10                  15
   Pro Tyr Ile Glu Tyr Phe Leu Asp Asn Tyr Thr Arg Trp Tyr Tyr Phe
               20                  25                  30
   Ile Pro Leu Val Leu Leu Ser Leu Asn Phe Ile Ser Leu Leu His Thr
           35                  40                  45
   Arg Tyr Leu Glu Arg Arg Phe His Ala Lys Pro Leu Gly Asn Phe Val
       50                  55                  60
   Arg Asp Pro Thr Phe Gly Ile Ala Thr Pro Leu Leu Leu Ile Tyr Leu
   65                  70                  75                  80
   Lys Ser Lys Gly Thr Val Met Lys Phe Ala Trp Gly Leu Trp Asn Asn
                   85                  90                  95
   Lys Tyr Ile Val Arg Asp Pro Lys Tyr Lys Thr Thr Gly Leu Arg Ile
               100                 105                 110
   Val Gly Leu Pro Leu Ile Glu Thr Met Asp Pro Glu Asn Ile Lys Ala
           115                 120                 125
   Val Leu Ala Thr Gln Phe Asn Asp Phe Ser Leu Gly Thr Arg His Asp
       130                 135                 140
   Phe Leu Tyr Ser Leu Leu Gly Asp Gly Ile Phe Thr Leu Asp Gly Ala
   145                 150                 155                 160
   Gly Trp Lys His Ser Arg Thr Met Leu Arg Pro Gln Phe Ala Arg Glu
                   165                 170                 175
   Gln Val Ser His Val Lys Leu Leu Glu Pro His Val Gln Val Phe Phe
               180                 185                 190
   Lys His Val Arg Lys His Arg Gly Gln Thr Phe Asp Ile Gln Glu Leu
           195                 200                 205
   Phe Phe Arg Leu Thr Val Asp Ser Ala Thr Glu Phe Leu Phe Gly Glu
       210                 215                 220
   Ser Ala Glu Ser Leu Arg Asp Glu Ser Ile Gly Leu Thr Pro Thr Thr
   225                 230                 235                 240
   Lys Asp Phe Asp Gly Arg Arg Asp Phe Ala Asp Ala Phe Asn Tyr Ser
                   245                 250                 255
   Gln Thr Tyr Gln Ala Tyr Arg Phe Leu Leu Gln Gln Met Tyr Trp Ile
               260                 265                 270
   Leu Asn Gly Ser Glu Phe Arg Lys Ser Ile Ala Val Val His Lys Phe
           275                 280                 285
   Ala Asp His Tyr Val Gln Lys Ala Leu Glu Leu Thr Asp Asp Asp Leu
       290                 295                 300
   Gln Lys Gln Asp Gly Tyr Val Phe Leu Tyr Glu Leu Ala Lys Gln Thr
   305                 310                 315                 320
   Arg Asp Pro Lys Val Leu Arg Asp Gln Leu Leu Asn Ile Leu Val Ala
                   325                 330                 335
   Gly Arg Asp Thr Thr Ala Gly Leu Leu Ser Phe Val Phe Tyr Glu Leu
               340                 345                 350
   Ser Arg Asn Pro Glu Val Phe Ala Lys Leu Arg Glu Glu Val Glu Asn
           355                 360                 365
   Arg Phe Gly Leu Gly Glu Glu Ala Arg Val Glu Glu Ile Ser Phe Glu
       370                 375                 380
   Ser Leu Lys Ser Cys Glu Tyr Leu Lys Ala Val Ile Asn Glu Thr Leu
   385                 390                 395                 400
   Arg Leu Tyr Pro Ser Val Pro His Asn Phe Arg Val Ala Thr Arg Asn
                   405                 410                 415
   Thr Thr Leu Pro Arg Gly Gly Gly Glu Asp Gly Tyr Ser Pro Ile Val
               420                 425                 430
   Val Lys Lys Gly Gln Val Val Met Tyr Thr Val Ile Ala Thr His Arg
           435                 440                 445
   Asp Pro Ser Ile Tyr Gly Ala Asp Ala Asp Val Phe Arg Pro Glu Arg
       450                 455                 460
   Trp Phe Glu Pro Glu Thr Arg Lys Leu Gly Trp Ala Tyr Val Pro Phe
   465                 470                 475                 480
   Asn Gly Gly Pro Arg Ile Cys Leu Gly Gln Gln Phe Ala Leu Thr Glu
                   485                 490                 495
   Ala Ser Tyr Val Thr Val Arg Leu Leu Gln Glu Phe Ala His Leu Ser
               500                 505                 510
   Met Asp Pro Asp Thr Glu Tyr Pro Pro Lys Leu Gln Asn Thr Leu Thr
           515                 520                 525
   Leu Ser Leu Phe Asp Gly Ala Asp Val Arg Met Tyr
       530                 535                 540
<210> 11
   <211> 1623
   <212> DNA
   <213> Candida tropicalis
<220>
   <221> CDS
   <222> (1)..(1623)
<400> 11
   atg tcg tct tct cca tcg ttt gct cag gag gtt ctc gct acc act agt       48
   Met Ser Ser Ser Pro Ser Phe Ala Gln Glu Val Leu Ala Thr Thr Ser
   1               5                   10                  15
   cct tac atc gag tac ttt ctt gac aac tac acc aga tgg tac tac ttc      96
   Pro Tyr Ile Glu Tyr Phe Leu Asp Asn Tyr Thr Arg Trp Tyr Tyr Phe
               20                  25                  30
   atc cct ttg gtg ctt ctt tcg ttg aac ttc atc agc ttg ctc cac aca     144
   Ile Pro Leu Val Leu Leu Ser Leu Asn Phe Ile Ser Leu Leu His Thr
           35                  40                  45
   aag tac ttg gaa cgc agg ttc cac gcc aag ccg ctc ggt aac gtc gtg     192
   Lys Tyr Leu Glu Arg Arg Phe His Ala Lys Pro Leu Gly Asn Val Val
       50                  55                  60
   ttg gat cct acg ttt ggt atc gct act ccg ttg atc ttg atc tac tta     240
   Leu Asp Pro Thr Phe Gly Ile Ala Thr Pro Leu Ile Leu Ile Tyr Leu
   65                  70                  75                  80
   aag tcg aaa ggt aca gtc atg aag ttt gcc tgg agc ttc tgg aac aac     288
   Lys Ser Lys Gly Thr Val Met Lys Phe Ala Trp Ser Phe Trp Asn Asn
                   85                  90                  95
   aag tac att gtc aaa gac cca aag tac aag acc act ggc ctt aga att     336
   Lys Tyr Ile Val Lys Asp Pro Lys Tyr Lys Thr Thr Gly Leu Arg Ile
               100                 105                 110
   gtc ggc ctc cca ttg att gaa acc ata gac cca gag aac atc aaa gct     384
   Val Gly Leu Pro Leu Ile Glu Thr Ile Asp Pro Glu Asn Ile Lys Ala
           115                 120                 125
   gtg ttg gct act cag ttc aac gat ttc tcc ttg gga act aga cac gat     432
   Val Leu Ala Thr Gln Phe Asn Asp Phe Ser Leu Gly Thr Arg His Asp
       130                 135                 140
   ttc ttg tac tcc ttg ttg ggc gat ggt att ttt acc ttg gac ggt gct     480
   Phe Leu Tyr Ser Leu Leu Gly Asp Gly Ile Phe Thr Leu Asp Gly Ala
   145                 150                 155                 160
   ggc tgg aaa cac agt aga act atg ttg aga cca cag ttt gct aga gaa     528
   Gly Trp Lys His Ser Arg Thr Met Leu Arg Pro Gln Phe Ala Arg Glu
                   165                 170                 175
   cag gtt tcc cac gtc aag ttg ttg gaa cca cac gtt cag gtg ttc ttc     576
   Gln Val Ser His Val Lys Leu Leu Glu Pro His Val Gln Val Phe Phe
               180                 185                 190
   aag cac gtt aga aaa cac cgc ggt cag act ttt gac atc caa gaa ttg     624
   Lys His Val Arg Lys His Arg Gly Gln Thr Phe Asp Ile Gln Glu Leu
           195                 200                 205
   ttc ttc aga ttg acc gtc gac tcc gcc acc gag ttc ttg ttt ggt gag     672
   Phe Phe Arg Leu Thr Val Asp Ser Ala Thr Glu Phe Leu Phe Gly Glu
       210                 215                 220
   tct gct gaa tcc ttg aga gac gac tct gtt ggt ttg acc cca acc acc     720
   Ser Ala Glu Ser Leu Arg Asp Asp Ser Val Gly Leu Thr Pro Thr Thr
   225                 230                 235                 240
   aag gat ttc gaa ggc aga gga gat ttc gct gac gct ttc aac tac tcg     768
   Lys Asp Phe Glu Gly Arg Gly Asp Phe Ala Asp Ala Phe Asn Tyr Ser
                   245                 250                 255
   cag act tac cag gcc tac aga ttt ttg ttg caa caa atg tac tgg att     816
   Gln Thr Tyr Gln Ala Tyr Arg Phe Leu Leu Gln Gln Met Tyr Trp Ile
               260                 265                 270
   ttg aat ggc gcg gaa ttc aga aag tcg att gcc atc gtg cac aag ttt       864
   Leu Asn Gly Ala Glu Phe Arg Lys Ser Ile Ala Ile Val His Lys Phe
           275                 280                 285
   gct gac cac tat gtg caa aag gct ttg gag ttg acc gac gat gac ttg       912
   Ala Asp His Tyr Val Gln Lys Ala Leu Glu Leu Thr Asp Asp Asp Leu
       290                 295                 300
   cag aaa caa gac ggc tat gtg ttc ttg tac gag ttg gct aag caa act       960
   Gln Lys Gln Asp Gly Tyr Val Phe Leu Tyr Glu Leu Ala Lys Gln Thr
   305                 310                 315                 320
   aga gac cca aag gtc ttg aga gac cag ttg ttg aac att ttg gtt gcc      1008
   Arg Asp Pro Lys Val Leu Arg Asp Gln Leu Leu Asn Ile Leu Val Ala
                   325                 330                 335
   ggt aga gac acg acc gcc ggt ttg ttg tcg ttt gtg ttc tac gag ttg      1056
   Gly Arg Asp Thr Thr Ala Gly Leu Leu Ser Phe Val Phe Tyr Glu Leu
               340                 345                 350
   tcg aga aac cct gaa gtg ttt gcc aag ttg aga gag gag gtg gaa aac      1104
   Ser Arg Asn Pro Glu Val Phe Ala Lys Leu Arg Glu Glu Val Glu Asn
           355                 360                 365
   aga ttt gga ctc ggc gaa gag gct cgt gtt gaa gag atc tct ttt gag      1152
   Arg Phe Gly Leu Gly Glu Glu Ala Arg Val Glu Glu Ile Ser Phe Glu
       370                 375                 380
   tcc ttg aag tcc tgt gag tac ttg aag gct gtc atc aat gaa gcc ttg      1200
   Ser Leu Lys Ser Cys Glu Tyr Leu Lys Ala Val Ile Asn Glu Ala Leu
   385                 390                 395                 400
   aga ttg tac cca tct gtt cca cac aac ttc aga gtt gcc acc aga aac      1248
   Arg Leu Tyr Pro Ser Val Pro His Asn Phe Arg Val Ala Thr Arg Asn
                   405                 410                 415
   act acc ctt cca aga ggc ggt ggt aaa gac gga tgc tcg cca att gtt      1296
   Thr Thr Leu Pro Arg Gly Gly Gly Lys Asp Gly Cys Ser Pro Ile Val
               420                 425                 430
   gtc aag aag ggt caa gtt gtc atg tac act gtc att ggt acc cac aga      1344
   Val Lys Lys Gly Gln Val Val Met Tyr Thr Val Ile Gly Thr His Arg
           435                 440                 445
   gac cca agt atc tac ggt gcc gac gcc gac gtc ttc aga cca gaa aga      1392
   Asp Pro Ser Ile Tyr Gly Ala Asp Ala Asp Val Phe Arg Pro Glu Arg
       450                 455                 460
   tgg ttc gag cca gaa act aga aag ttg ggc tgg gca tat gtt cca ttc      1440
   Trp Phe Glu Pro Glu Thr Arg Lys Leu Gly Trp Ala Tyr Val Pro Phe
   465                 470                 475                 480
   aat ggt ggt cca aga atc tgt ttg ggt cag cag ttt gcc ttg act gaa      1488
   Asn Gly Gly Pro Arg Ile Cys Leu Gly Gln Gln Phe Ala Leu Thr Glu
                   485                 490                 495
   gct tca tac gtc act gtc aga ttg ctc caa gag ttt gga aac ttg tcc      1536
   Ala Ser Tyr Val Thr Val Arg Leu Leu Gln Glu Phe Gly Asn Leu Ser
               500                 505                 510
   ctg gat cca aac gct gag tac cca cca aaa ttg cag aac acc ttg acc      1584
   Leu Asp Pro Asn Ala Glu Tyr Pro Pro Lys Leu Gln Asn Thr Leu Thr
       515                 520                 525
   ttg tca ctc ttt gat ggt gct gac gtt aga atg ttc taa                 1623
   Leu Ser Leu Phe Asp Gly Ala Asp Val Arg Met Phe
   530                 535                 540
<210> 12
   <211> 540
   <212> PRT
   <213> Candida tropicalis
<400> 12
   Met Ser Ser Ser Pro Ser Phe Ala Gln Glu Val Leu Ala Thr Thr Ser
   1               5                   10                  15
   Pro Tyr Ile Glu Tyr Phe Leu Asp Asn Tyr Thr Arg Trp Tyr Tyr Phe
               20                  25                  30
   Ile Pro Leu Val Leu Leu Ser Leu Asn Phe Ile Ser Leu Leu His Thr
           35                  40                  45
   Lys Tyr Leu Glu Arg Arg Phe His Ala Lys Pro Leu Gly Asn Val Val
       50                  55                  60
   Leu Asp Pro Thr Phe Gly Ile Ala Thr Pro Leu Ile Leu Ile Tyr Leu
   65                  70                  75                  80
   Lys Ser Lys Gly Thr Val Met Lys Phe Ala Trp Ser Phe Trp Asn Asn
                   85                  90                  95
   Lys Tyr Ile Val Lys Asp Pro Lys Tyr Lys Thr Thr Gly Leu Arg Ile
               100                 105                 110
   Val Gly Leu Pro Leu Ile Glu Thr Ile Asp Pro Glu Asn Ile Lys Ala
           115                 120                 125
   Val Leu Ala Thr Gln Phe Asn Asp Phe Ser Leu Gly Thr Arg His Asp
       130                 135                 140
   Phe Leu Tyr Ser Leu Leu Gly Asp Gly Ile Phe Thr Leu Asp Gly Ala
   145                 150                 155                 160
   Gly Trp Lys His Ser Arg Thr Met Leu Arg Pro Gln Phe Ala Arg Glu
                   165                 170                 175
   Gln Val Ser His Val Lys Leu Leu Glu Pro His Val Gln Val Phe Phe
               180                 185                 190
   Lys His Val Arg Lys His Arg Gly Gln Thr Phe Asp Ile Gln Glu Leu
           195                 200                 205
   Phe Phe Arg Leu Thr Val Asp Ser Ala Thr Glu Phe Leu Phe Gly Glu
       210                 215                 220
   Ser Ala Glu Ser Leu Arg Asp Asp Ser Val Gly Leu Thr Pro Thr Thr
   225                 230                 235                 240
   Lys Asp Phe Glu Gly Arg Gly Asp Phe Ala Asp Ala Phe Asn Tyr Ser
                   245                 250                 255
   Gln Thr Tyr Gln Ala Tyr Arg Phe Leu Leu Gln Gln Met Tyr Trp Ile
               260                 265                 270
   Leu Asn Gly Ala Glu Phe Arg Lys Ser Ile Ala Ile Val His Lys Phe
           275                 280                 285
   Ala Asp His Tyr Val Gln Lys Ala Leu Glu Leu Thr Asp Asp Asp Leu
       290                 295                 300
   Gln Lys Gln Asp Gly Tyr Val Phe Leu Tyr Glu Leu Ala Lys Gln Thr
   305                 310                 315                 320
   Arg Asp Pro Lys Val Leu Arg Asp Gln Leu Leu Asn Ile Leu Val Ala
                   325                 330                 335
   Gly Arg Asp Thr Thr Ala Gly Leu Leu Ser Phe Val Phe Tyr Glu Leu
               340                 345                 350
   Ser Arg Asn Pro Glu Val Phe Ala Lys Leu Arg Glu Glu Val Glu Asn
           355                 360                 365
   Arg Phe Gly Leu Gly Glu Glu Ala Arg Val Glu Glu Ile Ser Phe Glu
       370                 375                 380
   Ser Leu Lys Ser Cys Glu Tyr Leu Lys Ala Val Ile Asn Glu Ala Leu
   385                 390                 395                 400
   Arg Leu Tyr Pro Ser Val Pro His Asn Phe Arg Val Ala Thr Arg Asn
                   405                 410                 415
   Thr Thr Leu Pro Arg Gly Gly Gly Lys Asp Gly Cys Ser Pro Ile Val
               420                 425                 430
   Val Lys Lys Gly Gln Val Val Met Tyr Thr Val Ile Gly Thr His Arg
           435                 440                 445
   Asp Pro Ser Ile Tyr Gly Ala Asp Ala Asp Val Phe Arg Pro Glu Arg
       450                 455                 460
   Trp Phe Glu Pro Glu Thr Arg Lys Leu Gly Trp Ala Tyr Val Pro Phe
   465                 470                 475                 480
   Asn Gly Gly Pro Arg Ile Cys Leu Gly Gln Gln Phe Ala Leu Thr Glu
                   485                 490                 495
   Ala Ser Tyr Val Thr Val Arg Leu Leu Gln Glu Phe Gly Asn Leu Ser
               500                 505                 510
   Leu Asp Pro Asn Ala Glu Tyr Pro Pro Lys Leu Gln Asn Thr Leu Thr
           515                 520                 525
   Leu Ser Leu Phe Asp Gly Ala Asp Val Arg Met Phe
       530                 535                 540
<210> 13
   <211> 1554
   <212> DNA
   <213> Candida tropicalis
<220>
   <221> CDS
   <222> (1)..(1554)
<400> 13
   atg att gaa caa ctc cta gaa tat tgg tat gtc gtt gtg cca gtg ttg         48
   Met Ile Glu Gln Leu Leu Glu Tyr Trp Tyr Val Val Val Pro Val Leu
   1               5                   10                  15
   tac atc atc aaa caa ctc ctt gca tac aca aag act cgc gtc ttg atg         96
   Tyr Ile Ile Lys Gln Leu Leu Ala Tyr Thr Lys Thr Arg Val Leu Met
               20                  25                  30
   aaa aag ttg ggt gct gct cca gtc aca aac aag ttg tac gac aac gct        144
   Lys Lys Leu Gly Ala Ala Pro Val Thr Asn Lys Leu Tyr Asp Asn Ala
           35                  40                  45
   ttc ggt atc gtc aat gga tgg aag gct ctc cag ttc aag aaa gag ggc        192
   Phe Gly Ile Val Asn Gly Trp Lys Ala Leu Gln Phe Lys Lys Glu Gly
       50                  55                  60
   agg gct caa gag tac aac gat tac aag ttt gac cac tcc aag aac cca        240
   Arg Ala Gln Glu Tyr Asn Asp Tyr Lys Phe Asp His Ser Lys Asn Pro
   65                  70                  75                  80
   agc gtg ggc acc tac gtc agt att ctt ttc ggc acc agg atc gtc gtg        288
   Ser Val Gly Thr Tyr Val Ser Ile Leu Phe Gly Thr Arg Ile Val Val
                   85                  90                  95
   acc aaa gat cca gag aat atc aaa gct att ttg gca acc cag ttt ggt        336
   Thr Lys Asp Pro Glu Asn Ile Lys Ala Ile Leu Ala Thr Gln Phe Gly
               100                 105                 110
   gat ttt tct ttg ggc aag agg cac act ctt ttt aag cct ttg tta ggt       384
   Asp Phe Ser Leu Gly Lys Arg His Thr Leu Phe Lys Pro Leu Leu Gly
           115                 120                 125
   gat ggg atc ttc aca ttg gac ggc gaa ggc tgg aag cac agc aga gcc       432
   Asp Gly Ile Phe Thr Leu Asp Gly Glu Gly Trp Lys His Ser Arg Ala
       130                 135                 140
   atg ttg aga cca cag ttt gcc aga gaa caa gtt gct cat gtg acg tcg       480
   Met Leu Arg Pro Gln Phe Ala Arg Glu Gln Val Ala His Val Thr Ser
   145                 150                 155                 160
   ttg gaa cca cac ttc cag ttg ttg aag aag cat att ctt aag cac aag       528
   Leu Glu Pro His Phe Gln Leu Leu Lys Lys His Ile Leu Lys His Lys
                   165                 170                 175
   ggt gaa tac ttt gat atc cag gaa ttg ttc ttt aga ttt acc gtt gat       576
   Gly Glu Tyr Phe Asp Ile Gln Glu Leu Phe Phe Arg Phe Thr Val Asp
               180                 185                 190
   tcg gcc acg gag ttc tta ttt ggt gag tcc gtg cac tcc tta aag gac       624
   Ser Ala Thr Glu Phe Leu Phe Gly Glu Ser Val His Ser Leu Lys Asp
           195                 200                 205
   gaa tct att ggt atc aac caa gac gat ata gat ttt gct ggt aga aag       672
   Glu Ser Ile Gly Ile Asn Gln Asp Asp Ile Asp Phe Ala Gly Arg Lys
       210                 215                 220
   gac ttt gct gag tcg ttc aac aaa gcc cag gaa tac ttg gct att aga       720
   Asp Phe Ala Glu Ser Phe Asn Lys Ala Gln Glu Tyr Leu Ala Ile Arg
   225                 230                 235                 240
   acc ttg gtg cag acg ttc tac tgg ttg gtc aac aac aag gag ttt aga       768
   Thr Leu Val Gln Thr Phe Tyr Trp Leu Val Asn Asn Lys Glu Phe Arg
                   245                 250                 255
   gac tgt acc aag ctg gtg cac aag ttc acc aac tac tat gtt cag aaa       816
   Asp Cys Thr Lys Leu Val His Lys Phe Thr Asn Tyr Tyr Val Gln Lys
               260                 265                 270
   gct ttg gat gct agc cca gaa gag ctt gaa aag caa agt ggg tat gtg       864
   Ala Leu Asp Ala Ser Pro Glu Glu Leu Glu Lys Gln Ser Gly Tyr Val
           275                 280                 285
   ttc ttg tac gag ctt gtc aag cag aca aga gac ccc aat gtg ttg cgt       912
   Phe Leu Tyr Glu Leu Val Lys Gln Thr Arg Asp Pro Asn Val Leu Arg
       290                 295                 300
   gac cag tct ttg aac atc ttg ttg gcc gga aga gac acc act gct ggg       960
   Asp Gln Ser Leu Asn Ile Leu Leu Ala Gly Arg Asp Thr Thr Ala Gly
   305                 310                 315                 320
   ttg ttg tcg ttt gct gtc ttt gag ttg gcc aga cac cca gag atc tgg      1008
   Leu Leu Ser Phe Ala Val Phe Glu Leu Ala Arg His Pro Glu Ile Trp
                   325                 330                 335
   gcc aag ttg aga gag gaa att gaa caa cag ttt ggt ctt gga gaa gac      1056
   Ala Lys Leu Arg Glu Glu Ile Glu Gln Gln Phe Gly Leu Gly Glu Asp
               340                 345                 350
   tct cgt gtt gaa gag att acc ttt gag agc ttg aag aga tgt gag tac      1104
   Ser Arg Val Glu Glu Ile Thr Phe Glu Ser Leu Lys Arg Cys Glu Tyr
           355                 360                 365
   ttg aaa gcg ttc ctt aat gaa acc ttg cgt att tac cca agt gtc cca      1152
   Leu Lys Ala Phe Leu Asn Glu Thr Leu Arg Ile Tyr Pro Ser Val Pro
       370                 375                 380
   aga aac ttc aga atc gcc acc aag aac acg aca ttg cca agg ggc ggt      1200
   Arg Asn Phe Arg Ile Ala Thr Lys Asn Thr Thr Leu Pro Arg Gly Gly
   385                 390                 395                 400
   ggt tca gac ggt acc tcg cca atc ttg atc caa aag gga gaa gct gtg      1248
   Gly Ser Asp Gly Thr Ser Pro Ile Leu Ile Gln Lys Gly Glu Ala Val
                   405                 410                 415
   tcg tat ggt atc aac tct act cat ttg gac cct gtc tat tac ggc cct      1296
   Ser Tyr Gly Ile Asn Ser Thr His Leu Asp Pro Val Tyr Tyr Gly Pro
               420                 425                 430
   gat gct gct gag ttc aga cca gag aga tgg ttt gag cca tca acc aaa      1344
   Asp Ala Ala Glu Phe Arg Pro Glu Arg Trp Phe Glu Pro Ser Thr Lys
           435                 440                 445
   aag ctc ggc tgg gct tac ttg cca ttc aac ggt ggt cca aga atc tgt      1392
   Lys Leu Gly Trp Ala Tyr Leu Pro Phe Asn Gly Gly Pro Arg Ile Cys
       450                 455                 460
   ttg ggt cag cag ttt gcc ttg acg gaa gct ggc tat gtg ttg gtt aga      1440
   Leu Gly Gln Gln Phe Ala Leu Thr Glu Ala Gly Tyr Val Leu Val Arg
   465                 470                 475                 480
   ttg gtg caa gag ttc tcc cac gtt agg ctg gac cca gac gag gtg tac      1488
   Leu Val Gln Glu Phe Ser His Val Arg Leu Asp Pro Asp Glu Val Tyr
                   485                 490                 495
   ccg cca aag agg ttg acc aac ttg acc atg tgt ttg cag gat ggt gct      1536
   Pro Pro Lys Arg Leu Thr Asn Leu Thr Met Cys Leu Gln Asp Gly Ala
               500                 505                 510
   att gtc aag ttt gac tag                                              1554
   Ile Val Lys Phe Asp
           515
<210> 14
   <211> 517
   <212> PRT
   <213> Candida tropicalis
<400> 14
   Met Ile Glu Gln Leu Leu Glu Tyr Trp Tyr Val Val Val Pro Val Leu
   1               5                   10                  15
   Tyr Ile Ile Lys Gln Leu Leu Ala Tyr Thr Lys Thr Arg Val Leu Met
               20                  25                  30
   Lys Lys Leu Gly Ala Ala Pro Val Thr Asn Lys Leu Tyr Asp Asn Ala
           35                  40                  45
   Phe Gly Ile Val Asn Gly Trp Lys Ala Leu Gln Phe Lys Lys Glu Gly
   50 55 60
   Arg Ala Gln Glu Tyr Asn Asp Tyr Lys Phe Asp His Ser Lys Asn Pro
   65 70 75 80
   Ser Val Gly Thr Tyr Val Ser Ile Leu Phe Gly Thr Arg Ile Val Val
   85 90 95
   Thr Lys Asp Pro Glu Asn Ile Lys Ala Ile Leu Ala Thr Gln Phe Gly
   100 105 110
   Asp Phe Ser Leu Gly Lys Arg His Thr Leu Phe Lys Pro Leu Leu Gly
   115 120 125
   Asp Gly Ile Phe Thr Leu Asp Gly Glu Gly Trp Lys His Ser Arg Ala
   130 135 140
   Met Leu Arg Pro Gln Phe Ala Arg Glu Gln Val Ala His Val Thr Ser
   145 150 155 160
   Leu Glu Pro His Phe Gln Leu Leu Lys Lys His Ile Leu Lys His Lys
   165 170 175
   Gly Glu Tyr Phe Asp Ile Gln Glu Leu Phe Phe Arg Phe Thr Val Asp
   180 185 190
   Ser Ala Thr Glu Phe Leu Phe Gly Glu Ser Val His Ser Leu Lys Asp
   195 200 205
   Glu Ser Ile Gly Ile Asn Gln Asp Asp Ile Asp Phe Ala Gly Arg Lys
   210 215 220
   Asp Phe Ala Glu Ser Phe Asn Lys Ala Gln Glu Tyr Leu Ala Ile Arg
   225 230 235 240
   Thr Leu Val Gln Thr Phe Tyr Trp Leu Val Asn Asn Lys Glu Phe Arg
   245 250 255
   Asp Cys Thr Lys Leu Val His Lys Phe Thr Asn Tyr Tyr Val Gln Lys
   260 265 270
   Ala Leu Asp Ala Ser Pro Glu Glu Leu Glu Lys Gln Ser Gly Tyr Val
   275 280 285
   Phe Leu Tyr Glu Leu Val Lys Gln Thr Arg Asp Pro Asn Val Leu Arg
   290 295 300
   Asp Gln Ser Leu Asn Ile Leu Leu Ala Gly Arg Asp Thr Thr Ala Gly
   305                 310                 315                 320
   Leu Leu Ser Phe Ala Val Phe Glu Leu Ala Arg His Pro Glu Ile Trp
                   325                 330                 335
   Ala Lys Leu Arg Glu Glu Ile Glu Gln Gln Phe Gly Leu Gly Glu Asp
               340                 345                 350
   Ser Arg Val Glu Glu Ile Thr Phe Glu Ser Leu Lys Arg Cys Glu Tyr
           355                 360                 365
   Leu Lys Ala Phe Leu Asn Glu Thr Leu Arg Ile Tyr Pro Ser Val Pro
       370                 375                 380
   Arg Asn Phe Arg Ile Ala Thr Lys Asn Thr Thr Leu Pro Arg Gly Gly
   385                 390                 395                 400
   Gly Ser Asp Gly Thr Ser Pro Ile Leu Ile Gln Lys Gly Glu Ala Val
                   405                 410                 415
   Ser Tyr Gly Ile Asn Ser Thr His Leu Asp Pro Val Tyr Tyr Gly Pro
               420                 425                 430
   Asp Ala Ala Glu Phe Arg Pro Glu Arg Trp Phe Glu Pro Ser Thr Lys
           435                 440                 445
   Lys Leu Gly Trp Ala Tyr Leu Pro Phe Asn Gly Gly Pro Arg Ile Cys
       450                 455                 460
   Leu Gly Gln Gln Phe Ala Leu Thr Glu Ala Gly Tyr Val Leu Val Arg
   465                 470                 475                 480
   Leu Val Gln Glu Phe Ser His Val Arg Leu Asp Pro Asp Glu Val Tyr
                   485                 490                 495
   Pro Pro Lys Arg Leu Thr Asn Leu Thr Met Cys Leu Gln Asp Gly Ala
               500                 505                 510
   Ile Val Lys Phe Asp
           515
<210> 15
   <211> 1554
   <212> DNA
   <213> Candida tropicalis
<220>
   <221> CDS
   <222> (1)..(1554)
<400> 15
   atg att gaa caa atc cta gaa tat tgg tat att gtt gtg cct gtg ttg      48
   Met Ile Glu Gln Ile Leu Glu Tyr Trp Tyr Ile Val Val Pro Val Leu
   1               5                   10                  15
   tac atc atc aaa caa ctc att gcc tac agc aag act cgc gtc ttg atg      96
   Tyr Ile Ile Lys Gln Leu Ile Ala Tyr Ser Lys Thr Arg Val Leu Met
               20                  25                  30
   aaa cag ttg ggt gct gct cca atc aca aac cag ttg tac gac aac gtt     144
   Lys Gln Leu Gly Ala Ala Pro Ile Thr Asn Gln Leu Tyr Asp Asn Val
           35                  40                  45
   ttc ggt atc gtc aac gga tgg aag gct ctc cag ttc aag aaa gag ggc     192
   Phe Gly Ile Val Asn Gly Trp Lys Ala Leu Gln Phe Lys Lys Glu Gly
       50                  55                  60
   aga gct caa gag tac aac gat cac aag ttt gac agc tcc aag aac cca     240
   Arg Ala Gln Glu Tyr Asn Asp His Lys Phe Asp Ser Ser Lys Asn Pro
   65                  70                  75                  80
   agc gtc ggc acc tat gtc agt att ctt ttt ggc acc aag att gtc gtg     288
   Ser Val Gly Thr Tyr Val Ser Ile Leu Phe Gly Thr Lys Ile Val Val
                   85                  90                  95
   acc aag gat cca gag aat atc aaa gct att ttg gca acc cag ttt ggc     336
   Thr Lys Asp Pro Glu Asn Ile Lys Ala Ile Leu Ala Thr Gln Phe Gly
               100                 105                 110
   gat ttt tct ttg ggc aag aga cac gct ctt ttt aaa cct ttg tta ggt     384
   Asp Phe Ser Leu Gly Lys Arg His Ala Leu Phe Lys Pro Leu Leu Gly
           115                 120                 125
   gat ggg atc ttc acc ttg gac ggc gaa ggc tgg aag cat agc aga tcc     432
   Asp Gly Ile Phe Thr Leu Asp Gly Glu Gly Trp Lys His Ser Arg Ser
       130                 135                 140
   atg tta aga cca cag ttt gcc aga gaa caa gtt gct cat gtg acg tcg     480
   Met Leu Arg Pro Gln Phe Ala Arg Glu Gln Val Ala His Val Thr Ser
   145                 150                 155                 160
   ttg gaa cca cac ttc cag ttg ttg aag aag cat atc ctt aaa cac aag     528
   Leu Glu Pro His Phe Gln Leu Leu Lys Lys His Ile Leu Lys His Lys
                   165                 170                 175
   ggt gag tac ttt gat atc cag gaa ttg ttc ttt aga ttt act gtc gac     576
   Gly Glu Tyr Phe Asp Ile Gln Glu Leu Phe Phe Arg Phe Thr Val Asp
               180                 185                 190
   tcg gcc acg gag ttc tta ttt ggt gag tcc gtg cac tcc tta aag gac     624
   Ser Ala Thr Glu Phe Leu Phe Gly Glu Ser Val His Ser Leu Lys Asp
           195                 200                 205
   gaa act atc ggt atc aac caa gac gat ata gat ttt gct ggt aga aag     672
   Glu Thr Ile Gly Ile Asn Gln Asp Asp Ile Asp Phe Ala Gly Arg Lys
       210                 215                 220
   gac ttt gct gag tcg ttc aac aaa gcc cag gag tat ttg tct att aga     720
   Asp Phe Ala Glu Ser Phe Asn Lys Ala Gln Glu Tyr Leu Ser Ile Arg
   225                 230                 235                 240
   att ttg gtg cag acc ttc tac tgg ttg atc aac aac aag gag ttt aga      768
   Ile Leu Val Gln Thr Phe Tyr Trp Leu Ile Asn Asn Lys Glu Phe Arg
                   245                 250                 255
   gac tgt acc aag ctg gtg cac aag ttt acc aac tac tat gtt cag aaa      816
   Asp Cys Thr Lys Leu Val His Lys Phe Thr Asn Tyr Tyr Val Gln Lys
               260                 265                 270
   gct ttg gat gct acc cca gag gaa ctt gaa aag caa ggc ggg tat gtg      864
   Ala Leu Asp Ala Thr Pro Glu Glu Leu Glu Lys Gln Gly Gly Tyr Val
           275                 280                 285
   ttc ttg tat gag ctt gtc aag cag acg aga gac ccc aag gtg ttg cgt      912
   Phe Leu Tyr Glu Leu Val Lys Gln Thr Arg Asp Pro Lys Val Leu Arg
       290                 295                 300
   gac cag tct ttg aac atc ttg ttg gca gga aga gac acc act gct ggg      960
   Asp Gln Ser Leu Asn Ile Leu Leu Ala Gly Arg Asp Thr Thr Ala Gly
   305                 310                 315                 320
   ttg ttg tcc ttt gct gtg ttt gag ttg gcc aga aac cca cac atc tgg     1008
   Leu Leu Ser Phe Ala Val Phe Glu Leu Ala Arg Asn Pro His Ile Trp
                   325                 330                 335
   gcc aag ttg aga gag gaa att gaa cag cag ttt ggt ctt gga gaa gac     1056
   Ala Lys Leu Arg Glu Glu Ile Glu Gln Gln Phe Gly Leu Gly Glu Asp
               340                 345                 350
   tct cgt gtt gaa gag att acc ttt gag agc ttg aag aga tgt gag tac     1104
   Ser Arg Val Glu Glu Ile Thr Phe Glu Ser Leu Lys Arg Cys Glu Tyr
           355                 360                 365
   ttg aaa gcg ttc ctt aac gaa acc ttg cgt gtt tac cca agt gtc cca     1152
   Leu Lys Ala Phe Leu Asn Glu Thr Leu Arg Val Tyr Pro Ser Val Pro
       370                 375                 380
   aga aac ttc aga atc gcc acc aag aat aca aca ttg cca agg ggt ggt     1200
   Arg Asn Phe Arg Ile Ala Thr Lys Asn Thr Thr Leu Pro Arg Gly Gly
   385                 390                 395                 400
   ggt cca gac ggt acc cag cca atc ttg atc caa aag gga gaa ggt gtg     1248
   Gly Pro Asp Gly Thr Gln Pro Ile Leu Ile Gln Lys Gly Glu Gly Val
                   405                 410                 415
   tcg tat ggt atc aac tct acc cac tta gat cct gtc tat tat ggc cct     1296
   Ser Tyr Gly Ile Asn Ser Thr His Leu Asp Pro Val Tyr Tyr Gly Pro
               420                 425                 430
   gat gct gct gag ttc aga cca gag aga tgg ttt gag cca tca acc aga     1344
   Asp Ala Ala Glu Phe Arg Pro Glu Arg Trp Phe Glu Pro Ser Thr Arg
           435                 440                 445
   aag ctc ggc tgg gct tac ttg cca ttc aac ggt ggg cca cga atc tgt     1392
   Lys Leu Gly Trp Ala Tyr Leu Pro Phe Asn Gly Gly Pro Arg Ile Cys
       450                 455                 460
   ttg ggt cag cag ttt gcc ttg acc gaa gct ggt tac gtt ttg gtc aga     1440
   Leu Gly Gln Gln Phe Ala Leu Thr Glu Ala Gly Tyr Val Leu Val Arg
   465                 470                 475                 480
   ttg gtg caa gag ttc tcc cac att agg ctg gac cca gat gaa gtg tat     1488
   Leu Val Gln Glu Phe Ser His Ile Arg Leu Asp Pro Asp Glu Val Tyr
                   485                 490                 495
   cca cca aag agg ttg acc aac ttg acc atg tgt ttg cag gat ggt gct      1536
   Pro Pro Lys Arg Leu Thr Asn Leu Thr Met Cys Leu Gln Asp Gly Ala
               500                 505                 510
   att gtc aag ttt gac tag                                              1554
   Ile Val Lys Phe Asp
           515
<210> 16
   <211> 517
   <212> PRT
   <213> Candida tropicalis
<400> 16
   Met Ile Glu Gln Ile Leu Glu Tyr Trp Tyr Ile Val Val Pro Val Leu
   1               5                   10                  15
   Tyr Ile Ile Lys Gln Leu Ile Ala Tyr Ser Lys Thr Arg Val Leu Met
               20                  25                  30
   Lys Gln Leu Gly Ala Ala Pro Ile Thr Asn Gln Leu Tyr Asp Asn Val
           35                  40                  45
   Phe Gly Ile Val Asn Gly Trp Lys Ala Leu Gln Phe Lys Lys Glu Gly
       50                  55                  60
   Arg Ala Gln Glu Tyr Asn Asp His Lys Phe Asp Ser Ser Lys Asn Pro
   65                  70                  75                  80
   Ser Val Gly Thr Tyr Val Ser Ile Leu Phe Gly Thr Lys Ile Val Val
                   85                  90                  95
   Thr Lys Asp Pro Glu Asn Ile Lys Ala Ile Leu Ala Thr Gln Phe Gly
               100                 105                 110
   Asp Phe Ser Leu Gly Lys Arg His Ala Leu Phe Lys Pro Leu Leu Gly
           115                 120                 125
   Asp Gly Ile Phe Thr Leu Asp Gly Glu Gly Trp Lys His Ser Arg Ser
       130                 135                 140
   Met Leu Arg Pro Gln Phe Ala Arg Glu Gln Val Ala His Val Thr Ser
   145                 150                 155                 160
   Leu Glu Pro His Phe Gln Leu Leu Lys Lys His Ile Leu Lys His Lys
                   165                 170                 175
   Gly Glu Tyr Phe Asp Ile Gln Glu Leu Phe Phe Arg Phe Thr Val Asp
   180 185 190
   Ser Ala Thr Glu Phe Leu Phe Gly Glu Ser Val His Ser Leu Lys Asp
   195 200 205
   Glu Thr Ile Gly Ile Asn Gln Asp Asp Ile Asp Phe Ala Gly Arg Lys
   210 215 220
   Asp Phe Ala Glu Ser Phe Asn Lys Ala Gln Glu Tyr Leu Ser Ile Arg
   225 230 235 240
   Ile Leu Val Gln Thr Phe Tyr Trp Leu Ile Asn Asn Lys Glu Phe Arg
   245 250 255
   Asp Cys Thr Lys Leu Val His Lys Phe Thr Asn Tyr Tyr Val Gln Lys
   260 265 270
   Ala Leu Asp Ala Thr Pro Glu Glu Leu Glu Lys Gln Gly Gly Tyr Val
   275 280 285
   Phe Leu Tyr Glu Leu Val Lys Gln Thr Arg Asp Pro Lys Val Leu Arg
   290 295 300
   Asp Gln Ser Leu Asn Ile Leu Leu Ala Gly Arg Asp Thr Thr Ala Gly
   305 310 315 320
   Leu Leu Ser Phe Ala Val Phe Glu Leu Ala Arg Asn Pro His Ile Trp
   325 330 335
   Ala Lys Leu Arg Glu Glu Ile Glu Gln Gln Phe Gly Leu Gly Glu Asp
   340 345 350
   Ser Arg Val Glu Glu Ile Thr Phe Glu Ser Leu Lys Arg Cys Glu Tyr
   355 360 365
   Leu Lys Ala Phe Leu Asn Glu Thr Leu Arg Val Tyr Pro Ser Val Pro
   370 375 380
   Arg Asn Phe Arg Ile Ala Thr Lys Asn Thr Thr Leu Pro Arg Gly Gly
   385 390 395 400
   Gly Pro Asp Gly Thr Gln Pro Ile Leu Ile Gln Lys Gly Glu Gly Val
   405 410 415
   Ser Tyr Gly Ile Asn Ser Thr His Leu Asp Pro Val Tyr Tyr Gly Pro
   420 425 430
   Asp Ala Ala Glu Phe Arg Pro Glu Arg Trp Phe Glu Pro Ser Thr Arg
           435                 440                 445
   Lys Leu Gly Trp Ala Tyr Leu Pro Phe Asn Gly Gly Pro Arg Ile Cys
       450                 455                 460
   Leu Gly Gln Gln Phe Ala Leu Thr Glu Ala Gly Tyr Val Leu Val Arg
   465                 470                 475                 480
   Leu Val Gln Glu Phe Ser His Ile Arg Leu Asp Pro Asp Glu Val Tyr
                   485                 490                 495
   Pro Pro Lys Arg Leu Thr Asn Leu Thr Met Cys Leu Gln Asp Gly Ala
               500                 505                 510
   Ile Val Lys Phe Asp
           515
<210> 17
   <211> 1539
   <212> DNA
   <213> Candida tropicalis
<220>
   <221> CDS
   <222> (1)..(1539)
<400> 17
   atg ctc gat cag atc tta cat tac tgg tac att gtc ttg cca ttg ttg        48
   Met Leu Asp Gln Ile Leu His Tyr Trp Tyr Ile Val Leu Pro Leu Leu
   1               5                   10                  15
   gcc att atc aac cag atc gtg gct cat gtc agg acc aat tat ttg atg        96
   Ala Ile Ile Asn Gln Ile Val Ala His Val Arg Thr Asn Tyr Leu Met
               20                  25                  30
   aag aaa ttg ggt gct aag cca ttc aca cac gtc caa cgt gac ggg tgg       144
   Lys Lys Leu Gly Ala Lys Pro Phe Thr His Val Gln Arg Asp Gly Trp
           35                  40                  45
   ttg ggc ttc aaa ttc ggc cgt gaa ttc ctc aaa gca aaa agt gct ggg       192
   Leu Gly Phe Lys Phe Gly Arg Glu Phe Leu Lys Ala Lys Ser Ala Gly
       50                  55                  60
   aga ctg gtt gat tta atc atc tcc cgt ttc cac gat aat gag gac act       240
   Arg Leu Val Asp Leu Ile Ile Ser Arg Phe His Asp Asn Glu Asp Thr
   65                  70                  75                  80
   ttc tcc agc tat gct ttt ggc aac cat gtg gtg ttc acc agg gac ccc       288
   Phe Ser Ser Tyr Ala Phe Gly Asn His Val Val Phe Thr Arg Asp Pro
                   85                  90                  95
   gag aat atc aag gcg ctt ttg gca acc cag ttt ggt gat ttt tca ttg       336
   Glu Asn Ile Lys Ala Leu Leu Ala Thr Gln Phe Gly Asp Phe Ser Leu
               100                 105                 110
   ggc agc agg gtc aag ttc ttc aaa cca tta ttg ggg tac ggt atc ttc      384
   Gly Ser Arg Val Lys Phe Phe Lys Pro Leu Leu Gly Tyr Gly Ile Phe
           115                 120                 125
   aca ttg gac gcc gaa ggc tgg aag cac agc aga gcc atg ttg aga cca      432
   Thr Leu Asp Ala Glu Gly Trp Lys His Ser Arg Ala Met Leu Arg Pro
       130                 135                 140
   cag ttt gcc aga gaa caa gtt gct cat gtg acg tcg ttg gaa cca cac      480
   Gln Phe Ala Arg Glu Gln Val Ala His Val Thr Ser Leu Glu Pro His
   145                 150                 155                 160
   ttc cag ttg ttg aag aag cat atc ctt aaa cac aag ggt gag tac ttt      528
   Phe Gln Leu Leu Lys Lys His Ile Leu Lys His Lys Gly Glu Tyr Phe
                   165                 170                 175
   gat atc cag gaa ttg ttc ttt aga ttt act gtc gac tcg gcc acg gag      576
   Asp Ile Gln Glu Leu Phe Phe Arg Phe Thr Val Asp Ser Ala Thr Glu
               180                 185                 190
   ttc tta ttt ggt gag tcc gtg cac tcc tta aag gac gag gaa att ggc      624
   Phe Leu Phe Gly Glu Ser Val His Ser Leu Lys Asp Glu Glu Ile Gly
           195                 200                 205
   tac gac acg aaa gac atg tct gaa gaa aga cgc aga ttt gcc gac gcg      672
   Tyr Asp Thr Lys Asp Met Ser Glu Glu Arg Arg Arg Phe Ala Asp Ala
       210                 215                 220
   ttc aac aag tcg caa gtc tac gtg gcc acc aga gtt gct tta cag aac      720
   Phe Asn Lys Ser Gln Val Tyr Val Ala Thr Arg Val Ala Leu Gln Asn
   225                 230                 235                 240
   ttg tac tgg ttg gtc aac aac aaa gag ttc aag gag tgc aat gac att      768
   Leu Tyr Trp Leu Val Asn Asn Lys Glu Phe Lys Glu Cys Asn Asp Ile
                   245                 250                 255
   gtc cac aag ttt acc aac tac tat gtt cag aaa gcc ttg gat gct acc      816
   Val His Lys Phe Thr Asn Tyr Tyr Val Gln Lys Ala Leu Asp Ala Thr
               260                 265                 270
   cca gag gaa ctt gaa aag caa ggc ggg tat gtg ttc ttg tat gag ctt      864
   Pro Glu Glu Leu Glu Lys Gln Gly Gly Tyr Val Phe Leu Tyr Glu Leu
           275                 280                 285
   gtc aag cag acg aga gac ccc aag gtg ttg cgt gac cag tct ttg aac      912
   Val Lys Gln Thr Arg Asp Pro Lys Val Leu Arg Asp Gln Ser Leu Asn
       290                 295                 300
   atc ttg ttg gca gga aga gac acc act gct ggg ttg ttg tcc ttt gct      960
   Ile Leu Leu Ala Gly Arg Asp Thr Thr Ala Gly Leu Leu Ser Phe Ala
   305                 310                 315                 320
   gtg ttt gag ttg gcc aga aac cca cac atc tgg gcc aag ttg aga gag     1008
   Val Phe Glu Leu Ala Arg Asn Pro His Ile Trp Ala Lys Leu Arg Glu
                   325                 330                 335
   gaa att gaa cag cag ttt ggt ctt gga gaa gac tct cgt gtt gaa gag     1056
   Glu Ile Glu Gln Gln Phe Gly Leu Gly Glu Asp Ser Arg Val Glu Glu
               340                 345                 350
   att acc ttt gag agc ttg aag aga tgt gag tac ttg aag gcc gtg ttg     1104
   Ile Thr Phe Glu Ser Leu Lys Arg Cys Glu Tyr Leu Lys Ala Val Leu
           355                 360                 365
   aac gaa act ttg aga tta cac cca agt gtc cca aga aac gca aga ttt      1152
   Asn Glu Thr Leu Arg Leu His Pro Ser Val Pro Arg Asn Ala Arg Phe
       370                 375                 380
   gcg att aaa gac acg act tta cca aga ggc ggt ggc ccc aac ggc aag      1200
   Ala Ile Lys Asp Thr Thr Leu Pro Arg Gly Gly Gly Pro Asn Gly Lys
   385                 390                 395                 400
   gat cct atc ttg atc agg aag gat gag gtg gtg cag tac tcc atc tcg      1248
   Asp Pro Ile Leu Ile Arg Lys Asp Glu Val Val Gln Tyr Ser Ile Ser
                   405                 410                 415
   gca act cag aca aat cct gct tat tat ggc gcc gat gct gct gat ttt      1296
   Ala Thr Gln Thr Asn Pro Ala Tyr Tyr Gly Ala Asp Ala Ala Asp Phe
               420                 425                 430
   aga ccg gaa aga tgg ttt gaa cca tca act aga aac ttg gga tgg gct      1344
   Arg Pro Glu Arg Trp Phe Glu Pro Ser Thr Arg Asn Leu Gly Trp Ala
           435                 440                 445
   ttc ttg cca ttc aac ggt ggt cca aga atc tgt ttg gga caa cag ttt      1392
   Phe Leu Pro Phe Asn Gly Gly Pro Arg Ile Cys Leu Gly Gln Gln Phe
       450                 455                 460
   gct ttg act gaa gcc ggt tac gtt ttg gtt aga ctt gtt cag gag ttt      1440
   Ala Leu Thr Glu Ala Gly Tyr Val Leu Val Arg Leu Val Gln Glu Phe
   465                 470                 475                 480
   cca aac ttg tca caa gac ccc gaa acc aag tac cca cca cct aga ttg      1488
   Pro Asn Leu Ser Gln Asp Pro Glu Thr Lys Tyr Pro Pro Pro Arg Leu
                   485                 490                 495
   gca cac ttg acg atg tgc ttg ttt gac ggt gca cac gtc aag atg tca      1536
   Ala His Leu Thr Met Cys Leu Phe Asp Gly Ala His Val Lys Met Ser
               500                 505                 510
   tag                                                                  1539
<210> 18
   <211> 512
   <212> PRT
   <213> Candida tropicalis
<400> 18
   Met Leu Asp Gln Ile Leu His Tyr Trp Tyr Ile Val Leu Pro Leu Leu
   1               5                   10                  15
   Ala Ile Ile Asn Gln Ile Val Ala His Val Arg Thr Asn Tyr Leu Met
               20                  25                  30
   Lys Lys Leu Gly Ala Lys Pro Phe Thr His Val Gln Arg Asp Gly Trp
           35                  40                  45
   Leu Gly Phe Lys Phe Gly Arg Glu Phe Leu Lys Ala Lys Ser Ala Gly
       50                  55                  60
   Arg Leu Val Asp Leu Ile Ile Ser Arg Phe His Asp Asn Glu Asp Thr
   65                  70                  75                  80
   Phe Ser Ser Tyr Ala Phe Gly Asn His Val Val Phe Thr Arg Asp Pro
                   85                  90                  95
   Glu Asn Ile Lys Ala Leu Leu Ala Thr Gln Phe Gly Asp Phe Ser Leu
               100                 105                 110
   Gly Ser Arg Val Lys Phe Phe Lys Pro Leu Leu Gly Tyr Gly Ile Phe
           115                 120                 125
   Thr Leu Asp Ala Glu Gly Trp Lys His Ser Arg Ala Met Leu Arg Pro
       130                 135                 140
   Gln Phe Ala Arg Glu Gln Val Ala His Val Thr Ser Leu Glu Pro His
   145                 150                 155                 160
   Phe Gln Leu Leu Lys Lys His Ile Leu Lys His Lys Gly Glu Tyr Phe
                   165                 170                 175
   Asp Ile Gln Glu Leu Phe Phe Arg Phe Thr Val Asp Ser Ala Thr Glu
               180                 185                 190
   Phe Leu Phe Gly Glu Ser Val His Ser Leu Lys Asp Glu Glu Ile Gly
           195                 200                 205
   Tyr Asp Thr Lys Asp Met Ser Glu Glu Arg Arg Arg Phe Ala Asp Ala
       210                 215                 220
   Phe Asn Lys Ser Gln Val Tyr Val Ala Thr Arg Val Ala Leu Gln Asn
   225                 230                 235                 240
   Leu Tyr Trp Leu Val Asn Asn Lys Glu Phe Lys Glu Cys Asn Asp Ile
                   245                 250                 255
   Val His Lys Phe Thr Asn Tyr Tyr Val Gln Lys Ala Leu Asp Ala Thr
               260                 265                 270
   Pro Glu Glu Leu Glu Lys Gln Gly Gly Tyr Val Phe Leu Tyr Glu Leu
           275                 280                 285
   Val Lys Gln Thr Arg Asp Pro Lys Val Leu Arg Asp Gln Ser Leu Asn
       290                 295                 300
   Ile Leu Leu Ala Gly Arg Asp Thr Thr Ala Gly Leu Leu Ser Phe Ala
   305                 310                 315                 320
   Val Phe Glu Leu Ala Arg Asn Pro His Ile Trp Ala Lys Leu Arg Glu
                   325                 330                 335
   Glu Ile Glu Gln Gln Phe Gly Leu Gly Glu Asp Ser Arg Val Glu Glu
               340                 345                 350
   Ile Thr Phe Glu Ser Leu Lys Arg Cys Glu Tyr Leu Lys Ala Val Leu
           355                 360                 365
   Asn Glu Thr Leu Arg Leu His Pro Ser Val Pro Arg Asn Ala Arg Phe
       370                 375                 380
   Ala Ile Lys Asp Thr Thr Leu Pro Arg Gly Gly Gly Pro Asn Gly Lys
   385                 390                 395                 400
   Asp Pro Ile Leu Ile Arg Lys Asp Glu Val Val Gln Tyr Ser Ile Ser
                   405                 410                 415
   Ala Thr Gln Thr Asn Pro Ala Tyr Tyr Gly Ala Asp Ala Ala Asp Phe
               420                 425                 430
   Arg Pro Glu Arg Trp Phe Glu Pro Ser Thr Arg Asn Leu Gly Trp Ala
           435                 440                 445
   Phe Leu Pro Phe Asn Gly Gly Pro Arg Ile Cys Leu Gly Gln Gln Phe
       450                 455                 460
   Ala Leu Thr Glu Ala Gly Tyr Val Leu Val Arg Leu Val Gln Glu Phe
   465                 470                 475                 480
   Pro Asn Leu Ser Gln Asp Pro Glu Thr Lys Tyr Pro Pro Pro Arg Leu
                   485                 490                 495
   Ala His Leu Thr Met Cys Leu Phe Asp Gly Ala His Val Lys Met Ser
               500                 505                 510
<210> 19
   <211> 1539
   <212> DNA
   <213> Candida tropicalis
<220>
   <221> CDS
   <222> (1)..(1539)
<400> 19
   atg ctc gac cag atc ttc cat tac tgg tac att gtc ttg cca ttg ttg       48
   Met Leu Asp Gln Ile Phe His Tyr Trp Tyr Ile Val Leu Pro Leu Leu
   1               5                   10                  15
   gtc att atc aag cag atc gtg gct cat gcc agg acc aat tat ttg atg      96
   Val Ile Ile Lys Gln Ile Val Ala His Ala Arg Thr Asn Tyr Leu Met
               20                  25                  30
   aag aag ttg ggc gct aag cca ttc aca cat gtc caa cta gac ggg tgg     144
   Lys Lys Leu Gly Ala Lys Pro Phe Thr His Val Gln Leu Asp Gly Trp
           35                  40                  45
   ttt ggc ttc aaa ttt ggc cgt gaa ttc ctc aaa gct aaa agt gct ggg     192
   Phe Gly Phe Lys Phe Gly Arg Glu Phe Leu Lys Ala Lys Ser Ala Gly
       50                  55                  60
   agg cag gtt gat tta atc atc tcc cgt ttc cac gat aat gag gac act     240
   Arg Gln Val Asp Leu Ile Ile Ser Arg Phe His Asp Asn Glu Asp Thr
   65                  70                  75                  80
   ttc tcc agc tat gct ttt ggc aac cat gtg gtg ttc acc agg gac ccc     288
   Phe Ser Ser Tyr Ala Phe Gly Asn His Val Val Phe Thr Arg Asp Pro
                   85                  90                  95
   gag aat atc aag gcg ctt ttg gca acc cag ttt ggt gat ttt tca ttg     336
   Glu Asn Ile Lys Ala Leu Leu Ala Thr Gln Phe Gly Asp Phe Ser Leu
               100                 105                 110
   gga agc agg gtc aaa ttc ttc aaa cca ttg ttg ggg tac ggt atc ttc     384
   Gly Ser Arg Val Lys Phe Phe Lys Pro Leu Leu Gly Tyr Gly Ile Phe
           115                 120                 125
   acc ttg gac ggc gaa ggc tgg aag cac agc aga gcc atg ttg aga cca     432
   Thr Leu Asp Gly Glu Gly Trp Lys His Ser Arg Ala Met Leu Arg Pro
       130                 135                 140
   cag ttt gcc aga gag caa gtt gct cat gtg acg tcg ttg gaa cca cat     480
   Gln Phe Ala Arg Glu Gln Val Ala His Val Thr Ser Leu Glu Pro His
   145                 150                 155                 160
   ttc cag ttg ttg aag aag cat att ctt aag cac aag ggt gaa tac ttt     528
   Phe Gln Leu Leu Lys Lys His Ile Leu Lys His Lys Gly Glu Tyr Phe
                   165                 170                 175
   gat atc cag gaa ttg ttc ttt aga ttt acc gtt gat tca gcg acg gag     576
   Asp Ile Gln Glu Leu Phe Phe Arg Phe Thr Val Asp Ser Ala Thr Glu
               180                 185                 190
   ttc tta ttt ggt gag tcc gtg cac tcc tta agg gac gag gaa att ggc     624
   Phe Leu Phe Gly Glu Ser Val His Ser Leu Arg Asp Glu Glu Ile Gly
           195                 200                 205
   tac gat acg aag gac atg gct gaa gaa aga cgc aaa ttt gcc gac gcg     672
   Tyr Asp Thr Lys Asp Met Ala Glu Glu Arg Arg Lys Phe Ala Asp Ala
       210                 215                 220
   ttc aac aag tcg caa gtc tat ttg tcc acc aga gtt gct tta cag aca     720
   Phe Asn Lys Ser Gln Val Tyr Leu Ser Thr Arg Val Ala Leu Gln Thr
   225                 230                 235                 240
   ttg tac tgg ttg gtc aac aac aaa gag ttc aag gag tgc aac gac att     768
   Leu Tyr Trp Leu Val Asn Asn Lys Glu Phe Lys Glu Cys Asn Asp Ile
                   245                 250                 255
   gtc cac aag ttc acc aac tac tat gtt cag aaa gcc ttg gat gct acc     816
   Val His Lys Phe Thr Asn Tyr Tyr Val Gln Lys Ala Leu Asp Ala Thr
               260                 265                 270
   cca gag gaa ctt gaa aaa caa ggc ggg tat gtg ttc ttg tac gag ctt       864
   Pro Glu Glu Leu Glu Lys Gln Gly Gly Tyr Val Phe Leu Tyr Glu Leu
           275                 280                 285
   gcc aag cag acg aaa gac ccc aat gtg ttg cgt gac cag tct ttg aac       912
   Ala Lys Gln Thr Lys Asp Pro Asn Val Leu Arg Asp Gln Ser Leu Asn
       290                 295                 300
   atc ttg ttg gct gga agg gac acc act gct ggg ttg ttg tcc ttt gct       960
   Ile Leu Leu Ala Gly Arg Asp Thr Thr Ala Gly Leu Leu Ser Phe Ala
   305                 310                 315                 320
   gtg ttt gag ttg gcc agg aac cca cac atc tgg gcc aag ttg aga gag      1008
   Val Phe Glu Leu Ala Arg Asn Pro His Ile Trp Ala Lys Leu Arg Glu
                   325                 330                 335
   gaa att gaa tca cac ttt ggg ctg ggt gag gac tct cgt gtt gaa gag      1056
   Glu Ile Glu Ser His Phe Gly Leu Gly Glu Asp Ser Arg Val Glu Glu
               340                 345                 350
   att acc ttt gag agc ttg aag aga tgt gag tac ttg aaa gcc gtg ttg      1104
   Ile Thr Phe Glu Ser Leu Lys Arg Cys Glu Tyr Leu Lys Ala Val Leu
           355                 360                 365
   aac gaa acg ttg aga tta cac cca agt gtc cca aga aac gca aga ttt      1152
   Asn Glu Thr Leu Arg Leu His Pro Ser Val Pro Arg Asn Ala Arg Phe
       370                 375                 380
   gcg att aaa gac acg act tta cca aga ggc ggt ggc ccc aac ggc aag      1200
   Ala Ile Lys Asp Thr Thr Leu Pro Arg Gly Gly Gly Pro Asn Gly Lys
   385                 390                 395                 400
   gat cct atc ttg atc aga aag aat gag gtg gtg caa tac tcc atc tcg      1248
   Asp Pro Ile Leu Ile Arg Lys Asn Glu Val Val Gln Tyr Ser Ile Ser
                   405                 410                 415
   gca act cag aca aat cct gct tat tat ggc gcc gat gct gct gat ttt      1296
   Ala Thr Gln Thr Asn Pro Ala Tyr Tyr Gly Ala Asp Ala Ala Asp Phe
               420                 425                 430
   aga ccg gaa aga tgg ttt gag cca tca act aga aac ttg gga tgg gct      1344
   Arg Pro Glu Arg Trp Phe Glu Pro Ser Thr Arg Asn Leu Gly Trp Ala
           435                 440                 445
   tac ttg cca ttc aac ggt ggt cca aga atc tgc ttg gga caa cag ttt      1392
   Tyr Leu Pro Phe Asn Gly Gly Pro Arg Ile Cys Leu Gly Gln Gln Phe
       450                 455                 460
   gct ttg acc gaa gcc ggt tac gtt ttg gtt aga ctt gtt cag gaa ttc      1440
   Ala Leu Thr Glu Ala Gly Tyr Val Leu Val Arg Leu Val Gln Glu Phe
   465                 470                 475                 480
   cct agc ttg tca cag gac ccc gaa act gag tac cca cca cct aga ttg      1488
   Pro Ser Leu Ser Gln Asp Pro Glu Thr Glu Tyr Pro Pro Pro Arg Leu
                   485                 490                 495
   gca cac ttg acg atg tgc ttg ttt gac ggg gca tac gtc aag atg caa      1536
   Ala His Leu Thr Met Cys Leu Phe Asp Gly Ala Tyr Val Lys Met Gln
               500                 505                 510
   tag                                                                  1539
<210> 20
   <211> 512
   <212> PRT
   <213> Candida tropicalis
<400> 20
   Met Leu Asp Gln Ile Phe His Tyr Trp Tyr Ile Val Leu Pro Leu Leu
   1               5                   10                  15
   Val Ile Ile Lys Gln Ile Val Ala His Ala Arg Thr Asn Tyr Leu Met
               20                  25                  30
   Lys Lys Leu Gly Ala Lys Pro Phe Thr His Val Gln Leu Asp Gly Trp
           35                  40                  45
   Phe Gly Phe Lys Phe Gly Arg Glu Phe Leu Lys Ala Lys Ser Ala Gly
       50                  55                  60
   Arg Gln Val Asp Leu Ile Ile Ser Arg Phe His Asp Asn Glu Asp Thr
   65                  70                  75                  80
   Phe Ser Ser Tyr Ala Phe Gly Asn His Val Val Phe Thr Arg Asp Pro
                   85                  90                  95
   Glu Asn Ile Lys Ala Leu Leu Ala Thr Gln Phe Gly Asp Phe Ser Leu
               100                 105                 110
   Gly Ser Arg Val Lys Phe Phe Lys Pro Leu Leu Gly Tyr Gly Ile Phe
           115                 120                 125
   Thr Leu Asp Gly Glu Gly Trp Lys His Ser Arg Ala Met Leu Arg Pro
       130                 135                 140
   Gln Phe Ala Arg Glu Gln Val Ala His Val Thr Ser Leu Glu Pro His
   145                 150                 155                 160
   Phe Gln Leu Leu Lys Lys His Ile Leu Lys His Lys Gly Glu Tyr Phe
                   165                 170                 175
   Asp Ile Gln Glu Leu Phe Phe Arg Phe Thr Val Asp Ser Ala Thr Glu
               180                 185                 190
   Phe Leu Phe Gly Glu Ser Val His Ser Leu Arg Asp Glu Glu Ile Gly
           195                 200                 205
   Tyr Asp Thr Lys Asp Met Ala Glu Glu Arg Arg Lys Phe Ala Asp Ala
       210                 215                 220
   Phe Asn Lys Ser Gln Val Tyr Leu Ser Thr Arg Val Ala Leu Gln Thr
   225                 230                 235                 240
   Leu Tyr Trp Leu Val Asn Asn Lys Glu Phe Lys Glu Cys Asn Asp Ile
                   245                 250                 255
   Val His Lys Phe Thr Asn Tyr Tyr Val Gln Lys Ala Leu Asp Ala Thr
               260                 265                 270
   Pro Glu Glu Leu Glu Lys Gln Gly Gly Tyr Val Phe Leu Tyr Glu Leu
           275                 280                 285
   Ala Lys Gln Thr Lys Asp Pro Asn Val Leu Arg Asp Gln Ser Leu Asn
       290                 295                 300
   Ile Leu Leu Ala Gly Arg Asp Thr Thr Ala Gly Leu Leu Ser Phe Ala
   305                 310                 315                 320
   Val Phe Glu Leu Ala Arg Asn Pro His Ile Trp Ala Lys Leu Arg Glu
                   325                 330                 335
   Glu Ile Glu Ser His Phe Gly Leu Gly Glu Asp Ser Arg Val Glu Glu
               340                 345                 350
   Ile Thr Phe Glu Ser Leu Lys Arg Cys Glu Tyr Leu Lys Ala Val Leu
           355                 360                 365
   Asn Glu Thr Leu Arg Leu His Pro Ser Val Pro Arg Asn Ala Arg Phe
       370                 375                 380
   Ala Ile Lys Asp Thr Thr Leu Pro Arg Gly Gly Gly Pro Asn Gly Lys
   385                 390                 395                 400
   Asp Pro Ile Leu Ile Arg Lys Asn Glu Val Val Gln Tyr Ser Ile Ser
                   405                 410                 415
   Ala Thr Gln Thr Asn Pro Ala Tyr Tyr Gly Ala Asp Ala Ala Asp Phe
               420                 425                 430
   Arg Pro Glu Arg Trp Phe Glu Pro Ser Thr Arg Asn Leu Gly Trp Ala
           435                 440                 445
   Tyr Leu Pro Phe Asn Gly Gly Pro Arg Ile Cys Leu Gly Gln Gln Phe
       450                 455                 460
   Ala Leu Thr Glu Ala Gly Tyr Val Leu Val Arg Leu Val Gln Glu Phe
   465 470 475 480
   Pro Ser Leu Ser Gln Asp Pro Glu Thr Glu Tyr Pro Pro Pro Arg Leu
   485 490 495
   Ala His Leu Thr Met Cys Leu Phe Asp Gly Ala Tyr Val Lys Met Gln
   500 505 510
<210> 21
   <211> 390
   <212> DNA
   <213> Candida tropicalis
<220>
   <221> CDS
   <222> (1)..(390)
<400> 21
   atg acc gac aca gac acc acg acc acc atc tac acc cac gaa gag gtt       48
   Met Thr Asp Thr Asp Thr Thr Thr Thr Ile Tyr Thr His Glu Glu Val
   1               5                   10                  15
   gcc cag cac acc acc cac gac gac ttg tgg gtt att ctc aat ggt aag       96
   Ala Gln His Thr Thr His Asp Asp Leu Trp Val Ile Leu Asn Gly Lys
               20                  25                  30
   gtc tac aac atc tcc aac tat ata gac gag cac cca ggt ggt gaa gaa      144
   Val Tyr Asn Ile Ser Asn Tyr Ile Asp Glu His Pro Gly Gly Glu Glu
           35                  40                  45
   gtc att ctt gat tgc gcc ggc aca gac gcc act gaa gcc ttt gac gac      192
   Val Ile Leu Asp Cys Ala Gly Thr Asp Ala Thr Glu Ala Phe Asp Asp
       50                  55                  60
   att ggc cac tcc gac gag gcc cac gag atc ttg gaa aag ttg tac att      240
   Ile Gly His Ser Asp Glu Ala His Glu Ile Leu Glu Lys Leu Tyr Ile
   65                  70                  75                  80
   ggt aac ttg aag ggc gct aag att gtt gag gcc aag cac gcg cag tcg      288
   Gly Asn Leu Lys Gly Ala Lys Ile Val Glu Ala Lys His Ala Gln Ser
                   85                  90                  95
   ttc agc acg gaa gaa gac tcg ggt atc aac ttc cca ttg att gct gtt      336
   Phe Ser Thr Glu Glu Asp Ser Gly Ile Asn Phe Pro Leu Ile Ala Val
               100                 105                 110
   ggt gtg ttt ttg gct gct ttc ggt gtc tac tac tac aag acc aac ttt      384
   Gly Val Phe Leu Ala Ala Phe Gly Val Tyr Tyr Tyr Lys Thr Asn Phe
           115                 120                 125
   gcc taa                                                              390
   Ala
<210> 22
   <211> 129
   <212> PRT
   <213> Candida tropicalis
<400> 22
   Met Thr Asp Thr Asp Thr Thr Thr Thr Ile Tyr Thr His Glu Glu Val
   1               5                   10                  15
   Ala Gln His Thr Thr His Asp Asp Leu Trp Val Ile Leu Asn Gly Lys
               20                  25                  30
   Val Tyr Asn Ile Ser Asn Tyr Ile Asp Glu His Pro Gly Gly Glu Glu
           35                  40                  45
   Val Ile Leu Asp Cys Ala Gly Thr Asp Ala Thr Glu Ala Phe Asp Asp
       50                  55                  60
   Ile Gly His Ser Asp Glu Ala His Glu Ile Leu Glu Lys Leu Tyr Ile
   65                  70                  75                  80
   Gly Asn Leu Lys Gly Ala Lys Ile Val Glu Ala Lys His Ala Gln Ser
                   85                  90                  95
   Phe Ser Thr Glu Glu Asp Ser Gly Ile Asn Phe Pro Leu Ile Ala Val
               100                 105                 110
   Gly Val Phe Leu Ala Ala Phe Gly Val Tyr Tyr Tyr Lys Thr Asn Phe
           115                 120                 125
   Ala
<210> 23
   <211> 2115
   <212> DNA
   <213> Candida tropicalis
<220>
   <221> CDS
   <222> (1)..(2115)
<400> 23
   atg gct cca ttt ttg ccc gac cag gtc gac tac aaa cac gtc gac acc       48
   Met Ala Pro Phe Leu Pro Asp Gln Val Asp Tyr Lys His Val Asp Thr
   1               5                   10                  15
   ctt atg tta tta tgt gac ggg atc atc cac gaa acc acc gtg gac gaa       96
   Leu Met Leu Leu Cys Asp Gly Ile Ile His Glu Thr Thr Val Asp Glu
               20                  25                  30
   atc aaa gac gtc att gcc cct gac ttc ccc gcc gac aaa tac gag gag      144
   Ile Lys Asp Val Ile Ala Pro Asp Phe Pro Ala Asp Lys Tyr Glu Glu
           35                  40                  45
   tac gtc agg aca ttc acc aaa ccc tcc gaa acc cca ggg ttc agg gaa      192
   Tyr Val Arg Thr Phe Thr Lys Pro Ser Glu Thr Pro Gly Phe Arg Glu
       50                  55                  60
   acc gtc tac aac acc gtc aac gca aac acc atg gat gca atc cac cag      240
   Thr Val Tyr Asn Thr Val Asn Ala Asn Thr Met Asp Ala Ile His Gln
   65                  70                  75                  80
   ttc att atc ttg acc aat gtt ttg gga tca agg gtc ttg gca cca gct      288
   Phe Ile Ile Leu Thr Asn Val Leu Gly Ser Arg Val Leu Ala Pro Ala
                   85                  90                  95
   ttg acc aac tcg ttg act cct atc aag gac atg agc ttg gaa gac cgt      336
   Leu Thr Asn Ser Leu Thr Pro Ile Lys Asp Met Ser Leu Glu Asp Arg
               100                 105                 110
   gaa aag ttg tta gcc tcg tgg cgt gac tcc cct att gct gct aaa agg      384
   Glu Lys Leu Leu Ala Ser Trp Arg Asp Ser Pro Ile Ala Ala Lys Arg
           115                 120                 125
   aag ttg ttc agg ttg gtt tct acg ctt acc ttg gtc acg ttc acg aga      432
   Lys Leu Phe Arg Leu Val Ser Thr Leu Thr Leu Val Thr Phe Thr Arg
       130                 135                 140
   ttg gcc aat gag ttg cat ttg aaa gcc att cat tat cca gga aga gaa      480
   Leu Ala Asn Glu Leu His Leu Lys Ala Ile His Tyr Pro Gly Arg Glu
   145                 150                 155                 160
   gac cgt gaa aag gct tat gaa acc cag gag att gac cct ttt aag tac      528
   Asp Arg Glu Lys Ala Tyr Glu Thr Gln Glu Ile Asp Pro Phe Lys Tyr
                   165                 170                 175
   cag ttt ttg gaa aaa ccg aag ttt tac ggc gct gag ttg tac ttg cca      576
   Gln Phe Leu Glu Lys Pro Lys Phe Tyr Gly Ala Glu Leu Tyr Leu Pro
               180                 185                 190
   gat att gat gtg atc att att gga tct ggg gcc ggt gct ggt gtc gtg      624
   Asp Ile Asp Val Ile Ile Ile Gly Ser Gly Ala Gly Ala Gly Val Val
           195                 200                 205
   gcc cac act ttg acc aac gac ggc ttc aag agt ttg gtt ttg gaa aag      672
   Ala His Thr Leu Thr Asn Asp Gly Phe Lys Ser Leu Val Leu Glu Lys
       210                 215                 220
   ggc aga tac ttt agc aac tcc gag ttg aac ttt gat gac aag gac ggg      720
   Gly Arg Tyr Phe Ser Asn Ser Glu Leu Asn Phe Asp Asp Lys Asp Gly
   225                 230                 235                 240
   gtt caa gaa tta tac caa agt gga ggt act ttg acc acc gtc aac cag      768
   Val Gln Glu Leu Tyr Gln Ser Gly Gly Thr Leu Thr Thr Val Asn Gln
                   245                 250                 255
   cag ttg ttt gtt ctt gct ggt tcc act ttt ggt ggt ggt acc act gtc      816
   Gln Leu Phe Val Leu Ala Gly Ser Thr Phe Gly Gly Gly Thr Thr Val
               260                 265                 270
   aat tgg tcg gcc tgt ctt aaa acg cca ttc aag gtg cgt aag gaa tgg      864
   Asn Trp Ser Ala Cys Leu Lys Thr Pro Phe Lys Val Arg Lys Glu Trp
           275                 280                 285
   tat gat gag ttt ggc gtt gac ttt gct gcc gat gaa gcc tac gac aaa      912
   Tyr Asp Glu Phe Gly Val Asp Phe Ala Ala Asp Glu Ala Tyr Asp Lys
       290                 295                 300
   gca cag gat tat gtt tgg cag caa atg gga gct tct acc gaa ggc atc 960
   Ala Gln Asp Tyr Val Trp Gln Gln Met Gly Ala Ser Thr Glu Gly Ile
   305 310 315 320
   acc cac tct ttg gct aac gag att att att gaa ggt ggc aag aaa tta 1008
   Thr His Ser Leu Ala Asn Glu Ile Ile Ile Glu Gly Gly Lys Lys Leu
   325 330 335
   ggt tac aag gcc aag gta tta gac caa aac agc ggt ggt cat cct cat 1056
   Gly Tyr Lys Ala Lys Val Leu Asp Gln Asn Ser Gly Gly His Pro His
   340 345 350
   cac aga tgc ggt ttc tgt tat ttg ggt tgt aag cac ggt atc aag cag 1104
   His Arg Cys Gly Phe Cys Tyr Leu Gly Cys Lys His Gly Ile Lys Gln
   355 360 365
   ggc tct gtt aat aac tgg ttt aga gac gca gct gcc cac ggt tct cag 1152
   Gly Ser Val Asn Asn Trp Phe Arg Asp Ala Ala Ala His Gly Ser Gln
   370 375 380
   ttc atg caa cag gtt aga gtt ttg caa atc ctt aac aag aag ggc atc 1200
   Phe Met Gln Gln Val Arg Val Leu Gln Ile Leu Asn Lys Lys Gly Ile
   385 390 395 400
   gct tat ggt atc ttg tgt gag gat gtt gta acc ggt gcc aag ttc acc 1248
   Ala Tyr Gly Ile Leu Cys Glu Asp Val Val Thr Gly Ala Lys Phe Thr
   405 410 415
   att act ggc ccc aaa aag ttt gtt gtt gcc gcc ggc gcc tta aac act 1296
   Ile Thr Gly Pro Lys Lys Phe Val Val Ala Ala Gly Ala Leu Asn Thr
   420 425 430
   cca tct gtg ttg gtc aac tcc gga ttc aag aac aag aac atc ggt aag 1344
   Pro Ser Val Leu Val Asn Ser Gly Phe Lys Asn Lys Asn Ile Gly Lys
   435 440 445
   aac tta act ttg cat cca gtt tct gtc gtg ttt ggt gat ttt ggc aaa 1392
   Asn Leu Thr Leu His Pro Val Ser Val Val Phe Gly Asp Phe Gly Lys
   450 455 460
   gac gtt caa gca gat cac ttc cac aac tcc atc atg act gct ctt tgt 1440
   Asp Val Gln Ala Asp His Phe His Asn Ser Ile Met Thr Ala Leu Cys
   465 470 475 480
   tca gaa gcc gct gat tta gac ggc aag ggt cat gga tgc aga att gaa 1488
   Ser Glu Ala Ala Asp Leu Asp Gly Lys Gly His Gly Cys Arg Ile Glu
   485 490 495
   acc atc ttg aac gct cca ttc atc cag gct tca ttc tta cca tgg aga 1536
   Thr Ile Leu Asn Ala Pro Phe Ile Gln Ala Ser Phe Leu Pro Trp Arg
   500 505 510
   ggt agt aac gag gct aga cga gac ttg ttg cgt tac aac aac atg gtg 1584
   Gly Ser Asn Glu Ala Arg Arg Asp Leu Leu Arg Tyr Asn Asn Met Val
   515 520 525
   gcc atg tta ctt ctt agt cgt gat acc acc agt ggt tcc gtt tcg tcc 1632
   Ala Met Leu Leu Leu Ser Arg Asp Thr Thr Ser Gly Ser Val Ser Ser
   530 535 540
   cat cca act aaa cct gaa gca tta gtt gtc gag tac gac gtg aac aag 1680
   His Pro Thr Lys Pro Glu Ala Leu Val Val Glu Tyr Asp Val Asn Lys
   545 550 555 560
   ttt gac aga aac tcc atc ttg cag gca ttg ttg gtc act gct gac ttg      1728
   Phe Asp Arg Asn Ser Ile Leu Gln Ala Leu Leu Val Thr Ala Asp Leu
                   565                 570                 575
   ttg tac att caa ggt gcc aag aga atc ctt agt ccc caa cca tgg gtg      1776
   Leu Tyr Ile Gln Gly Ala Lys Arg Ile Leu Ser Pro Gln Pro Trp Val
               580                 585                 590
   cca att ttt gaa tcc gac aag cca aag gat aag aga tca atc aag gac      1824
   Pro Ile Phe Glu Ser Asp Lys Pro Lys Asp Lys Arg Ser Ile Lys Asp
           595                 600                 605
   gag gac tat gtc gaa tgg aga gcc aag gtt gcc aag att cct ttt gac      1872
   Glu Asp Tyr Val Glu Trp Arg Ala Lys Val Ala Lys Ile Pro Phe Asp
       610                 615                 620
   acc tac ggc tcg cct tat ggt tcg gcg cat caa atg tct tct tgt cgt      1920
   Thr Tyr Gly Ser Pro Tyr Gly Ser Ala His Gln Met Ser Ser Cys Arg
   625                 630                 635                 640
   atg tca ggt aag ggt cct aaa tac ggt gct gtt gat acc gat ggt aga      1968
   Met Ser Gly Lys Gly Pro Lys Tyr Gly Ala Val Asp Thr Asp Gly Arg
                   645                 650                 655
   ttg ttt gaa tgt tcg aat gtt tat gtt gct gac gct agt ctt ttg cca      2016
   Leu Phe Glu Cys Ser Asn Val Tyr Val Ala Asp Ala Ser Leu Leu Pro
               660                 665                 670
   act gct agc ggt gct aat cct atg gtc acc acc atg act ctt gca aga      2064
   Thr Ala Ser Gly Ala Asn Pro Met Val Thr Thr Met Thr Leu Ala Arg
           675                 680                 685
   cat gtt gcg tta ggt ttg gca gac tcc ttg aag acc aag gcc aag ttg      2112
   His Val Ala Leu Gly Leu Ala Asp Ser Leu Lys Thr Lys Ala Lys Leu
       690                 695                 700
   tag                                                                  2115
<210> 24
   <211> 704
   <212> PRT
   <213> Candida tropicalis
<400> 24
   Met Ala Pro Phe Leu Pro Asp Gln Val Asp Tyr Lys His Val Asp Thr
   1               5                   10                  15
   Leu Met Leu Leu Cys Asp Gly Ile Ile His Glu Thr Thr Val Asp Glu
               20                  25                  30
   Ile Lys Asp Val Ile Ala Pro Asp Phe Pro Ala Asp Lys Tyr Glu Glu
           35                  40                  45
   Tyr Val Arg Thr Phe Thr Lys Pro Ser Glu Thr Pro Gly Phe Arg Glu
       50                  55                  60
   Thr Val Tyr Asn Thr Val Asn Ala Asn Thr Met Asp Ala Ile His Gln
   65                  70                  75                  80
   Phe Ile Ile Leu Thr Asn Val Leu Gly Ser Arg Val Leu Ala Pro Ala
                   85                  90                  95
   Leu Thr Asn Ser Leu Thr Pro Ile Lys Asp Met Ser Leu Glu Asp Arg
               100                 105                 110
   Glu Lys Leu Leu Ala Ser Trp Arg Asp Ser Pro Ile Ala Ala Lys Arg
           115                 120                 125
   Lys Leu Phe Arg Leu Val Ser Thr Leu Thr Leu Val Thr Phe Thr Arg
       130                 135                 140
   Leu Ala Asn Glu Leu His Leu Lys Ala Ile His Tyr Pro Gly Arg Glu
   145                 150                 155                 160
   Asp Arg Glu Lys Ala Tyr Glu Thr Gln Glu Ile Asp Pro Phe Lys Tyr
                   165                 170                 175
   Gln Phe Leu Glu Lys Pro Lys Phe Tyr Gly Ala Glu Leu Tyr Leu Pro
               180                 185                 190
   Asp Ile Asp Val Ile Ile Ile Gly Ser Gly Ala Gly Ala Gly Val Val
           195                 200                 205
   Ala His Thr Leu Thr Asn Asp Gly Phe Lys Ser Leu Val Leu Glu Lys
       210                 215                 220
   Gly Arg Tyr Phe Ser Asn Ser Glu Leu Asn Phe Asp Asp Lys Asp Gly
   225                 230                 235                 240
   Val Gln Glu Leu Tyr Gln Ser Gly Gly Thr Leu Thr Thr Val Asn Gln
                   245                 250                 255
   Gln Leu Phe Val Leu Ala Gly Ser Thr Phe Gly Gly Gly Thr Thr Val
               260                 265                 270
   Asn Trp Ser Ala Cys Leu Lys Thr Pro Phe Lys Val Arg Lys Glu Trp
           275                 280                 285
   Tyr Asp Glu Phe Gly Val Asp Phe Ala Ala Asp Glu Ala Tyr Asp Lys
       290                 295                 300
   Ala Gln Asp Tyr Val Trp Gln Gln Met Gly Ala Ser Thr Glu Gly Ile
   305                 310                 315                 320
   Thr His Ser Leu Ala Asn Glu Ile Ile Ile Glu Gly Gly Lys Lys Leu
                   325                 330                 335
   Gly Tyr Lys Ala Lys Val Leu Asp Gln Asn Ser Gly Gly His Pro His
               340                 345                 350
   His Arg Cys Gly Phe Cys Tyr Leu Gly Cys Lys His Gly Ile Lys Gln
           355                 360                 365
   Gly Ser Val Asn Asn Trp Phe Arg Asp Ala Ala Ala His Gly Ser Gln
       370                 375                 380
   Phe Met Gln Gln Val Arg Val Leu Gln Ile Leu Asn Lys Lys Gly Ile
   385                 390                 395                 400
   Ala Tyr Gly Ile Leu Cys Glu Asp Val Val Thr Gly Ala Lys Phe Thr
                   405                 410                 415
   Ile Thr Gly Pro Lys Lys Phe Val Val Ala Ala Gly Ala Leu Asn Thr
               420                 425                 430
   Pro Ser Val Leu Val Asn Ser Gly Phe Lys Asn Lys Asn Ile Gly Lys
           435                 440                 445
   Asn Leu Thr Leu His Pro Val Ser Val Val Phe Gly Asp Phe Gly Lys
       450                 455                 460
   Asp Val Gln Ala Asp His Phe His Asn Ser Ile Met Thr Ala Leu Cys
   465                 470                 475                 480
   Ser Glu Ala Ala Asp Leu Asp Gly Lys Gly His Gly Cys Arg Ile Glu
                   485                 490                 495
   Thr Ile Leu Asn Ala Pro Phe Ile Gln Ala Ser Phe Leu Pro Trp Arg
               500                 505                 510
   Gly Ser Asn Glu Ala Arg Arg Asp Leu Leu Arg Tyr Asn Asn Met Val
           515                 520                 525
   Ala Met Leu Leu Leu Ser Arg Asp Thr Thr Ser Gly Ser Val Ser Ser
       530                 535                 540
   His Pro Thr Lys Pro Glu Ala Leu Val Val Glu Tyr Asp Val Asn Lys
   545                 550                 555                 560
   Phe Asp Arg Asn Ser Ile Leu Gln Ala Leu Leu Val Thr Ala Asp Leu
                   565                 570                 575
   Leu Tyr Ile Gln Gly Ala Lys Arg Ile Leu Ser Pro Gln Pro Trp Val
   580 585 590
   Pro Ile Phe Glu Ser Asp Lys Pro Lys Asp Lys Arg Ser Ile Lys Asp
   595 600 605
   Glu Asp Tyr Val Glu Trp Arg Ala Lys Val Ala Lys Ile Pro Phe Asp
   610 615 620
   Thr Tyr Gly Ser Pro Tyr Gly Ser Ala His Gln Met Ser Ser Cys Arg
   625 630 635 640
   Met Ser Gly Lys Gly Pro Lys Tyr Gly Ala Val Asp Thr Asp Gly Arg
   645 650 655
   Leu Phe Glu Cys Ser Asn Val Tyr Val Ala Asp Ala Ser Leu Leu Pro
   660 665 670
   Thr Ala Ser Gly Ala Asn Pro Met Val Thr Thr Met Thr Leu Ala Arg
   675 680 685
   His Val Ala Leu Gly Leu Ala Asp Ser Leu Lys Thr Lys Ala Lys Leu
   690 695 700
<210> 25
   <211> 2115
   <212> DNA
   <213> Candida tropicalis
<220>
   <221> CDS
   <222> (1)..(2115)
<400> 25
   atg aat acc ttc ttg cca gac gtg ctc gaa tac aaa cac gtc gac acc       48
   Met Asn Thr Phe Leu Pro Asp Val Leu Glu Tyr Lys His Val Asp Thr
   1               5                   10                  15
   ctt ttg tta ttg tgt gac ggg atc atc cac gaa acc aca gtc gat cag       96
   Leu Leu Leu Leu Cys Asp Gly Ile Ile His Glu Thr Thr Val Asp Gln
               20                  25                  30
   atc aag gac gcc att gct ccc gac ttc cct gag gac cag tac gag gag      144
   Ile Lys Asp Ala Ile Ala Pro Asp Phe Pro Glu Asp Gln Tyr Glu Glu
           35                  40                  45
   tat ctc aag acc ttc acc aag cca tct gag acc cct ggg ttc aga gaa      192
   Tyr Leu Lys Thr Phe Thr Lys Pro Ser Glu Thr Pro Gly Phe Arg Glu
       50                  55                  60
   gcc gtc tac gac acg atc aac gcc acc cca acc gat gcc gtg cac atg      240
   Ala Val Tyr Asp Thr Ile Asn Ala Thr Pro Thr Asp Ala Val His Met
   65                  70                  75                  80
   tgt att gtc ttg acc acc gca ttg gac tcc aga atc ttg gcc ccc acg        288
   Cys Ile Val Leu Thr Thr Ala Leu Asp Ser Arg Ile Leu Ala Pro Thr
                   85                  90                  95
   ttg acc aac tcg ttg acg cct atc aag gat atg acc ttg aag gag cgt        336
   Leu Thr Asn Ser Leu Thr Pro Ile Lys Asp Met Thr Leu Lys Glu Arg
               100                 105                 110
   gaa caa ttg ttg gcc tct tgg cgt gat tcc ccg att gcg gca aag aga        384
   Glu Gln Leu Leu Ala Ser Trp Arg Asp Ser Pro Ile Ala Ala Lys Arg
           115                 120                 125
   aga ttg ttc aga ttg att tcc tcg ctt acc ttg acg acg ttt acg aga        432
   Arg Leu Phe Arg Leu Ile Ser Ser Leu Thr Leu Thr Thr Phe Thr Arg
       130                 135                 140
   ttg gcc agc gaa ttg cac ttg aaa gcc atc cac tac cct ggc aga gac        480
   Leu Ala Ser Glu Leu His Leu Lys Ala Ile His Tyr Pro Gly Arg Asp
   145                 150                 155                 160
   ttg cgt gaa aag gcg tat gaa acc cag gtg gtt gac cct ttc agg tac        528
   Leu Arg Glu Lys Ala Tyr Glu Thr Gln Val Val Asp Pro Phe Arg Tyr
                   165                 170                 175
   ctg ttt atg gag aaa cca aag ttt gac ggc gcc gaa ttg tac ttg cca        576
   Leu Phe Met Glu Lys Pro Lys Phe Asp Gly Ala Glu Leu Tyr Leu Pro
               180                 185                 190
   gat atc gac gtc atc atc att gga tca ggc gcc ggt gct ggt gtc atg        624
   Asp Ile Asp Val Ile Ile Ile Gly Ser Gly Ala Gly Ala Gly Val Met
           195                 200                 205
   gcc cac act ctc gcc aac gac ggg ttc aag acc ttg gtt ttg gaa aag        672
   Ala His Thr Leu Ala Asn Asp Gly Phe Lys Thr Leu Val Leu Glu Lys
       210                 215                 220
   gga aag tat ttc agc aac tcc gag ttg aac ttt aat gac gct gat ggc        720
   Gly Lys Tyr Phe Ser Asn Ser Glu Leu Asn Phe Asn Asp Ala Asp Gly
   225                 230                 235                 240
   gtg aaa gag ttg tac caa ggt aaa ggt gct ttg gcc acc acc aat cag        768
   Val Lys Glu Leu Tyr Gln Gly Lys Gly Ala Leu Ala Thr Thr Asn Gln
                   245                 250                 255
   cag atg ttt att ctt gcc ggt tcc act ttg ggc ggt ggt acc act gtc        816
   Gln Met Phe Ile Leu Ala Gly Ser Thr Leu Gly Gly Gly Thr Thr Val
               260                 265                 270
   aac tgg tct gct tgc ctt aaa aca cca ttt aaa gtg cgt aag gag tgg        864
   Asn Trp Ser Ala Cys Leu Lys Thr Pro Phe Lys Val Arg Lys Glu Trp
           275                 280                 285
   tac gac gag ttt ggt ctt gaa ttt gct gcc gat gaa gcc tac gac aaa        912
   Tyr Asp Glu Phe Gly Leu Glu Phe Ala Ala Asp Glu Ala Tyr Asp Lys
       290                 295                 300
   gcg cag gat tat gtt tgg aaa caa atg ggt gct tca aca gat gga atc        960
   Ala Gln Asp Tyr Val Trp Lys Gln Met Gly Ala Ser Thr Asp Gly Ile
   305                 310                 315                 320
   act cac tcc ttg gcc aac gaa gtt gtg gtt gaa gga ggt aag aag ttg       1008
   Thr His Ser Leu Ala Asn Glu Val Val Val Glu Gly Gly Lys Lys Leu
                   325                 330                 335
   ggc tac aag agc aag gaa att gag cag aac aac ggt ggc cac cct gac      1056
   Gly Tyr Lys Ser Lys Glu Ile Glu Gln Asn Asn Gly Gly His Pro Asp
               340                 345                 350
   cac cca tgt ggt ttc tgt tac ttg ggc tgt aag tac ggt att aaa cag      1104
   His Pro Cys Gly Phe Cys Tyr Leu Gly Cys Lys Tyr Gly Ile Lys Gln
           355                 360                 365
   ggt tct gtg aat aac tgg ttt aga gac gca gct gcc cac ggg tcc aag      1152
   Gly Ser Val Asn Asn Trp Phe Arg Asp Ala Ala Ala His Gly Ser Lys
       370                 375                 380
   ttc atg caa caa gtc aga gtt gtg caa atc ctc aac aag aat ggc gtc      1200
   Phe Met Gln Gln Val Arg Val Val Gln Ile Leu Asn Lys Asn Gly Val
   385                 390                 395                 400
   gct tat ggt atc ttg tgt gag gat gtc gaa acc gga gtc agg ttc act      1248
   Ala Tyr Gly Ile Leu Cys Glu Asp Val Glu Thr Gly Val Arg Phe Thr
                   405                 410                 415
   att agt ggc ccc aaa aag ttt gtt gtt tct gct ggt tct ttg aac acg      1296
   Ile Ser Gly Pro Lys Lys Phe Val Val Ser Ala Gly Ser Leu Asn Thr
               420                 425                 430
   cca act gtg ttg acc aac tcc gga ttc aag aac aag cac att ggt aag      1344
   Pro Thr Val Leu Thr Asn Ser Gly Phe Lys Asn Lys His Ile Gly Lys
           435                 440                 445
   aac ttg acg ttg cac cca gtt tcc acc gtg ttt ggt gac ttt ggc aga      1392
   Asn Leu Thr Leu His Pro Val Ser Thr Val Phe Gly Asp Phe Gly Arg
       450                 455                 460
   gac gtg caa gcc gac cat ttc cac aaa tct att atg act tcg ctt tgt      1440
   Asp Val Gln Ala Asp His Phe His Lys Ser Ile Met Thr Ser Leu Cys
   465                 470                 475                 480
   tac gag gtt gct gac ttg gac ggc aag ggc cac gga tgc aga atc gaa      1488
   Tyr Glu Val Ala Asp Leu Asp Gly Lys Gly His Gly Cys Arg Ile Glu
                   485                 490                 495
   acc atc ttg aac gct cca ttc atc caa gct tct ttg ttg cca tgg aga      1536
   Thr Ile Leu Asn Ala Pro Phe Ile Gln Ala Ser Leu Leu Pro Trp Arg
               500                 505                 510
   gga agt gac gag gtc aga aga gac ttg ttg cgt tac aac aac atg gtg      1584
   Gly Ser Asp Glu Val Arg Arg Asp Leu Leu Arg Tyr Asn Asn Met Val
           515                 520                 525
   gcc atg ttg ctt atc acg cgt gat acc acc agt ggt tca gtt tct gct      1632
   Ala Met Leu Leu Ile Thr Arg Asp Thr Thr Ser Gly Ser Val Ser Ala
       530                 535                 540
   gac cca aag aag ccc gac gct ttg att gtc gac tat gag att aac aag      1680
   Asp Pro Lys Lys Pro Asp Ala Leu Ile Val Asp Tyr Glu Ile Asn Lys
   545                 550                 555                 560
   ttt gac aag aat gcc atc ttg caa gct ttc ttg atc act tcc gac atg      1728
   Phe Asp Lys Asn Ala Ile Leu Gln Ala Phe Leu Ile Thr Ser Asp Met
                   565                 570                 575
   ttg tac att gaa ggt gcc aag aga atc ctc agt cca cag cca tgg gtg      1776
   Leu Tyr Ile Glu Gly Ala Lys Arg Ile Leu Ser Pro Gln Pro Trp Val
               580                 585                 590
   cca atc ttt gag tcg aac aag cca aag gag caa aga acg atc aag gac      1824
   Pro Ile Phe Glu Ser Asn Lys Pro Lys Glu Gln Arg Thr Ile Lys Asp
           595                 600                 605
   aag gac tat gtt gag tgg aga gcc aag gct gct aag ata cct ttc gac      1872
   Lys Asp Tyr Val Glu Trp Arg Ala Lys Ala Ala Lys Ile Pro Phe Asp
       610                 615                 620
   acc tac ggt tct gca tat ggg tcc gca cat caa atg tcc acc tgt cgt      1920
   Thr Tyr Gly Ser Ala Tyr Gly Ser Ala His Gln Met Ser Thr Cys Arg
   625                 630                 635                 640
   atg tcc gga aag ggt cct aaa tac ggt gct gtt gat act gat ggt aga      1968
   Met Ser Gly Lys Gly Pro Lys Tyr Gly Ala Val Asp Thr Asp Gly Arg
                   645                 650                 655
   ttg ttt gaa tgt tcg aat gtc tat gtt gct gat gct agt gtt ttg cct      2016
   Leu Phe Glu Cys Ser Asn Val Tyr Val Ala Asp Ala Ser Val Leu Pro
               660                 665                 670
   act gcc agc ggt gcc aac cca atg ata tcc acc atg acc ttt gct aga      2064
   Thr Ala Ser Gly Ala Asn Pro Met Ile Ser Thr Met Thr Phe Ala Arg
           675                 680                 685
   cag att gcg tta ggt ttg gct gac tcc ttg aag acc aaa ccc aag ttg      2112
   Gln Ile Ala Leu Gly Leu Ala Asp Ser Leu Lys Thr Lys Pro Lys Leu
       690                 695                 700
   tag                                                                  2115
<210> 26
   <211> 704
   <212> PRT
   <213> Candida tropicalis
<400> 26
   Met Asn Thr Phe Leu Pro Asp Val Leu Glu Tyr Lys His Val Asp Thr
   1               5                   10                  15
   Leu Leu Leu Leu Cys Asp Gly Ile Ile His Glu Thr Thr Val Asp Gln
               20                  25                  30
   Ile Lys Asp Ala Ile Ala Pro Asp Phe Pro Glu Asp Gln Tyr Glu Glu
           35                  40                  45
   Tyr Leu Lys Thr Phe Thr Lys Pro Ser Glu Thr Pro Gly Phe Arg Glu
       50                  55                  60
   Ala Val Tyr Asp Thr Ile Asn Ala Thr Pro Thr Asp Ala Val His Met
   65                  70                  75                  80
   Cys Ile Val Leu Thr Thr Ala Leu Asp Ser Arg Ile Leu Ala Pro Thr
   85 90 95
   Leu Thr Asn Ser Leu Thr Pro Ile Lys Asp Met Thr Leu Lys Glu Arg
   100 105 110
   Glu Gln Leu Leu Ala Ser Trp Arg Asp Ser Pro Ile Ala Ala Lys Arg
   115 120 125
   Arg Leu Phe Arg Leu Ile Ser Ser Leu Thr Leu Thr Thr Phe Thr Arg
   130 135 140
   Leu Ala Ser Glu Leu His Leu Lys Ala Ile His Tyr Pro Gly Arg Asp
   145 150 155 160
   Leu Arg Glu Lys Ala Tyr Glu Thr Gln Val Val Asp Pro Phe Arg Tyr
   165 170 175
   Leu Phe Met Glu Lys Pro Lys Phe Asp Gly Ala Glu Leu Tyr Leu Pro
   180 185 190
   Asp Ile Asp Val Ile Ile Ile Gly Ser Gly Ala Gly Ala Gly Val Met
   195 200 205
   Ala His Thr Leu Ala Asn Asp Gly Phe Lys Thr Leu Val Leu Glu Lys
   210 215 220
   Gly Lys Tyr Phe Ser Asn Ser Glu Leu Asn Phe Asn Asp Ala Asp Gly
   225 230 235 240
   Val Lys Glu Leu Tyr Gln Gly Lys Gly Ala Leu Ala Thr Thr Asn Gln
   245 250 255
   Gln Met Phe Ile Leu Ala Gly Ser Thr Leu Gly Gly Gly Thr Thr Val
   260 265 270
   Asn Trp Ser Ala Cys Leu Lys Thr Pro Phe Lys Val Arg Lys Glu Trp
   275 280 285
   Tyr Asp Glu Phe Gly Leu Glu Phe Ala Ala Asp Glu Ala Tyr Asp Lys
   290 295 300
   Ala Gln Asp Tyr Val Trp Lys Gln Met Gly Ala Ser Thr Asp Gly Ile
   305 310 315 320
   Thr His Ser Leu Ala Asn Glu Val Val Val Glu Gly Gly Lys Lys Leu
   325 330 335
   Gly Tyr Lys Ser Lys Glu Ile Glu Gln Asn Asn Gly Gly His Pro Asp
   340 345 350
   His Pro Cys Gly Phe Cys Tyr Leu Gly Cys Lys Tyr Gly Ile Lys Gln
   355 360 365
   Gly Ser Val Asn Asn Trp Phe Arg Asp Ala Ala Ala His Gly Ser Lys
   370 375 380
   Phe Met Gln Gln Val Arg Val Val Gln Ile Leu Asn Lys Asn Gly Val
   385 390 395 400
   Ala Tyr Gly Ile Leu Cys Glu Asp Val Glu Thr Gly Val Arg Phe Thr
   405 410 415
   Ile Ser Gly Pro Lys Lys Phe Val Val Ser Ala Gly Ser Leu Asn Thr
   420 425 430
   Pro Thr Val Leu Thr Asn Ser Gly Phe Lys Asn Lys His Ile Gly Lys
   435 440 445
   Asn Leu Thr Leu His Pro Val Ser Thr Val Phe Gly Asp Phe Gly Arg
   450 455 460
   Asp Val Gln Ala Asp His Phe His Lys Ser Ile Met Thr Ser Leu Cys
   465 470 475 480
   Tyr Glu Val Ala Asp Leu Asp Gly Lys Gly His Gly Cys Arg Ile Glu
   485 490 495
   Thr Ile Leu Asn Ala Pro Phe Ile Gln Ala Ser Leu Leu Pro Trp Arg
   500 505 510
   Gly Ser Asp Glu Val Arg Arg Asp Leu Leu Arg Tyr Asn Asn Met Val
   515 520 525
   Ala Met Leu Leu Ile Thr Arg Asp Thr Thr Ser Gly Ser Val Ser Ala
   530 535 540
   Asp Pro Lys Lys Pro Asp Ala Leu Ile Val Asp Tyr Glu Ile Asn Lys
   545 550 555 560
   Phe Asp Lys Asn Ala Ile Leu Gln Ala Phe Leu Ile Thr Ser Asp Met
   565 570 575
   Leu Tyr Ile Glu Gly Ala Lys Arg Ile Leu Ser Pro Gln Pro Trp Val
   580 585 590
   Pro Ile Phe Glu Ser Asn Lys Pro Lys Glu Gln Arg Thr Ile Lys Asp
           595                 600                 605
   Lys Asp Tyr Val Glu Trp Arg Ala Lys Ala Ala Lys Ile Pro Phe Asp
       610                 615                 620
   Thr Tyr Gly Ser Ala Tyr Gly Ser Ala His Gln Met Ser Thr Cys Arg
   625                 630                 635                 640
   Met Ser Gly Lys Gly Pro Lys Tyr Gly Ala Val Asp Thr Asp Gly Arg
                   645                 650                 655
   Leu Phe Glu Cys Ser Asn Val Tyr Val Ala Asp Ala Ser Val Leu Pro
               660                 665                 670
   Thr Ala Ser Gly Ala Asn Pro Met Ile Ser Thr Met Thr Phe Ala Arg
           675                 680                 685
   Gln Ile Ala Leu Gly Leu Ala Asp Ser Leu Lys Thr Lys Pro Lys Leu
       690                 695                 700
<210> 27
   <211> 2115
   <212> DNA
   <213> Candida tropicalis
<220>
   <221> CDS
   <222> (1)..(2115)
<400> 27
   atg aat acc ttc ttg cca gac gtg ctc gaa tac aaa cac gtc gat acc      48
   Met Asn Thr Phe Leu Pro Asp Val Leu Glu Tyr Lys His Val Asp Thr
   1               5                   10                  15
   ctt ttg tta tta tgt gac ggg atc atc cac gaa acc aca gtc gac cag      96
   Leu Leu Leu Leu Cys Asp Gly Ile Ile His Glu Thr Thr Val Asp Gln
               20                  25                  30
   atc agg gac gcc att gct ccc gac ttc cct gaa gac cag tac gag gag     144
   Ile Arg Asp Ala Ile Ala Pro Asp Phe Pro Glu Asp Gln Tyr Glu Glu
           35                  40                  45
   tat ctc aag acc ttc acc aag cca tct gag acc cct ggg ttc aga gaa     192
   Tyr Leu Lys Thr Phe Thr Lys Pro Ser Glu Thr Pro Gly Phe Arg Glu
       50                  55                  60
   gcc gtc tac gac acg atc aac agc acc cca acc gag gct gtg cac atg     240
   Ala Val Tyr Asp Thr Ile Asn Ser Thr Pro Thr Glu Ala Val His Met
   65                  70                  75                  80
   tgt att gta ttg acc acc gca ttg gac tcg aga atc ttg gcc ccc acg     288
   Cys Ile Val Leu Thr Thr Ala Leu Asp Ser Arg Ile Leu Ala Pro Thr
                   85                  90                  95
   ttg acc aac tcg ttg acg cct atc aag gat atg acc ttg aaa gag cgt       336
   Leu Thr Asn Ser Leu Thr Pro Ile Lys Asp Met Thr Leu Lys Glu Arg
               100                 105                 110
   gaa caa ttg ttg gct gcc tgg cgt gat tcc ccg atc gcg gcc aag aga       384
   Glu Gln Leu Leu Ala Ala Trp Arg Asp Ser Pro Ile Ala Ala Lys Arg
           115                 120                 125
   aga ttg ttc aga ttg att tcc tca ctt acc ttg acg acc ttt acg aga       432
   Arg Leu Phe Arg Leu Ile Ser Ser Leu Thr Leu Thr Thr Phe Thr Arg
       130                 135                 140
   ttg gcc agc gac ttg cac ttg aga gcc atc cac tac cct ggc aga gac       480
   Leu Ala Ser Asp Leu His Leu Arg Ala Ile His Tyr Pro Gly Arg Asp
   145                 150                 155                 160
   ttg cgt gaa aag gca tat gaa acc cag gtg gtt gac cct ttc agg tac       528
   Leu Arg Glu Lys Ala Tyr Glu Thr Gln Val Val Asp Pro Phe Arg Tyr
                   165                 170                 175
   ctg ttt atg gaa aaa cca aag ttt gac ggc acc gag ttg tac ttg cca       576
   Leu Phe Met Glu Lys Pro Lys Phe Asp Gly Thr Glu Leu Tyr Leu Pro
               180                 185                 190
   gat atc gac gtc atc atc att gga tcc ggt gcc ggt gct ggt gtc atg       624
   Asp Ile Asp Val Ile Ile Ile Gly Ser Gly Ala Gly Ala Gly Val Met
           195                 200                 205
   gcc cac act tta gcc aac gac ggg tac aag acc ttg gtt ttg gaa aag       672
   Ala His Thr Leu Ala Asn Asp Gly Tyr Lys Thr Leu Val Leu Glu Lys
       210                 215                 220
   gga aag tat ttc agc aac tcc gag ttg aac ttt aat gat gcc gat ggt       720
   Gly Lys Tyr Phe Ser Asn Ser Glu Leu Asn Phe Asn Asp Ala Asp Gly
   225                 230                 235                 240
   atg aaa gag ttg tac caa ggt aaa tgt gcg ttg acc acc acg aac cag       768
   Met Lys Glu Leu Tyr Gln Gly Lys Cys Ala Leu Thr Thr Thr Asn Gln
                   245                 250                 255
   cag atg ttt att ctt gcc ggt tcc act ttg ggc ggt ggt acc act gtt       816
   Gln Met Phe Ile Leu Ala Gly Ser Thr Leu Gly Gly Gly Thr Thr Val
               260                 265                 270
   aac tgg tct gct tgt ctt aaa aca cca ttt aaa gtg cgt aag gag tgg       864
   Asn Trp Ser Ala Cys Leu Lys Thr Pro Phe Lys Val Arg Lys Glu Trp
           275                 280                 285
   tac gac gag ttt ggt ctt gaa ttt gct gcc gac gaa gcc tac gac aaa       912
   Tyr Asp Glu Phe Gly Leu Glu Phe Ala Ala Asp Glu Ala Tyr Asp Lys
       290                 295                 300
   gca caa gac tat gtt tgg aaa caa atg ggc gct tct acc gaa gga atc       960
   Ala Gln Asp Tyr Val Trp Lys Gln Met Gly Ala Ser Thr Glu Gly Ile
   305                 310                 315                 320
   act cac tct ttg gcg aac gcg gtt gtg gtt gaa gga ggt aag aag ttg      1008
   Thr His Ser Leu Ala Asn Ala Val Val Val Glu Gly Gly Lys Lys Leu
                   325                 330                 335
   ggt tac aag agc aag gaa atc gag cag aac aat ggt ggc cat cct gac      1056
   Gly Tyr Lys Ser Lys Glu Ile Glu Gln Asn Asn Gly Gly His Pro Asp
               340                 345                 350
   cac ccc tgt ggt ttc tgt tac ttg ggc tgt aag tac ggt att aag cag      1104
   His Pro Cys Gly Phe Cys Tyr Leu Gly Cys Lys Tyr Gly Ile Lys Gln
           355                 360                 365
   ggt tct gtg aat aac tgg ttt aga gac gca gct gcc cac ggg tcc aag      1152
   Gly Ser Val Asn Asn Trp Phe Arg Asp Ala Ala Ala His Gly Ser Lys
       370                 375                 380
   ttc atg caa caa gtc aga gtt gtg caa atc ctc cac aat aaa ggc gtc      1200
   Phe Met Gln Gln Val Arg Val Val Gln Ile Leu His Asn Lys Gly Val
   385                 390                 395                 400
   gct tat ggc atc ttg tgt gag gat gtc gag acc gga gtc aaa ttc act      1248
   Ala Tyr Gly Ile Leu Cys Glu Asp Val Glu Thr Gly Val Lys Phe Thr
                   405                 410                 415
   atc agt ggc ccc aaa aag ttt gtt gtt tct gca ggt tct ttg aac acg      1296
   Ile Ser Gly Pro Lys Lys Phe Val Val Ser Ala Gly Ser Leu Asn Thr
               420                 425                 430
   cca acg gtg ttg acc aac tcc gga ttc aag aac aaa cac atc ggt aag      1344
   Pro Thr Val Leu Thr Asn Ser Gly Phe Lys Asn Lys His Ile Gly Lys
           435                 440                 445
   aac ttg acg ttg cac cca gtt tcg acc gtg ttt ggt gac ttt ggc aga      1392
   Asn Leu Thr Leu His Pro Val Ser Thr Val Phe Gly Asp Phe Gly Arg
       450                 455                 460
   gac gtg caa gcc gac cat ttc cac aaa tct att atg act tcg ctc tgt      1440
   Asp Val Gln Ala Asp His Phe His Lys Ser Ile Met Thr Ser Leu Cys
   465                 470                 475                 480
   tac gaa gtc gct gac ttg gac ggc aag ggc cac gga tgc aga atc gag      1488
   Tyr Glu Val Ala Asp Leu Asp Gly Lys Gly His Gly Cys Arg Ile Glu
                   485                 490                 495
   acc atc ttg aac gct cca ttc atc caa gct tct ttg ttg cca tgg aga      1536
   Thr Ile Leu Asn Ala Pro Phe Ile Gln Ala Ser Leu Leu Pro Trp Arg
               500                 505                 510
   gga agc gac gag gtc aga aga gac ttg ttg cgt tac aac aac atg gtg      1584
   Gly Ser Asp Glu Val Arg Arg Asp Leu Leu Arg Tyr Asn Asn Met Val
           515                 520                 525
   gcc atg ttg ctt atc acc cgt gac acc acc agt ggt tca gtt tct gct      1632
   Ala Met Leu Leu Ile Thr Arg Asp Thr Thr Ser Gly Ser Val Ser Ala
       530                 535                 540
   gac cca aag aag ccc gac gct ttg att gtc gac tat gac atc aac aag      1680
   Asp Pro Lys Lys Pro Asp Ala Leu Ile Val Asp Tyr Asp Ile Asn Lys
   545                 550                 555                 560
   ttt gac aag aat gcc atc ttg caa gct ttc ttg atc acc tcc gac atg      1728
   Phe Asp Lys Asn Ala Ile Leu Gln Ala Phe Leu Ile Thr Ser Asp Met
                   565                 570                 575
   ttg tac atc gaa ggt gcc aag aga atc ctc agt cca cag gca tgg gtg      1776
   Leu Tyr Ile Glu Gly Ala Lys Arg Ile Leu Ser Pro Gln Ala Trp Val
               580                 585                 590
   cca atc ttt gag tcg aac aag cca aag gag caa aga aca atc aag gac      1824
   Pro Ile Phe Glu Ser Asn Lys Pro Lys Glu Gln Arg Thr Ile Lys Asp
           595                 600                 605
   aag gac tat gtc gaa tgg aga gcc aag gct gcc aag ata cct ttc gac      1872
   Lys Asp Tyr Val Glu Trp Arg Ala Lys Ala Ala Lys Ile Pro Phe Asp
       610                 615                 620
   acc tac ggt tct gcc tat ggg tcc gca cat caa atg tcc acc tgt cgt      1920
   Thr Tyr Gly Ser Ala Tyr Gly Ser Ala His Gln Met Ser Thr Cys Arg
   625                 630                 635                 640
   atg tcc gga aag ggt cct aaa tac ggc gcc gtt gat acc gat ggt aga      1968
   Met Ser Gly Lys Gly Pro Lys Tyr Gly Ala Val Asp Thr Asp Gly Arg
                   645                 650                 655
   ttg ttt gaa tgt tcg aat gtc tat gtt gct gat gct agt gtt ttg cct      2016
   Leu Phe Glu Cys Ser Asn Val Tyr Val Ala Asp Ala Ser Val Leu Pro
               660                 665                 670
   act gcc agc ggt gcc aac cca atg atc tcc acc atg acg ttt gct aga      2064
   Thr Ala Ser Gly Ala Asn Pro Met Ile Ser Thr Met Thr Phe Ala Arg
           675                 680                 685
   cag att gcg tta ggt ttg gct gac tct ttg aag acc aaa ccc aag ttg      2112
   Gln Ile Ala Leu Gly Leu Ala Asp Ser Leu Lys Thr Lys Pro Lys Leu
       690                 695                 700
   tag                                                                  2115
<210> 28
   <211> 704
   <212> PRT
   <213> Candida tropicalis
<400> 28
   Met Asn Thr Phe Leu Pro Asp Val Leu Glu Tyr Lys His Val Asp Thr
   1               5                   10                  15
   Leu Leu Leu Leu Cys Asp Gly Ile Ile His Glu Thr Thr Val Asp Gln
               20                  25                  30
   Ile Arg Asp Ala Ile Ala Pro Asp Phe Pro Glu Asp Gln Tyr Glu Glu
           35                  40                  45
   Tyr Leu Lys Thr Phe Thr Lys Pro Ser Glu Thr Pro Gly Phe Arg Glu
       50                  55                  60
   Ala Val Tyr Asp Thr Ile Asn Ser Thr Pro Thr Glu Ala Val His Met
   65                  70                  75                  80
   Cys Ile Val Leu Thr Thr Ala Leu Asp Ser Arg Ile Leu Ala Pro Thr
                   85                  90                  95
   Leu Thr Asn Ser Leu Thr Pro Ile Lys Asp Met Thr Leu Lys Glu Arg
               100                 105                 110
   Glu Gln Leu Leu Ala Ala Trp Arg Asp Ser Pro Ile Ala Ala Lys Arg
   115 120 125
   Arg Leu Phe Arg Leu Ile Ser Ser Leu Thr Leu Thr Thr Phe Thr Arg
   130 135 140
   Leu Ala Ser Asp Leu His Leu Arg Ala Ile His Tyr Pro Gly Arg Asp
   145 150 155 160
   Leu Arg Glu Lys Ala Tyr Glu Thr Gln Val Val Asp Pro Phe Arg Tyr
   165 170 175
   Leu Phe Met Glu Lys Pro Lys Phe Asp Gly Thr Glu Leu Tyr Leu Pro
   180 185 190
   Asp Ile Asp Val Ile Ile Ile Gly Ser Gly Ala Gly Ala Gly Val Met
   195 200 205
   Ala His Thr Leu Ala Asn Asp Gly Tyr Lys Thr Leu Val Leu Glu Lys
   210 215 220
   Gly Lys Tyr Phe Ser Asn Ser Glu Leu Asn Phe Asn Asp Ala Asp Gly
   225 230 235 240
   Met Lys Glu Leu Tyr Gln Gly Lys Cys Ala Leu Thr Thr Thr Asn Gln
   245 250 255
   Gln Met Phe Ile Leu Ala Gly Ser Thr Leu Gly Gly Gly Thr Thr Val
   260 265 270
   Asn Trp Ser Ala Cys Leu Lys Thr Pro Phe Lys Val Arg Lys Glu Trp
   275 280 285
   Tyr Asp Glu Phe Gly Leu Glu Phe Ala Ala Asp Glu Ala Tyr Asp Lys
   290 295 300
   Ala Gln Asp Tyr Val Trp Lys Gln Met Gly Ala Ser Thr Glu Gly Ile
   305 310 315 320
   Thr His Ser Leu Ala Asn Ala Val Val Val Glu Gly Gly Lys Lys Leu
   325 330 335
   Gly Tyr Lys Ser Lys Glu Ile Glu Gln Asn Asn Gly Gly His Pro Asp
   340 345 350
   His Pro Cys Gly Phe Cys Tyr Leu Gly Cys Lys Tyr Gly Ile Lys Gln
   355 360 365
   Gly Ser Val Asn Asn Trp Phe Arg Asp Ala Ala Ala His Gly Ser Lys
   370 375 380
   Phe Met Gln Gln Val Arg Val Val Gln Ile Leu His Asn Lys Gly Val
   385 390 395 400
   Ala Tyr Gly Ile Leu Cys Glu Asp Val Glu Thr Gly Val Lys Phe Thr
   405 410 415
   Ile Ser Gly Pro Lys Lys Phe Val Val Ser Ala Gly Ser Leu Asn Thr
   420 425 430
   Pro Thr Val Leu Thr Asn Ser Gly Phe Lys Asn Lys His Ile Gly Lys
   435 440 445
   Asn Leu Thr Leu His Pro Val Ser Thr Val Phe Gly Asp Phe Gly Arg
   450 455 460
   Asp Val Gln Ala Asp His Phe His Lys Ser Ile Met Thr Ser Leu Cys
   465 470 475 480
   Tyr Glu Val Ala Asp Leu Asp Gly Lys Gly His Gly Cys Arg Ile Glu
   485 490 495
   Thr Ile Leu Asn Ala Pro Phe Ile Gln Ala Ser Leu Leu Pro Trp Arg
   500 505 510
   Gly Ser Asp Glu Val Arg Arg Asp Leu Leu Arg Tyr Asn Asn Met Val
   515 520 525
   Ala Met Leu Leu Ile Thr Arg Asp Thr Thr Ser Gly Ser Val Ser Ala
   530 535 540
   Asp Pro Lys Lys Pro Asp Ala Leu Ile Val Asp Tyr Asp Ile Asn Lys
   545 550 555 560
   Phe Asp Lys Asn Ala Ile Leu Gln Ala Phe Leu Ile Thr Ser Asp Met
   565 570 575
   Leu Tyr Ile Glu Gly Ala Lys Arg Ile Leu Ser Pro Gln Ala Trp Val
   580 585 590
   Pro Ile Phe Glu Ser Asn Lys Pro Lys Glu Gln Arg Thr Ile Lys Asp
   595 600 605
   Lys Asp Tyr Val Glu Trp Arg Ala Lys Ala Ala Lys Ile Pro Phe Asp
   610 615 620
   Thr Tyr Gly Ser Ala Tyr Gly Ser Ala His Gln Met Ser Thr Cys Arg
   625 630 635 640
   Met Ser Gly Lys Gly Pro Lys Tyr Gly Ala Val Asp Thr Asp Gly Arg
   645 650 655
   Leu Phe Glu Cys Ser Asn Val Tyr Val Ala Asp Ala Ser Val Leu Pro
   660 665 670
   Thr Ala Ser Gly Ala Asn Pro Met Ile Ser Thr Met Thr Phe Ala Arg
   675 680 685
   Gln Ile Ala Leu Gly Leu Ala Asp Ser Leu Lys Thr Lys Pro Lys Leu
   690 695 700
<210> 29
   <211> 1446
   <212> DNA
   <213> Candida tropicalis
<220>
   <221> CDS
   <222> (1)..(1446)
<400> 29
   atg ata atg ttt gat atc gtc aaa tac acc tta att ggg tta ttt agc       48
   Met Ile Met Phe Asp Ile Val Lys Tyr Thr Leu Ile Gly Leu Phe Ser
   1               5                   10                  15
   tat ttg cta tat gta att ctc gat ata gta cta cca cca ttc aat ttt       96
   Tyr Leu Leu Tyr Val Ile Leu Asp Ile Val Leu Pro Pro Phe Asn Phe
               20                  25                  30
   ccc aaa aat atc cca aca atc cca ttc tat gtt tcc ttc tta ggt gct      144
   Pro Lys Asn Ile Pro Thr Ile Pro Phe Tyr Val Ser Phe Leu Gly Ala
           35                  40                  45
   tac act aac ttg gat cag agg gat att tac aat ttg tat ttg aga gag      192
   Tyr Thr Asn Leu Asp Gln Arg Asp Ile Tyr Asn Leu Tyr Leu Arg Glu
       50                  55                  60
   aag ttg gaa aag tac ggg gca gta aag ata tat ttt gct tca aga tgg      240
   Lys Leu Glu Lys Tyr Gly Ala Val Lys Ile Tyr Phe Ala Ser Arg Trp
   65                  70                  75                  80
   aac ata ctc att act agg cca gaa tat ctt ctt gaa atg ttt aga aat      288
   Asn Ile Leu Ile Thr Arg Pro Glu Tyr Leu Leu Glu Met Phe Arg Asn
                   85                  90                  95
   gaa gat gtg tac tca aaa cgg gga aac cac cta aaa atc cct ggt tca      336
   Glu Asp Val Tyr Ser Lys Arg Gly Asn His Leu Lys Ile Pro Gly Ser
               100                 105                 110
   gtg atg gct aca tac act ggt gat aat atc att agt gct cat gga gaa      384
   Val Met Ala Thr Tyr Thr Gly Asp Asn Ile Ile Ser Ala His Gly Glu
           115                 120                 125
   tta tgg aaa tta tat cga gaa gtt att gcc aaa agt att caa ttt ccc      432
   Leu Trp Lys Leu Tyr Arg Glu Val Ile Ala Lys Ser Ile Gln Phe Pro
       130                 135                 140
   gat ttt gaa cca att acg aaa aat act aaa tca tta ctt gaa att att      480
   Asp Phe Glu Pro Ile Thr Lys Asn Thr Lys Ser Leu Leu Glu Ile Ile
   145                 150                 155                 160
   gat ggt atg att gac agt gat aaa aat cat gca att att cca atc aca      528
   Asp Gly Met Ile Asp Ser Asp Lys Asn His Ala Ile Ile Pro Ile Thr
                   165                 170                 175
   gat tta ttt caa aaa tat tca tta gca aac gtt aca gaa tct ata ctt      576
   Asp Leu Phe Gln Lys Tyr Ser Leu Ala Asn Val Thr Glu Ser Ile Leu
               180                 185                 190
   gga gta aat ttt aag gtt ctt gaa ggc gat caa tca atc atg cat caa      624
   Gly Val Asn Phe Lys Val Leu Glu Gly Asp Gln Ser Ile Met His Gln
           195                 200                 205
   aaa ata aag tac gtc aag ctg caa ata ttc aaa cct ttt ttc ttg aac      672
   Lys Ile Lys Tyr Val Lys Leu Gln Ile Phe Lys Pro Phe Phe Leu Asn
       210                 215                 220
   ttt cct tat ttt gat agc ttt cct att cca agt agg tta caa gca aga      720
   Phe Pro Tyr Phe Asp Ser Phe Pro Ile Pro Ser Arg Leu Gln Ala Arg
   225                 230                 235                 240
   aag gaa gtt att aat ttt agg aat tgg tat ggt caa agt att att gac      768
   Lys Glu Val Ile Asn Phe Arg Asn Trp Tyr Gly Gln Ser Ile Ile Asp
                   245                 250                 255
   aag cat gat cca caa ctt cct aat agt gca gct aca aaa tta gtt gat      816
   Lys His Asp Pro Gln Leu Pro Asn Ser Ala Ala Thr Lys Leu Val Asp
               260                 265                 270
   ggc ttg atg caa gaa aaa ctc act gaa aaa caa ttt ttg gat aat gcc      864
   Gly Leu Met Gln Glu Lys Leu Thr Glu Lys Gln Phe Leu Asp Asn Ala
           275                 280                 285
   att att gtg atg att gct gga cat gaa aat cca ctt ttg tta atg tta      912
   Ile Ile Val Met Ile Ala Gly His Glu Asn Pro Leu Leu Leu Met Leu
       290                 295                 300
   tcg ttg atg ttt gtt gct gca aaa tat cca aaa gtt cag gag gcc ata      960
   Ser Leu Met Phe Val Ala Ala Lys Tyr Pro Lys Val Gln Glu Ala Ile
   305                 310                 315                 320
   cgt tca gaa ata gat cca aca aaa cct tat cta cac tct gtt att tat     1008
   Arg Ser Glu Ile Asp Pro Thr Lys Pro Tyr Leu His Ser Val Ile Tyr
                   325                 330                 335
   gaa act tta aga atg tat cca cca ttg gga tta atc att aat cgt tat     1056
   Glu Thr Leu Arg Met Tyr Pro Pro Leu Gly Leu Ile Ile Asn Arg Tyr
               340                 345                 350
   acc acc aga cct act aaa cta ggc aac ata gta att ccc aaa ggt gtt     1104
   Thr Thr Arg Pro Thr Lys Leu Gly Asn Ile Val Ile Pro Lys Gly Val
           355                 360                 365
   tac tgt ggc tat aat aat ttt ggt acg ggt aga gac aga aat gtt tgg      1152
   Tyr Cys Gly Tyr Asn Asn Phe Gly Thr Gly Arg Asp Arg Asn Val Trp
       370                 375                 380
   gga cca gac tcg gat gag ttt aaa cca gag aga tgg gga agg gat aat      1200
   Gly Pro Asp Ser Asp Glu Phe Lys Pro Glu Arg Trp Gly Arg Asp Asn
   385                 390                 395                 400
   att gaa gaa ata aat cgc aat tat gct aat gcc aaa aga tca gct gaa      1248
   Ile Glu Glu Ile Asn Arg Asn Tyr Ala Asn Ala Lys Arg Ser Ala Glu
                   405                 410                 415
   tta cct gcg ttt cat ggc aga aag aga gct tgt tta gga gaa aag tat      1296
   Leu Pro Ala Phe His Gly Arg Lys Arg Ala Cys Leu Gly Glu Lys Tyr
               420                 425                 430
   gcc tta tat gaa gtt aaa gaa ttg cta act agt att tta gga cat tac      1344
   Ala Leu Tyr Glu Val Lys Glu Leu Leu Thr Ser Ile Leu Gly His Tyr
           435                 440                 445
   aaa gtt act tta gat gca agt tgg aaa gag aaa ata acc cct gct gga      1392
   Lys Val Thr Leu Asp Ala Ser Trp Lys Glu Lys Ile Thr Pro Ala Gly
       450                 455                 460
   cct att agt cca ttt ggt ttg aag gtg aaa ttt gaa aag ctt att gtt      1440
   Pro Ile Ser Pro Phe Gly Leu Lys Val Lys Phe Glu Lys Leu Ile Val
   465                 470                 475                 480
   gca taa                                                              1446
   Ala
<210> 30
   <211> 481
   <212> PRT
   <213> Candida tropicalis
<400> 30
   Met Ile Met Phe Asp Ile Val Lys Tyr Thr Leu Ile Gly Leu Phe Ser
   1               5                   10                  15
   Tyr Leu Leu Tyr Val Ile Leu Asp Ile Val Leu Pro Pro Phe Asn Phe
               20                  25                  30
   Pro Lys Asn Ile Pro Thr Ile Pro Phe Tyr Val Ser Phe Leu Gly Ala
           35                  40                  45
   Tyr Thr Asn Leu Asp Gln Arg Asp Ile Tyr Asn Leu Tyr Leu Arg Glu
       50                  55                  60
   Lys Leu Glu Lys Tyr Gly Ala Val Lys Ile Tyr Phe Ala Ser Arg Trp
   65                  70                  75                  80
   Asn Ile Leu Ile Thr Arg Pro Glu Tyr Leu Leu Glu Met Phe Arg Asn
                   85                  90                  95
   Glu Asp Val Tyr Ser Lys Arg Gly Asn His Leu Lys Ile Pro Gly Ser
               100                 105                 110
   Val Met Ala Thr Tyr Thr Gly Asp Asn Ile Ile Ser Ala His Gly Glu
           115                 120                 125
   Leu Trp Lys Leu Tyr Arg Glu Val Ile Ala Lys Ser Ile Gln Phe Pro
       130                 135                 140
   Asp Phe Glu Pro Ile Thr Lys Asn Thr Lys Ser Leu Leu Glu Ile Ile
   145                 150                 155                 160
   Asp Gly Met Ile Asp Ser Asp Lys Asn His Ala Ile Ile Pro Ile Thr
                   165                 170                 175
   Asp Leu Phe Gln Lys Tyr Ser Leu Ala Asn Val Thr Glu Ser Ile Leu
               180                 185                 190
   Gly Val Asn Phe Lys Val Leu Glu Gly Asp Gln Ser Ile Met His Gln
           195                 200                 205
   Lys Ile Lys Tyr Val Lys Leu Gln Ile Phe Lys Pro Phe Phe Leu Asn
       210                 215                 220
   Phe Pro Tyr Phe Asp Ser Phe Pro Ile Pro Ser Arg Leu Gln Ala Arg
   225                 230                 235                 240
   Lys Glu Val Ile Asn Phe Arg Asn Trp Tyr Gly Gln Ser Ile Ile Asp
                   245                 250                 255
   Lys His Asp Pro Gln Leu Pro Asn Ser Ala Ala Thr Lys Leu Val Asp
               260                 265                 270
   Gly Leu Met Gln Glu Lys Leu Thr Glu Lys Gln Phe Leu Asp Asn Ala
           275                 280                 285
   Ile Ile Val Met Ile Ala Gly His Glu Asn Pro Leu Leu Leu Met Leu
       290                 295                 300
   Ser Leu Met Phe Val Ala Ala Lys Tyr Pro Lys Val Gln Glu Ala Ile
   305                 310                 315                 320
   Arg Ser Glu Ile Asp Pro Thr Lys Pro Tyr Leu His Ser Val Ile Tyr
                   325                 330                 335
   Glu Thr Leu Arg Met Tyr Pro Pro Leu Gly Leu Ile Ile Asn Arg Tyr
   340 345 350
   Thr Thr Arg Pro Thr Lys Leu Gly Asn Ile Val Ile Pro Lys Gly Val
   355 360 365
   Tyr Cys Gly Tyr Asn Asn Phe Gly Thr Gly Arg Asp Arg Asn Val Trp
   370 375 380
   Gly Pro Asp Ser Asp Glu Phe Lys Pro Glu Arg Trp Gly Arg Asp Asn
   385 390 395 400
   Ile Glu Glu Ile Asn Arg Asn Tyr Ala Asn Ala Lys Arg Ser Ala Glu
   405 410 415
   Leu Pro Ala Phe His Gly Arg Lys Arg Ala Cys Leu Gly Glu Lys Tyr
   420 425 430
   Ala Leu Tyr Glu Val Lys Glu Leu Leu Thr Ser Ile Leu Gly His Tyr
   435 440 445
   Lys Val Thr Leu Asp Ala Ser Trp Lys Glu Lys Ile Thr Pro Ala Gly
   450 455 460
   Pro Ile Ser Pro Phe Gly Leu Lys Val Lys Phe Glu Lys Leu Ile Val
   465 470 475 480
   Ala
<210> 31
   <211> 1623
   <212> DNA
   <213> Candida tropicalis
<220>
   <221> CDS
   <222> (1)..(1623)
<400> 31
   atg tcg tcg tct cca tct att gct caa gaa ttt ctc gca acc att act       48
   Met Ser Ser Ser Pro Ser Ile Ala Gln Glu Phe Leu Ala Thr Ile Thr
   1               5                   10                  15
   cca tat gtt gag tat tgt caa gag aat tat acc aaa tgg tac tac ttc       96
   Pro Tyr Val Glu Tyr Cys Gln Glu Asn Tyr Thr Lys Trp Tyr Tyr Phe
               20                  25                  30
   att cca ttg gtg att ctt tcg ttg aat ctt atc tcc atg ctt cat aca      144
   Ile Pro Leu Val Ile Leu Ser Leu Asn Leu Ile Ser Met Leu His Thr
           35                  40                  45
   aag tat ttg gaa cgt aaa ttt aag gct aaa ccg ctt gct gtc tat gtt      192
   Lys Tyr Leu Glu Arg Lys Phe Lys Ala Lys Pro Leu Ala Val Tyr Val
       50                  55                  60
   caa gat tat acc ttt ggt ctt att act cca ctt gtt ttg atc tac tac      240
   Gln Asp Tyr Thr Phe Gly Leu Ile Thr Pro Leu Val Leu Ile Tyr Tyr
   65                  70                  75                  80
   aag tct aaa ggt acc gtg atg caa ttt gcc tgt gat tta tgg gac aag      288
   Lys Ser Lys Gly Thr Val Met Gln Phe Ala Cys Asp Leu Trp Asp Lys
                   85                  90                  95
   aaa ctc att gtc agt gat cca aag gca aag act att ggt ctt aag att      336
   Lys Leu Ile Val Ser Asp Pro Lys Ala Lys Thr Ile Gly Leu Lys Ile
               100                 105                 110
   tta ggt att cca ttg att gaa act aaa gac cca gag aat gtc aag gct      384
   Leu Gly Ile Pro Leu Ile Glu Thr Lys Asp Pro Glu Asn Val Lys Ala
           115                 120                 125
   att tta gcc acc cag ttt aat gat ttt tct ttg gga act aga cat gat      432
   Ile Leu Ala Thr Gln Phe Asn Asp Phe Ser Leu Gly Thr Arg His Asp
       130                 135                 140
   ttc ttg tat tca ttg tta gga gat ggt att ttc act tta gat ggt gct      480
   Phe Leu Tyr Ser Leu Leu Gly Asp Gly Ile Phe Thr Leu Asp Gly Ala
   145                 150                 155                 160
   ggc tgg aaa cac agc aga acc atg ttg aga cca caa ttc gct aga gag      528
   Gly Trp Lys His Ser Arg Thr Met Leu Arg Pro Gln Phe Ala Arg Glu
                   165                 170                 175
   caa gtt tcg cat gta aag ttg ttg gaa ccc cat atg caa gtt tta ttt      576
   Gln Val Ser His Val Lys Leu Leu Glu Pro His Met Gln Val Leu Phe
               180                 185                 190
   aaa cac att aga aaa cat cat ggc caa act ttt gat atc caa gaa ttg      624
   Lys His Ile Arg Lys His His Gly Gln Thr Phe Asp Ile Gln Glu Leu
           195                 200                 205
   ttt ttc aga tta act gtt gat tcg gct act gag ttt ttg ttt ggt gaa      672
   Phe Phe Arg Leu Thr Val Asp Ser Ala Thr Glu Phe Leu Phe Gly Glu
       210                 215                 220
   tct gct gaa tct tta aga gac gag tct gtc ggt tta act cca aca acc      720
   Ser Ala Glu Ser Leu Arg Asp Glu Ser Val Gly Leu Thr Pro Thr Thr
   225                 230                 235                 240
   aaa gat ttt gat ggt aga aat gag ttt gcc gat gcg ttc aac tat tct      768
   Lys Asp Phe Asp Gly Arg Asn Glu Phe Ala Asp Ala Phe Asn Tyr Ser
                   245                 250                 255
   caa act tac caa gcg tac aga ttc ttg ttg caa caa atg tat tgg att      816
   Gln Thr Tyr Gln Ala Tyr Arg Phe Leu Leu Gln Gln Met Tyr Trp Ile
               260                 265                 270
   ttg aat ggt tct gaa ttt aga aaa tct att gct att gtc cac aag ttt      864
   Leu Asn Gly Ser Glu Phe Arg Lys Ser Ile Ala Ile Val His Lys Phe
           275                 280                 285
   gct gac cac tat gtt caa aag gca ttg gaa tta act gat gaa gat ttg      912
   Ala Asp His Tyr Val Gln Lys Ala Leu Glu Leu Thr Asp Glu Asp Leu
       290                 295                 300
   gaa aag aaa gaa ggc tat gta ttt tta ttc gag tta gcc aaa caa acc      960
   Glu Lys Lys Glu Gly Tyr Val Phe Leu Phe Glu Leu Ala Lys Gln Thr
   305                 310                 315                 320
   aga gat cca aag gtt ttg aga gat caa ttg tta aat atc ttg gtt gct     1008
   Arg Asp Pro Lys Val Leu Arg Asp Gln Leu Leu Asn Ile Leu Val Ala
                   325                 330                 335
   ggt aga gat acc aca gct ggc ttg ttg tcg ttt ctt ttc ttt gaa ttg     1056
   Gly Arg Asp Thr Thr Ala Gly Leu Leu Ser Phe Leu Phe Phe Glu Leu
               340                 345                 350
   tct aga aac cca gaa ata ttt gca aaa ttg aga gaa gaa atc gaa aac     1104
   Ser Arg Asn Pro Glu Ile Phe Ala Lys Leu Arg Glu Glu Ile Glu Asn
           355                 360                 365
   aag ttt ggt ctt gga caa gat gct cgt gtt gaa gag att tct ttt gaa     1152
   Lys Phe Gly Leu Gly Gln Asp Ala Arg Val Glu Glu Ile Ser Phe Glu
       370                 375                 380
   aca ttg aaa tct tgt gaa tac ttg aag gct gtt atc aat gaa act ttg     1200
   Thr Leu Lys Ser Cys Glu Tyr Leu Lys Ala Val Ile Asn Glu Thr Leu
   385                 390                 395                 400
   aga att tat cct tcc gtc cca cat aat ttt aga gtt gct act aga aac     1248
   Arg Ile Tyr Pro Ser Val Pro His Asn Phe Arg Val Ala Thr Arg Asn
                   405                 410                 415
   aca act tta cca aga ggt ggt ggt gaa ggt ggt tta tcc cca att gct     1296
   Thr Thr Leu Pro Arg Gly Gly Gly Glu Gly Gly Leu Ser Pro Ile Ala
               420                 425                 430
   att aag aag ggc caa gtt gtt atg tac acg att ctt gct act cac aga     1344
   Ile Lys Lys Gly Gln Val Val Met Tyr Thr Ile Leu Ala Thr His Arg
           435                 440                 445
   gat aaa gac att tat ggt gaa gat gct tat gtt ttc agg cca gaa aga     1392
   Asp Lys Asp Ile Tyr Gly Glu Asp Ala Tyr Val Phe Arg Pro Glu Arg
       450                 455                 460
   tgg ttt gaa cct gaa acc aga aaa ttg ggc tgg gca tat gtt cca ttc     1440
   Trp Phe Glu Pro Glu Thr Arg Lys Leu Gly Trp Ala Tyr Val Pro Phe
   465                 470                 475                 480
   aat ggc ggt cca aga att tgt ttg ggt caa cag ttt gct tta act gaa     1488
   Asn Gly Gly Pro Arg Ile Cys Leu Gly Gln Gln Phe Ala Leu Thr Glu
                   485                 490                 495
   gca tca tat gtc act gtt aga ttg ctt caa gaa ttt ggt aac ttg aaa     1536
   Ala Ser Tyr Val Thr Val Arg Leu Leu Gln Glu Phe Gly Asn Leu Lys
               500                 505                 510
   caa gat cca aat act gaa tat cca cca aaa tta caa aac aca ttg act     1584
   Gln Asp Pro Asn Thr Glu Tyr Pro Pro Lys Leu Gln Asn Thr Leu Thr
           515                 520                 525
   ttg tct ctt ttt gaa ggt gct gaa gta caa atg tat taa                 1623
   Leu Ser Leu Phe Glu Gly Ala Glu Val Gln Met Tyr
       530                 535                 540
<210> 32
   <211> 540
   <212> PRT
   <213> Candida tropicalis
<400> 32
   Met Ser Ser Ser Pro Ser Ile Ala Gln Glu Phe Leu Ala Thr Ile Thr
   1               5                   10                  15
   Pro Tyr Val Glu Tyr Cys Gln Glu Asn Tyr Thr Lys Trp Tyr Tyr Phe
               20                  25                  30
   Ile Pro Leu Val Ile Leu Ser Leu Asn Leu Ile Ser Met Leu His Thr
           35                  40                  45
   Lys Tyr Leu Glu Arg Lys Phe Lys Ala Lys Pro Leu Ala Val Tyr Val
       50                  55                  60
   Gln Asp Tyr Thr Phe Gly Leu Ile Thr Pro Leu Val Leu Ile Tyr Tyr
   65                  70                  75                  80
   Lys Ser Lys Gly Thr Val Met Gln Phe Ala Cys Asp Leu Trp Asp Lys
                   85                  90                  95
   Lys Leu Ile Val Ser Asp Pro Lys Ala Lys Thr Ile Gly Leu Lys Ile
               100                 105                 110
   Leu Gly Ile Pro Leu Ile Glu Thr Lys Asp Pro Glu Asn Val Lys Ala
           115                 120                 125
   Ile Leu Ala Thr Gln Phe Asn Asp Phe Ser Leu Gly Thr Arg His Asp
       130                 135                 140
   Phe Leu Tyr Ser Leu Leu Gly Asp Gly Ile Phe Thr Leu Asp Gly Ala
   145                 150                 155                 160
   Gly Trp Lys His Ser Arg Thr Met Leu Arg Pro Gln Phe Ala Arg Glu
                   165                 170                 175
   Gln Val Ser His Val Lys Leu Leu Glu Pro His Met Gln Val Leu Phe
               180                 185                 190
   Lys His Ile Arg Lys His His Gly Gln Thr Phe Asp Ile Gln Glu Leu
           195                 200                 205
   Phe Phe Arg Leu Thr Val Asp Ser Ala Thr Glu Phe Leu Phe Gly Glu
       210                 215                 220
   Ser Ala Glu Ser Leu Arg Asp Glu Ser Val Gly Leu Thr Pro Thr Thr
   225                 230                 235                 240
   Lys Asp Phe Asp Gly Arg Asn Glu Phe Ala Asp Ala Phe Asn Tyr Ser
   245 250 255
   Gln Thr Tyr Gln Ala Tyr Arg Phe Leu Leu Gln Gln Met Tyr Trp Ile
   260 265 270
   Leu Asn Gly Ser Glu Phe Arg Lys Ser Ile Ala Ile Val His Lys Phe
   275 280 285
   Ala Asp His Tyr Val Gln Lys Ala Leu Glu Leu Thr Asp Glu Asp Leu
   290 295 300
   Glu Lys Lys Glu Gly Tyr Val Phe Leu Phe Glu Leu Ala Lys Gln Thr
   305 310 315 320
   Arg Asp Pro Lys Val Leu Arg Asp Gln Leu Leu Asn Ile Leu Val Ala
   325 330 335
   Gly Arg Asp Thr Thr Ala Gly Leu Leu Ser Phe Leu Phe Phe Glu Leu
   340 345 350
   Ser Arg Asn Pro Glu Ile Phe Ala Lys Leu Arg Glu Glu Ile Glu Asn
   355 360 365
   Lys Phe Gly Leu Gly Gln Asp Ala Arg Val Glu Glu Ile Ser Phe Glu
   370 375 380
   Thr Leu Lys Ser Cys Glu Tyr Leu Lys Ala Val Ile Asn Glu Thr Leu
   385 390 395 400
   Arg Ile Tyr Pro Ser Val Pro His Asn Phe Arg Val Ala Thr Arg Asn
   405 410 415
   Thr Thr Leu Pro Arg Gly Gly Gly Glu Gly Gly Leu Ser Pro Ile Ala
   420 425 430
   Ile Lys Lys Gly Gln Val Val Met Tyr Thr Ile Leu Ala Thr His Arg
   435 440 445
   Asp Lys Asp Ile Tyr Gly Glu Asp Ala Tyr Val Phe Arg Pro Glu Arg
   450 455 460
   Trp Phe Glu Pro Glu Thr Arg Lys Leu Gly Trp Ala Tyr Val Pro Phe
   465 470 475 480
   Asn Gly Gly Pro Arg Ile Cys Leu Gly Gln Gln Phe Ala Leu Thr Glu
   485 490 495
   Ala Ser Tyr Val Thr Val Arg Leu Leu Gln Glu Phe Gly Asn Leu Lys
   500 505 510
   Gln Asp Pro Asn Thr Glu Tyr Pro Pro Lys Leu Gln Asn Thr Leu Thr
   515 520 525
   Leu Ser Leu Phe Glu Gly Ala Glu Val Gln Met Tyr
   530 535 540
<210> 33
   <211> 1569
   <212> DNA
   <213> Candida tropicalis
<220>
   <221> CDS
   <222> (1)..(1569)
<400> 33
   atg agt att caa gat att gtt gaa act tac agt acc aaa tgg tac gtt        48
   Met Ser Ile Gln Asp Ile Val Glu Thr Tyr Ser Thr Lys Trp Tyr Val
   1               5                   10                  15
   gtt gta ctg gtt gct ttg att gta tac aag gtt ttt gat ttc ttt tat        96
   Val Val Leu Val Ala Leu Ile Val Tyr Lys Val Phe Asp Phe Phe Tyr
               20                  25                  30
   gct aga tat ttg atg tat aag ctt ggt gct aag cca ttc ctt caa agt       144
   Ala Arg Tyr Leu Met Tyr Lys Leu Gly Ala Lys Pro Phe Leu Gln Ser
           35                  40                  45
   caa acc gac ggt tat ctt ggt ttc aga gtt cca ttt gaa ttg atg gga       192
   Gln Thr Asp Gly Tyr Leu Gly Phe Arg Val Pro Phe Glu Leu Met Gly
       50                  55                  60
   aag aag agt gaa ggt aca ctt ata gac ttt aca tat caa cgt act ttg       240
   Lys Lys Ser Glu Gly Thr Leu Ile Asp Phe Thr Tyr Gln Arg Thr Leu
   65                  70                  75                  80
   gag ctt gac aat ccc gat att cca aca ttt aca ttc cca ata ttt tct       288
   Glu Leu Asp Asn Pro Asp Ile Pro Thr Phe Thr Phe Pro Ile Phe Ser
                   85                  90                  95
   gtt ctg att atc tca act ctt gaa cca gac aac atc aaa gct att ttg       336
   Val Leu Ile Ile Ser Thr Leu Glu Pro Asp Asn Ile Lys Ala Ile Leu
               100                 105                 110
   gcc aca caa ttc aat gac ttt tca tta ggt aca aga cat tca cat ttt       384
   Ala Thr Gln Phe Asn Asp Phe Ser Leu Gly Thr Arg His Ser His Phe
           115                 120                 125
   gct cct tta tta ggt gac ggt att ttc act tta gat ggt gct ggt tgg       432
   Ala Pro Leu Leu Gly Asp Gly Ile Phe Thr Leu Asp Gly Ala Gly Trp
       130                 135                 140
   aaa cat agt aga tct atg ttg aga cca caa ttt gca aga gaa cag gtt       480
   Lys His Ser Arg Ser Met Leu Arg Pro Gln Phe Ala Arg Glu Gln Val
   145                 150                 155                 160
   tct cac gtt aag ttg ttg gaa cca cat atg caa gtt ttt ttc aaa cat      528
   Ser His Val Lys Leu Leu Glu Pro His Met Gln Val Phe Phe Lys His
                   165                 170                 175
   att aga aaa cat cat ggc caa acc ttt gat ata caa gaa ttg ttt ttc      576
   Ile Arg Lys His His Gly Gln Thr Phe Asp Ile Gln Glu Leu Phe Phe
               180                 185                 190
   aga tta act gtt gat tct gcc act gag ttt ttg ttt ggt gaa tct gtt      624
   Arg Leu Thr Val Asp Ser Ala Thr Glu Phe Leu Phe Gly Glu Ser Val
           195                 200                 205
   gag tct tta aga gac gag tcc atc ggt atg tta aac gat gct ctt gat      672
   Glu Ser Leu Arg Asp Glu Ser Ile Gly Met Leu Asn Asp Ala Leu Asp
       210                 215                 220
   ttt gat ggt aag gct gga ttt gct gat gcc ttt aac tat tct caa aac      720
   Phe Asp Gly Lys Ala Gly Phe Ala Asp Ala Phe Asn Tyr Ser Gln Asn
   225                 230                 235                 240
   tat ttg gct tct cga gct ctt atg caa caa atg tac tgg att ttg aac      768
   Tyr Leu Ala Ser Arg Ala Leu Met Gln Gln Met Tyr Trp Ile Leu Asn
                   245                 250                 255
   gga aaa aag ttt aaa gaa tgt aat gcc aag gtt cac aag ttt gct gat      816
   Gly Lys Lys Phe Lys Glu Cys Asn Ala Lys Val His Lys Phe Ala Asp
               260                 265                 270
   tat tat gtt gaa aaa gca ttg gaa tta act ccg gac caa ttg gaa aaa      864
   Tyr Tyr Val Glu Lys Ala Leu Glu Leu Thr Pro Asp Gln Leu Glu Lys
           275                 280                 285
   caa gat ggg tat gtt ttc ttg tat gaa ttg gta aaa caa acc aga gac      912
   Gln Asp Gly Tyr Val Phe Leu Tyr Glu Leu Val Lys Gln Thr Arg Asp
       290                 295                 300
   aga caa gtc ttg aga gat cag tta ttg aat ata tta gtt gct ggt aga      960
   Arg Gln Val Leu Arg Asp Gln Leu Leu Asn Ile Leu Val Ala Gly Arg
   305                 310                 315                 320
   gat acc act gct ggt tta tta tca ttt gtg ttt ttt gaa ttg gca aga     1008
   Asp Thr Thr Ala Gly Leu Leu Ser Phe Val Phe Phe Glu Leu Ala Arg
                   325                 330                 335
   act cca aga gta gca aat aaa tta aga gaa gaa atc gaa gac aaa ttt     1056
   Thr Pro Arg Val Ala Asn Lys Leu Arg Glu Glu Ile Glu Asp Lys Phe
               340                 345                 350
   ggc ctt gga caa gat gct cgt gtt gaa gaa att tcc ttt gaa tct tta     1104
   Gly Leu Gly Gln Asp Ala Arg Val Glu Glu Ile Ser Phe Glu Ser Leu
           355                 360                 365
   aaa tca tgt gaa tat ttg aag gca gtg ctt aat gaa tgt tta aga tta     1152
   Lys Ser Cys Glu Tyr Leu Lys Ala Val Leu Asn Glu Cys Leu Arg Leu
       370                 375                 380
   tac cca tct gtt cca caa aac ttt aga gtt gct acc aga aat acc aca     1200
   Tyr Pro Ser Val Pro Gln Asn Phe Arg Val Ala Thr Arg Asn Thr Thr
   385                 390                 395                 400
   tta cca aga ggt ggt ggc aag gat ggt tta tca cca gta tta gtt aga      1248
   Leu Pro Arg Gly Gly Gly Lys Asp Gly Leu Ser Pro Val Leu Val Arg
                   405                 410                 415
   aag ggt caa act gtg atg tac agt gtg tat gct gcc cat aga aac aaa      1296
   Lys Gly Gln Thr Val Met Tyr Ser Val Tyr Ala Ala His Arg Asn Lys
               420                 425                 430
   caa att tat ggt gaa gac gca ctt gaa ttc agg cca gaa aga tgg ttt      1344
   Gln Ile Tyr Gly Glu Asp Ala Leu Glu Phe Arg Pro Glu Arg Trp Phe
           435                 440                 445
   gaa cca gag aca aag aaa ttg ggc tgg gcc ttc tta cct ttt aat ggt      1392
   Glu Pro Glu Thr Lys Lys Leu Gly Trp Ala Phe Leu Pro Phe Asn Gly
       450                 455                 460
   ggt cca aga att tgt ttg ggt caa caa ttt gct ttg act gaa gct tct      1440
   Gly Pro Arg Ile Cys Leu Gly Gln Gln Phe Ala Leu Thr Glu Ala Ser
   465                 470                 475                 480
   tat gtt act gtt aga tta ctt caa gag ttt agc cac ttg aca atg gat      1488
   Tyr Val Thr Val Arg Leu Leu Gln Glu Phe Ser His Leu Thr Met Asp
                   485                 490                 495
   cca aac act gaa tac ccg cca aag aaa atg tcc cat ttg aca atg tct      1536
   Pro Asn Thr Glu Tyr Pro Pro Lys Lys Met Ser His Leu Thr Met Ser
               500                 505                 510
   cta ttt gat ggt gcc aac att caa atg tat tag                          1569
   Leu Phe Asp Gly Ala Asn Ile Gln Met Tyr
           515                 520
<210> 34
   <211> 522
   <212> PRT
   <213> Candida tropicalis
<400> 34
   Met Ser Ile Gln Asp Ile Val Glu Thr Tyr Ser Thr Lys Trp Tyr Val
   1               5                   10                  15
   Val Val Leu Val Ala Leu Ile Val Tyr Lys Val Phe Asp Phe Phe Tyr
               20                  25                  30
   Ala Arg Tyr Leu Met Tyr Lys Leu Gly Ala Lys Pro Phe Leu Gln Ser
           35                  40                  45
   Gln Thr Asp Gly Tyr Leu Gly Phe Arg Val Pro Phe Glu Leu Met Gly
       50                  55                  60
   Lys Lys Ser Glu Gly Thr Leu Ile Asp Phe Thr Tyr Gln Arg Thr Leu
   65                  70                  75                  80
   Glu Leu Asp Asn Pro Asp Ile Pro Thr Phe Thr Phe Pro Ile Phe Ser
                   85                  90                  95
   Val Leu Ile Ile Ser Thr Leu Glu Pro Asp Asn Ile Lys Ala Ile Leu
   100 105 110
   Ala Thr Gln Phe Asn Asp Phe Ser Leu Gly Thr Arg His Ser His Phe
   115 120 125
   Ala Pro Leu Leu Gly Asp Gly Ile Phe Thr Leu Asp Gly Ala Gly Trp
   130 135 140
   Lys His Ser Arg Ser Met Leu Arg Pro Gln Phe Ala Arg Glu Gln Val
   145 150 155 160
   Ser His Val Lys Leu Leu Glu Pro His Met Gln Val Phe Phe Lys His
   165 170 175
   Ile Arg Lys His His Gly Gln Thr Phe Asp Ile Gln Glu Leu Phe Phe
   180 185 190
   Arg Leu Thr Val Asp Ser Ala Thr Glu Phe Leu Phe Gly Glu Ser Val
   195 200 205
   Glu Ser Leu Arg Asp Glu Ser Ile Gly Met Leu Asn Asp Ala Leu Asp
   210 215 220
   Phe Asp Gly Lys Ala Gly Phe Ala Asp Ala Phe Asn Tyr Ser Gln Asn
   225 230 235 240
   Tyr Leu Ala Ser Arg Ala Leu Met Gln Gln Met Tyr Trp Ile Leu Asn
   245 250 255
   Gly Lys Lys Phe Lys Glu Cys Asn Ala Lys Val His Lys Phe Ala Asp
   260 265 270
   Tyr Tyr Val Glu Lys Ala Leu Glu Leu Thr Pro Asp Gln Leu Glu Lys
   275 280 285
   Gln Asp Gly Tyr Val Phe Leu Tyr Glu Leu Val Lys Gln Thr Arg Asp
   290 295 300
   Arg Gln Val Leu Arg Asp Gln Leu Leu Asn Ile Leu Val Ala Gly Arg
   305 310 315 320
   Asp Thr Thr Ala Gly Leu Leu Ser Phe Val Phe Phe Glu Leu Ala Arg
   325 330 335
   Thr Pro Arg Val Ala Asn Lys Leu Arg Glu Glu Ile Glu Asp Lys Phe
   340 345 350
   Gly Leu Gly Gln Asp Ala Arg Val Glu Glu Ile Ser Phe Glu Ser Leu
           355                 360                 365
   Lys Ser Cys Glu Tyr Leu Lys Ala Val Leu Asn Glu Cys Leu Arg Leu
       370                 375                 380
   Tyr Pro Ser Val Pro Gln Asn Phe Arg Val Ala Thr Arg Asn Thr Thr
   385                 390                 395                 400
   Leu Pro Arg Gly Gly Gly Lys Asp Gly Leu Ser Pro Val Leu Val Arg
                   405                 410                 415
   Lys Gly Gln Thr Val Met Tyr Ser Val Tyr Ala Ala His Arg Asn Lys
               420                 425                 430
   Gln Ile Tyr Gly Glu Asp Ala Leu Glu Phe Arg Pro Glu Arg Trp Phe
           435                 440                 445
   Glu Pro Glu Thr Lys Lys Leu Gly Trp Ala Phe Leu Pro Phe Asn Gly
       450                 455                 460
   Gly Pro Arg Ile Cys Leu Gly Gln Gln Phe Ala Leu Thr Glu Ala Ser
   465                 470                 475                 480
   Tyr Val Thr Val Arg Leu Leu Gln Glu Phe Ser His Leu Thr Met Asp
                   485                 490                 495
   Pro Asn Thr Glu Tyr Pro Pro Lys Lys Met Ser His Leu Thr Met Ser
               500                 505                 510
   Leu Phe Asp Gly Ala Asn Ile Gln Met Tyr
           515                 520
<210> 35
   <211> 1572
   <212> DNA
   <213> Candida tropicalis
<220>
   <221> CDS
   <222> (1)..(1572)
<400> 35
   atg gcc aca caa gaa att att gat tct gca ctt ccg tac ttg aca aag      48
   Met Ala Thr Gln Glu Ile Ile Asp Ser Ala Leu Pro Tyr Leu Thr Lys
   1               5                   10                  15
   tgg tat act gtt atc act tta gca gct ttg gtt ttc tta att tca tct      96
   Trp Tyr Thr Val Ile Thr Leu Ala Ala Leu Val Phe Leu Ile Ser Ser
               20                  25                  30
   aat att aaa aat tac gtc aag gct aag aag ttg aaa tgc aga gat cct      144
   Asn Ile Lys Asn Tyr Val Lys Ala Lys Lys Leu Lys Cys Arg Asp Pro
           35                  40                  45
   cca tat ttc aaa gga gcc ggt tgg aca ggt att agt cca tta att gaa      192
   Pro Tyr Phe Lys Gly Ala Gly Trp Thr Gly Ile Ser Pro Leu Ile Glu
       50                  55                  60
   att att aaa gtt aaa ggt aat ggt aga ttg gca gat ttt gcc gat aaa      240
   Ile Ile Lys Val Lys Gly Asn Gly Arg Leu Ala Asp Phe Ala Asp Lys
   65                  70                  75                  80
   aca ttc gac gac tat cca aac cat act ttt tac atg tct att att ggt      288
   Thr Phe Asp Asp Tyr Pro Asn His Thr Phe Tyr Met Ser Ile Ile Gly
                   85                  90                  95
   gct ttg aaa atc gtc ttg act gtt gat cca gaa aat att aaa gct gtt      336
   Ala Leu Lys Ile Val Leu Thr Val Asp Pro Glu Asn Ile Lys Ala Val
               100                 105                 110
   ttg gct act caa ttt act gat ttc tcc tta ggt acc aga cat gct cat      384
   Leu Ala Thr Gln Phe Thr Asp Phe Ser Leu Gly Thr Arg His Ala His
           115                 120                 125
   ttc tat cca tta tta ggt gat ggt att ttt act ttg gat ggt gaa ggt      432
   Phe Tyr Pro Leu Leu Gly Asp Gly Ile Phe Thr Leu Asp Gly Glu Gly
       130                 135                 140
   tgg aaa cat agt aga gct atg ttg aga cca caa ttt gct aga gat caa      480
   Trp Lys His Ser Arg Ala Met Leu Arg Pro Gln Phe Ala Arg Asp Gln
   145                 150                 155                 160
   att ggt cat gtt aaa gct ttg gaa cca cat att caa atc ttg gcc aaa      528
   Ile Gly His Val Lys Ala Leu Glu Pro His Ile Gln Ile Leu Ala Lys
                   165                 170                 175
   caa att aaa ttg aat aaa ggt aaa act ttt gat att caa gaa ttg ttt      576
   Gln Ile Lys Leu Asn Lys Gly Lys Thr Phe Asp Ile Gln Glu Leu Phe
               180                 185                 190
   ttc aga ttt act gtt gat act gct act gaa ttc ttg ttt ggt gaa tct      624
   Phe Arg Phe Thr Val Asp Thr Ala Thr Glu Phe Leu Phe Gly Glu Ser
           195                 200                 205
   gtt cac tct ttg tat gat gaa aaa tta ggt att cct act cca aat gaa      672
   Val His Ser Leu Tyr Asp Glu Lys Leu Gly Ile Pro Thr Pro Asn Glu
       210                 215                 220
   att cca ggt aga gat aat ttt gca act gct ttt aac act tct caa cat      720
   Ile Pro Gly Arg Asp Asn Phe Ala Thr Ala Phe Asn Thr Ser Gln His
   225                 230                 235                 240
   tat ttg gct acc aga aca tac tcc caa act ttc tac ttt tta act aac      768
   Tyr Leu Ala Thr Arg Thr Tyr Ser Gln Thr Phe Tyr Phe Leu Thr Asn
                   245                 250                 255
   cct aag gaa ttt aga gac tgt aat gct aaa gtt cat tac ttg gct aaa      816
   Pro Lys Glu Phe Arg Asp Cys Asn Ala Lys Val His Tyr Leu Ala Lys
               260                 265                 270
   tat ttt gtc aat aaa gct ttg aat ttc act ccg gaa gaa att gaa gaa      864
   Tyr Phe Val Asn Lys Ala Leu Asn Phe Thr Pro Glu Glu Ile Glu Glu
           275                 280                 285
   aag tcc aaa tct ggt tat gtt ttc ttg tat gaa ttg gtt aaa caa acc      912
   Lys Ser Lys Ser Gly Tyr Val Phe Leu Tyr Glu Leu Val Lys Gln Thr
       290                 295                 300
   aga gat cca aaa gtt tta caa gat caa tta ttg aac att atg gtt gcc      960
   Arg Asp Pro Lys Val Leu Gln Asp Gln Leu Leu Asn Ile Met Val Ala
   305                 310                 315                 320
   ggt aga gat acc act gct ggt tta tta tca ttt gca atg ttt gaa tta     1008
   Gly Arg Asp Thr Thr Ala Gly Leu Leu Ser Phe Ala Met Phe Glu Leu
                   325                 330                 335
   gct aga cat cca gaa att tgg tct aaa tta aga gaa gaa att gaa gtt     1056
   Ala Arg His Pro Glu Ile Trp Ser Lys Leu Arg Glu Glu Ile Glu Val
               340                 345                 350
   aac ttt ggt gtt ggt gaa gaa tct cgt gtt gaa gaa att act ttt gaa     1104
   Asn Phe Gly Val Gly Glu Glu Ser Arg Val Glu Glu Ile Thr Phe Glu
           355                 360                 365
   tct ttg aag aga tgt gaa tac ttg aaa gct att ctt aat gaa act ttg     1152
   Ser Leu Lys Arg Cys Glu Tyr Leu Lys Ala Ile Leu Asn Glu Thr Leu
       370                 375                 380
   cgt atg tat cct tct gtt cca gtc aat tcc aga aca gcc act aga gat     1200
   Arg Met Tyr Pro Ser Val Pro Val Asn Ser Arg Thr Ala Thr Arg Asp
   385                 390                 395                 400
   acc aca tta cca aga ggt ggt ggt cca aat ggt act gat cca att ttt     1248
   Thr Thr Leu Pro Arg Gly Gly Gly Pro Asn Gly Thr Asp Pro Ile Phe
                   405                 410                 415
   att cca aag ggt tcc act gtt gct tat att gtt tac aaa act cat cgt     1296
   Ile Pro Lys Gly Ser Thr Val Ala Tyr Ile Val Tyr Lys Thr His Arg
               420                 425                 430
   tta gaa gaa tat tat ggt aaa gat gct gat gat ttc aga cca gaa aga     1344
   Leu Glu Glu Tyr Tyr Gly Lys Asp Ala Asp Asp Phe Arg Pro Glu Arg
           435                 440                 445
   tgg ttt gaa cca tca act aaa aag tta ggt tgg gct tat gtt cca ttt     1392
   Trp Phe Glu Pro Ser Thr Lys Lys Leu Gly Trp Ala Tyr Val Pro Phe
       450                 455                 460
   aat ggt ggt cca aga att tgt tta ggc caa caa ttt gct tta act gaa     1440
   Asn Gly Gly Pro Arg Ile Cys Leu Gly Gln Gln Phe Ala Leu Thr Glu
   465                 470                 475                 480
   gct tct tat gtt att acc aga ttg gta caa atg ttt gaa act gtt tct     1488
   Ala Ser Tyr Val Ile Thr Arg Leu Val Gln Met Phe Glu Thr Val Ser
                   485                 490                 495
   tct tcc cca gat gtt gaa tac cct cca cca aaa tgt att cat ttg act     1536
   Ser Ser Pro Asp Val Glu Tyr Pro Pro Pro Lys Cys Ile His Leu Thr
               500                 505                 510
   atg agt cat gat gat ggt gtt ttc gtt aaa atg taa                     1572
   Met Ser His Asp Asp Gly Val Phe Val Lys Met
           515                 520
<210> 36
   <211> 523
   <212> PRT
   <213> Candida tropicalis
<400> 36
   Met Ala Thr Gln Glu Ile Ile Asp Ser Ala Leu Pro Tyr Leu Thr Lys
   1               5                   10                  15
   Trp Tyr Thr Val Ile Thr Leu Ala Ala Leu Val Phe Leu Ile Ser Ser
               20                  25                  30
   Asn Ile Lys Asn Tyr Val Lys Ala Lys Lys Leu Lys Cys Arg Asp Pro
           35                  40                  45
   Pro Tyr Phe Lys Gly Ala Gly Trp Thr Gly Ile Ser Pro Leu Ile Glu
       50                  55                  60
   Ile Ile Lys Val Lys Gly Asn Gly Arg Leu Ala Asp Phe Ala Asp Lys
   65                  70                  75                  80
   Thr Phe Asp Asp Tyr Pro Asn His Thr Phe Tyr Met Ser Ile Ile Gly
                   85                  90                  95
   Ala Leu Lys Ile Val Leu Thr Val Asp Pro Glu Asn Ile Lys Ala Val
               100                 105                 110
   Leu Ala Thr Gln Phe Thr Asp Phe Ser Leu Gly Thr Arg His Ala His
           115                 120                 125
   Phe Tyr Pro Leu Leu Gly Asp Gly Ile Phe Thr Leu Asp Gly Glu Gly
       130                 135                 140
   Trp Lys His Ser Arg Ala Met Leu Arg Pro Gln Phe Ala Arg Asp Gln
   145                 150                 155                 160
   Ile Gly His Val Lys Ala Leu Glu Pro His Ile Gln Ile Leu Ala Lys
                   165                 170                 175
   Gln Ile Lys Leu Asn Lys Gly Lys Thr Phe Asp Ile Gln Glu Leu Phe
               180                 185                 190
   Phe Arg Phe Thr Val Asp Thr Ala Thr Glu Phe Leu Phe Gly Glu Ser
           195                 200                 205
   Val His Ser Leu Tyr Asp Glu Lys Leu Gly Ile Pro Thr Pro Asn Glu
       210                 215                 220
   Ile Pro Gly Arg Asp Asn Phe Ala Thr Ala Phe Asn Thr Ser Gln His
   225 230 235 240
   Tyr Leu Ala Thr Arg Thr Tyr Ser Gln Thr Phe Tyr Phe Leu Thr Asn
   245 250 255
   Pro Lys Glu Phe Arg Asp Cys Asn Ala Lys Val His Tyr Leu Ala Lys
   260 265 270
   Tyr Phe Val Asn Lys Ala Leu Asn Phe Thr Pro Glu Glu Ile Glu Glu
   275 280 285
   Lys Ser Lys Ser Gly Tyr Val Phe Leu Tyr Glu Leu Val Lys Gln Thr
   290 295 300
   Arg Asp Pro Lys Val Leu Gln Asp Gln Leu Leu Asn Ile Met Val Ala
   305 310 315 320
   Gly Arg Asp Thr Thr Ala Gly Leu Leu Ser Phe Ala Met Phe Glu Leu
   325 330 335
   Ala Arg His Pro Glu Ile Trp Ser Lys Leu Arg Glu Glu Ile Glu Val
   340 345 350
   Asn Phe Gly Val Gly Glu Glu Ser Arg Val Glu Glu Ile Thr Phe Glu
   355 360 365
   Ser Leu Lys Arg Cys Glu Tyr Leu Lys Ala Ile Leu Asn Glu Thr Leu
   370 375 380
   Arg Met Tyr Pro Ser Val Pro Val Asn Ser Arg Thr Ala Thr Arg Asp
   385 390 395 400
   Thr Thr Leu Pro Arg Gly Gly Gly Pro Asn Gly Thr Asp Pro Ile Phe
   405 410 415
   Ile Pro Lys Gly Ser Thr Val Ala Tyr Ile Val Tyr Lys Thr His Arg
   420 425 430
   Leu Glu Glu Tyr Tyr Gly Lys Asp Ala Asp Asp Phe Arg Pro Glu Arg
   435 440 445
   Trp Phe Glu Pro Ser Thr Lys Lys Leu Gly Trp Ala Tyr Val Pro Phe
   450 455 460
   Asn Gly Gly Pro Arg Ile Cys Leu Gly Gln Gln Phe Ala Leu Thr Glu
   465 470 475 480
   Ala Ser Tyr Val Ile Thr Arg Leu Val Gln Met Phe Glu Thr Val Ser
                   485                 490                 495
   Ser Ser Pro Asp Val Glu Tyr Pro Pro Pro Lys Cys Ile His Leu Thr
               500                 505                 510
   Met Ser His Asp Asp Gly Val Phe Val Lys Met
           515                 520
<210> 37
   <211> 1512
   <212> DNA
   <213> Candida tropicalis
<220>
   <221> CDS
   <222> (1)..(1512)
<400> 37
   atg tca tta aca gaa aca act gct acg ttt att tac aat tat tgg tac       48
   Met Ser Leu Thr Glu Thr Thr Ala Thr Phe Ile Tyr Asn Tyr Trp Tyr
   1               5                   10                  15
   atc ata ttc att ata ttc tac aca acc agc aaa atc atc aag tat cat       96
   Ile Ile Phe Ile Ile Phe Tyr Thr Thr Ser Lys Ile Ile Lys Tyr His
               20                  25                  30
   cac acg aca tat ctt atg ata aag ttc aaa gct tct ccg cct ttg aat      144
   His Thr Thr Tyr Leu Met Ile Lys Phe Lys Ala Ser Pro Pro Leu Asn
           35                  40                  45
   tac ata aat aaa ggt ttt ttt gga att cag gcg acg ttc acg gaa ttg      192
   Tyr Ile Asn Lys Gly Phe Phe Gly Ile Gln Ala Thr Phe Thr Glu Leu
       50                  55                  60
   aaa cat ctt ata tgt cac aca tcg att gat tac gcc atc gat caa ttc      240
   Lys His Leu Ile Cys His Thr Ser Ile Asp Tyr Ala Ile Asp Gln Phe
   65                  70                  75                  80
   aat aac gtc cca ttc cca cat gtt cat act ttt gta acc aaa gtt ctt      288
   Asn Asn Val Pro Phe Pro His Val His Thr Phe Val Thr Lys Val Leu
                   85                  90                  95
   ggt aat gag tta atc atg aca aaa gat cct gaa aat att aaa gtt tta      336
   Gly Asn Glu Leu Ile Met Thr Lys Asp Pro Glu Asn Ile Lys Val Leu
               100                 105                 110
   ttg agt tcc agt ttt gat aag ttt gat tat gga aca cgt tca agt gcc      384
   Leu Ser Ser Ser Phe Asp Lys Phe Asp Tyr Gly Thr Arg Ser Ser Ala
           115                 120                 125
   gtg caa cca tct tta gga atg ggg ata ttc act ctt gaa gga gaa aat      432
   Val Gln Pro Ser Leu Gly Met Gly Ile Phe Thr Leu Glu Gly Glu Asn
       130                 135                 140
   tgg aaa gca aca aga agt gtt tta agg aac atg ttt gat aga aaa tca      480
   Trp Lys Ala Thr Arg Ser Val Leu Arg Asn Met Phe Asp Arg Lys Ser
   145                 150                 155                 160
   att gac aag gta cat gat ttt gaa cca cat ttc aaa acc ctt cag aaa      528
   Ile Asp Lys Val His Asp Phe Glu Pro His Phe Lys Thr Leu Gln Lys
                   165                 170                 175
   aga ata gat ggg aag gtt gga tat ttt gac atc caa cag gag ttt tta      576
   Arg Ile Asp Gly Lys Val Gly Tyr Phe Asp Ile Gln Gln Glu Phe Leu
               180                 185                 190
   aaa tta gga ttg gaa ttg agt att gag ttt att ttt ggt caa gtt gta      624
   Lys Leu Gly Leu Glu Leu Ser Ile Glu Phe Ile Phe Gly Gln Val Val
           195                 200                 205
   tcg gaa gat gtc cca cat tat gat gat ttt acc cag gct tgg gat aga      672
   Ser Glu Asp Val Pro His Tyr Asp Asp Phe Thr Gln Ala Trp Asp Arg
       210                 215                 220
   tgt caa gac tat atg atg cta aga tta ttg ttg ggg gat ttt tat tgg      720
   Cys Gln Asp Tyr Met Met Leu Arg Leu Leu Leu Gly Asp Phe Tyr Trp
   225                 230                 235                 240
   ata gct aat gac tgg aga tat aaa cag tcc aat caa att gtg caa gct      768
   Ile Ala Asn Asp Trp Arg Tyr Lys Gln Ser Asn Gln Ile Val Gln Ala
                   245                 250                 255
   ttt tgt gat tat ttg gtg caa aaa tca ctt gaa aat aca tgc aac gac      816
   Phe Cys Asp Tyr Leu Val Gln Lys Ser Leu Glu Asn Thr Cys Asn Asp
               260                 265                 270
   aaa ttt gtc ttt gta cag gaa ctt gca aaa cac acg acc aac aaa acg      864
   Lys Phe Val Phe Val Gln Glu Leu Ala Lys His Thr Thr Asn Lys Thr
           275                 280                 285
   ttc att aga gat caa gca tta agc ttg att atg gct tca aga gac aca      912
   Phe Ile Arg Asp Gln Ala Leu Ser Leu Ile Met Ala Ser Arg Asp Thr
       290                 295                 300
   act gct gag ttg atg gca ttc acc ata cta gaa tta tcc aga aat ccc      960
   Thr Ala Glu Leu Met Ala Phe Thr Ile Leu Glu Leu Ser Arg Asn Pro
   305                 310                 315                 320
   act atc tgg gaa aga tta aga gag gaa ata gat gcc aat ttt gga ttg     1008
   Thr Ile Trp Glu Arg Leu Arg Glu Glu Ile Asp Ala Asn Phe Gly Leu
                   325                 330                 335
   gaa tca ccc gac ttg ctt aca ttt gat tca ctt cgc aag ttc aaa tat     1056
   Glu Ser Pro Asp Leu Leu Thr Phe Asp Ser Leu Arg Lys Phe Lys Tyr
               340                 345                 350
   gtt caa gct atc ttg aat gag act ctt agg atg tac cct gga gtt cca     1104
   Val Gln Ala Ile Leu Asn Glu Thr Leu Arg Met Tyr Pro Gly Val Pro
           355                 360                 365
   aga aat atg aaa act gct aaa tgt act act aca tta cca aaa gga gga     1152
   Arg Asn Met Lys Thr Ala Lys Cys Thr Thr Thr Leu Pro Lys Gly Gly
       370                 375                 380
   ggg aaa gat ggt caa gat cca att cta gtt aaa aag ggt caa tct gtt     1200
   Gly Lys Asp Gly Gln Asp Pro Ile Leu Val Lys Lys Gly Gln Ser Val
   385                 390                 395                 400
   ggg ttt att tcc ata gct act cat ttg gat ccg gtt tat ttt ggt agt     1248
   Gly Phe Ile Ser Ile Ala Thr His Leu Asp Pro Val Tyr Phe Gly Ser
                   405                 410                 415
   gat gcc cat gtg ttt aga cca gac cgc tgg ttt gat tct agt atg aaa      1296
   Asp Ala His Val Phe Arg Pro Asp Arg Trp Phe Asp Ser Ser Met Lys
               420                 425                 430
   aat ttg ggg tgt aaa tac ttg ccc ttt aac gtc ggt cca aga acg tgt      1344
   Asn Leu Gly Cys Lys Tyr Leu Pro Phe Asn Val Gly Pro Arg Thr Cys
           435                 440                 445
   ttg gga caa cag tac act ttg att gag gca agc tac ttg tta gtt cgt      1392
   Leu Gly Gln Gln Tyr Thr Leu Ile Glu Ala Ser Tyr Leu Leu Val Arg
       450                 455                 460
   cta gcg caa aca tat gaa aca gtt gaa tca cat ccc gat tca gtt tat      1440
   Leu Ala Gln Thr Tyr Glu Thr Val Glu Ser His Pro Asp Ser Val Tyr
   465                 470                 475                 480
   cca cca agg aag aaa gcg ttg atc aat atg tgt gct gca gac ggt gtt      1488
   Pro Pro Arg Lys Lys Ala Leu Ile Asn Met Cys Ala Ala Asp Gly Val
                   485                 490                 495
   gat gtc aag ttc cat aga tta taa                                      1512
   Asp Val Lys Phe His Arg Leu
               500
<210> 38
   <211> 503
   <212> PRT
   <213> Candida tropicalis
<400> 38
   Met Ser Leu Thr Glu Thr Thr Ala Thr Phe Ile Tyr Asn Tyr Trp Tyr
   1               5                   10                  15
   Ile Ile Phe Ile Ile Phe Tyr Thr Thr Ser Lys Ile Ile Lys Tyr His
               20                  25                  30
   His Thr Thr Tyr Leu Met Ile Lys Phe Lys Ala Ser Pro Pro Leu Asn
           35                  40                  45
   Tyr Ile Asn Lys Gly Phe Phe Gly Ile Gln Ala Thr Phe Thr Glu Leu
       50                  55                  60
   Lys His Leu Ile Cys His Thr Ser Ile Asp Tyr Ala Ile Asp Gln Phe
   65                  70                  75                  80
   Asn Asn Val Pro Phe Pro His Val His Thr Phe Val Thr Lys Val Leu
                   85                  90                  95
   Gly Asn Glu Leu Ile Met Thr Lys Asp Pro Glu Asn Ile Lys Val Leu
               100                 105                 110
   Leu Ser Ser Ser Phe Asp Lys Phe Asp Tyr Gly Thr Arg Ser Ser Ala
           115                 120                 125
   Val Gln Pro Ser Leu Gly Met Gly Ile Phe Thr Leu Glu Gly Glu Asn
       130                 135                 140
   Trp Lys Ala Thr Arg Ser Val Leu Arg Asn Met Phe Asp Arg Lys Ser
   145                 150                 155                 160
   Ile Asp Lys Val His Asp Phe Glu Pro His Phe Lys Thr Leu Gln Lys
                   165                 170                 175
   Arg Ile Asp Gly Lys Val Gly Tyr Phe Asp Ile Gln Gln Glu Phe Leu
               180                 185                 190
   Lys Leu Gly Leu Glu Leu Ser Ile Glu Phe Ile Phe Gly Gln Val Val
           195                 200                 205
   Ser Glu Asp Val Pro His Tyr Asp Asp Phe Thr Gln Ala Trp Asp Arg
       210                 215                 220
   Cys Gln Asp Tyr Met Met Leu Arg Leu Leu Leu Gly Asp Phe Tyr Trp
   225                 230                 235                 240
   Ile Ala Asn Asp Trp Arg Tyr Lys Gln Ser Asn Gln Ile Val Gln Ala
                   245                 250                 255
   Phe Cys Asp Tyr Leu Val Gln Lys Ser Leu Glu Asn Thr Cys Asn Asp
               260                 265                 270
   Lys Phe Val Phe Val Gln Glu Leu Ala Lys His Thr Thr Asn Lys Thr
           275                 280                 285
   Phe Ile Arg Asp Gln Ala Leu Ser Leu Ile Met Ala Ser Arg Asp Thr
       290                 295                 300
   Thr Ala Glu Leu Met Ala Phe Thr Ile Leu Glu Leu Ser Arg Asn Pro
   305                 310                 315                 320
   Thr Ile Trp Glu Arg Leu Arg Glu Glu Ile Asp Ala Asn Phe Gly Leu
                   325                 330                 335
   Glu Ser Pro Asp Leu Leu Thr Phe Asp Ser Leu Arg Lys Phe Lys Tyr
               340                 345                 350
   Val Gln Ala Ile Leu Asn Glu Thr Leu Arg Met Tyr Pro Gly Val Pro
           355                 360                 365
   Arg Asn Met Lys Thr Ala Lys Cys Thr Thr Thr Leu Pro Lys Gly Gly
   370 375 380
   Gly Lys Asp Gly Gln Asp Pro Ile Leu Val Lys Lys Gly Gln Ser Val
   385 390 395 400
   Gly Phe Ile Ser Ile Ala Thr His Leu Asp Pro Val Tyr Phe Gly Ser
   405 410 415
   Asp Ala His Val Phe Arg Pro Asp Arg Trp Phe Asp Ser Ser Met Lys
   420 425 430
   Asn Leu Gly Cys Lys Tyr Leu Pro Phe Asn Val Gly Pro Arg Thr Cys
   435 440 445
   Leu Gly Gln Gln Tyr Thr Leu Ile Glu Ala Ser Tyr Leu Leu Val Arg
   450 455 460
   Leu Ala Gln Thr Tyr Glu Thr Val Glu Ser His Pro Asp Ser Val Tyr
   465 470 475 480
   Pro Pro Arg Lys Lys Ala Leu Ile Asn Met Cys Ala Ala Asp Gly Val
   485 490 495
   Asp Val Lys Phe His Arg Leu
   500
<210> 39
   <211> 1554
   <212> DNA
   <213> Candida tropicalis
<220>
   <221> CDS
   <222> (1)..(1554)
<400> 39
   atg atc gaa caa gtt gtt gaa tac tgg tac gtg gtc tta cca ttg gta       48
   Met Ile Glu Gln Val Val Glu Tyr Trp Tyr Val Val Leu Pro Leu Val
   1               5                   10                  15
   ttt atc ctt cat aaa gta ttt gac atg tgg cac act cgt cgg ttg atg       96
   Phe Ile Leu His Lys Val Phe Asp Met Trp His Thr Arg Arg Leu Met
               20                  25                  30
   aag caa ttg ggc gct gct cct gtc aca aac caa tta cac gac aat ttt      144
   Lys Gln Leu Gly Ala Ala Pro Val Thr Asn Gln Leu His Asp Asn Phe
           35                  40                  45
   ttt ggt att atc aac gga tgg aaa gca ctt aag ttc aag aaa gaa ggt      192
   Phe Gly Ile Ile Asn Gly Trp Lys Ala Leu Lys Phe Lys Lys Glu Gly
       50                  55                  60
   aga gct caa gaa tat aat gat tat aaa ttt gcc aat tcg aaa att cca      240
   Arg Ala Gln Glu Tyr Asn Asp Tyr Lys Phe Ala Asn Ser Lys Ile Pro
   65                  70                  75                  80
   agt gtg ggt act tat gtt agt acc atc ttt gga aca aag ctc ctc gtc      288
   Ser Val Gly Thr Tyr Val Ser Thr Ile Phe Gly Thr Lys Leu Leu Val
                   85                  90                  95
   aca aaa gat ccg gag aat atc aaa gct tta tta gca acc caa ttc agt      336
   Thr Lys Asp Pro Glu Asn Ile Lys Ala Leu Leu Ala Thr Gln Phe Ser
               100                 105                 110
   gat ttt tcc ttg ggt aag agg cat aca ctt ttc aaa cca tta tta ggt      384
   Asp Phe Ser Leu Gly Lys Arg His Thr Leu Phe Lys Pro Leu Leu Gly
           115                 120                 125
   gac ggt att ttc act ttg gat gga gaa ggt tgg aaa cat agt cga gct      432
   Asp Gly Ile Phe Thr Leu Asp Gly Glu Gly Trp Lys His Ser Arg Ala
       130                 135                 140
   atg ttg aga cca cag ttt gca aga gaa caa gtt gcc cat gtc act tct      480
   Met Leu Arg Pro Gln Phe Ala Arg Glu Gln Val Ala His Val Thr Ser
   145                 150                 155                 160
   tta gag cca cat ttc caa ttg ttg aaa aaa cat atc ctc aag aac aaa      528
   Leu Glu Pro His Phe Gln Leu Leu Lys Lys His Ile Leu Lys Asn Lys
                   165                 170                 175
   gga ggt ttt ttt gat atc cag gaa ttg ttt ttc cga ttc act gtt gat      576
   Gly Gly Phe Phe Asp Ile Gln Glu Leu Phe Phe Arg Phe Thr Val Asp
               180                 185                 190
   tca gct act gag ttt ttg ttt ggt gaa tca gta cac tct ttg aag gat      624
   Ser Ala Thr Glu Phe Leu Phe Gly Glu Ser Val His Ser Leu Lys Asp
           195                 200                 205
   gaa aca att ggg tat aac caa gat gat atc gac ttt gtt ggt aga aag      672
   Glu Thr Ile Gly Tyr Asn Gln Asp Asp Ile Asp Phe Val Gly Arg Lys
       210                 215                 220
   gat ttt gcg gaa tcg ttc aac aag gca caa gag tat ctt gct att aga      720
   Asp Phe Ala Glu Ser Phe Asn Lys Ala Gln Glu Tyr Leu Ala Ile Arg
   225                 230                 235                 240
   act ttg gtg caa gat ttt tat tat ctt gta aac aat cag gaa ttc aga      768
   Thr Leu Val Gln Asp Phe Tyr Tyr Leu Val Asn Asn Gln Glu Phe Arg
                   245                 250                 255
   gat tgc aat aaa ctg gta cac aag ttt acc aac tac tat gtc caa aga      816
   Asp Cys Asn Lys Leu Val His Lys Phe Thr Asn Tyr Tyr Val Gln Arg
               260                 265                 270
   gca ttg gat gcc act cct gaa gag ctt gaa aag caa agt gga tat gtt      864
   Ala Leu Asp Ala Thr Pro Glu Glu Leu Glu Lys Gln Ser Gly Tyr Val
           275                 280                 285
   ttc ttg tat gaa ttg gtt aaa caa acc aga gac cct aat gta ttg aga      912
   Phe Leu Tyr Glu Leu Val Lys Gln Thr Arg Asp Pro Asn Val Leu Arg
       290                 295                 300
   gat caa tca ttg aac atc tta tta gct ggt aga gat acc act gct ggg      960
   Asp Gln Ser Leu Asn Ile Leu Leu Ala Gly Arg Asp Thr Thr Ala Gly
   305                 310                 315                 320
   ttg ttg tca ttt gcg gta ttt gaa ctt gct agg aat cca cat att tgg      1008
   Leu Leu Ser Phe Ala Val Phe Glu Leu Ala Arg Asn Pro His Ile Trp
                   325                 330                 335
   gcc aaa tta aga gaa gat gtt gaa tcc caa ttt ggt ctt ggt gaa gaa      1056
   Ala Lys Leu Arg Glu Asp Val Glu Ser Gln Phe Gly Leu Gly Glu Glu
               340                 345                 350
   tct cgc att gaa gag att acc ttt gaa agt tta aaa cga tgt gaa tac      1104
   Ser Arg Ile Glu Glu Ile Thr Phe Glu Ser Leu Lys Arg Cys Glu Tyr
           355                 360                 365
   ttg aaa gct ttc ctt aac gaa aca tta cgt gtt tat cca agt gtt cca      1152
   Leu Lys Ala Phe Leu Asn Glu Thr Leu Arg Val Tyr Pro Ser Val Pro
       370                 375                 380
   aga aat ttc aga att gct acc aaa aac acc act tta cca aga ggt ggt      1200
   Arg Asn Phe Arg Ile Ala Thr Lys Asn Thr Thr Leu Pro Arg Gly Gly
   385                 390                 395                 400
   ggt tca gac ggc aat tct cct gtt ttg gtc aaa aag ggc gag gct gtt      1248
   Gly Ser Asp Gly Asn Ser Pro Val Leu Val Lys Lys Gly Glu Ala Val
                   405                 410                 415
   tca tat ggt ata aat tct act cac tta gat cct gtc tat tat ggt gac      1296
   Ser Tyr Gly Ile Asn Ser Thr His Leu Asp Pro Val Tyr Tyr Gly Asp
               420                 425                 430
   gat gct gca gaa ttt aga cca gaa aga tgg aac gag cca tca aca aga      1344
   Asp Ala Ala Glu Phe Arg Pro Glu Arg Trp Asn Glu Pro Ser Thr Arg
           435                 440                 445
   aaa ttg gga tgg gca tat tta ccg ttc aac gga ggc cca aga att tgt      1392
   Lys Leu Gly Trp Ala Tyr Leu Pro Phe Asn Gly Gly Pro Arg Ile Cys
       450                 455                 460
   tta ggt caa caa ttt gct tta acc gaa gcg ggt tat gta ttg gtt aga      1440
   Leu Gly Gln Gln Phe Ala Leu Thr Glu Ala Gly Tyr Val Leu Val Arg
   465                 470                 475                 480
   ttg gcc caa agt ttt gac acc ttg gaa ttg aag cca cca gtt gtg tat      1488
   Leu Ala Gln Ser Phe Asp Thr Leu Glu Leu Lys Pro Pro Val Val Tyr
                   485                 490                 495
   cca cca aag aga tta aca aac ttg act atg tct tta caa gac gga act      1536
   Pro Pro Lys Arg Leu Thr Asn Leu Thr Met Ser Leu Gln Asp Gly Thr
               500                 505                 510
   att gtc aag atc gat tag                                              1554
   Ile Val Lys Ile Asp
           515
<210> 40
   <211> 517
   <212> PRT
   <213> Candida tropicalis
<400> 40
   Met Ile Glu Gln Val Val Glu Tyr Trp Tyr Val Val Leu Pro Leu Val
   1               5                   10                  15
   Phe Ile Leu His Lys Val Phe Asp Met Trp His Thr Arg Arg Leu Met
               20                  25                  30
   Lys Gln Leu Gly Ala Ala Pro Val Thr Asn Gln Leu His Asp Asn Phe
           35                  40                  45
   Phe Gly Ile Ile Asn Gly Trp Lys Ala Leu Lys Phe Lys Lys Glu Gly
       50                  55                  60
   Arg Ala Gln Glu Tyr Asn Asp Tyr Lys Phe Ala Asn Ser Lys Ile Pro
   65                  70                  75                  80
   Ser Val Gly Thr Tyr Val Ser Thr Ile Phe Gly Thr Lys Leu Leu Val
                   85                  90                  95
   Thr Lys Asp Pro Glu Asn Ile Lys Ala Leu Leu Ala Thr Gln Phe Ser
               100                 105                 110
   Asp Phe Ser Leu Gly Lys Arg His Thr Leu Phe Lys Pro Leu Leu Gly
           115                 120                 125
   Asp Gly Ile Phe Thr Leu Asp Gly Glu Gly Trp Lys His Ser Arg Ala
       130                 135                 140
   Met Leu Arg Pro Gln Phe Ala Arg Glu Gln Val Ala His Val Thr Ser
   145                 150                 155                 160
   Leu Glu Pro His Phe Gln Leu Leu Lys Lys His Ile Leu Lys Asn Lys
                   165                 170                 175
   Gly Gly Phe Phe Asp Ile Gln Glu Leu Phe Phe Arg Phe Thr Val Asp
               180                 185                 190
   Ser Ala Thr Glu Phe Leu Phe Gly Glu Ser Val His Ser Leu Lys Asp
           195                 200                 205
   Glu Thr Ile Gly Tyr Asn Gln Asp Asp Ile Asp Phe Val Gly Arg Lys
       210                 215                 220
   Asp Phe Ala Glu Ser Phe Asn Lys Ala Gln Glu Tyr Leu Ala Ile Arg
   225                 230                 235                 240
   Thr Leu Val Gln Asp Phe Tyr Tyr Leu Val Asn Asn Gln Glu Phe Arg
                   245                 250                 255
   Asp Cys Asn Lys Leu Val His Lys Phe Thr Asn Tyr Tyr Val Gln Arg
               260                 265                 270
   Ala Leu Asp Ala Thr Pro Glu Glu Leu Glu Lys Gln Ser Gly Tyr Val
           275                 280                 285
   Phe Leu Tyr Glu Leu Val Lys Gln Thr Arg Asp Pro Asn Val Leu Arg
       290                 295                 300
   Asp Gln Ser Leu Asn Ile Leu Leu Ala Gly Arg Asp Thr Thr Ala Gly
   305                 310                 315                 320
   Leu Leu Ser Phe Ala Val Phe Glu Leu Ala Arg Asn Pro His Ile Trp
                   325                 330                 335
   Ala Lys Leu Arg Glu Asp Val Glu Ser Gln Phe Gly Leu Gly Glu Glu
               340                 345                 350
   Ser Arg Ile Glu Glu Ile Thr Phe Glu Ser Leu Lys Arg Cys Glu Tyr
           355                 360                 365
   Leu Lys Ala Phe Leu Asn Glu Thr Leu Arg Val Tyr Pro Ser Val Pro
       370                 375                 380
   Arg Asn Phe Arg Ile Ala Thr Lys Asn Thr Thr Leu Pro Arg Gly Gly
   385                 390                 395                 400
   Gly Ser Asp Gly Asn Ser Pro Val Leu Val Lys Lys Gly Glu Ala Val
                   405                 410                 415
   Ser Tyr Gly Ile Asn Ser Thr His Leu Asp Pro Val Tyr Tyr Gly Asp
               420                 425                 430
   Asp Ala Ala Glu Phe Arg Pro Glu Arg Trp Asn Glu Pro Ser Thr Arg
           435                 440                 445
   Lys Leu Gly Trp Ala Tyr Leu Pro Phe Asn Gly Gly Pro Arg Ile Cys
       450                 455                 460
   Leu Gly Gln Gln Phe Ala Leu Thr Glu Ala Gly Tyr Val Leu Val Arg
   465                 470                 475                 480
   Leu Ala Gln Ser Phe Asp Thr Leu Glu Leu Lys Pro Pro Val Val Tyr
                   485                 490                 495
   Pro Pro Lys Arg Leu Thr Asn Leu Thr Met Ser Leu Gln Asp Gly Thr
               500                 505                 510
   Ile Val Lys Ile Asp
                   515
<210> 41
   <211> 1539
   <212> DNA
   <213> Candida tropicalis
<220>
   <221> CDS
   <222> (1)..(1539)
<400> 41
   atg att gaa cag gtt tta cat tat tgg tat att gtt tta cct gca ttt      48
   Met Ile Glu Gln Val Leu His Tyr Trp Tyr Ile Val Leu Pro Ala Phe
   1               5                   10                  15
   ata att ttt cat tgg att gta tct gca att cat aca aat tcc ttg cgt      96
   Ile Ile Phe His Trp Ile Val Ser Ala Ile His Thr Asn Ser Leu Arg
               20                  25                  30
   aga aaa cta ggt gcc aaa cct ttc act cat aca caa ctt gat ggt ttt     144
   Arg Lys Leu Gly Ala Lys Pro Phe Thr His Thr Gln Leu Asp Gly Phe
           35                  40                  45
   tat gga ttt aaa ttt ggc cgt gat ttt ctt aaa gct aaa agg att ggt     192
   Tyr Gly Phe Lys Phe Gly Arg Asp Phe Leu Lys Ala Lys Arg Ile Gly
       50                  55                  60
   agg caa gtt gat tta atc aat tcc cgt ttc cca gac gat att gac aca     240
   Arg Gln Val Asp Leu Ile Asn Ser Arg Phe Pro Asp Asp Ile Asp Thr
   65                  70                  75                  80
   ttt tca agt tat act ttc ggc aac cac gtg att ttt aca cgt gat cca     288
   Phe Ser Ser Tyr Thr Phe Gly Asn His Val Ile Phe Thr Arg Asp Pro
                   85                  90                  95
   gaa aat atc aaa gct tta ttg gca aca caa ttt aat gat ttt tca tta     336
   Glu Asn Ile Lys Ala Leu Leu Ala Thr Gln Phe Asn Asp Phe Ser Leu
               100                 105                 110
   ggt ggt aga att aag ttt ttc aaa cca ttg ttg gga tat gga atc ttt     384
   Gly Gly Arg Ile Lys Phe Phe Lys Pro Leu Leu Gly Tyr Gly Ile Phe
           115                 120                 125
   act ttg gat gga gaa ggt tgg aaa cat agt cga gct atg ttg aga cca     432
   Thr Leu Asp Gly Glu Gly Trp Lys His Ser Arg Ala Met Leu Arg Pro
       130                 135                 140
   cag ttt gca aga gaa caa gtt gcc cat gtc act tct tta gaa cca cat     480
   Gln Phe Ala Arg Glu Gln Val Ala His Val Thr Ser Leu Glu Pro His
   145                 150                 155                 160
   ttc caa ttg ttg aaa aag cat atc ctc aag aac aaa ggt ggg ttt ttt     528
   Phe Gln Leu Leu Lys Lys His Ile Leu Lys Asn Lys Gly Gly Phe Phe
                   165                 170                 175
   gat atc cag gaa ttg ttt ttc cga ttc acc gtt gat tca gct act gag     576
   Asp Ile Gln Glu Leu Phe Phe Arg Phe Thr Val Asp Ser Ala Thr Glu
               180                 185                 190
   ttt ttg ttt ggt gaa tca gtg aac tct ttg aaa agt gca tca att ggt      624
   Phe Leu Phe Gly Glu Ser Val Asn Ser Leu Lys Ser Ala Ser Ile Gly
           195                 200                 205
   tgt gac gag gaa acc gag ctt gag gaa aga aag aaa ttt gcg gaa gca      672
   Cys Asp Glu Glu Thr Glu Leu Glu Glu Arg Lys Lys Phe Ala Glu Ala
       210                 215                 220
   ttc aat aaa gcg caa gag tat att tct act cga gtt gct ttg caa caa      720
   Phe Asn Lys Ala Gln Glu Tyr Ile Ser Thr Arg Val Ala Leu Gln Gln
   225                 230                 235                 240
   tta tat tgg ttt gtt aat aat agc gaa ttc aag gaa tgt aac gaa att      768
   Leu Tyr Trp Phe Val Asn Asn Ser Glu Phe Lys Glu Cys Asn Glu Ile
                   245                 250                 255
   gtt cat aag ttt acc aat tat tat gta caa aag gca ttg gat gct act      816
   Val His Lys Phe Thr Asn Tyr Tyr Val Gln Lys Ala Leu Asp Ala Thr
               260                 265                 270
   cct gaa gag ctt gaa aag caa agt gga tat gtt ttc ttg tat gaa ttg      864
   Pro Glu Glu Leu Glu Lys Gln Ser Gly Tyr Val Phe Leu Tyr Glu Leu
           275                 280                 285
   gtt aaa caa acc aga gac cct aat gta ttg aga gat caa tca ttg aat      912
   Val Lys Gln Thr Arg Asp Pro Asn Val Leu Arg Asp Gln Ser Leu Asn
       290                 295                 300
   atc tta tta gct ggt aga gat acc act gct ggg ttg ttg tca ttt gcg      960
   Ile Leu Leu Ala Gly Arg Asp Thr Thr Ala Gly Leu Leu Ser Phe Ala
   305                 310                 315                 320
   gta ttt gaa ctt gct agg aat cca cat att tgg gcc aaa tta aga gaa     1008
   Val Phe Glu Leu Ala Arg Asn Pro His Ile Trp Ala Lys Leu Arg Glu
                   325                 330                 335
   gat gtc gaa tcc caa ttt ggt ctt ggt gaa gaa tct cgc att gaa gag     1056
   Asp Val Glu Ser Gln Phe Gly Leu Gly Glu Glu Ser Arg Ile Glu Glu
               340                 345                 350
   att acc ttt gaa agt tta aaa cga tgt gaa tat ttg aaa gcc gtg atg     1104
   Ile Thr Phe Glu Ser Leu Lys Arg Cys Glu Tyr Leu Lys Ala Val Met
           355                 360                 365
   aat gaa aca ttg aga ttg cat cca agt gtt cca aga aat gct aga ttt     1152
   Asn Glu Thr Leu Arg Leu His Pro Ser Val Pro Arg Asn Ala Arg Phe
       370                 375                 380
   gca ctt aag gat aca act tta cct aga ggt gga ggt cca gat gga aaa     1200
   Ala Leu Lys Asp Thr Thr Leu Pro Arg Gly Gly Gly Pro Asp Gly Lys
   385                 390                 395                 400
   gac ccg att tta gtt aga aaa aat gaa gtt gtt caa tat tcc att tct     1248
   Asp Pro Ile Leu Val Arg Lys Asn Glu Val Val Gln Tyr Ser Ile Ser
                   405                 410                 415
   ggc aca caa att gat cca aaa cat tat ggc aaa gat gct aaa ttg ttt     1296
   Gly Thr Gln Ile Asp Pro Lys His Tyr Gly Lys Asp Ala Lys Leu Phe
               420                 425                 430
   aga cca gaa aga tgg ttt gaa tca agt aca aga aat tta ggt tgg gca     1344
   Arg Pro Glu Arg Trp Phe Glu Ser Ser Thr Arg Asn Leu Gly Trp Ala
           435                 440                 445
   tac tta cca ttc aac ggg ggt ccg aga att tgt tta ggt caa caa ttt     1392
   Tyr Leu Pro Phe Asn Gly Gly Pro Arg Ile Cys Leu Gly Gln Gln Phe
       450                 455                 460
   gct tta acc gaa gca ggt tac ata ttg gtt aga ttg gct caa agt ttt     1440
   Ala Leu Thr Glu Ala Gly Tyr Ile Leu Val Arg Leu Ala Gln Ser Phe
   465                 470                 475                 480
   gac acc ttg gaa ttg aaa cca gat aca gaa tac cct cca cca aga tta     1488
   Asp Thr Leu Glu Leu Lys Pro Asp Thr Glu Tyr Pro Pro Pro Arg Leu
                   485                 490                 495
   gcc cat ttg act atg tgt ttg ttt gat ggt gcg ctt gtc aag atg gat     1536
   Ala His Leu Thr Met Cys Leu Phe Asp Gly Ala Leu Val Lys Met Asp
               500                 505                 510
   taa                                                                 1539
<210> 42
   <211> 512
   <212> PRT
   <213> Candida tropicalis
<400> 42
   Met Ile Glu Gln Val Leu His Tyr Trp Tyr Ile Val Leu Pro Ala Phe
   1               5                   10                  15
   Ile Ile Phe His Trp Ile Val Ser Ala Ile His Thr Asn Ser Leu Arg
               20                  25                  30
   Arg Lys Leu Gly Ala Lys Pro Phe Thr His Thr Gln Leu Asp Gly Phe
           35                  40                  45
   Tyr Gly Phe Lys Phe Gly Arg Asp Phe Leu Lys Ala Lys Arg Ile Gly
       50                  55                  60
   Arg Gln Val Asp Leu Ile Asn Ser Arg Phe Pro Asp Asp Ile Asp Thr
   65                  70                  75                  80
   Phe Ser Ser Tyr Thr Phe Gly Asn His Val Ile Phe Thr Arg Asp Pro
                   85                  90                  95
   Glu Asn Ile Lys Ala Leu Leu Ala Thr Gln Phe Asn Asp Phe Ser Leu
               100                 105                 110
   Gly Gly Arg Ile Lys Phe Phe Lys Pro Leu Leu Gly Tyr Gly Ile Phe
           115                 120                 125
   Thr Leu Asp Gly Glu Gly Trp Lys His Ser Arg Ala Met Leu Arg Pro
       130                 135                 140
   Gln Phe Ala Arg Glu Gln Val Ala His Val Thr Ser Leu Glu Pro His
   145 150 155 160
   Phe Gln Leu Leu Lys Lys His Ile Leu Lys Asn Lys Gly Gly Phe Phe
   165 170 175
   Asp Ile Gln Glu Leu Phe Phe Arg Phe Thr Val Asp Ser Ala Thr Glu
   180 185 190
   Phe Leu Phe Gly Glu Ser Val Asn Ser Leu Lys Ser Ala Ser Ile Gly
   195 200 205
   Cys Asp Glu Glu Thr Glu Leu Glu Glu Arg Lys Lys Phe Ala Glu Ala
   210 215 220
   Phe Asn Lys Ala Gln Glu Tyr Ile Ser Thr Arg Val Ala Leu Gln Gln
   225 230 235 240
   Leu Tyr Trp Phe Val Asn Asn Ser Glu Phe Lys Glu Cys Asn Glu Ile
   245 250 255
   Val His Lys Phe Thr Asn Tyr Tyr Val Gln Lys Ala Leu Asp Ala Thr
   260 265 270
   Pro Glu Glu Leu Glu Lys Gln Ser Gly Tyr Val Phe Leu Tyr Glu Leu
   275 280 285
   Val Lys Gln Thr Arg Asp Pro Asn Val Leu Arg Asp Gln Ser Leu Asn
   290 295 300
   Ile Leu Leu Ala Gly Arg Asp Thr Thr Ala Gly Leu Leu Ser Phe Ala
   305 310 315 320
   Val Phe Glu Leu Ala Arg Asn Pro His Ile Trp Ala Lys Leu Arg Glu
   325 330 335
   Asp Val Glu Ser Gln Phe Gly Leu Gly Glu Glu Ser Arg Ile Glu Glu
   340 345 350
   Ile Thr Phe Glu Ser Leu Lys Arg Cys Glu Tyr Leu Lys Ala Val Met
   355 360 365
   Asn Glu Thr Leu Arg Leu His Pro Ser Val Pro Arg Asn Ala Arg Phe
   370 375 380
   Ala Leu Lys Asp Thr Thr Leu Pro Arg Gly Gly Gly Pro Asp Gly Lys
   385 390 395 400
   Asp Pro Ile Leu Val Arg Lys Asn Glu Val Val Gln Tyr Ser Ile Ser
                   405                 410                 415
   Gly Thr Gln Ile Asp Pro Lys His Tyr Gly Lys Asp Ala Lys Leu Phe
               420                 425                 430
   Arg Pro Glu Arg Trp Phe Glu Ser Ser Thr Arg Asn Leu Gly Trp Ala
           435                 440                 445
   Tyr Leu Pro Phe Asn Gly Gly Pro Arg Ile Cys Leu Gly Gln Gln Phe
       450                 455                 460
   Ala Leu Thr Glu Ala Gly Tyr Ile Leu Val Arg Leu Ala Gln Ser Phe
   465                 470                 475                 480
   Asp Thr Leu Glu Leu Lys Pro Asp Thr Glu Tyr Pro Pro Pro Arg Leu
                   485                 490                 495
   Ala His Leu Thr Met Cys Leu Phe Asp Gly Ala Leu Val Lys Met Asp
               500                 505                 510
<210> 43
   <211> 1572
   <212> DNA
   <213> Candida tropicalis
<220>
   <221> CDS
   <222> (1)..(1572)
<400> 43
   atg att aaa gaa ata gtg tat ttt gta tat tat tat gga att acc cac      48
   Met Ile Lys Glu Ile Val Tyr Phe Val Tyr Tyr Tyr Gly Ile Thr His
   1               5                   10                  15
   tca gtt tca gtg cag gtc acc gct ctt gtc ctt atc gtg aca tac ttt      96
   Ser Val Ser Val Gln Val Thr Ala Leu Val Leu Ile Val Thr Tyr Phe
               20                  25                  30
   ttt gtt att cgt cca att aac tca cct tta tgg aga gtt ccc gga cca     144
   Phe Val Ile Arg Pro Ile Asn Ser Pro Leu Trp Arg Val Pro Gly Pro
           35                  40                  45
   tat tta cat cgg gta act tac ttt cca tgt tta aat gcc caa cga aag     192
   Tyr Leu His Arg Val Thr Tyr Phe Pro Cys Leu Asn Ala Gln Arg Lys
       50                  55                  60
   gga gaa tgg atc tcc aaa gtt tat gat ttg cac aag aaa tat ggt gat     240
   Gly Glu Trp Ile Ser Lys Val Tyr Asp Leu His Lys Lys Tyr Gly Asp
   65                  70                  75                  80
   gta gtt tta ctt tct cca aat gaa atc agc gtc aat ggt gac cca aaa     288
   Val Val Leu Leu Ser Pro Asn Glu Ile Ser Val Asn Gly Asp Pro Lys
                   85                  90                  95
   tat ttg act gat att tat gta aag aac ctc cca aag tca aag ttt tat      336
   Tyr Leu Thr Asp Ile Tyr Val Lys Asn Leu Pro Lys Ser Lys Phe Tyr
               100                 105                 110
   gaa aac ttt aga aat cat gga ttc cag gat aat att ttt gcc agt ttg      384
   Glu Asn Phe Arg Asn His Gly Phe Gln Asp Asn Ile Phe Ala Ser Leu
           115                 120                 125
   gaa aat gat aga cat atc aag tat aaa aga atg ata aat aac ttg tac      432
   Glu Asn Asp Arg His Ile Lys Tyr Lys Arg Met Ile Asn Asn Leu Tyr
       130                 135                 140
   agt aaa tct tcc atc ttc tcc aaa gaa aac cac aca aga tca gtt tta      480
   Ser Lys Ser Ser Ile Phe Ser Lys Glu Asn His Thr Arg Ser Val Leu
   145                 150                 155                 160
   ttt gac acc aca aaa aca tta gtc gat gca gtt gct aga gaa tct ccg      528
   Phe Asp Thr Thr Lys Thr Leu Val Asp Ala Val Ala Arg Glu Ser Pro
                   165                 170                 175
   tca att gat gtg ttt acg tta ttt ggt tcc ttg gcc atg gat gtg gtt      576
   Ser Ile Asp Val Phe Thr Leu Phe Gly Ser Leu Ala Met Asp Val Val
               180                 185                 190
   tca agg ttt gaa ctt ggt aga gac aat gga acg gat tta ttg aat cat      624
   Ser Arg Phe Glu Leu Gly Arg Asp Asn Gly Thr Asp Leu Leu Asn His
           195                 200                 205
   cca caa gaa agg cat att att gaa tct cat aga aag gtg tct tgt atg      672
   Pro Gln Glu Arg His Ile Ile Glu Ser His Arg Lys Val Ser Cys Met
       210                 215                 220
   ggg ttc tgg aca aca ttg atg cca agc ttc ttt tgg aat ttg gca gca      720
   Gly Phe Trp Thr Thr Leu Met Pro Ser Phe Phe Trp Asn Leu Ala Ala
   225                 230                 235                 240
   acc aag gcc acc ttg caa gct gtt gat gat att tgc aat ttc caa ttg      768
   Thr Lys Ala Thr Leu Gln Ala Val Asp Asp Ile Cys Asn Phe Gln Leu
                   245                 250                 255
   ggt tta tat aaa att gca gaa tcc aat ctt gtg tcc aat ggt aaa aac      816
   Gly Leu Tyr Lys Ile Ala Glu Ser Asn Leu Val Ser Asn Gly Lys Asn
               260                 265                 270
   cta act acg ata caa aca ctc aag aag tac gga ttg gaa gga aat tct      864
   Leu Thr Thr Ile Gln Thr Leu Lys Lys Tyr Gly Leu Glu Gly Asn Ser
           275                 280                 285
   gcg tat tct ttt ctc acg gat aat tta ttt gct gga cat gaa act aca      912
   Ala Tyr Ser Phe Leu Thr Asp Asn Leu Phe Ala Gly His Glu Thr Thr
       290                 295                 300
   gct gtt caa ttg aca tat ttg tgt tat gaa tta tca aga cca gct aat      960
   Ala Val Gln Leu Thr Tyr Leu Cys Tyr Glu Leu Ser Arg Pro Ala Asn
   305                 310                 315                 320
   tac aaa att cag aat aga tta aga tat gag ctt caa gaa gca ttt cca     1008
   Tyr Lys Ile Gln Asn Arg Leu Arg Tyr Glu Leu Gln Glu Ala Phe Pro
                   325                 330                 335
   agt ggt caa att gaa gat ttg gaa gtg gtt gat aat ctt ccg tat ctt     1056
   Ser Gly Gln Ile Glu Asp Leu Glu Val Val Asp Asn Leu Pro Tyr Leu
               340                 345                 350
   aat gcg ttg ttg tcc gaa aat ggt cga att cac acc tca att cct gga      1104
   Asn Ala Leu Leu Ser Glu Asn Gly Arg Ile His Thr Ser Ile Pro Gly
           355                 360                 365
   gct gag cca cgt gtg gtt gca aaa ccg tat act att ggc aaa ttg ctt      1152
   Ala Glu Pro Arg Val Val Ala Lys Pro Tyr Thr Ile Gly Lys Leu Leu
       370                 375                 380
   att cca gtt ggt acg gtt atc tct tgt ctt cct tat gcg tat cat aga      1200
   Ile Pro Val Gly Thr Val Ile Ser Cys Leu Pro Tyr Ala Tyr His Arg
   385                 390                 395                 400
   aat ccg tcg gta ttt acc aat cct gat aaa ttt atc ccc gaa aga tgg      1248
   Asn Pro Ser Val Phe Thr Asn Pro Asp Lys Phe Ile Pro Glu Arg Trp
                   405                 410                 415
   tta gtt gac aac gaa gaa gac aag aaa cga gtc aaa caa caa gcg aag      1296
   Leu Val Asp Asn Glu Glu Asp Lys Lys Arg Val Lys Gln Gln Ala Lys
               420                 425                 430
   tat atg atg cca ttt ggt aaa ggt gta aga atg tgt ctt ggg atg aac      1344
   Tyr Met Met Pro Phe Gly Lys Gly Val Arg Met Cys Leu Gly Met Asn
           435                 440                 445
   ttg gca ctc att gaa atg aag ttg gcc att gca agt ttg tat tta aac      1392
   Leu Ala Leu Ile Glu Met Lys Leu Ala Ile Ala Ser Leu Tyr Leu Asn
       450                 455                 460
   ttt tct tct tcc att gac gaa gac tgg tgt ggc aaa gta tta gaa aat      1440
   Phe Ser Ser Ser Ile Asp Glu Asp Trp Cys Gly Lys Val Leu Glu Asn
   465                 470                 475                 480
   gat gac ccc ata ggt atc ggg aat tca tgc act cat gag act gat cag      1488
   Asp Asp Pro Ile Gly Ile Gly Asn Ser Cys Thr His Glu Thr Asp Gln
                   485                 490                 495
   gat aaa atg aaa atg tac gat gct tat act acg aga cca atg cta gat      1536
   Asp Lys Met Lys Met Tyr Asp Ala Tyr Thr Thr Arg Pro Met Leu Asp
               500                 505                 510
   gaa tgt tac ttg aag tgg aca aga tta act cct tga                      1572
   Glu Cys Tyr Leu Lys Trp Thr Arg Leu Thr Pro
           515                 520
<210> 44
   <211> 523
   <212> PRT
   <213> Candida tropicalis
<400> 44
   Met Ile Lys Glu Ile Val Tyr Phe Val Tyr Tyr Tyr Gly Ile Thr His
   1               5                   10                  15
   Ser Val Ser Val Gln Val Thr Ala Leu Val Leu Ile Val Thr Tyr Phe
               20                  25                  30
   Phe Val Ile Arg Pro Ile Asn Ser Pro Leu Trp Arg Val Pro Gly Pro
           35                  40                  45
   Tyr Leu His Arg Val Thr Tyr Phe Pro Cys Leu Asn Ala Gln Arg Lys
       50                  55                  60
   Gly Glu Trp Ile Ser Lys Val Tyr Asp Leu His Lys Lys Tyr Gly Asp
   65                  70                  75                  80
   Val Val Leu Leu Ser Pro Asn Glu Ile Ser Val Asn Gly Asp Pro Lys
                   85                  90                  95
   Tyr Leu Thr Asp Ile Tyr Val Lys Asn Leu Pro Lys Ser Lys Phe Tyr
               100                 105                 110
   Glu Asn Phe Arg Asn His Gly Phe Gln Asp Asn Ile Phe Ala Ser Leu
           115                 120                 125
   Glu Asn Asp Arg His Ile Lys Tyr Lys Arg Met Ile Asn Asn Leu Tyr
       130                 135                 140
   Ser Lys Ser Ser Ile Phe Ser Lys Glu Asn His Thr Arg Ser Val Leu
   145                 150                 155                 160
   Phe Asp Thr Thr Lys Thr Leu Val Asp Ala Val Ala Arg Glu Ser Pro
                   165                 170                 175
   Ser Ile Asp Val Phe Thr Leu Phe Gly Ser Leu Ala Met Asp Val Val
               180                 185                 190
   Ser Arg Phe Glu Leu Gly Arg Asp Asn Gly Thr Asp Leu Leu Asn His
           195                 200                 205
   Pro Gln Glu Arg His Ile Ile Glu Ser His Arg Lys Val Ser Cys Met
       210                 215                 220
   Gly Phe Trp Thr Thr Leu Met Pro Ser Phe Phe Trp Asn Leu Ala Ala
   225                 230                 235                 240
   Thr Lys Ala Thr Leu Gln Ala Val Asp Asp Ile Cys Asn Phe Gln Leu
                   245                 250                 255
   Gly Leu Tyr Lys Ile Ala Glu Ser Asn Leu Val Ser Asn Gly Lys Asn
               260                 265                 270
   Leu Thr Thr Ile Gln Thr Leu Lys Lys Tyr Gly Leu Glu Gly Asn Ser
           275                 280                 285
   Ala Tyr Ser Phe Leu Thr Asp Asn Leu Phe Ala Gly His Glu Thr Thr
   290 295 300
   Ala Val Gln Leu Thr Tyr Leu Cys Tyr Glu Leu Ser Arg Pro Ala Asn
   305 310 315 320
   Tyr Lys Ile Gln Asn Arg Leu Arg Tyr Glu Leu Gln Glu Ala Phe Pro
   325 330 335
   Ser Gly Gln Ile Glu Asp Leu Glu Val Val Asp Asn Leu Pro Tyr Leu
   340 345 350
   Asn Ala Leu Leu Ser Glu Asn Gly Arg Ile His Thr Ser Ile Pro Gly
   355 360 365
   Ala Glu Pro Arg Val Val Ala Lys Pro Tyr Thr Ile Gly Lys Leu Leu
   370 375 380
   Ile Pro Val Gly Thr Val Ile Ser Cys Leu Pro Tyr Ala Tyr His Arg
   385 390 395 400
   Asn Pro Ser Val Phe Thr Asn Pro Asp Lys Phe Ile Pro Glu Arg Trp
   405 410 415
   Leu Val Asp Asn Glu Glu Asp Lys Lys Arg Val Lys Gln Gln Ala Lys
   420 425 430
   Tyr Met Met Pro Phe Gly Lys Gly Val Arg Met Cys Leu Gly Met Asn
   435 440 445
   Leu Ala Leu Ile Glu Met Lys Leu Ala Ile Ala Ser Leu Tyr Leu Asn
   450 455 460
   Phe Ser Ser Ser Ile Asp Glu Asp Trp Cys Gly Lys Val Leu Glu Asn
   465 470 475 480
   Asp Asp Pro Ile Gly Ile Gly Asn Ser Cys Thr His Glu Thr Asp Gln
   485 490 495
   Asp Lys Met Lys Met Tyr Asp Ala Tyr Thr Thr Arg Pro Met Leu Asp
   500 505 510
   Glu Cys Tyr Leu Lys Trp Thr Arg Leu Thr Pro
   515 520
<210> 45
   <211> 1728
   <212> DNA
   <213> Candida tropicalis
<220>
   <221> CDS
   <222> (1)..(1728)
<400> 45
   atg ctt ctg agc ata cca tgg gat caa tca ttg cta acg ttg cta aca          48
   Met Leu Leu Ser Ile Pro Trp Asp Gln Ser Leu Leu Thr Leu Leu Thr
   1               5                   10                  15
   tat ctt gat acc cat cca ata gct acg ata ttc acc att att tta acc          96
   Tyr Leu Asp Thr His Pro Ile Ala Thr Ile Phe Thr Ile Ile Leu Thr
               20                  25                  30
   att tta aca att gga att tta ttt gat tat tgt cta tca cct aaa gag         144
   Ile Leu Thr Ile Gly Ile Leu Phe Asp Tyr Cys Leu Ser Pro Lys Glu
           35                  40                  45
   att gcc aat att ttc agt att cct gga gat ttg cca ttt att ggt cat         192
   Ile Ala Asn Ile Phe Ser Ile Pro Gly Asp Leu Pro Phe Ile Gly His
       50                  55                  60
   tta cat tta ata cta gat aat cca gca ttg att tat tta aca tgg tat         240
   Leu His Leu Ile Leu Asp Asn Pro Ala Leu Ile Tyr Leu Thr Trp Tyr
   65                  70                  75                  80
   aaa tta tat aat aaa ctg gtt ttt caa att cgt att gga aat aaa cgt         288
   Lys Leu Tyr Asn Lys Leu Val Phe Gln Ile Arg Ile Gly Asn Lys Arg
                   85                  90                  95
   gta gtt gtt gtt aat tca ttt gat gat gtt gtt gga tta tgg ata aat         336
   Val Val Val Val Asn Ser Phe Asp Asp Val Val Gly Leu Trp Ile Asn
               100                 105                 110
   cat agt tgt caa aat aat tca aga cct tta agt tat act ttt cat gga         384
   His Ser Cys Gln Asn Asn Ser Arg Pro Leu Ser Tyr Thr Phe His Gly
           115                 120                 125
   tta gtt tca gca tta caa ggt ttt aca gtt ggt tct act cct gca agt         432
   Leu Val Ser Ala Leu Gln Gly Phe Thr Val Gly Ser Thr Pro Ala Ser
       130                 135                 140
   tta aca ttt ctg aga aag aag aaa gtt att tca ctg tgt tta aga aag         480
   Leu Thr Phe Leu Arg Lys Lys Lys Val Ile Ser Leu Cys Leu Arg Lys
   145                 150                 155                 160
   aaa gaa att gat gaa aaa gta tgt ctc ata gat aat gaa att tgt gtt         528
   Lys Glu Ile Asp Glu Lys Val Cys Leu Ile Asp Asn Glu Ile Cys Val
                   165                 170                 175
   atg att aaa gaa ata att aaa aag aag gat ata tca act gat gta aat         576
   Met Ile Lys Glu Ile Ile Lys Lys Lys Asp Ile Ser Thr Asp Val Asn
               180                 185                 190
   atg tta cct tat tta caa aaa ttc ata tta aaa aca gct att ctt atg         624
   Met Leu Pro Tyr Leu Gln Lys Phe Ile Leu Lys Thr Ala Ile Leu Met
           195                 200                 205
   agt tat gga att gaa ttg gat tgt tat aat aaa gat gtt aaa tta tgt         672
   Ser Tyr Gly Ile Glu Leu Asp Cys Tyr Asn Lys Asp Val Lys Leu Cys
       210                 215                 220
   caa gag att atc acc gtg gag aat aat atc ata aga tta aga tca cca       720
   Gln Glu Ile Ile Thr Val Glu Asn Asn Ile Ile Arg Leu Arg Ser Pro
   225                 230                 235                 240
   ata tct aat ctt caa gat tct gta cct ttc tta aga cta ata cca tgg       768
   Ile Ser Asn Leu Gln Asp Ser Val Pro Phe Leu Arg Leu Ile Pro Trp
                   245                 250                 255
   ttt aat aat cga gaa ttt gcc ctt cgt tgt gga aat aga agg aat aaa       816
   Phe Asn Asn Arg Glu Phe Ala Leu Arg Cys Gly Asn Arg Arg Asn Lys
               260                 265                 270
   tat atg gat caa tta tat aat agg tta caa aat gga tta gct gaa aat       864
   Tyr Met Asp Gln Leu Tyr Asn Arg Leu Gln Asn Gly Leu Ala Glu Asn
           275                 280                 285
   gat cca aat ata gct aat agt att ctt gga caa tta att ctt aat aat       912
   Asp Pro Asn Ile Ala Asn Ser Ile Leu Gly Gln Leu Ile Leu Asn Asn
       290                 295                 300
   gat aat aat aat tct aac agc tta act agt caa gaa ata caa agt att       960
   Asp Asn Asn Asn Ser Asn Ser Leu Thr Ser Gln Glu Ile Gln Ser Ile
   305                 310                 315                 320
   tgt tta aca tta gta agt gct gga tta gat aat acc cca ctt aat ttg      1008
   Cys Leu Thr Leu Val Ser Ala Gly Leu Asp Asn Thr Pro Leu Asn Leu
                   325                 330                 335
   aat tat ctt att gga ata tta tca caa cca aga ata ggt aag ata ttt      1056
   Asn Tyr Leu Ile Gly Ile Leu Ser Gln Pro Arg Ile Gly Lys Ile Phe
               340                 345                 350
   caa gat aaa gct ata aaa gat att tta aat cat gca aat gga gat att      1104
   Gln Asp Lys Ala Ile Lys Asp Ile Leu Asn His Ala Asn Gly Asp Ile
           355                 360                 365
   att caa gca tgg aat caa ctg aat gaa gaa aat cga gat tgt aaa tat      1152
   Ile Gln Ala Trp Asn Gln Leu Asn Glu Glu Asn Arg Asp Cys Lys Tyr
       370                 375                 380
   att caa gct tta att ctt gaa act tta aga cat ttt aca gta tta cca      1200
   Ile Gln Ala Leu Ile Leu Glu Thr Leu Arg His Phe Thr Val Leu Pro
   385                 390                 395                 400
   tta agt tta cct aga tta act aca aaa cca ata tat tat aaa aat ttt      1248
   Leu Ser Leu Pro Arg Leu Thr Thr Lys Pro Ile Tyr Tyr Lys Asn Phe
                   405                 410                 415
   atg att cct aaa aat act cat atg ttt atg aat gca tat tct gca aat      1296
   Met Ile Pro Lys Asn Thr His Met Phe Met Asn Ala Tyr Ser Ala Asn
               420                 425                 430
   cat gat gaa tta ata ttc aaa aat cca ttt aaa ttt gat cca gaa aga      1344
   His Asp Glu Leu Ile Phe Lys Asn Pro Phe Lys Phe Asp Pro Glu Arg
           435                 440                 445
   tgg tta gat tca gaa act aat gaa att aaa tca aaa ata ctt gct act      1392
   Trp Leu Asp Ser Glu Thr Asn Glu Ile Lys Ser Lys Ile Leu Ala Thr
       450                 455                 460
   act tct tcg tct tct tct tcg aca cat cat ggt ggt gga aat gga ata      1440
   Thr Ser Ser Ser Ser Ser Ser Thr His His Gly Gly Gly Asn Gly Ile
   465                 470                 475                 480
   aat gta cag aat ttt cat ttt gca ttt ggt gct gga tca aga atg tgt      1488
   Asn Val Gln Asn Phe His Phe Ala Phe Gly Ala Gly Ser Arg Met Cys
                   485                 490                 495
   tca ggt tat aat cta gtt atg aaa gaa atg tat atg atg ata att aaa      1536
   Ser Gly Tyr Asn Leu Val Met Lys Glu Met Tyr Met Met Ile Ile Lys
               500                 505                 510
   tta tta tta tta ttt gaa att aat cct cct gat aat aat aat aat aat      1584
   Leu Leu Leu Leu Phe Glu Ile Asn Pro Pro Asp Asn Asn Asn Asn Asn
           515                 520                 525
   ggg aaa tat tta atg gaa atg aat cct ttt gtt aat aat ctg aat cct      1632
   Gly Lys Tyr Leu Met Glu Met Asn Pro Phe Val Asn Asn Leu Asn Pro
       530                 535                 540
   aga ggt act tca ttt gaa cca cga ata cat aat att aaa tta caa tat      1680
   Arg Gly Thr Ser Phe Glu Pro Arg Ile His Asn Ile Lys Leu Gln Tyr
   545                 550                 555                 560
   aga aaa cta cct aat tat gaa act cta cac gaa ata gtt ctc aaa tag      1728
   Arg Lys Leu Pro Asn Tyr Glu Thr Leu His Glu Ile Val Leu Lys
                   565                 570                 575
<210> 46
   <211> 575
   <212> PRT
   <213> Candida tropicalis
<400> 46
   Met Leu Leu Ser Ile Pro Trp Asp Gln Ser Leu Leu Thr Leu Leu Thr
   1               5                   10                  15
   Tyr Leu Asp Thr His Pro Ile Ala Thr Ile Phe Thr Ile Ile Leu Thr
               20                  25                  30
   Ile Leu Thr Ile Gly Ile Leu Phe Asp Tyr Cys Leu Ser Pro Lys Glu
           35                  40                  45
   Ile Ala Asn Ile Phe Ser Ile Pro Gly Asp Leu Pro Phe Ile Gly His
       50                  55                  60
   Leu His Leu Ile Leu Asp Asn Pro Ala Leu Ile Tyr Leu Thr Trp Tyr
   65                  70                  75                  80
   Lys Leu Tyr Asn Lys Leu Val Phe Gln Ile Arg Ile Gly Asn Lys Arg
                   85                  90                  95
   Val Val Val Val Asn Ser Phe Asp Asp Val Val Gly Leu Trp Ile Asn
               100                 105                 110
   His Ser Cys Gln Asn Asn Ser Arg Pro Leu Ser Tyr Thr Phe His Gly
   115 120 125
   Leu Val Ser Ala Leu Gln Gly Phe Thr Val Gly Ser Thr Pro Ala Ser
   130 135 140
   Leu Thr Phe Leu Arg Lys Lys Lys Val Ile Ser Leu Cys Leu Arg Lys
   145 150 155 160
   Lys Glu Ile Asp Glu Lys Val Cys Leu Ile Asp Asn Glu Ile Cys Val
   165 170 175
   Met Ile Lys Glu Ile Ile Lys Lys Lys Asp Ile Ser Thr Asp Val Asn
   180 185 190
   Met Leu Pro Tyr Leu Gln Lys Phe Ile Leu Lys Thr Ala Ile Leu Met
   195 200 205
   Ser Tyr Gly Ile Glu Leu Asp Cys Tyr Asn Lys Asp Val Lys Leu Cys
   210 215 220
   Gln Glu Ile Ile Thr Val Glu Asn Asn Ile Ile Arg Leu Arg Ser Pro
   225 230 235 240
   Ile Ser Asn Leu Gln Asp Ser Val Pro Phe Leu Arg Leu Ile Pro Trp
   245 250 255
   Phe Asn Asn Arg Glu Phe Ala Leu Arg Cys Gly Asn Arg Arg Asn Lys
   260 265 270
   Tyr Met Asp Gln Leu Tyr Asn Arg Leu Gln Asn Gly Leu Ala Glu Asn
   275 280 285
   Asp Pro Asn Ile Ala Asn Ser Ile Leu Gly Gln Leu Ile Leu Asn Asn
   290 295 300
   Asp Asn Asn Asn Ser Asn Ser Leu Thr Ser Gln Glu Ile Gln Ser Ile
   305 310 315 320
   Cys Leu Thr Leu Val Ser Ala Gly Leu Asp Asn Thr Pro Leu Asn Leu
   325 330 335
   Asn Tyr Leu Ile Gly Ile Leu Ser Gln Pro Arg Ile Gly Lys Ile Phe
   340 345 350
   Gln Asp Lys Ala Ile Lys Asp Ile Leu Asn His Ala Asn Gly Asp Ile
   355 360 365
   Ile Gln Ala Trp Asn Gln Leu Asn Glu Glu Asn Arg Asp Cys Lys Tyr
       370                 375                 380
   Ile Gln Ala Leu Ile Leu Glu Thr Leu Arg His Phe Thr Val Leu Pro
   385                 390                 395                 400
   Leu Ser Leu Pro Arg Leu Thr Thr Lys Pro Ile Tyr Tyr Lys Asn Phe
                   405                 410                 415
   Met Ile Pro Lys Asn Thr His Met Phe Met Asn Ala Tyr Ser Ala Asn
               420                 425                 430
   His Asp Glu Leu Ile Phe Lys Asn Pro Phe Lys Phe Asp Pro Glu Arg
           435                 440                 445
   Trp Leu Asp Ser Glu Thr Asn Glu Ile Lys Ser Lys Ile Leu Ala Thr
       450                 455                 460
   Thr Ser Ser Ser Ser Ser Ser Thr His His Gly Gly Gly Asn Gly Ile
   465                 470                 475                 480
   Asn Val Gln Asn Phe His Phe Ala Phe Gly Ala Gly Ser Arg Met Cys
                   485                 490                 495
   Ser Gly Tyr Asn Leu Val Met Lys Glu Met Tyr Met Met Ile Ile Lys
               500                 505                 510
   Leu Leu Leu Leu Phe Glu Ile Asn Pro Pro Asp Asn Asn Asn Asn Asn
           515                 520                 525
   Gly Lys Tyr Leu Met Glu Met Asn Pro Phe Val Asn Asn Leu Asn Pro
       530                 535                 540
   Arg Gly Thr Ser Phe Glu Pro Arg Ile His Asn Ile Lys Leu Gln Tyr
   545                 550                 555                 560
   Arg Lys Leu Pro Asn Tyr Glu Thr Leu His Glu Ile Val Leu Lys
                   565                 570                 575
<210> 47
   <211> 1518
   <212> DNA
   <213> Candida tropicalis
<220>
   <221> CDS
   <222> (1)..(1518)
<400> 47
   atg tat caa tta ttt tgt ttt ctt gct ggt ata att gtt gta tat aaa       48
   Met Tyr Gln Leu Phe Cys Phe Leu Ala Gly Ile Ile Val Val Tyr Lys
   1               5                   10                  15
   gca gca caa tac tac aag aga agg aca ctt gtg act aaa ttt cat tgc       96
   Ala Ala Gln Tyr Tyr Lys Arg Arg Thr Leu Val Thr Lys Phe His Cys
               20                  25                  30
   aaa caa gca cgt att tcc cca aac aag tca tgg ttg gaa tat tta ggc      144
   Lys Gln Ala Arg Ile Ser Pro Asn Lys Ser Trp Leu Glu Tyr Leu Gly
           35                  40                  45
   att gcc tcc gtt gta cat gcc aat gaa atg att aga aaa gga gga ttg      192
   Ile Ala Ser Val Val His Ala Asn Glu Met Ile Arg Lys Gly Gly Leu
       50                  55                  60
   tat tca gaa att gat gga aga ttt aaa tcc ctt gat gtt tca aca ttc      240
   Tyr Ser Glu Ile Asp Gly Arg Phe Lys Ser Leu Asp Val Ser Thr Phe
   65                  70                  75                  80
   aag tct ata act ttg gga aag aca acg tat gtt acc aaa gat att gaa      288
   Lys Ser Ile Thr Leu Gly Lys Thr Thr Tyr Val Thr Lys Asp Ile Glu
                   85                  90                  95
   aac atc cgt cat atc ttg agt gca acg gaa atg aac tca tgg aat ctt      336
   Asn Ile Arg His Ile Leu Ser Ala Thr Glu Met Asn Ser Trp Asn Leu
               100                 105                 110
   ggt gcc cgt cca att gcg tta aga ccc ttg att ggt gat gga att ttt      384
   Gly Ala Arg Pro Ile Ala Leu Arg Pro Leu Ile Gly Asp Gly Ile Phe
           115                 120                 125
   gct agt gaa ggt caa tct tgg aaa cat agt cga atc atg ctt aga cca      432
   Ala Ser Glu Gly Gln Ser Trp Lys His Ser Arg Ile Met Leu Arg Pro
       130                 135                 140
   gta ttt gca aaa gaa cac gtt aaa caa atc act tca atg gaa cca tat      480
   Val Phe Ala Lys Glu His Val Lys Gln Ile Thr Ser Met Glu Pro Tyr
   145                 150                 155                 160
   gta caa ctg ttg atc aaa atc atc aag aac cat gaa ggg gaa cca tta      528
   Val Gln Leu Leu Ile Lys Ile Ile Lys Asn His Glu Gly Glu Pro Leu
                   165                 170                 175
   gag ttt caa acc tta gcc cat ctt ttt aca ata gat tat tct act gat      576
   Glu Phe Gln Thr Leu Ala His Leu Phe Thr Ile Asp Tyr Ser Thr Asp
               180                 185                 190
   ttc cta tta ggt gaa agt tgt gat agt ttg aag gat ttc cta gga gaa      624
   Phe Leu Leu Gly Glu Ser Cys Asp Ser Leu Lys Asp Phe Leu Gly Glu
           195                 200                 205
   gag tcc aat tcc aca tta gat aca tcg ttg aga ctg gca ttt gca tca      672
   Glu Ser Asn Ser Thr Leu Asp Thr Ser Leu Arg Leu Ala Phe Ala Ser
       210                 215                 220
   cag ttt aat aaa acc cag cag caa atg aca att cga ttc atg ttg gga      720
   Gln Phe Asn Lys Thr Gln Gln Gln Met Thr Ile Arg Phe Met Leu Gly
   225                 230                 235                 240
   aaa ttg gcc ttt ctc atg tat cca aag agt ttt caa aat agt att caa      768
   Lys Leu Ala Phe Leu Met Tyr Pro Lys Ser Phe Gln Asn Ser Ile Gln
                   245                 250                 255
   atg caa aag gac ttt gtt gat gaa tat atc gac aga gta gta ggt atg      816
   Met Gln Lys Asp Phe Val Asp Glu Tyr Ile Asp Arg Val Val Gly Met
               260                 265                 270
   tcc gaa gaa gaa ttg aac aat cat cca aag agc tat gtt ttg ttg tac      864
   Ser Glu Glu Glu Leu Asn Asn His Pro Lys Ser Tyr Val Leu Leu Tyr
           275                 280                 285
   caa tta gca aga caa act aag aat cgt gat ata tta caa gat gaa ttg      912
   Gln Leu Ala Arg Gln Thr Lys Asn Arg Asp Ile Leu Gln Asp Glu Leu
       290                 295                 300
   atg tcc att tta ctt gca ggt aga gac acc act gcc agt ttg ttg act      960
   Met Ser Ile Leu Leu Ala Gly Arg Asp Thr Thr Ala Ser Leu Leu Thr
   305                 310                 315                 320
   ttt ttg ttt ttc gaa tta agt cac cat cca gaa gta ttt aac aaa tta     1008
   Phe Leu Phe Phe Glu Leu Ser His His Pro Glu Val Phe Asn Lys Leu
                   325                 330                 335
   aaa gag gaa atc gaa aga cac ttt cct gat gtt gaa tcc gtt aca ttt     1056
   Lys Glu Glu Ile Glu Arg His Phe Pro Asp Val Glu Ser Val Thr Phe
               340                 345                 350
   gga act atc cag aga tgc gac tat ctt caa tgg tgt att aac gaa act     1104
   Gly Thr Ile Gln Arg Cys Asp Tyr Leu Gln Trp Cys Ile Asn Glu Thr
           355                 360                 365
   atg aga ctc cat cca tca gtt cct ttt aat ttc aga act gca gcc aat     1152
   Met Arg Leu His Pro Ser Val Pro Phe Asn Phe Arg Thr Ala Ala Asn
       370                 375                 380
   gac aca gta ata cca aga ggt gga ggt aaa tcc tgt aca gat cct att     1200
   Asp Thr Val Ile Pro Arg Gly Gly Gly Lys Ser Cys Thr Asp Pro Ile
   385                 390                 395                 400
   ctt gtc cat aag ggt gaa caa gta tta ttc agt ttc tat tct gta aac     1248
   Leu Val His Lys Gly Glu Gln Val Leu Phe Ser Phe Tyr Ser Val Asn
                   405                 410                 415
   aga gaa gaa aag tat ttt ggt aca aat acc gac aag ttt gct cca gaa     1296
   Arg Glu Glu Lys Tyr Phe Gly Thr Asn Thr Asp Lys Phe Ala Pro Glu
               420                 425                 430
   aga tgg agt gaa tca tta agg aga act gag ttc ata cca ttt tct gct     1344
   Arg Trp Ser Glu Ser Leu Arg Arg Thr Glu Phe Ile Pro Phe Ser Ala
           435                 440                 445
   gga cct cgt gcc tgt ttg ggt caa cag tta gct aga gtt gaa gct tca     1392
   Gly Pro Arg Ala Cys Leu Gly Gln Gln Leu Ala Arg Val Glu Ala Ser
       450                 455                 460
   tat gtt act att aga ttg ctt caa acc ttt cat ggg ttg cat aat gcc     1440
   Tyr Val Thr Ile Arg Leu Leu Gln Thr Phe His Gly Leu His Asn Ala
   465                 470                 475                 480
   agt aaa caa tac cca cca aat aga gtg gtt gca gct aca atg aga ttg     1488
   Ser Lys Gln Tyr Pro Pro Asn Arg Val Val Ala Ala Thr Met Arg Leu
                   485                 490                 495
   act gac ggt tgt aac gtt tgt ttt atc tag                             1518
   Thr Asp Gly Cys Asn Val Cys Phe Ile
   500 505
<210> 48
   <211> 505
   <212> PRT
   <213> Candida tropicalis
<400> 48
   Met Tyr Gln Leu Phe Cys Phe Leu Ala Gly Ile Ile Val Val Tyr Lys
   1 5 10 15
   Ala Ala Gln Tyr Tyr Lys Arg Arg Thr Leu Val Thr Lys Phe His Cys
   20 25 30
   Lys Gln Ala Arg Ile Ser Pro Asn Lys Ser Trp Leu Glu Tyr Leu Gly
   35 40 45
   Ile Ala Ser Val Val His Ala Asn Glu Met Ile Arg Lys Gly Gly Leu
   50 55 60
   Tyr Ser Glu Ile Asp Gly Arg Phe Lys Ser Leu Asp Val Ser Thr Phe
   65 70 75 80
   Lys Ser Ile Thr Leu Gly Lys Thr Thr Tyr Val Thr Lys Asp Ile Glu
   85 90 95
   Asn Ile Arg His Ile Leu Ser Ala Thr Glu Met Asn Ser Trp Asn Leu
   100 105 110
   Gly Ala Arg Pro Ile Ala Leu Arg Pro Leu Ile Gly Asp Gly Ile Phe
   115 120 125
   Ala Ser Glu Gly Gln Ser Trp Lys His Ser Arg Ile Met Leu Arg Pro
   130 135 140
   Val Phe Ala Lys Glu His Val Lys Gln Ile Thr Ser Met Glu Pro Tyr
   145 150 155 160
   Val Gln Leu Leu Ile Lys Ile Ile Lys Asn His Glu Gly Glu Pro Leu
   165 170 175
   Glu Phe Gln Thr Leu Ala His Leu Phe Thr Ile Asp Tyr Ser Thr Asp
   180 185 190
   Phe Leu Leu Gly Glu Ser Cys Asp Ser Leu Lys Asp Phe Leu Gly Glu
   195 200 205
   Glu Ser Asn Ser Thr Leu Asp Thr Ser Leu Arg Leu Ala Phe Ala Ser
       210                 215                 220
   Gln Phe Asn Lys Thr Gln Gln Gln Met Thr Ile Arg Phe Met Leu Gly
   225                 230                 235                 240
   Lys Leu Ala Phe Leu Met Tyr Pro Lys Ser Phe Gln Asn Ser Ile Gln
                   245                 250                 255
   Met Gln Lys Asp Phe Val Asp Glu Tyr Ile Asp Arg Val Val Gly Met
               260                 265                 270
   Ser Glu Glu Glu Leu Asn Asn His Pro Lys Ser Tyr Val Leu Leu Tyr
           275                 280                 285
   Gln Leu Ala Arg Gln Thr Lys Asn Arg Asp Ile Leu Gln Asp Glu Leu
       290                 295                 300
   Met Ser Ile Leu Leu Ala Gly Arg Asp Thr Thr Ala Ser Leu Leu Thr
   305                 310                 315                 320
   Phe Leu Phe Phe Glu Leu Ser His His Pro Glu Val Phe Asn Lys Leu
                   325                 330                 335
   Lys Glu Glu Ile Glu Arg His Phe Pro Asp Val Glu Ser Val Thr Phe
               340                 345                 350
   Gly Thr Ile Gln Arg Cys Asp Tyr Leu Gln Trp Cys Ile Asn Glu Thr
           355                 360                 365
   Met Arg Leu His Pro Ser Val Pro Phe Asn Phe Arg Thr Ala Ala Asn
       370                 375                 380
   Asp Thr Val Ile Pro Arg Gly Gly Gly Lys Ser Cys Thr Asp Pro Ile
   385                 390                 395                 400
   Leu Val His Lys Gly Glu Gln Val Leu Phe Ser Phe Tyr Ser Val Asn
                   405                 410                 415
   Arg Glu Glu Lys Tyr Phe Gly Thr Asn Thr Asp Lys Phe Ala Pro Glu
               420                 425                 430
   Arg Trp Ser Glu Ser Leu Arg Arg Thr Glu Phe Ile Pro Phe Ser Ala
           435                 440                 445
   Gly Pro Arg Ala Cys Leu Gly Gln Gln Leu Ala Arg Val Glu Ala Ser
       450                 455                 460
   Tyr Val Thr Ile Arg Leu Leu Gln Thr Phe His Gly Leu His Asn Ala
   465 470 475 480
   Ser Lys Gln Tyr Pro Pro Asn Arg Val Val Ala Ala Thr Met Arg Leu
   485 490 495
   Thr Asp Gly Cys Asn Val Cys Phe Ile
   500 505
<210> 49
   <211> 1584
   <212> DNA
   <213> Candida tropicalis
<220>
   <221> CDS
   <222> (1)..(1584)
<400> 49
   atg agt gtc aat act act tct cca gtt gtg gat agt att agt gat aat        48
   Met Ser Val Asn Thr Thr Ser Pro Val Val Asp Ser Ile Ser Asp Asn
   1               5                   10                  15
   gct act aca tat ttg agt aca aaa tat atc atc gat agt tta tat tca        96
   Ala Thr Thr Tyr Leu Ser Thr Lys Tyr Ile Ile Asp Ser Leu Tyr Ser
               20                  25                  30
   act tat caa caa gct tca tgg tta caa atc att tta acc tca atc att       144
   Thr Tyr Gln Gln Ala Ser Trp Leu Gln Ile Ile Leu Thr Ser Ile Ile
           35                  40                  45
   ctc att ttg act tat gat caa atc ctg tat caa atc aat aaa ggt tca       192
   Leu Ile Leu Thr Tyr Asp Gln Ile Leu Tyr Gln Ile Asn Lys Gly Ser
       50                  55                  60
   att gct ggt cca aaa ttc aaa ttt tgg cca att att ggt cca ttc ttg       240
   Ile Ala Gly Pro Lys Phe Lys Phe Trp Pro Ile Ile Gly Pro Phe Leu
   65                  70                  75                  80
   gaa tct tta gat cca aaa ttc gaa gaa tat aaa gct aaa tgg gat tct       288
   Glu Ser Leu Asp Pro Lys Phe Glu Glu Tyr Lys Ala Lys Trp Asp Ser
                   85                  90                  95
   ggt gaa ttg agt tgt gtt tcc att ttc cat aaa ttc gtt gtt att gct       336
   Gly Glu Leu Ser Cys Val Ser Ile Phe His Lys Phe Val Val Ile Ala
               100                 105                 110
   tct tct cgt gat tta gct aga aag att tta gca tct cca aaa tac gtt       384
   Ser Ser Arg Asp Leu Ala Arg Lys Ile Leu Ala Ser Pro Lys Tyr Val
           115                 120                 125
   aaa cct tgt gtt gtt gat gtt gct gtt aaa att tta aga cct tca aat       432
   Lys Pro Cys Val Val Asp Val Ala Val Lys Ile Leu Arg Pro Ser Asn
       130                 135                 140
   tgg gtt ttc tta gac ggt aaa gca cat act gat tac cgt cgt tct ttg       480
   Trp Val Phe Leu Asp Gly Lys Ala His Thr Asp Tyr Arg Arg Ser Leu
   145                 150                 155                 160
   aat ggt tta ttc tcc caa aga gct ttg gaa att tac att cca gtt caa       528
   Asn Gly Leu Phe Ser Gln Arg Ala Leu Glu Ile Tyr Ile Pro Val Gln
                   165                 170                 175
   gaa aaa tac atg gat att tat tta gat aga ttc tgt aag tac gac ggt       576
   Glu Lys Tyr Met Asp Ile Tyr Leu Asp Arg Phe Cys Lys Tyr Asp Gly
               180                 185                 190
   cca cgt gaa ttc ttc cca gaa ttt aga gaa ttg ttg tgt gct tta tct       624
   Pro Arg Glu Phe Phe Pro Glu Phe Arg Glu Leu Leu Cys Ala Leu Ser
           195                 200                 205
   ttg aga act ttc tgt ggg gat tac atc act gaa gat caa att gct tta       672
   Leu Arg Thr Phe Cys Gly Asp Tyr Ile Thr Glu Asp Gln Ile Ala Leu
       210                 215                 220
   gtt gct gat aac tat tac aga gtc act gct gct ttg gaa ttg gtc aat       720
   Val Ala Asp Asn Tyr Tyr Arg Val Thr Ala Ala Leu Glu Leu Val Asn
   225                 230                 235                 240
   ttc cca atc att att cct tac act aaa act tgg tac ggt aag aag att       768
   Phe Pro Ile Ile Ile Pro Tyr Thr Lys Thr Trp Tyr Gly Lys Lys Ile
                   245                 250                 255
   gct gat gat acc atg aag att ttt gaa aat tgt gct gct atg tcc aag       816
   Ala Asp Asp Thr Met Lys Ile Phe Glu Asn Cys Ala Ala Met Ser Lys
               260                 265                 270
   aaa cac att aat gaa aat aat ggt act cca ggt tgt gtt atg gat gaa       864
   Lys His Ile Asn Glu Asn Asn Gly Thr Pro Gly Cys Val Met Asp Glu
           275                 280                 285
   tgg att tac ttg atg aaa gaa gct aaa gaa aaa cac tct gat gat cca       912
   Trp Ile Tyr Leu Met Lys Glu Ala Lys Glu Lys His Ser Asp Asp Pro
       290                 295                 300
   gat tcc aaa tta ttg att aga gaa ttc tcc aac cgt gaa att tcc gaa       960
   Asp Ser Lys Leu Leu Ile Arg Glu Phe Ser Asn Arg Glu Ile Ser Glu
   305                 310                 315                 320
   gcc att ttc act ttc ttg ttt gct tct caa gat gcc tct tct tct ttg      1008
   Ala Ile Phe Thr Phe Leu Phe Ala Ser Gln Asp Ala Ser Ser Ser Leu
                   325                 330                 335
   gct tgt tgg tta ttc caa att gtc gcc gac aga cca gat gtt gtt gcc      1056
   Ala Cys Trp Leu Phe Gln Ile Val Ala Asp Arg Pro Asp Val Val Ala
               340                 345                 350
   aag att aga gaa gaa caa ttg aga gtt aga aac aat gac cca tct gtt      1104
   Lys Ile Arg Glu Glu Gln Leu Arg Val Arg Asn Asn Asp Pro Ser Val
           355                 360                 365
   aaa ttg tcc ttg gat ttg att aat gaa atg act tac acc aat gat gtt      1152
   Lys Leu Ser Leu Asp Leu Ile Asn Glu Met Thr Tyr Thr Asn Asp Val
       370                 375                 380
   gtt aaa gaa tct ttg aga tac cgt cca cca gtc ttg atg gtt cca tat      1200
   Val Lys Glu Ser Leu Arg Tyr Arg Pro Pro Val Leu Met Val Pro Tyr
   385                 390                 395                 400
   gtt gtt aag aaa gct ttc cca gtt act gaa aaa tac act gct cca aag      1248
   Val Val Lys Lys Ala Phe Pro Val Thr Glu Lys Tyr Thr Ala Pro Lys
                   405                 410                 415
   ggt tct atg ctt atc cca act ttg tac cct gct tta cat gat cct gaa      1296
   Gly Ser Met Leu Ile Pro Thr Leu Tyr Pro Ala Leu His Asp Pro Glu
               420                 425                 430
   gtt tat gat gaa cca gat tct ttc att cca gaa aga tgg gcc act gct      1344
   Val Tyr Asp Glu Pro Asp Ser Phe Ile Pro Glu Arg Trp Ala Thr Ala
           435                 440                 445
   tct ggt gat atg tac aaa cgt aac tgg ttg gtc ttc ggt act ggt cca      1392
   Ser Gly Asp Met Tyr Lys Arg Asn Trp Leu Val Phe Gly Thr Gly Pro
       450                 455                 460
   cac gtt tgt ttg ggt aag aac tat gtc atg atg ttg ttt act ggt atg      1440
   His Val Cys Leu Gly Lys Asn Tyr Val Met Met Leu Phe Thr Gly Met
   465                 470                 475                 480
   ttg ggt aaa ttt gtc atg aac tct gat atc att cat cac aaa act gca      1488
   Leu Gly Lys Phe Val Met Asn Ser Asp Ile Ile His His Lys Thr Ala
                   485                 490                 495
   tta tct gaa gaa atc aaa gtt ttc gct act att ttc cct aag gac gat      1536
   Leu Ser Glu Glu Ile Lys Val Phe Ala Thr Ile Phe Pro Lys Asp Asp
               500                 505                 510
   gtt att tta gaa tgg aaa aag aga gat ccg tta gct gct tct aat taa      1584
   Val Ile Leu Glu Trp Lys Lys Arg Asp Pro Leu Ala Ala Ser Asn
           515                 520                 525
<210> 50
   <211> 527
   <212> PRT
   <213> Candida tropicalis
<400> 50
   Met Ser Val Asn Thr Thr Ser Pro Val Val Asp Ser Ile Ser Asp Asn
   1               5                   10                  15
   Ala Thr Thr Tyr Leu Ser Thr Lys Tyr Ile Ile Asp Ser Leu Tyr Ser
               20                  25                  30
   Thr Tyr Gln Gln Ala Ser Trp Leu Gln Ile Ile Leu Thr Ser Ile Ile
           35                  40                  45
   Leu Ile Leu Thr Tyr Asp Gln Ile Leu Tyr Gln Ile Asn Lys Gly Ser
       50                  55                  60
   Ile Ala Gly Pro Lys Phe Lys Phe Trp Pro Ile Ile Gly Pro Phe Leu
   65                  70                  75                  80
   Glu Ser Leu Asp Pro Lys Phe Glu Glu Tyr Lys Ala Lys Trp Asp Ser
                   85                  90                  95
   Gly Glu Leu Ser Cys Val Ser Ile Phe His Lys Phe Val Val Ile Ala
   100 105 110
   Ser Ser Arg Asp Leu Ala Arg Lys Ile Leu Ala Ser Pro Lys Tyr Val
   115 120 125
   Lys Pro Cys Val Val Asp Val Ala Val Lys Ile Leu Arg Pro Ser Asn
   130 135 140
   Trp Val Phe Leu Asp Gly Lys Ala His Thr Asp Tyr Arg Arg Ser Leu
   145 150 155 160
   Asn Gly Leu Phe Ser Gln Arg Ala Leu Glu Ile Tyr Ile Pro Val Gln
   165 170 175
   Glu Lys Tyr Met Asp Ile Tyr Leu Asp Arg Phe Cys Lys Tyr Asp Gly
   180 185 190
   Pro Arg Glu Phe Phe Pro Glu Phe Arg Glu Leu Leu Cys Ala Leu Ser
   195 200 205
   Leu Arg Thr Phe Cys Gly Asp Tyr Ile Thr Glu Asp Gln Ile Ala Leu
   210 215 220
   Val Ala Asp Asn Tyr Tyr Arg Val Thr Ala Ala Leu Glu Leu Val Asn
   225 230 235 240
   Phe Pro Ile Ile Ile Pro Tyr Thr Lys Thr Trp Tyr Gly Lys Lys Ile
   245 250 255
   Ala Asp Asp Thr Met Lys Ile Phe Glu Asn Cys Ala Ala Met Ser Lys
   260 265 270
   Lys His Ile Asn Glu Asn Asn Gly Thr Pro Gly Cys Val Met Asp Glu
   275 280 285
   Trp Ile Tyr Leu Met Lys Glu Ala Lys Glu Lys His Ser Asp Asp Pro
   290 295 300
   Asp Ser Lys Leu Leu Ile Arg Glu Phe Ser Asn Arg Glu Ile Ser Glu
   305 310 315 320
   Ala Ile Phe Thr Phe Leu Phe Ala Ser Gln Asp Ala Ser Ser Ser Leu
   325 330 335
   Ala Cys Trp Leu Phe Gln Ile Val Ala Asp Arg Pro Asp Val Val Ala
   340 345 350
   Lys Ile Arg Glu Glu Gln Leu Arg Val Arg Asn Asn Asp Pro Ser Val
           355                 360                 365
   Lys Leu Ser Leu Asp Leu Ile Asn Glu Met Thr Tyr Thr Asn Asp Val
       370                 375                 380
   Val Lys Glu Ser Leu Arg Tyr Arg Pro Pro Val Leu Met Val Pro Tyr
   385                 390                 395                 400
   Val Val Lys Lys Ala Phe Pro Val Thr Glu Lys Tyr Thr Ala Pro Lys
                   405                 410                 415
   Gly Ser Met Leu Ile Pro Thr Leu Tyr Pro Ala Leu His Asp Pro Glu
               420                 425                 430
   Val Tyr Asp Glu Pro Asp Ser Phe Ile Pro Glu Arg Trp Ala Thr Ala
           435                 440                 445
   Ser Gly Asp Met Tyr Lys Arg Asn Trp Leu Val Phe Gly Thr Gly Pro
       450                 455                 460
   His Val Cys Leu Gly Lys Asn Tyr Val Met Met Leu Phe Thr Gly Met
   465                 470                 475                 480
   Leu Gly Lys Phe Val Met Asn Ser Asp Ile Ile His His Lys Thr Ala
                   485                 490                 495
   Leu Ser Glu Glu Ile Lys Val Phe Ala Thr Ile Phe Pro Lys Asp Asp
               500                 505                 510
   Val Ile Leu Glu Trp Lys Lys Arg Asp Pro Leu Ala Ala Ser Asn
           515                 520                 525
<210> 51
   <211> 1587
   <212> DNA
   <213> Candida tropicalis
<220>
   <221> CDS
   <222> (1)..(1587)
<400> 51
   atg gct att gtt gat act gcc att gat ggc atc aat tat ttc tta tcc        48
   Met Ala Ile Val Asp Thr Ala Ile Asp Gly Ile Asn Tyr Phe Leu Ser
   1               5                   10                  15
   tta tca tta act caa caa atc acc atc ttg gtt gtt ttc cca ttc atc        96
   Leu Ser Leu Thr Gln Gln Ile Thr Ile Leu Val Val Phe Pro Phe Ile
               20                  25                  30
   tac aac ata gca tgg caa tta ctt tac tcc tta aga aaa gat aga gtt      144
   Tyr Asn Ile Ala Trp Gln Leu Leu Tyr Ser Leu Arg Lys Asp Arg Val
           35                  40                  45
   cca atg gtt ttc tac tgg atc cca tgg ttt ggt tct gct gct agt tat      192
   Pro Met Val Phe Tyr Trp Ile Pro Trp Phe Gly Ser Ala Ala Ser Tyr
       50                  55                  60
   ggt atg caa cca tac gaa ttc ttt gaa aag tgc aga ttg aaa tat ggt      240
   Gly Met Gln Pro Tyr Glu Phe Phe Glu Lys Cys Arg Leu Lys Tyr Gly
   65                  70                  75                  80
   gat gtt ttt tca ttt atg tta tta ggt aaa gtt atg act gtt tat ttg      288
   Asp Val Phe Ser Phe Met Leu Leu Gly Lys Val Met Thr Val Tyr Leu
                   85                  90                  95
   ggt cca aaa ggt cac gaa ttc att tac aat gct aaa tta tcc gat gtt      336
   Gly Pro Lys Gly His Glu Phe Ile Tyr Asn Ala Lys Leu Ser Asp Val
               100                 105                 110
   tct gct gaa gaa gct tat acc cat ttg act act cct gtt ttt ggt aaa      384
   Ser Ala Glu Glu Ala Tyr Thr His Leu Thr Thr Pro Val Phe Gly Lys
           115                 120                 125
   ggt gtt att tat gat tgt cca aac tct aga tta atg gaa caa aag aag      432
   Gly Val Ile Tyr Asp Cys Pro Asn Ser Arg Leu Met Glu Gln Lys Lys
       130                 135                 140
   ttt gct aaa ttt gct ttg act act gat tct ttc aaa acc tat gtt cca      480
   Phe Ala Lys Phe Ala Leu Thr Thr Asp Ser Phe Lys Thr Tyr Val Pro
   145                 150                 155                 160
   aag atc aga gaa gaa gtt ttg aat tat ttt gtt aac gat gtt agt ttc      528
   Lys Ile Arg Glu Glu Val Leu Asn Tyr Phe Val Asn Asp Val Ser Phe
                   165                 170                 175
   aaa acc aag gaa aga gac cat ggt gtt gct agt gtt atg aaa act caa      576
   Lys Thr Lys Glu Arg Asp His Gly Val Ala Ser Val Met Lys Thr Gln
               180                 185                 190
   cca gaa atc act att ttc act gct tct cgt tgt tta ttt ggt gat gaa      624
   Pro Glu Ile Thr Ile Phe Thr Ala Ser Arg Cys Leu Phe Gly Asp Glu
           195                 200                 205
   atg aga aag agt ttc gac aga tca ttt gct caa ttg tat gct gac ttg      672
   Met Arg Lys Ser Phe Asp Arg Ser Phe Ala Gln Leu Tyr Ala Asp Leu
       210                 215                 220
   gat aaa ggt ttc acc cca atc aac ttt gtt ttc cca aac ttg cca tta      720
   Asp Lys Gly Phe Thr Pro Ile Asn Phe Val Phe Pro Asn Leu Pro Leu
   225                 230                 235                 240
   cct cat tac tgg aga cgt gac gct gct caa aga aag ata tct gct cat      768
   Pro His Tyr Trp Arg Arg Asp Ala Ala Gln Arg Lys Ile Ser Ala His
                   245                 250                 255
   tac atg aag gaa att aag aga aga aga gaa agc ggt gat att gat cca      816
   Tyr Met Lys Glu Ile Lys Arg Arg Arg Glu Ser Gly Asp Ile Asp Pro
               260                 265                 270
   aag aga gat ttg att gat tcc ttg ttg gtt aac tct act tat aaa gat      864
   Lys Arg Asp Leu Ile Asp Ser Leu Leu Val Asn Ser Thr Tyr Lys Asp
           275                 280                 285
   ggt gtt aaa atg act gat caa gaa att gct aac ctt tta att ggt gtt      912
   Gly Val Lys Met Thr Asp Gln Glu Ile Ala Asn Leu Leu Ile Gly Val
       290                 295                 300
   ttg atg ggt ggt caa cat act tct gct tcc act tct gcc tgg ttc ttg      960
   Leu Met Gly Gly Gln His Thr Ser Ala Ser Thr Ser Ala Trp Phe Leu
   305                 310                 315                 320
   ttg cat ttg gct gaa caa cca caa tta caa gat gat ctt tac gaa gaa     1008
   Leu His Leu Ala Glu Gln Pro Gln Leu Gln Asp Asp Leu Tyr Glu Glu
                   325                 330                 335
   ttg acc aac ttg ttg aaa gaa aag ggt ggt gac ttg aac gat ttg act     1056
   Leu Thr Asn Leu Leu Lys Glu Lys Gly Gly Asp Leu Asn Asp Leu Thr
               340                 345                 350
   tac gaa gac ttg caa aaa tta cca ttg gtt aac aac act att aaa gaa     1104
   Tyr Glu Asp Leu Gln Lys Leu Pro Leu Val Asn Asn Thr Ile Lys Glu
           355                 360                 365
   act ttg aga atg cac atg cca ttg cat tct att ttc aga aaa gtt atg     1152
   Thr Leu Arg Met His Met Pro Leu His Ser Ile Phe Arg Lys Val Met
       370                 375                 380
   aac cca ttg aga gtc cca aat acc aaa tat gtt att cca aaa ggt cac     1200
   Asn Pro Leu Arg Val Pro Asn Thr Lys Tyr Val Ile Pro Lys Gly His
   385                 390                 395                 400
   tat gtc tta gtt tct gcc ggt tat gct cat acc agt gat aga tgg ttt     1248
   Tyr Val Leu Val Ser Ala Gly Tyr Ala His Thr Ser Asp Arg Trp Phe
                   405                 410                 415
   gaa cac cca gaa cat ttc aac cca aga aga tgg gaa tct gat gat acc     1296
   Glu His Pro Glu His Phe Asn Pro Arg Arg Trp Glu Ser Asp Asp Thr
               420                 425                 430
   aag gct agt gct gtt tct ttc aat tct gaa gat act gtt gat tat ggt     1344
   Lys Ala Ser Ala Val Ser Phe Asn Ser Glu Asp Thr Val Asp Tyr Gly
           435                 440                 445
   ttc ggt aaa att tcc aaa ggt gtc tcc tct cca tac ttg cca ttc ggt     1392
   Phe Gly Lys Ile Ser Lys Gly Val Ser Ser Pro Tyr Leu Pro Phe Gly
       450                 455                 460
   ggt ggt aga cac aga tgt att ggt gaa caa ttt gct tat gtt caa ttg     1440
   Gly Gly Arg His Arg Cys Ile Gly Glu Gln Phe Ala Tyr Val Gln Leu
   465                 470                 475                 480
   gga act att ttg acc act tat atc tac aac ttc aaa tgg aga tta aac     1488
   Gly Thr Ile Leu Thr Thr Tyr Ile Tyr Asn Phe Lys Trp Arg Leu Asn
                   485                 490                 495
   ggt gat aag gtt cca gat gtt gat tac caa tcc atg gtt acc tta cca     1536
   Gly Asp Lys Val Pro Asp Val Asp Tyr Gln Ser Met Val Thr Leu Pro
               500                 505                 510
   tta gaa cct gct gaa atc gtt tgg gaa aag aga gat act tgt atg gtt     1584
   Leu Glu Pro Ala Glu Ile Val Trp Glu Lys Arg Asp Thr Cys Met Val
           515                 520                 525
   tag                                                                 1587
<210> 52
   <211> 528
   <212> PRT
   <213> Candida tropicalis
<400> 52
   Met Ala Ile Val Asp Thr Ala Ile Asp Gly Ile Asn Tyr Phe Leu Ser
   1               5                   10                  15
   Leu Ser Leu Thr Gln Gln Ile Thr Ile Leu Val Val Phe Pro Phe Ile
               20                  25                  30
   Tyr Asn Ile Ala Trp Gln Leu Leu Tyr Ser Leu Arg Lys Asp Arg Val
           35                  40                  45
   Pro Met Val Phe Tyr Trp Ile Pro Trp Phe Gly Ser Ala Ala Ser Tyr
       50                  55                  60
   Gly Met Gln Pro Tyr Glu Phe Phe Glu Lys Cys Arg Leu Lys Tyr Gly
   65                  70                  75                  80
   Asp Val Phe Ser Phe Met Leu Leu Gly Lys Val Met Thr Val Tyr Leu
                   85                  90                  95
   Gly Pro Lys Gly His Glu Phe Ile Tyr Asn Ala Lys Leu Ser Asp Val
               100                 105                 110
   Ser Ala Glu Glu Ala Tyr Thr His Leu Thr Thr Pro Val Phe Gly Lys
           115                 120                 125
   Gly Val Ile Tyr Asp Cys Pro Asn Ser Arg Leu Met Glu Gln Lys Lys
       130                 135                 140
   Phe Ala Lys Phe Ala Leu Thr Thr Asp Ser Phe Lys Thr Tyr Val Pro
   145                 150                 155                 160
   Lys Ile Arg Glu Glu Val Leu Asn Tyr Phe Val Asn Asp Val Ser Phe
                   165                 170                 175
   Lys Thr Lys Glu Arg Asp His Gly Val Ala Ser Val Met Lys Thr Gln
               180                 185                 190
   Pro Glu Ile Thr Ile Phe Thr Ala Ser Arg Cys Leu Phe Gly Asp Glu
           195                 200                 205
   Met Arg Lys Ser Phe Asp Arg Ser Phe Ala Gln Leu Tyr Ala Asp Leu
       210                 215                 220
   Asp Lys Gly Phe Thr Pro Ile Asn Phe Val Phe Pro Asn Leu Pro Leu
   225 230 235 240
   Pro His Tyr Trp Arg Arg Asp Ala Ala Gln Arg Lys Ile Ser Ala His
   245 250 255
   Tyr Met Lys Glu Ile Lys Arg Arg Arg Glu Ser Gly Asp Ile Asp Pro
   260 265 270
   Lys Arg Asp Leu Ile Asp Ser Leu Leu Val Asn Ser Thr Tyr Lys Asp
   275 280 285
   Gly Val Lys Met Thr Asp Gln Glu Ile Ala Asn Leu Leu Ile Gly Val
   290 295 300
   Leu Met Gly Gly Gln His Thr Ser Ala Ser Thr Ser Ala Trp Phe Leu
   305 310 315 320
   Leu His Leu Ala Glu Gln Pro Gln Leu Gln Asp Asp Leu Tyr Glu Glu
   325 330 335
   Leu Thr Asn Leu Leu Lys Glu Lys Gly Gly Asp Leu Asn Asp Leu Thr
   340 345 350
   Tyr Glu Asp Leu Gln Lys Leu Pro Leu Val Asn Asn Thr Ile Lys Glu
   355 360 365
   Thr Leu Arg Met His Met Pro Leu His Ser Ile Phe Arg Lys Val Met
   370 375 380
   Asn Pro Leu Arg Val Pro Asn Thr Lys Tyr Val Ile Pro Lys Gly His
   385 390 395 400
   Tyr Val Leu Val Ser Ala Gly Tyr Ala His Thr Ser Asp Arg Trp Phe
   405 410 415
   Glu His Pro Glu His Phe Asn Pro Arg Arg Trp Glu Ser Asp Asp Thr
   420 425 430
   Lys Ala Ser Ala Val Ser Phe Asn Ser Glu Asp Thr Val Asp Tyr Gly
   435 440 445
   Phe Gly Lys Ile Ser Lys Gly Val Ser Ser Pro Tyr Leu Pro Phe Gly
   450 455 460
   Gly Gly Arg His Arg Cys Ile Gly Glu Gln Phe Ala Tyr Val Gln Leu
   465 470 475 480
   Gly Thr Ile Leu Thr Thr Tyr Ile Tyr Asn Phe Lys Trp Arg Leu Asn
                   485                 490                 495
   Gly Asp Lys Val Pro Asp Val Asp Tyr Gln Ser Met Val Thr Leu Pro
               500                 505                 510
   Leu Glu Pro Ala Glu Ile Val Trp Glu Lys Arg Asp Thr Cys Met Val
           515                 520                 525
<210> 53
   <211> 2097
   <212> DNA
   <213> Candida cloacae
<220>
   <221> CDS
   <222> (1)..(2097)
<400> 53
   atg tcc cat caa gtt gaa gac cac gac tta gac gtg ttc tgt tta ttg      48
   Met Ser His Gln Val Glu Asp His Asp Leu Asp Val Phe Cys Leu Leu
   1               5                   10                  15
   gcc gat gcc gtg ctc cat gaa att cct ccc agc gaa atc gtg gag tac      96
   Ala Asp Ala Val Leu His Glu Ile Pro Pro Ser Glu Ile Val Glu Tyr
               20                  25                  30
   ctt cat cct gac ttc ccc aaa gat aag atc gaa gag tat ttg aca ggc     144
   Leu His Pro Asp Phe Pro Lys Asp Lys Ile Glu Glu Tyr Leu Thr Gly
           35                  40                  45
   ttt tcc cgt ccg tct gct gtt cct cag ttt aga caa tgt gcc aag aag     192
   Phe Ser Arg Pro Ser Ala Val Pro Gln Phe Arg Gln Cys Ala Lys Lys
       50                  55                  60
   ctt atc aac aga ggc tcc gag ctg tcg atc aag ttg ttt ttg tac ttg     240
   Leu Ile Asn Arg Gly Ser Glu Leu Ser Ile Lys Leu Phe Leu Tyr Leu
   65                  70                  75                  80
   acc act gcg ttg gac tca aga atc ctt gca cct gcc ttg acc aat tcg     288
   Thr Thr Ala Leu Asp Ser Arg Ile Leu Ala Pro Ala Leu Thr Asn Ser
                   85                  90                  95
   ttg act ttg atc agg gat atg gat ctt tcc caa aga gag gag ttg ttg     336
   Leu Thr Leu Ile Arg Asp Met Asp Leu Ser Gln Arg Glu Glu Leu Leu
               100                 105                 110
   aga tca tgg aga gac tct cct tta act gca aaa aga aga tta ttt aga     384
   Arg Ser Trp Arg Asp Ser Pro Leu Thr Ala Lys Arg Arg Leu Phe Arg
           115                 120                 125
   gtg tat gcc tct ttt acc ttg tct act ttt aac aag ttg gga aca gac     432
   Val Tyr Ala Ser Phe Thr Leu Ser Thr Phe Asn Lys Leu Gly Thr Asp
       130                 135                 140
   ttg cac ttt aag gcg ttg ggc tac cca ggt aga gag ctc aga acg caa     480
   Leu His Phe Lys Ala Leu Gly Tyr Pro Gly Arg Glu Leu Arg Thr Gln
   145                 150                 155                 160
   att caa gac tac gaa gtc gac cct ttt aga tat tcg ttt atg gag aaa      528
   Ile Gln Asp Tyr Glu Val Asp Pro Phe Arg Tyr Ser Phe Met Glu Lys
                   165                 170                 175
   ctt aaa cac gag ggc cac gaa ttg ttc ctt cct gat att gac gtt tta      576
   Leu Lys His Glu Gly His Glu Leu Phe Leu Pro Asp Ile Asp Val Leu
               180                 185                 190
   atc atc ggg tcg gga tca gga gca ggt gtg gtt gca caa act ctt act      624
   Ile Ile Gly Ser Gly Ser Gly Ala Gly Val Val Ala Gln Thr Leu Thr
           195                 200                 205
   gaa agt ggc ctc aaa tca ttg gtt ttg gaa aag ggc aaa tac ttt gcc      672
   Glu Ser Gly Leu Lys Ser Leu Val Leu Glu Lys Gly Lys Tyr Phe Ala
       210                 215                 220
   agt gaa gaa ttg tgc atg acg gac ttg gac ggt aac gag gca tta ttc      720
   Ser Glu Glu Leu Cys Met Thr Asp Leu Asp Gly Asn Glu Ala Leu Phe
   225                 230                 235                 240
   gaa agt gga gga aca att cct tcc acc aac caa caa ttg ttc atg att      768
   Glu Ser Gly Gly Thr Ile Pro Ser Thr Asn Gln Gln Leu Phe Met Ile
                   245                 250                 255
   gca ggt tcg act ttt ggt ggt ggt tct aca gtt aat tgg tct gcc tgt      816
   Ala Gly Ser Thr Phe Gly Gly Gly Ser Thr Val Asn Trp Ser Ala Cys
               260                 265                 270
   ttg aag acc cca ttc aaa gta aga aag gaa tgg tat gac gat ttc gga      864
   Leu Lys Thr Pro Phe Lys Val Arg Lys Glu Trp Tyr Asp Asp Phe Gly
           275                 280                 285
   ctt gat ttt gtc gct act caa caa tac gac gat tgt atg gat tac gtg      912
   Leu Asp Phe Val Ala Thr Gln Gln Tyr Asp Asp Cys Met Asp Tyr Val
       290                 295                 300
   tgg aag aaa atg ggt gct tcg acc gaa cat atc gaa cat tct gct gca      960
   Trp Lys Lys Met Gly Ala Ser Thr Glu His Ile Glu His Ser Ala Ala
   305                 310                 315                 320
   aat gcc gtc atc atg gac ggg gca gca aaa ctt ggc tac gca cac aga     1008
   Asn Ala Val Ile Met Asp Gly Ala Ala Lys Leu Gly Tyr Ala His Arg
                   325                 330                 335
   gca ctt gag cag aat acc ggg ggc cat gtt cac gac tgt ggg atg tgc     1056
   Ala Leu Glu Gln Asn Thr Gly Gly His Val His Asp Cys Gly Met Cys
               340                 345                 350
   cac ttg gga tgt aga ttc ggt atc aaa caa ggt ggt gta aat tgc tgg     1104
   His Leu Gly Cys Arg Phe Gly Ile Lys Gln Gly Gly Val Asn Cys Trp
           355                 360                 365
   ttc cgt gaa cct agt gaa aag ggt tct aag ttc atg gaa caa gtt gtt     1152
   Phe Arg Glu Pro Ser Glu Lys Gly Ser Lys Phe Met Glu Gln Val Val
       370                 375                 380
   gtt gaa aag att ttg cag cac aag ggt aaa gct act ggg att ttg tgt     1200
   Val Glu Lys Ile Leu Gln His Lys Gly Lys Ala Thr Gly Ile Leu Cys
   385                 390                 395                 400
   aga gat act gaa agt ggg att aaa ttc aaa atc act gga cca aag aaa     1248
   Arg Asp Thr Glu Ser Gly Ile Lys Phe Lys Ile Thr Gly Pro Lys Lys
                   405                 410                 415
   tac gtt gtt tcc ggt ggt tct ttg caa acc cca gtt ttg tta caa aaa      1296
   Tyr Val Val Ser Gly Gly Ser Leu Gln Thr Pro Val Leu Leu Gln Lys
               420                 425                 430
   tct ggt ttc aag aat aaa cat att gga gct aac tta aaa ctt cac cca      1344
   Ser Gly Phe Lys Asn Lys His Ile Gly Ala Asn Leu Lys Leu His Pro
           435                 440                 445
   gtc tcg gtt gcc ctt ggg gac ttt ggt aat gaa gtg gac ttt gaa gcc      1392
   Val Ser Val Ala Leu Gly Asp Phe Gly Asn Glu Val Asp Phe Glu Ala
       450                 455                 460
   tac aag aga cca ctt atg acc gcc gtt tgt aat gcc gtc gat gat tta      1440
   Tyr Lys Arg Pro Leu Met Thr Ala Val Cys Asn Ala Val Asp Asp Leu
   465                 470                 475                 480
   gat ggc aag gcc cat gga aca aga att gaa gcc att ttg cat gct cca      1488
   Asp Gly Lys Ala His Gly Thr Arg Ile Glu Ala Ile Leu His Ala Pro
                   485                 490                 495
   tac gtc act gcc cca ttt tac cca tgg caa tca ggt gct caa gca aga      1536
   Tyr Val Thr Ala Pro Phe Tyr Pro Trp Gln Ser Gly Ala Gln Ala Arg
               500                 505                 510
   aag aac ctc ttg aaa tat aaa caa act gtg ccg tta tta ctt ctt tct      1584
   Lys Asn Leu Leu Lys Tyr Lys Gln Thr Val Pro Leu Leu Leu Leu Ser
           515                 520                 525
   aga gat aca tca tca ggt acc gtt aca tat gat aaa caa aag cct gac      1632
   Arg Asp Thr Ser Ser Gly Thr Val Thr Tyr Asp Lys Gln Lys Pro Asp
       530                 535                 540
   gta ttg gta gtt gac tac act gtt aac aag ttt gac aga aat tcg att      1680
   Val Leu Val Val Asp Tyr Thr Val Asn Lys Phe Asp Arg Asn Ser Ile
   545                 550                 555                 560
   tta caa ggg ttt ttg gtt gct tcc gac atc ttg tat att gaa ggt gct      1728
   Leu Gln Gly Phe Leu Val Ala Ser Asp Ile Leu Tyr Ile Glu Gly Ala
                   565                 570                 575
   aaa gag att ttg tca cca caa gct tgg gta cca acc ttc aag agc aac      1776
   Lys Glu Ile Leu Ser Pro Gln Ala Trp Val Pro Thr Phe Lys Ser Asn
               580                 585                 590
   aaa cca aaa cat gct aga tcg atc aaa gac gaa gat tac gtc aaa tgg      1824
   Lys Pro Lys His Ala Arg Ser Ile Lys Asp Glu Asp Tyr Val Lys Trp
           595                 600                 605
   aga gaa acc gtg gcc aag atc cca ttt gac tcc tac ggt tcg cca tac      1872
   Arg Glu Thr Val Ala Lys Ile Pro Phe Asp Ser Tyr Gly Ser Pro Tyr
       610                 615                 620
   ggt tct gct cat caa atg agt tcg tgt aga atg tct ggt aag gga cca      1920
   Gly Ser Ala His Gln Met Ser Ser Cys Arg Met Ser Gly Lys Gly Pro
   625                 630                 635                 640
   gga tac ggt gct tgt gac act aaa gga aga tta ttt gaa tgt aac aac      1968
   Gly Tyr Gly Ala Cys Asp Thr Lys Gly Arg Leu Phe Glu Cys Asn Asn
                   645                 650                 655
   gtt tac gtt gct gat gct tcg gtt atg cct act gca tcg gga gtc aat      2016
   Val Tyr Val Ala Asp Ala Ser Val Met Pro Thr Ala Ser Gly Val Asn
               660                 665                 670
   cct atg atc act aca atg gct ttt gca aga cat gtg gcc tta tgt ctt      2064
   Pro Met Ile Thr Thr Met Ala Phe Ala Arg His Val Ala Leu Cys Leu
           675                 680                 685
   gct aaa gac ttg caa cca caa act aaa ctt tag                          2097
   Ala Lys Asp Leu Gln Pro Gln Thr Lys Leu
       690                 695
<210> 54
   <211> 698
   <212> PRT
   <213> Candida cloacae
<400> 54
   Met Ser His Gln Val Glu Asp His Asp Leu Asp Val Phe Cys Leu Leu
   1               5                   10                  15
   Ala Asp Ala Val Leu His Glu Ile Pro Pro Ser Glu Ile Val Glu Tyr
               20                  25                  30
   Leu His Pro Asp Phe Pro Lys Asp Lys Ile Glu Glu Tyr Leu Thr Gly
           35                  40                  45
   Phe Ser Arg Pro Ser Ala Val Pro Gln Phe Arg Gln Cys Ala Lys Lys
       50                  55                  60
   Leu Ile Asn Arg Gly Ser Glu Leu Ser Ile Lys Leu Phe Leu Tyr Leu
   65                  70                  75                  80
   Thr Thr Ala Leu Asp Ser Arg Ile Leu Ala Pro Ala Leu Thr Asn Ser
                   85                  90                  95
   Leu Thr Leu Ile Arg Asp Met Asp Leu Ser Gln Arg Glu Glu Leu Leu
               100                 105                 110
   Arg Ser Trp Arg Asp Ser Pro Leu Thr Ala Lys Arg Arg Leu Phe Arg
           115                 120                 125
   Val Tyr Ala Ser Phe Thr Leu Ser Thr Phe Asn Lys Leu Gly Thr Asp
       130                 135                 140
   Leu His Phe Lys Ala Leu Gly Tyr Pro Gly Arg Glu Leu Arg Thr Gln
   145                 150                 155                 160
   Ile Gln Asp Tyr Glu Val Asp Pro Phe Arg Tyr Ser Phe Met Glu Lys
                   165                 170                 175
   Leu Lys His Glu Gly His Glu Leu Phe Leu Pro Asp Ile Asp Val Leu
               180                 185                 190
   Ile Ile Gly Ser Gly Ser Gly Ala Gly Val Val Ala Gln Thr Leu Thr
           195                 200                 205
   Glu Ser Gly Leu Lys Ser Leu Val Leu Glu Lys Gly Lys Tyr Phe Ala
       210                 215                 220
   Ser Glu Glu Leu Cys Met Thr Asp Leu Asp Gly Asn Glu Ala Leu Phe
   225                 230                 235                 240
   Glu Ser Gly Gly Thr Ile Pro Ser Thr Asn Gln Gln Leu Phe Met Ile
                   245                 250                 255
   Ala Gly Ser Thr Phe Gly Gly Gly Ser Thr Val Asn Trp Ser Ala Cys
               260                 265                 270
   Leu Lys Thr Pro Phe Lys Val Arg Lys Glu Trp Tyr Asp Asp Phe Gly
           275                 280                 285
   Leu Asp Phe Val Ala Thr Gln Gln Tyr Asp Asp Cys Met Asp Tyr Val
       290                 295                 300
   Trp Lys Lys Met Gly Ala Ser Thr Glu His Ile Glu His Ser Ala Ala
   305                 310                 315                 320
   Asn Ala Val Ile Met Asp Gly Ala Ala Lys Leu Gly Tyr Ala His Arg
                   325                 330                 335
   Ala Leu Glu Gln Asn Thr Gly Gly His Val His Asp Cys Gly Met Cys
               340                 345                 350
   His Leu Gly Cys Arg Phe Gly Ile Lys Gln Gly Gly Val Asn Cys Trp
           355                 360                 365
   Phe Arg Glu Pro Ser Glu Lys Gly Ser Lys Phe Met Glu Gln Val Val
       370                 375                 380
   Val Glu Lys Ile Leu Gln His Lys Gly Lys Ala Thr Gly Ile Leu Cys
   385                 390                 395                 400
   Arg Asp Thr Glu Ser Gly Ile Lys Phe Lys Ile Thr Gly Pro Lys Lys
                   405                 410                 415
   Tyr Val Val Ser Gly Gly Ser Leu Gln Thr Pro Val Leu Leu Gln Lys
               420                 425                 430
   Ser Gly Phe Lys Asn Lys His Ile Gly Ala Asn Leu Lys Leu His Pro
   435 440 445
   Val Ser Val Ala Leu Gly Asp Phe Gly Asn Glu Val Asp Phe Glu Ala
   450 455 460
   Tyr Lys Arg Pro Leu Met Thr Ala Val Cys Asn Ala Val Asp Asp Leu
   465 470 475 480
   Asp Gly Lys Ala His Gly Thr Arg Ile Glu Ala Ile Leu His Ala Pro
   485 490 495
   Tyr Val Thr Ala Pro Phe Tyr Pro Trp Gln Ser Gly Ala Gln Ala Arg
   500 505 510
   Lys Asn Leu Leu Lys Tyr Lys Gln Thr Val Pro Leu Leu Leu Leu Ser
   515 520 525
   Arg Asp Thr Ser Ser Gly Thr Val Thr Tyr Asp Lys Gln Lys Pro Asp
   530 535 540
   Val Leu Val Val Asp Tyr Thr Val Asn Lys Phe Asp Arg Asn Ser Ile
   545 550 555 560
   Leu Gln Gly Phe Leu Val Ala Ser Asp Ile Leu Tyr Ile Glu Gly Ala
   565 570 575
   Lys Glu Ile Leu Ser Pro Gln Ala Trp Val Pro Thr Phe Lys Ser Asn
   580 585 590
   Lys Pro Lys His Ala Arg Ser Ile Lys Asp Glu Asp Tyr Val Lys Trp
   595 600 605
   Arg Glu Thr Val Ala Lys Ile Pro Phe Asp Ser Tyr Gly Ser Pro Tyr
   610 615 620
   Gly Ser Ala His Gln Met Ser Ser Cys Arg Met Ser Gly Lys Gly Pro
   625 630 635 640
   Gly Tyr Gly Ala Cys Asp Thr Lys Gly Arg Leu Phe Glu Cys Asn Asn
   645 650 655
   Val Tyr Val Ala Asp Ala Ser Val Met Pro Thr Ala Ser Gly Val Asn
   660 665 670
   Pro Met Ile Thr Thr Met Ala Phe Ala Arg His Val Ala Leu Cys Leu
   675 680 685
   Ala Lys Asp Leu Gln Pro Gln Thr Lys Leu
   690 695
<210> 55
   <211> 2097
   <212> DNA
   <213> Candida cloacae
<220>
   <221> CDS
   <222> (1)..(2097)
<400> 55
   atg aat ccc gtt gtt gaa gac agc cat tta gat gtg ttc tgc ttg tta        48
   Met Asn Pro Val Val Glu Asp Ser His Leu Asp Val Phe Cys Leu Leu
   1               5                   10                  15
   gcc gat gct gtg gtc cat gag ata cct cct agt gag atc gta gag tac        96
   Ala Asp Ala Val Val His Glu Ile Pro Pro Ser Glu Ile Val Glu Tyr
               20                  25                  30
   tta cat cct gat ttc cca aag gac aag gtt gaa gag tat ttg gct gag       144
   Leu His Pro Asp Phe Pro Lys Asp Lys Val Glu Glu Tyr Leu Ala Glu
           35                  40                  45
   ttt tct cat cct tca gca att ccc gaa ttt aga gaa gtt gca aaa aga       192
   Phe Ser His Pro Ser Ala Ile Pro Glu Phe Arg Glu Val Ala Lys Arg
       50                  55                  60
   att att aac aaa ggg act gtg ctg tca ata aag ttg ttt ttg ctc ttg       240
   Ile Ile Asn Lys Gly Thr Val Leu Ser Ile Lys Leu Phe Leu Leu Leu
   65                  70                  75                  80
   gca act gct cta gac tcg aga atc ctt gct cct gcg ttg acc aac tcg       288
   Ala Thr Ala Leu Asp Ser Arg Ile Leu Ala Pro Ala Leu Thr Asn Ser
                   85                  90                  95
   acg acg tta atc aga gat atg gat ctt tct caa aga gaa gaa tta ttg       336
   Thr Thr Leu Ile Arg Asp Met Asp Leu Ser Gln Arg Glu Glu Leu Leu
               100                 105                 110
   aga tca tgg aga gac tct cca ttc act aca aaa agg aaa ttg ttc agg       384
   Arg Ser Trp Arg Asp Ser Pro Phe Thr Thr Lys Arg Lys Leu Phe Arg
           115                 120                 125
   gtg tat aat tca ttc acc ttg aac gcg ttt agt aag acc gca aca gat       432
   Val Tyr Asn Ser Phe Thr Leu Asn Ala Phe Ser Lys Thr Ala Thr Asp
       130                 135                 140
   ttg cac ttc aaa gcg ttg gga tat cct ggt aga gag ctc agg act caa       480
   Leu His Phe Lys Ala Leu Gly Tyr Pro Gly Arg Glu Leu Arg Thr Gln
   145                 150                 155                 160
   att cag gac tat gag gtc gat cct ttc aga tat acg ttc ttg gaa aaa       528
   Ile Gln Asp Tyr Glu Val Asp Pro Phe Arg Tyr Thr Phe Leu Glu Lys
                   165                 170                 175
   ccc caa caa gac ggc cag gag tta cat ttt ccc gac att gat gtc ttg       576
   Pro Gln Gln Asp Gly Gln Glu Leu His Phe Pro Asp Ile Asp Val Leu
               180                 185                 190
   att atc ggg tct ggt tcg gga gca gga gtg gtt gct caa act ctt tcg      624
   Ile Ile Gly Ser Gly Ser Gly Ala Gly Val Val Ala Gln Thr Leu Ser
           195                 200                 205
   gaa aac gga ctt aaa tca ttg gtg ttg gaa aaa ggt aaa tac ttt tcc      672
   Glu Asn Gly Leu Lys Ser Leu Val Leu Glu Lys Gly Lys Tyr Phe Ser
       210                 215                 220
   aat gat gag ttg acc atg aat gat ttg gaa ggt agt gag gca tta ttc      720
   Asn Asp Glu Leu Thr Met Asn Asp Leu Glu Gly Ser Glu Ala Leu Phe
   225                 230                 235                 240
   gaa aat gga ggt gcc ctc agt agt acc aac caa cag ata ttt ata att      768
   Glu Asn Gly Gly Ala Leu Ser Ser Thr Asn Gln Gln Ile Phe Ile Ile
                   245                 250                 255
   gca ggt tcg act ttt ggg ggt ggt tct aca gtt aat tgg tct gcc tgt      816
   Ala Gly Ser Thr Phe Gly Gly Gly Ser Thr Val Asn Trp Ser Ala Cys
               260                 265                 270
   tta aaa act ccg ttc aaa gta aga aaa gag tgg tat gac aac ttc gga      864
   Leu Lys Thr Pro Phe Lys Val Arg Lys Glu Trp Tyr Asp Asn Phe Gly
           275                 280                 285
   ctt gat ttc gtc gca acc caa tac tac gaa gat tgt atg gat tat gtt      912
   Leu Asp Phe Val Ala Thr Gln Tyr Tyr Glu Asp Cys Met Asp Tyr Val
       290                 295                 300
   tgg aag aaa atg ggt gct tcg aac gaa aat atc gac cat tct ggt gct      960
   Trp Lys Lys Met Gly Ala Ser Asn Glu Asn Ile Asp His Ser Gly Ala
   305                 310                 315                 320
   aat agt gtt ata ttg gaa ggg tcc aaa aaa ctt ggc tac cct cac agg     1008
   Asn Ser Val Ile Leu Glu Gly Ser Lys Lys Leu Gly Tyr Pro His Arg
                   325                 330                 335
   gcc gtt gaa caa aat aat ggg ggc aaa att cat gac tgt ggt atg tgt     1056
   Ala Val Glu Gln Asn Asn Gly Gly Lys Ile His Asp Cys Gly Met Cys
               340                 345                 350
   cac ttg ggt tgt aga ttt ggt att aaa cag gga agt gta aat tgc tgg     1104
   His Leu Gly Cys Arg Phe Gly Ile Lys Gln Gly Ser Val Asn Cys Trp
           355                 360                 365
   ttc cgt ggt cca agt gaa aac gga tcc aag ttt atg caa caa gtt ctc     1152
   Phe Arg Gly Pro Ser Glu Asn Gly Ser Lys Phe Met Gln Gln Val Leu
       370                 375                 380
   gtg gat aag ata ttg caa cgt gac ggt aaa gca gtc ggt gtt ttg tgt     1200
   Val Asp Lys Ile Leu Gln Arg Asp Gly Lys Ala Val Gly Val Leu Cys
   385                 390                 395                 400
   aga gat gtt gta acc ggt gtt aag ttc aag atc act gga cca aag aaa     1248
   Arg Asp Val Val Thr Gly Val Lys Phe Lys Ile Thr Gly Pro Lys Lys
                   405                 410                 415
   att gtt gtt ttc tgg tgg ttc ttt gca aac tcc ggt ttt gtt aca aaa     1296
   Ile Val Val Phe Trp Trp Phe Phe Ala Asn Ser Gly Phe Val Thr Lys
               420                 425                 430
   tca ggt ttc aag aat aaa cac att ggt gct aac ttg aag ctc cat cca      1344
   Ser Gly Phe Lys Asn Lys His Ile Gly Ala Asn Leu Lys Leu His Pro
           435                 440                 445
   gtt tca ctt acg ctt gga gac ttc ggt aac aac gtg gat ttc gaa gct      1392
   Val Ser Leu Thr Leu Gly Asp Phe Gly Asn Asn Val Asp Phe Glu Ala
       450                 455                 460
   tac agg aaa cca att atg aca tca att tgt aat aaa gtc gaa gat tta      1440
   Tyr Arg Lys Pro Ile Met Thr Ser Ile Cys Asn Lys Val Glu Asp Leu
   465                 470                 475                 480
   gat gga aag gcc cat ggt aca aga att gaa gct atg ttg aat gct cca      1488
   Asp Gly Lys Ala His Gly Thr Arg Ile Glu Ala Met Leu Asn Ala Pro
                   485                 490                 495
   tat ggt gtt gca cca ttt ttc ccc tgg aaa tca ggc gct gaa tca aga      1536
   Tyr Gly Val Ala Pro Phe Phe Pro Trp Lys Ser Gly Ala Glu Ser Arg
               500                 505                 510
   aag gat ctc ttg aga tac aaa caa act gtg cct ata tta ctt ctt tcc      1584
   Lys Asp Leu Leu Arg Tyr Lys Gln Thr Val Pro Ile Leu Leu Leu Ser
           515                 520                 525
   aga gat act act tca ggg tct gtc aca tat gat aaa caa aaa cca gat      1632
   Arg Asp Thr Thr Ser Gly Ser Val Thr Tyr Asp Lys Gln Lys Pro Asp
       530                 535                 540
   gca ttg gtg att gat tac ctg tta aac aag ttt gac aga aac tca att      1680
   Ala Leu Val Ile Asp Tyr Leu Leu Asn Lys Phe Asp Arg Asn Ser Ile
   545                 550                 555                 560
   tta caa ggg ttt ttg ata gct tca gac ctt tta tat att gaa ggt gcc      1728
   Leu Gln Gly Phe Leu Ile Ala Ser Asp Leu Leu Tyr Ile Glu Gly Ala
                   565                 570                 575
   agt aga gat cat gtc acc tac aag ctt ggg tac caa tgg ttc aag agt      1776
   Ser Arg Asp His Val Thr Tyr Lys Leu Gly Tyr Gln Trp Phe Lys Ser
               580                 585                 590
   tct aaa cct aaa cat gcc aga tcg att gaa gat gaa gac tac gta aac      1824
   Ser Lys Pro Lys His Ala Arg Ser Ile Glu Asp Glu Asp Tyr Val Asn
           595                 600                 605
   tgg aga gct aaa gtt gca aag att cca ttt gat tcc tac gga tca cca      1872
   Trp Arg Ala Lys Val Ala Lys Ile Pro Phe Asp Ser Tyr Gly Ser Pro
       610                 615                 620
   tac ggt tca gct cac caa atg agt aca tgc aga atg tca ggt aag gga      1920
   Tyr Gly Ser Ala His Gln Met Ser Thr Cys Arg Met Ser Gly Lys Gly
   625                 630                 635                 640
   cca gga tat gga gct tgt gat acc aaa gga aaa tta ttt gaa tgt agc      1968
   Pro Gly Tyr Gly Ala Cys Asp Thr Lys Gly Lys Leu Phe Glu Cys Ser
                   645                 650                 655
   aat gtt tat gtt gca gat gct tcc act ttg cct act gca tca ggg gct      2016
   Asn Val Tyr Val Ala Asp Ala Ser Thr Leu Pro Thr Ala Ser Gly Ala
               660                 665                 670
   aat cca atg gtt agt acc atg tca ttt gca agg cac gtg tcc tta gga      2064
   Asn Pro Met Val Ser Thr Met Ser Phe Ala Arg His Val Ser Leu Gly
           675                 680                 685
   att gtt aaa gaa ttg caa caa agt aaa ctt tag                     2097
   Ile Val Lys Glu Leu Gln Gln Ser Lys Leu
       690                 695
<210> 56
   <211> 698
   <212> PRT
   <213> Candida cloacae
<400> 56
   Met Asn Pro Val Val Glu Asp Ser His Leu Asp Val Phe Cys Leu Leu
   1               5                   10                  15
   Ala Asp Ala Val Val His Glu Ile Pro Pro Ser Glu Ile Val Glu Tyr
               20                  25                  30
   Leu His Pro Asp Phe Pro Lys Asp Lys Val Glu Glu Tyr Leu Ala Glu
           35                  40                  45
   Phe Ser His Pro Ser Ala Ile Pro Glu Phe Arg Glu Val Ala Lys Arg
       50                  55                  60
   Ile Ile Asn Lys Gly Thr Val Leu Ser Ile Lys Leu Phe Leu Leu Leu
   65                  70                  75                  80
   Ala Thr Ala Leu Asp Ser Arg Ile Leu Ala Pro Ala Leu Thr Asn Ser
                   85                  90                  95
   Thr Thr Leu Ile Arg Asp Met Asp Leu Ser Gln Arg Glu Glu Leu Leu
               100                 105                 110
   Arg Ser Trp Arg Asp Ser Pro Phe Thr Thr Lys Arg Lys Leu Phe Arg
           115                 120                 125
   Val Tyr Asn Ser Phe Thr Leu Asn Ala Phe Ser Lys Thr Ala Thr Asp
       130                 135                 140
   Leu His Phe Lys Ala Leu Gly Tyr Pro Gly Arg Glu Leu Arg Thr Gln
   145                 150                 155                 160
   Ile Gln Asp Tyr Glu Val Asp Pro Phe Arg Tyr Thr Phe Leu Glu Lys
                   165                 170                 175
   Pro Gln Gln Asp Gly Gln Glu Leu His Phe Pro Asp Ile Asp Val Leu
               180                 185                 190
   Ile Ile Gly Ser Gly Ser Gly Ala Gly Val Val Ala Gln Thr Leu Ser
           195                 200                 205
   Glu Asn Gly Leu Lys Ser Leu Val Leu Glu Lys Gly Lys Tyr Phe Ser
       210                 215                 220
   Asn Asp Glu Leu Thr Met Asn Asp Leu Glu Gly Ser Glu Ala Leu Phe
   225                 230                 235                 240
   Glu Asn Gly Gly Ala Leu Ser Ser Thr Asn Gln Gln Ile Phe Ile Ile
                   245                 250                 255
   Ala Gly Ser Thr Phe Gly Gly Gly Ser Thr Val Asn Trp Ser Ala Cys
               260                 265                 270
   Leu Lys Thr Pro Phe Lys Val Arg Lys Glu Trp Tyr Asp Asn Phe Gly
           275                 280                 285
   Leu Asp Phe Val Ala Thr Gln Tyr Tyr Glu Asp Cys Met Asp Tyr Val
       290                 295                 300
   Trp Lys Lys Met Gly Ala Ser Asn Glu Asn Ile Asp His Ser Gly Ala
   305                 310                 315                 320
   Asn Ser Val Ile Leu Glu Gly Ser Lys Lys Leu Gly Tyr Pro His Arg
                   325                 330                 335
   Ala Val Glu Gln Asn Asn Gly Gly Lys Ile His Asp Cys Gly Met Cys
               340                 345                 350
   His Leu Gly Cys Arg Phe Gly Ile Lys Gln Gly Ser Val Asn Cys Trp
           355                 360                 365
   Phe Arg Gly Pro Ser Glu Asn Gly Ser Lys Phe Met Gln Gln Val Leu
       370                 375                 380
   Val Asp Lys Ile Leu Gln Arg Asp Gly Lys Ala Val Gly Val Leu Cys
   385                 390                 395                 400
   Arg Asp Val Val Thr Gly Val Lys Phe Lys Ile Thr Gly Pro Lys Lys
                   405                 410                 415
   Ile Val Val Phe Trp Trp Phe Phe Ala Asn Ser Gly Phe Val Thr Lys
               420                 425                 430
   Ser Gly Phe Lys Asn Lys His Ile Gly Ala Asn Leu Lys Leu His Pro
           435                 440                 445
   Val Ser Leu Thr Leu Gly Asp Phe Gly Asn Asn Val Asp Phe Glu Ala
       450                 455                 460
   Tyr Arg Lys Pro Ile Met Thr Ser Ile Cys Asn Lys Val Glu Asp Leu
   465                 470                 475                 480
   Asp Gly Lys Ala His Gly Thr Arg Ile Glu Ala Met Leu Asn Ala Pro
                   485                 490                 495
   Tyr Gly Val Ala Pro Phe Phe Pro Trp Lys Ser Gly Ala Glu Ser Arg
               500                 505                 510
   Lys Asp Leu Leu Arg Tyr Lys Gln Thr Val Pro Ile Leu Leu Leu Ser
           515                 520                 525
   Arg Asp Thr Thr Ser Gly Ser Val Thr Tyr Asp Lys Gln Lys Pro Asp
       530                 535                 540
   Ala Leu Val Ile Asp Tyr Leu Leu Asn Lys Phe Asp Arg Asn Ser Ile
   545                 550                 555                 560
   Leu Gln Gly Phe Leu Ile Ala Ser Asp Leu Leu Tyr Ile Glu Gly Ala
                   565                 570                 575
   Ser Arg Asp His Val Thr Tyr Lys Leu Gly Tyr Gln Trp Phe Lys Ser
               580                 585                 590
   Ser Lys Pro Lys His Ala Arg Ser Ile Glu Asp Glu Asp Tyr Val Asn
           595                 600                 605
   Trp Arg Ala Lys Val Ala Lys Ile Pro Phe Asp Ser Tyr Gly Ser Pro
       610                 615                 620
   Tyr Gly Ser Ala His Gln Met Ser Thr Cys Arg Met Ser Gly Lys Gly
   625                 630                 635                 640
   Pro Gly Tyr Gly Ala Cys Asp Thr Lys Gly Lys Leu Phe Glu Cys Ser
                   645                 650                 655
   Asn Val Tyr Val Ala Asp Ala Ser Thr Leu Pro Thr Ala Ser Gly Ala
               660                 665                 670
   Asn Pro Met Val Ser Thr Met Ser Phe Ala Arg His Val Ser Leu Gly
           675                 680                 685
   Ile Val Lys Glu Leu Gln Gln Ser Lys Leu
       690                 695
<210> 57
   <211> 948
   <212> DNA
   <213> Candida tropicalis
<220>
   <221> CDS
   <222> (1)..(948)
<400> 57
   cca ttg caa tac acc gat atc cca gtt cca gtc cct aag cca aac gaa       48
   Pro Leu Gln Tyr Thr Asp Ile Pro Val Pro Val Pro Lys Pro Asn Glu
   1               5                   10                  15
   ttg ctc gtc cac gtc aaa tac tcc ggt gtt tgt cac tca gat ata cac       96
   Leu Leu Val His Val Lys Tyr Ser Gly Val Cys His Ser Asp Ile His
               20                  25                  30
   gtc tgg aag ggt gac tgg ttc cca gca tcg aaa ttg ccc gtt gtt ggt      144
   Val Trp Lys Gly Asp Trp Phe Pro Ala Ser Lys Leu Pro Val Val Gly
           35                  40                  45
   ggt cac gaa ggt gcc ggt gtt gtc gtt gcc att ggt gaa aac gtc caa      192
   Gly His Glu Gly Ala Gly Val Val Val Ala Ile Gly Glu Asn Val Gln
       50                  55                  60
   ggc tgg aaa gta ggt gac ttg gca ggt ata aag atg ttg aat ggt tcc      240
   Gly Trp Lys Val Gly Asp Leu Ala Gly Ile Lys Met Leu Asn Gly Ser
   65                  70                  75                  80
   tgt atg aac tgt gaa tac tgt caa caa ggt gct gaa cca aac tgt ccc      288
   Cys Met Asn Cys Glu Tyr Cys Gln Gln Gly Ala Glu Pro Asn Cys Pro
                   85                  90                  95
   cac gct gat gtc tcg ggt tac tcc cac gac ggt act ttc caa cag tac      336
   His Ala Asp Val Ser Gly Tyr Ser His Asp Gly Thr Phe Gln Gln Tyr
               100                 105                 110
   gct acc gcc gat gct gtt caa gct gct aaa ttc cca gct ggt tct gat      384
   Ala Thr Ala Asp Ala Val Gln Ala Ala Lys Phe Pro Ala Gly Ser Asp
           115                 120                 125
   tta gct agc atc gca cct ata tcc tgc gcc ggt gtt act gtt tac aaa      432
   Leu Ala Ser Ile Ala Pro Ile Ser Cys Ala Gly Val Thr Val Tyr Lys
       130                 135                 140
   gca ttg aaa act gca ggc ttg cag cca ggt caa tgg gtt gcc atc tct      480
   Ala Leu Lys Thr Ala Gly Leu Gln Pro Gly Gln Trp Val Ala Ile Ser
   145                 150                 155                 160
   ggt gca gct ggt ggt ttg ggt tct ttg gct gtg caa tac gcc aag gcc      528
   Gly Ala Ala Gly Gly Leu Gly Ser Leu Ala Val Gln Tyr Ala Lys Ala
                   165                 170                 175
   atg ggt ttg aga gtc gtg gcc att gac ggt ggt gac gaa aga gga gtg      576
   Met Gly Leu Arg Val Val Ala Ile Asp Gly Gly Asp Glu Arg Gly Val
               180                 185                 190
   ttt gtc aaa tcg ttg ggt gct gaa gtt ttc gtt gat ttc acc aaa gag      624
   Phe Val Lys Ser Leu Gly Ala Glu Val Phe Val Asp Phe Thr Lys Glu
           195                 200                 205
   gcc aat gtc tct gag gct atc atc aag gct acc gac ggt ggt gcc cat      672
   Ala Asn Val Ser Glu Ala Ile Ile Lys Ala Thr Asp Gly Gly Ala His
       210                 215                 220
   ggc gtc atc aac gtt tcc att tct gaa aaa gcc atc aac cag tct gtt      720
   Gly Val Ile Asn Val Ser Ile Ser Glu Lys Ala Ile Asn Gln Ser Val
   225                 230                 235                 240
   gaa tat gtt aga act ttg gga act gtt gtc ttg gtt ggt ttg cca gct      768
   Glu Tyr Val Arg Thr Leu Gly Thr Val Val Leu Val Gly Leu Pro Ala
                   245                 250                 255
   ggt gca aag ctc gaa gct cct atc ttc aat gcc gtt gcc aaa tcc atc      816
   Gly Ala Lys Leu Glu Ala Pro Ile Phe Asn Ala Val Ala Lys Ser Ile
               260                 265                 270
   caa atc aaa ggt tct tac gtg gga aac aga aga gac act gct gag gct      864
   Gln Ile Lys Gly Ser Tyr Val Gly Asn Arg Arg Asp Thr Ala Glu Ala
           275                 280                 285
   gtt gat ttc ttc gct aga ggt ttg gtc aaa tgt cca att aag gtt gtt      912
   Val Asp Phe Phe Ala Arg Gly Leu Val Lys Cys Pro Ile Lys Val Val
       290                 295                 300
   ggg ttg agt gaa ttg cca gag att ttc aaa ttg ttg                      948
   Gly Leu Ser Glu Leu Pro Glu Ile Phe Lys Leu Leu
   305                 310                 315
<210> 58
   <211> 316
   <212> PRT
   <213> Candida tropicalis
<400> 58
   Pro Leu Gln Tyr Thr Asp Ile Pro Val Pro Val Pro Lys Pro Asn Glu
   1               5                   10                  15
   Leu Leu Val His Val Lys Tyr Ser Gly Val Cys His Ser Asp Ile His
               20                  25                  30
   Val Trp Lys Gly Asp Trp Phe Pro Ala Ser Lys Leu Pro Val Val Gly
           35                  40                  45
   Gly His Glu Gly Ala Gly Val Val Val Ala Ile Gly Glu Asn Val Gln
       50                  55                  60
   Gly Trp Lys Val Gly Asp Leu Ala Gly Ile Lys Met Leu Asn Gly Ser
   65                  70                  75                  80
   Cys Met Asn Cys Glu Tyr Cys Gln Gln Gly Ala Glu Pro Asn Cys Pro
                   85                  90                  95
   His Ala Asp Val Ser Gly Tyr Ser His Asp Gly Thr Phe Gln Gln Tyr
               100                 105                 110
   Ala Thr Ala Asp Ala Val Gln Ala Ala Lys Phe Pro Ala Gly Ser Asp
           115                 120                 125
   Leu Ala Ser Ile Ala Pro Ile Ser Cys Ala Gly Val Thr Val Tyr Lys
       130                 135                 140
   Ala Leu Lys Thr Ala Gly Leu Gln Pro Gly Gln Trp Val Ala Ile Ser
   145                 150                 155                 160
   Gly Ala Ala Gly Gly Leu Gly Ser Leu Ala Val Gln Tyr Ala Lys Ala
                   165                 170                 175
   Met Gly Leu Arg Val Val Ala Ile Asp Gly Gly Asp Glu Arg Gly Val
               180                 185                 190
   Phe Val Lys Ser Leu Gly Ala Glu Val Phe Val Asp Phe Thr Lys Glu
           195                 200                 205
   Ala Asn Val Ser Glu Ala Ile Ile Lys Ala Thr Asp Gly Gly Ala His
       210                 215                 220
   Gly Val Ile Asn Val Ser Ile Ser Glu Lys Ala Ile Asn Gln Ser Val
   225                 230                 235                 240
   Glu Tyr Val Arg Thr Leu Gly Thr Val Val Leu Val Gly Leu Pro Ala
                   245                 250                 255
   Gly Ala Lys Leu Glu Ala Pro Ile Phe Asn Ala Val Ala Lys Ser Ile
               260                 265                 270
   Gln Ile Lys Gly Ser Tyr Val Gly Asn Arg Arg Asp Thr Ala Glu Ala
           275                 280                 285
   Val Asp Phe Phe Ala Arg Gly Leu Val Lys Cys Pro Ile Lys Val Val
       290                 295                 300
   Gly Leu Ser Glu Leu Pro Glu Ile Phe Lys Leu Leu
   305                 310                 315
<210> 59
   <211> 948
   <212> DNA
   <213> Candida tropicalis
<220>
   <221> CDS
   <222> (1)..(948)
<400> 59
   aaa tta gaa tac aag gac atc cca gtt cca aag cca aag cca aac gaa        48
   Lys Leu Glu Tyr Lys Asp Ile Pro Val Pro Lys Pro Lys Pro Asn Glu
   1               5                   10                  15
   ttg ctc atc aac gtc aag tac tcc ggt gtc tgc cac act gat tta cac        96
   Leu Leu Ile Asn Val Lys Tyr Ser Gly Val Cys His Thr Asp Leu His
               20                  25                  30
   gcc tgg aag ggt gac tgg cca ttg gac acc aag ttg cca ttg gtg ggt       144
   Ala Trp Lys Gly Asp Trp Pro Leu Asp Thr Lys Leu Pro Leu Val Gly
           35                  40                  45
   ggt cac gaa ggt gct ggt gtt gtt gtt gcc att ggt gac aat gtc aag       192
   Gly His Glu Gly Ala Gly Val Val Val Ala Ile Gly Asp Asn Val Lys
       50                  55                  60
   gga tgg aag gtc ggt gat ttg gcc ggt gtc aag tgg ttg aac ggt tcc       240
   Gly Trp Lys Val Gly Asp Leu Ala Gly Val Lys Trp Leu Asn Gly Ser
   65                  70                  75                  80
   tgt atg aac tgt gag tac tgt caa cag ggt gcc gaa cca aac tgt cca       288
   Cys Met Asn Cys Glu Tyr Cys Gln Gln Gly Ala Glu Pro Asn Cys Pro
                   85                  90                  95
   cag gct gac ttg tct ggt tac acc cac gac ggt tct ttc cag caa tac       336
   Gln Ala Asp Leu Ser Gly Tyr Thr His Asp Gly Ser Phe Gln Gln Tyr
               100                 105                 110
   gcc act gca gat gcc gtg caa gcc gct aga att cca gct ggt act gat       384
   Ala Thr Ala Asp Ala Val Gln Ala Ala Arg Ile Pro Ala Gly Thr Asp
           115                 120                 125
   tta gcc aac gtt gcc ccc atc ttg tgt gct ggt gtc act gtt tac aag       432
   Leu Ala Asn Val Ala Pro Ile Leu Cys Ala Gly Val Thr Val Tyr Lys
       130                 135                 140
   gcc ttg aag acc gcc gac ttg cag cca ggt caa tgg gtc gcc att tcc       480
   Ala Leu Lys Thr Ala Asp Leu Gln Pro Gly Gln Trp Val Ala Ile Ser
   145                 150                 155                 160
   ggt gcc gct ggt ggt ttg ggt tct ttg gcc gtt caa tac gcc aag gcc       528
   Gly Ala Ala Gly Gly Leu Gly Ser Leu Ala Val Gln Tyr Ala Lys Ala
                   165                 170                 175
   atg ggc tac aga gtt gtc gcc atc gat ggt ggt gcc gac aag ggt gag       576
   Met Gly Tyr Arg Val Val Ala Ile Asp Gly Gly Ala Asp Lys Gly Glu
               180                 185                 190
   ttc gtc aag tct ttg ggc gct gag gtc ttt gtt gat ttc ctc aag gaa       624
   Phe Val Lys Ser Leu Gly Ala Glu Val Phe Val Asp Phe Leu Lys Glu
           195                 200                 205
   aag gac att gtt ggt gct gtc aag aag gca acc gat ggt ggc cca cac       672
   Lys Asp Ile Val Gly Ala Val Lys Lys Ala Thr Asp Gly Gly Pro His
       210                 215                 220
   ggt gcc gtt aac gtt tcc atc tcc gaa aag gcc atc aac caa tct gtc       720
   Gly Ala Val Asn Val Ser Ile Ser Glu Lys Ala Ile Asn Gln Ser Val
   225                 230                 235                 240
   gac tac gtt aga acc ttg ggt aag gtt gtc ttg gtc ggt ttg cca gct       768
   Asp Tyr Val Arg Thr Leu Gly Lys Val Val Leu Val Gly Leu Pro Ala
                   245                 250                 255
   ggc tcc aag gtt tct gct cca gtc ttt gac tcc gtc gtc aag tcc atc       816
   Gly Ser Lys Val Ser Ala Pro Val Phe Asp Ser Val Val Lys Ser Ile
               260                 265                 270
   caa atc aag ggt tcc tat gtc ggt aac aga aag gac act gcc gaa gct       864
   Gln Ile Lys Gly Ser Tyr Val Gly Asn Arg Lys Asp Thr Ala Glu Ala
           275                 280                 285
   gtt gac ttt ttc tcc aga ggc ttg atc aag tgt cca atc aag gtt gtc       912
   Val Asp Phe Phe Ser Arg Gly Leu Ile Lys Cys Pro Ile Lys Val Val
       290                 295                 300
   ggt ttg agt gaa ttg cca gaa gtc tac aag ttg atg                       948
   Gly Leu Ser Glu Leu Pro Glu Val Tyr Lys Leu Met
   305                 310                 315
<210> 60
   <211> 316
   <212> PRT
   <213> Candida tropicalis
<400> 60
   Lys Leu Glu Tyr Lys Asp Ile Pro Val Pro Lys Pro Lys Pro Asn Glu
   1               5                   10                  15
   Leu Leu Ile Asn Val Lys Tyr Ser Gly Val Cys His Thr Asp Leu His
               20                  25                  30
   Ala Trp Lys Gly Asp Trp Pro Leu Asp Thr Lys Leu Pro Leu Val Gly
           35                  40                  45
   Gly His Glu Gly Ala Gly Val Val Val Ala Ile Gly Asp Asn Val Lys
       50                  55                  60
   Gly Trp Lys Val Gly Asp Leu Ala Gly Val Lys Trp Leu Asn Gly Ser
   65                  70                  75                  80
   Cys Met Asn Cys Glu Tyr Cys Gln Gln Gly Ala Glu Pro Asn Cys Pro
                   85                  90                  95
   Gln Ala Asp Leu Ser Gly Tyr Thr His Asp Gly Ser Phe Gln Gln Tyr
               100                 105                 110
   Ala Thr Ala Asp Ala Val Gln Ala Ala Arg Ile Pro Ala Gly Thr Asp
           115                 120                 125
   Leu Ala Asn Val Ala Pro Ile Leu Cys Ala Gly Val Thr Val Tyr Lys
       130                 135                 140
   Ala Leu Lys Thr Ala Asp Leu Gln Pro Gly Gln Trp Val Ala Ile Ser
   145                 150                 155                 160
   Gly Ala Ala Gly Gly Leu Gly Ser Leu Ala Val Gln Tyr Ala Lys Ala
                   165                 170                 175
   Met Gly Tyr Arg Val Val Ala Ile Asp Gly Gly Ala Asp Lys Gly Glu
               180                 185                 190
   Phe Val Lys Ser Leu Gly Ala Glu Val Phe Val Asp Phe Leu Lys Glu
           195                 200                 205
   Lys Asp Ile Val Gly Ala Val Lys Lys Ala Thr Asp Gly Gly Pro His
       210                 215                 220
   Gly Ala Val Asn Val Ser Ile Ser Glu Lys Ala Ile Asn Gln Ser Val
   225                 230                 235                 240
   Asp Tyr Val Arg Thr Leu Gly Lys Val Val Leu Val Gly Leu Pro Ala
                   245                 250                 255
   Gly Ser Lys Val Ser Ala Pro Val Phe Asp Ser Val Val Lys Ser Ile
               260                 265                 270
   Gln Ile Lys Gly Ser Tyr Val Gly Asn Arg Lys Asp Thr Ala Glu Ala
           275                 280                 285
   Val Asp Phe Phe Ser Arg Gly Leu Ile Lys Cys Pro Ile Lys Val Val
       290                 295                 300
   Gly Leu Ser Glu Leu Pro Glu Val Tyr Lys Leu Met
   305                 310                 315
<210> 61
   <211> 456
   <212> DNA
   <213> Candida tropicalis
<220>
   <221> CDS
   <222> (1)..(456)
<400> 61
   aac tgt gag ttt tgc caa cag ggc gct gaa cct aat tgt cca aga gcc      48
   Asn Cys Glu Phe Cys Gln Gln Gly Ala Glu Pro Asn Cys Pro Arg Ala
   1               5                   10                  15
   gac atg tct gga tat acc cac gat ggg act ttc caa caa tat gct acc      96
   Asp Met Ser Gly Tyr Thr His Asp Gly Thr Phe Gln Gln Tyr Ala Thr
               20                  25                  30
   gcc gat gcc gtc caa gct gcc aag atc cca gaa ggc gca gac atg gct      144
   Ala Asp Ala Val Gln Ala Ala Lys Ile Pro Glu Gly Ala Asp Met Ala
           35                  40                  45
   agt atc gcc ccg atc ttg tgt gct ggt gtg acc gtg tac aag gct ttg      192
   Ser Ile Ala Pro Ile Leu Cys Ala Gly Val Thr Val Tyr Lys Ala Leu
       50                  55                  60
   aag aac gcc gac ttg ttg gct ggc caa tgg gtg gct atc tct ggt gct      240
   Lys Asn Ala Asp Leu Leu Ala Gly Gln Trp Val Ala Ile Ser Gly Ala
   65                  70                  75                  80
   ggt ggt ggt ttg ggc tcc ttg ggt gtg cag tac gct aaa gcc atg ggt      288
   Gly Gly Gly Leu Gly Ser Leu Gly Val Gln Tyr Ala Lys Ala Met Gly
                   85                  90                  95
   tac aga gtg tta gcc atc gat ggt ggt gat gag aga gga gag ttt gtc      336
   Tyr Arg Val Leu Ala Ile Asp Gly Gly Asp Glu Arg Gly Glu Phe Val
               100                 105                 110
   aag tca ttg ggc gcc gaa gtg tac att gac ttc ctt aag gaa cag gac      384
   Lys Ser Leu Gly Ala Glu Val Tyr Ile Asp Phe Leu Lys Glu Gln Asp
           115                 120                 125
   att gtt agt gcc att aga aag gca act ggt ggt ggc cca cac ggt gtt      432
   Ile Val Ser Ala Ile Arg Lys Ala Thr Gly Gly Gly Pro His Gly Val
       130                 135                 140
   att aac gtc tcg gtg tcc gaa aag                                      456
   Ile Asn Val Ser Val Ser Glu Lys
   145                 150
<210> 62
   <211> 152
   <212> PRT
   <213> Candida tropicalis
<400> 62
   Asn Cys Glu Phe Cys Gln Gln Gly Ala Glu Pro Asn Cys Pro Arg Ala
   1               5                   10                  15
   Asp Met Ser Gly Tyr Thr His Asp Gly Thr Phe Gln Gln Tyr Ala Thr
               20                  25                  30
   Ala Asp Ala Val Gln Ala Ala Lys Ile Pro Glu Gly Ala Asp Met Ala
           35                  40                  45
   Ser Ile Ala Pro Ile Leu Cys Ala Gly Val Thr Val Tyr Lys Ala Leu
       50                  55                  60
   Lys Asn Ala Asp Leu Leu Ala Gly Gln Trp Val Ala Ile Ser Gly Ala
   65                  70                  75                  80
   Gly Gly Gly Leu Gly Ser Leu Gly Val Gln Tyr Ala Lys Ala Met Gly
                   85                  90                  95
   Tyr Arg Val Leu Ala Ile Asp Gly Gly Asp Glu Arg Gly Glu Phe Val
               100                 105                 110
   Lys Ser Leu Gly Ala Glu Val Tyr Ile Asp Phe Leu Lys Glu Gln Asp
           115                 120                 125
   Ile Val Ser Ala Ile Arg Lys Ala Thr Gly Gly Gly Pro His Gly Val
       130                 135                 140
   Ile Asn Val Ser Val Ser Glu Lys
   145                 150
<210> 63
   <211> 948
   <212> DNA
   <213> Candida tropicalis
<220>
   <221> CDS
   <222> (1)..(948)
<400> 63
   aag tta gaa tac aaa gac gtg ccg gtc cct gtc cct aaa ccc aac gaa        48
   Lys Leu Glu Tyr Lys Asp Val Pro Val Pro Val Pro Lys Pro Asn Glu
   1               5                   10                  15
   ttg ctt gtc aac gtc aag tac tcg ggt gtg tgt cat tct gac ttg cat        96
   Leu Leu Val Asn Val Lys Tyr Ser Gly Val Cys His Ser Asp Leu His
               20                  25                  30
   gtc tgg aaa ggc gac tgg ccc att cct gcc aag ttg ccc ttg gtg gga       144
   Val Trp Lys Gly Asp Trp Pro Ile Pro Ala Lys Leu Pro Leu Val Gly
           35                  40                  45
   ggt cac gaa ggt gct ggt gtc gtt gtc ggc atg ggt gac aac gtc aag       192
   Gly His Glu Gly Ala Gly Val Val Val Gly Met Gly Asp Asn Val Lys
       50                  55                  60
   ggc tgg aag gtg ggg gac ttg gct ggt atc aag tgg ttg aat ggt tcg       240
   Gly Trp Lys Val Gly Asp Leu Ala Gly Ile Lys Trp Leu Asn Gly Ser
   65                  70                  75                  80
   tgt atg aac tgt gag ttt tgc caa cag ggc gca gaa cct aac tgt tca       288
   Cys Met Asn Cys Glu Phe Cys Gln Gln Gly Ala Glu Pro Asn Cys Ser
                   85                  90                  95
   aga gcc gac atg tct ggg tat acc cac gat gga act ttc caa caa tac       336
   Arg Ala Asp Met Ser Gly Tyr Thr His Asp Gly Thr Phe Gln Gln Tyr
               100                 105                 110
   gcc act gct gat gct gtc caa gct gcc aag atc cca gaa ggc gcc gac       384
   Ala Thr Ala Asp Ala Val Gln Ala Ala Lys Ile Pro Glu Gly Ala Asp
           115                 120                 125
   atg gct agt atc gcc ccg atc ttg tgc gct ggt gtg acc gtg tac aag       432
   Met Ala Ser Ile Ala Pro Ile Leu Cys Ala Gly Val Thr Val Tyr Lys
       130                 135                 140
   gct ttg aag aac gcc gac ttg ttg gct ggc caa tgg gtg gct atc tct      480
   Ala Leu Lys Asn Ala Asp Leu Leu Ala Gly Gln Trp Val Ala Ile Ser
   145                 150                 155                 160
   ggt gct ggt ggt ggt ttg ggc tcc ttg ggt gtg cag tac gct aaa gcc      528
   Gly Ala Gly Gly Gly Leu Gly Ser Leu Gly Val Gln Tyr Ala Lys Ala
                   165                 170                 175
   atg ggt tac aga gtg ttg gct atc gac ggt ggt gac gag aga gga gag      576
   Met Gly Tyr Arg Val Leu Ala Ile Asp Gly Gly Asp Glu Arg Gly Glu
               180                 185                 190
   ttt gtc aag tcc ttg ggc gcc gaa gtg tac att gac ttc ctt aag gaa      624
   Phe Val Lys Ser Leu Gly Ala Glu Val Tyr Ile Asp Phe Leu Lys Glu
           195                 200                 205
   cag gac atc gtt agt gct atc aga aag gca act ggt ggt ggt cca cac      672
   Gln Asp Ile Val Ser Ala Ile Arg Lys Ala Thr Gly Gly Gly Pro His
       210                 215                 220
   ggt gtt att aac gtg tca gtg tcc gaa aag gca atc aac cag tcg gtg      720
   Gly Val Ile Asn Val Ser Val Ser Glu Lys Ala Ile Asn Gln Ser Val
   225                 230                 235                 240
   gag tac gtc aga act ttg ggg aaa gtg gtt tta gtt agc ttg ccg gca      768
   Glu Tyr Val Arg Thr Leu Gly Lys Val Val Leu Val Ser Leu Pro Ala
                   245                 250                 255
   ggt ggt aaa ctc act gct cct ctt ttc gag tct gtt gct aga tca atc      816
   Gly Gly Lys Leu Thr Ala Pro Leu Phe Glu Ser Val Ala Arg Ser Ile
               260                 265                 270
   cag att aga act acg tgt gtt ggc aac aga aag gat act act gaa gct      864
   Gln Ile Arg Thr Thr Cys Val Gly Asn Arg Lys Asp Thr Thr Glu Ala
           275                 280                 285
   att gat ttc ttt gtt aga ggg ttg atc gat tgc cca att aaa gtc gct      912
   Ile Asp Phe Phe Val Arg Gly Leu Ile Asp Cys Pro Ile Lys Val Ala
       290                 295                 300
   ggt tta agt gaa gtg cca gag att ttt gac ttg atg                      948
   Gly Leu Ser Glu Val Pro Glu Ile Phe Asp Leu Met
   305                 310                 315
<210> 64
   <211> 316
   <212> PRT
   <213> Candida tropicalis
<400> 64
   Lys Leu Glu Tyr Lys Asp Val Pro Val Pro Val Pro Lys Pro Asn Glu
   1               5                   10                  15
   Leu Leu Val Asn Val Lys Tyr Ser Gly Val Cys His Ser Asp Leu His
               20                  25                  30
   Val Trp Lys Gly Asp Trp Pro Ile Pro Ala Lys Leu Pro Leu Val Gly
           35                  40                  45
   Gly His Glu Gly Ala Gly Val Val Val Gly Met Gly Asp Asn Val Lys
       50                  55                  60
   Gly Trp Lys Val Gly Asp Leu Ala Gly Ile Lys Trp Leu Asn Gly Ser
   65                  70                  75                  80
   Cys Met Asn Cys Glu Phe Cys Gln Gln Gly Ala Glu Pro Asn Cys Ser
                   85                  90                  95
   Arg Ala Asp Met Ser Gly Tyr Thr His Asp Gly Thr Phe Gln Gln Tyr
               100                 105                 110
   Ala Thr Ala Asp Ala Val Gln Ala Ala Lys Ile Pro Glu Gly Ala Asp
           115                 120                 125
   Met Ala Ser Ile Ala Pro Ile Leu Cys Ala Gly Val Thr Val Tyr Lys
       130                 135                 140
   Ala Leu Lys Asn Ala Asp Leu Leu Ala Gly Gln Trp Val Ala Ile Ser
   145                 150                 155                 160
   Gly Ala Gly Gly Gly Leu Gly Ser Leu Gly Val Gln Tyr Ala Lys Ala
                   165                 170                 175
   Met Gly Tyr Arg Val Leu Ala Ile Asp Gly Gly Asp Glu Arg Gly Glu
               180                 185                 190
   Phe Val Lys Ser Leu Gly Ala Glu Val Tyr Ile Asp Phe Leu Lys Glu
           195                 200                 205
   Gln Asp Ile Val Ser Ala Ile Arg Lys Ala Thr Gly Gly Gly Pro His
       210                 215                 220
   Gly Val Ile Asn Val Ser Val Ser Glu Lys Ala Ile Asn Gln Ser Val
   225                 230                 235                 240
   Glu Tyr Val Arg Thr Leu Gly Lys Val Val Leu Val Ser Leu Pro Ala
                   245                 250                 255
   Gly Gly Lys Leu Thr Ala Pro Leu Phe Glu Ser Val Ala Arg Ser Ile
               260                 265                 270
   Gln Ile Arg Thr Thr Cys Val Gly Asn Arg Lys Asp Thr Thr Glu Ala
           275                 280                 285
   Ile Asp Phe Phe Val Arg Gly Leu Ile Asp Cys Pro Ile Lys Val Ala
       290                 295                 300
   Gly Leu Ser Glu Val Pro Glu Ile Phe Asp Leu Met
   305                 310                 315
<210> 65
   <211> 948
   <212> DNA
   <213> Candida tropicalis
<220>
   <221> CDS
   <222> (1)..(948)
<400> 65
   gaa tta gaa tac aaa gat atc cca gtg cca acc cca aag gcc aac gaa      48
   Glu Leu Glu Tyr Lys Asp Ile Pro Val Pro Thr Pro Lys Ala Asn Glu
   1               5                   10                  15
   ttg ctc atc aac gtc aaa tac tcg ggt gtc tgc cac act gat ttg cac      96
   Leu Leu Ile Asn Val Lys Tyr Ser Gly Val Cys His Thr Asp Leu His
               20                  25                  30
   gcc tgg aag ggt gac tgg cca ttg gcc acc aag ttg cca ttg gtt ggt      144
   Ala Trp Lys Gly Asp Trp Pro Leu Ala Thr Lys Leu Pro Leu Val Gly
           35                  40                  45
   ggt cac gaa ggt gct ggt gtc gtt gtc ggc atg ggt gaa aac gtc aag      192
   Gly His Glu Gly Ala Gly Val Val Val Gly Met Gly Glu Asn Val Lys
       50                  55                  60
   ggc tgg aag att ggt gac ttc gcc ggt atc aaa tgg ttg aac ggt tcc      240
   Gly Trp Lys Ile Gly Asp Phe Ala Gly Ile Lys Trp Leu Asn Gly Ser
   65                  70                  75                  80
   tgt atg tcc tgt gag ttc tgt caa caa ggt gct gaa cca aac tgt ggt      288
   Cys Met Ser Cys Glu Phe Cys Gln Gln Gly Ala Glu Pro Asn Cys Gly
                   85                  90                  95
   gag gcc gac ttg tct ggt tac acc cac gat ggt tct ttc gaa caa tac      336
   Glu Ala Asp Leu Ser Gly Tyr Thr His Asp Gly Ser Phe Glu Gln Tyr
               100                 105                 110
   gcc act gct gat gct gtt caa gcc gcc aga atc cca gct ggt act gat      384
   Ala Thr Ala Asp Ala Val Gln Ala Ala Arg Ile Pro Ala Gly Thr Asp
           115                 120                 125
   ttg gcc gaa gtt gcc cca atc ttg tgt gcg ggt gtc acc gtc tac aaa      432
   Leu Ala Glu Val Ala Pro Ile Leu Cys Ala Gly Val Thr Val Tyr Lys
       130                 135                 140
   gcc ttg aag act gcc gac ttg gcc gct ggt caa tgg gtc gct atc tcc      480
   Ala Leu Lys Thr Ala Asp Leu Ala Ala Gly Gln Trp Val Ala Ile Ser
   145                 150                 155                 160
   ggt gct ggt ggt ggt ttg ggt tcc ttg gct gtc caa tac gcc gtc gcc      528
   Gly Ala Gly Gly Gly Leu Gly Ser Leu Ala Val Gln Tyr Ala Val Ala
                   165                 170                 175
   atg ggc ttg aga gtc gtt gcc att gac ggt ggt gac gaa aag ggt gcc      576
   Met Gly Leu Arg Val Val Ala Ile Asp Gly Gly Asp Glu Lys Gly Ala
               180                 185                 190
   ttt gtc aag tcc ttg ggt gct gaa gcc tac att gat ttc ctc aag gaa        624
   Phe Val Lys Ser Leu Gly Ala Glu Ala Tyr Ile Asp Phe Leu Lys Glu
           195                 200                 205
   aag gac att gtc tct gct gtc aag aag gcc acc gat gga ggt cca cac        672
   Lys Asp Ile Val Ser Ala Val Lys Lys Ala Thr Asp Gly Gly Pro His
       210                 215                 220
   ggt gct atc aat gtt tcc gtt tcc gaa aaa gcc att gac caa tcc gtc        720
   Gly Ala Ile Asn Val Ser Val Ser Glu Lys Ala Ile Asp Gln Ser Val
   225                 230                 235                 240
   gag tac gtt aga cca ttg ggt aag gtt gtt ttg gtt ggt ttg cca gct        768
   Glu Tyr Val Arg Pro Leu Gly Lys Val Val Leu Val Gly Leu Pro Ala
                   245                 250                 255
   ggc tcc aag gtc act gct ggt gtt ttc gaa gcc gtt gtc aag tcc att        816
   Gly Ser Lys Val Thr Ala Gly Val Phe Glu Ala Val Val Lys Ser Ile
               260                 265                 270
   gaa atc aag ggt tcc tat gtc ggt aac aga aag gat acc gcc gaa gcc        864
   Glu Ile Lys Gly Ser Tyr Val Gly Asn Arg Lys Asp Thr Ala Glu Ala
           275                 280                 285
   gtt gac ttt ttc tcc aga ggc ttg atc aag tgt cca atc aag att gtt        912
   Val Asp Phe Phe Ser Arg Gly Leu Ile Lys Cys Pro Ile Lys Ile Val
       290                 295                 300
   ggc ttg agt gaa ttg cca cag gtc ttc aag ttg atg                        948
   Gly Leu Ser Glu Leu Pro Gln Val Phe Lys Leu Met
   305                 310                 315
<210> 66
   <211> 316
   <212> PRT
   <213> Candida tropicalis
<400> 66
   Glu Leu Glu Tyr Lys Asp Ile Pro Val Pro Thr Pro Lys Ala Asn Glu
   1               5                   10                  15
   Leu Leu Ile Asn Val Lys Tyr Ser Gly Val Cys His Thr Asp Leu His
               20                  25                  30
   Ala Trp Lys Gly Asp Trp Pro Leu Ala Thr Lys Leu Pro Leu Val Gly
           35                  40                  45
   Gly His Glu Gly Ala Gly Val Val Val Gly Met Gly Glu Asn Val Lys
       50                  55                  60
   Gly Trp Lys Ile Gly Asp Phe Ala Gly Ile Lys Trp Leu Asn Gly Ser
   65                  70                  75                  80
   Cys Met Ser Cys Glu Phe Cys Gln Gln Gly Ala Glu Pro Asn Cys Gly
                   85                  90                  95
   Glu Ala Asp Leu Ser Gly Tyr Thr His Asp Gly Ser Phe Glu Gln Tyr
               100                 105                 110
   Ala Thr Ala Asp Ala Val Gln Ala Ala Arg Ile Pro Ala Gly Thr Asp
           115                 120                 125
   Leu Ala Glu Val Ala Pro Ile Leu Cys Ala Gly Val Thr Val Tyr Lys
       130                 135                 140
   Ala Leu Lys Thr Ala Asp Leu Ala Ala Gly Gln Trp Val Ala Ile Ser
   145                 150                 155                 160
   Gly Ala Gly Gly Gly Leu Gly Ser Leu Ala Val Gln Tyr Ala Val Ala
                   165                 170                 175
   Met Gly Leu Arg Val Val Ala Ile Asp Gly Gly Asp Glu Lys Gly Ala
               180                 185                 190
   Phe Val Lys Ser Leu Gly Ala Glu Ala Tyr Ile Asp Phe Leu Lys Glu
           195                 200                 205
   Lys Asp Ile Val Ser Ala Val Lys Lys Ala Thr Asp Gly Gly Pro His
       210                 215                 220
   Gly Ala Ile Asn Val Ser Val Ser Glu Lys Ala Ile Asp Gln Ser Val
   225                 230                 235                 240
   Glu Tyr Val Arg Pro Leu Gly Lys Val Val Leu Val Gly Leu Pro Ala
                   245                 250                 255
   Gly Ser Lys Val Thr Ala Gly Val Phe Glu Ala Val Val Lys Ser Ile
               260                 265                 270
   Glu Ile Lys Gly Ser Tyr Val Gly Asn Arg Lys Asp Thr Ala Glu Ala
           275                 280                 285
   Val Asp Phe Phe Ser Arg Gly Leu Ile Lys Cys Pro Ile Lys Ile Val
       290                 295                 300
   Gly Leu Ser Glu Leu Pro Gln Val Phe Lys Leu Met
   305                 310                 315
<210> 67
   <211> 1992
   <212> DNA
   <213> Candida tropicalis
<220>
   <221> CDS
   <222> (1)..(1992)
<400> 67
   ctt atg tta tta tgt gac ggg atc atc cac gaa acc acc gtc gac caa      48
   Leu Met Leu Leu Cys Asp Gly Ile Ile His Glu Thr Thr Val Asp Gln
   1               5                   10                  15
   atc aaa gac gtt att gct cct gac ttc cct gct gac aag tac gaa gag      96
   Ile Lys Asp Val Ile Ala Pro Asp Phe Pro Ala Asp Lys Tyr Glu Glu
               20                  25                  30
   tac gtc agg aca ttc acc aaa ccc tcc gaa acc cca ggg ttc agg gaa     144
   Tyr Val Arg Thr Phe Thr Lys Pro Ser Glu Thr Pro Gly Phe Arg Glu
           35                  40                  45
   acc gtc tac aac aca gtc aac gca aac acc acg gac gca atc cac cag     192
   Thr Val Tyr Asn Thr Val Asn Ala Asn Thr Thr Asp Ala Ile His Gln
       50                  55                  60
   ttc att atc ttg acc aat gtt ttg gca tcc agg gtc ttg gct cca gct     240
   Phe Ile Ile Leu Thr Asn Val Leu Ala Ser Arg Val Leu Ala Pro Ala
   65                  70                  75                  80
   ttg acc aac tcg ttg acg cct atc aag gac atg agc ttg gaa gac cgt     288
   Leu Thr Asn Ser Leu Thr Pro Ile Lys Asp Met Ser Leu Glu Asp Arg
                   85                  90                  95
   gaa aaa ttg ttg gcc tcg tgg cgc gac tcc cca atc gct gcc aaa agg     336
   Glu Lys Leu Leu Ala Ser Trp Arg Asp Ser Pro Ile Ala Ala Lys Arg
               100                 105                 110
   aaa ttg ttc agg ttg gtt tcc acg ctt acc ttg gtt act ttc acg aga     384
   Lys Leu Phe Arg Leu Val Ser Thr Leu Thr Leu Val Thr Phe Thr Arg
           115                 120                 125
   ttg gcc aat gag ttg cat ttg aaa gcc att cac tat cca gga aga gaa     432
   Leu Ala Asn Glu Leu His Leu Lys Ala Ile His Tyr Pro Gly Arg Glu
       130                 135                 140
   gac cgt gaa aag gct tat gaa acc cag gag att gac cct ttc aag tac     480
   Asp Arg Glu Lys Ala Tyr Glu Thr Gln Glu Ile Asp Pro Phe Lys Tyr
   145                 150                 155                 160
   cag ttt atg gaa aag cca aag ttt gac ggc gct gag ttg tac ttg cca     528
   Gln Phe Met Glu Lys Pro Lys Phe Asp Gly Ala Glu Leu Tyr Leu Pro
                   165                 170                 175
   gat att gat gtt atc att att gga tct ggt gcc ggt gct ggt gtt gtg     576
   Asp Ile Asp Val Ile Ile Ile Gly Ser Gly Ala Gly Ala Gly Val Val
               180                 185                 190
   gcc cac act ttg gcc aac gat ggc ttc aag agt ttg gtt ttg gaa aag     624
   Ala His Thr Leu Ala Asn Asp Gly Phe Lys Ser Leu Val Leu Glu Lys
           195                 200                 205
   ggc aaa tac ttt agc aac tcc gag ttg aac ttt gat gac aag gac ggc     672
   Gly Lys Tyr Phe Ser Asn Ser Glu Leu Asn Phe Asp Asp Lys Asp Gly
       210                 215                 220
   gtt caa gaa tta tac caa agt gga ggt act ttg act aca gtc aac caa       720
   Val Gln Glu Leu Tyr Gln Ser Gly Gly Thr Leu Thr Thr Val Asn Gln
   225                 230                 235                 240
   cag ttg ttt gtt ctt gct ggt tcc act ttt ggt ggc ggt acc act gtc       768
   Gln Leu Phe Val Leu Ala Gly Ser Thr Phe Gly Gly Gly Thr Thr Val
                   245                 250                 255
   aat tgg tca gcc tgt ctt aag acg cca ttc aag gtg cgt aag gaa tgg       816
   Asn Trp Ser Ala Cys Leu Lys Thr Pro Phe Lys Val Arg Lys Glu Trp
               260                 265                 270
   tat gat gag ttt ggt gtt gac ttt gct gct gat gaa gca tac gat aaa       864
   Tyr Asp Glu Phe Gly Val Asp Phe Ala Ala Asp Glu Ala Tyr Asp Lys
           275                 280                 285
   gcg cag gat tat gtt tgg cag caa atg gga gct tct acc gaa ggc atc       912
   Ala Gln Asp Tyr Val Trp Gln Gln Met Gly Ala Ser Thr Glu Gly Ile
       290                 295                 300
   acc cac tct ttg gct aac gag att att att gaa ggt ggt aag aaa tta       960
   Thr His Ser Leu Ala Asn Glu Ile Ile Ile Glu Gly Gly Lys Lys Leu
   305                 310                 315                 320
   ggt tac aag gcc aag gta tta gac caa aac agc ggt ggt cat cct cag      1008
   Gly Tyr Lys Ala Lys Val Leu Asp Gln Asn Ser Gly Gly His Pro Gln
                   325                 330                 335
   cac aga tgc ggt ttc tgt tat ttg ggc tgt aag cac ggt atc aag cag      1056
   His Arg Cys Gly Phe Cys Tyr Leu Gly Cys Lys His Gly Ile Lys Gln
               340                 345                 350
   ggt tct gtt aat aac tgg ttt aga gac gca gct gcc cac ggt tcc cag      1104
   Gly Ser Val Asn Asn Trp Phe Arg Asp Ala Ala Ala His Gly Ser Gln
           355                 360                 365
   ttc atg caa cag gtt aga gtt ttg caa ata ctt aac aag aag ggg atc      1152
   Phe Met Gln Gln Val Arg Val Leu Gln Ile Leu Asn Lys Lys Gly Ile
       370                 375                 380
   gct tac ggt atc ttg tgt gag gat gtt gta acc ggc gcc aag ttc acc      1200
   Ala Tyr Gly Ile Leu Cys Glu Asp Val Val Thr Gly Ala Lys Phe Thr
   385                 390                 395                 400
   att act ggc ccc aaa aag ttt gtt gtt gct gcc ggt gct ttg aac act      1248
   Ile Thr Gly Pro Lys Lys Phe Val Val Ala Ala Gly Ala Leu Asn Thr
                   405                 410                 415
   cca tct gtg ttg gtc aac tcc ggc ttc aag aac aag aac atc ggt aag      1296
   Pro Ser Val Leu Val Asn Ser Gly Phe Lys Asn Lys Asn Ile Gly Lys
               420                 425                 430
   aac tta act ttg cac cca gtt tct gtc gtg ttt ggt gat ttt ggc aaa      1344
   Asn Leu Thr Leu His Pro Val Ser Val Val Phe Gly Asp Phe Gly Lys
           435                 440                 445
   gac gtt caa gca gac cac ttc cac aac tcc atc atg act gcc ctt tgt      1392
   Asp Val Gln Ala Asp His Phe His Asn Ser Ile Met Thr Ala Leu Cys
       450                 455                 460
   tca gaa gcc gct gat tta gac ggc aag ggc cat gga tgc aga att gaa      1440
   Ser Glu Ala Ala Asp Leu Asp Gly Lys Gly His Gly Cys Arg Ile Glu
   465                 470                 475                 480
   acc atc ttg aac gct cca ttc atc cag gct tca ttc tta cca tgg aga     1488
   Thr Ile Leu Asn Ala Pro Phe Ile Gln Ala Ser Phe Leu Pro Trp Arg
                   485                 490                 495
   ggt agt aac gag gct aga cga gac ttg ttg cgt tac aac aac atg gtg     1536
   Gly Ser Asn Glu Ala Arg Arg Asp Leu Leu Arg Tyr Asn Asn Met Val
               500                 505                 510
   gcg atg ttg ctc ctt agt cgt gac acc acc agt ggt tcc gtt tct gct     1584
   Ala Met Leu Leu Leu Ser Arg Asp Thr Thr Ser Gly Ser Val Ser Ala
           515                 520                 525
   cat cca acc aaa cct gaa gct ttg gtt gtc gag tac gac gtg aac aag     1632
   His Pro Thr Lys Pro Glu Ala Leu Val Val Glu Tyr Asp Val Asn Lys
       530                 535                 540
   ttt gac aga aac tcg atc ttg cag gca ttg ttg gtc act gct gac ttg     1680
   Phe Asp Arg Asn Ser Ile Leu Gln Ala Leu Leu Val Thr Ala Asp Leu
   545                 550                 555                 560
   ttg tat atc caa ggt gcc aag aga atc ctt agt cca cag gca tgg gtg     1728
   Leu Tyr Ile Gln Gly Ala Lys Arg Ile Leu Ser Pro Gln Ala Trp Val
                   565                 570                 575
   cca att ttt gaa tcc gac aag cca aag gat aag aga tca atc aag gac     1776
   Pro Ile Phe Glu Ser Asp Lys Pro Lys Asp Lys Arg Ser Ile Lys Asp
               580                 585                 590
   gag gac tat gtc gaa tgg aga gcc aag gtt gcc aag att cct ttc gac     1824
   Glu Asp Tyr Val Glu Trp Arg Ala Lys Val Ala Lys Ile Pro Phe Asp
           595                 600                 605
   acc tac ggc tca cct tat ggt tcg gca cat caa atg tct tct tgc cgt     1872
   Thr Tyr Gly Ser Pro Tyr Gly Ser Ala His Gln Met Ser Ser Cys Arg
       610                 615                 620
   atg tca ggt aag ggt cct aaa tac ggt gct gtt gac acc gat ggt aga     1920
   Met Ser Gly Lys Gly Pro Lys Tyr Gly Ala Val Asp Thr Asp Gly Arg
   625                 630                 635                 640
   ttg ttt gaa tgt tcg aat gtt tat gtt gcc gat gca agt ctt ttg cca     1968
   Leu Phe Glu Cys Ser Asn Val Tyr Val Ala Asp Ala Ser Leu Leu Pro
                   645                 650                 655
   act gca agc ggt gct aat cct atg                                     1992
   Thr Ala Ser Gly Ala Asn Pro Met
               660
<210> 68
   <211> 664
   <212> PRT
   <213> Candida tropicalis
<400> 68
   Leu Met Leu Leu Cys Asp Gly Ile Ile His Glu Thr Thr Val Asp Gln
   1               5                   10                  15
   Ile Lys Asp Val Ile Ala Pro Asp Phe Pro Ala Asp Lys Tyr Glu Glu
               20                  25                  30
   Tyr Val Arg Thr Phe Thr Lys Pro Ser Glu Thr Pro Gly Phe Arg Glu
           35                  40                  45
   Thr Val Tyr Asn Thr Val Asn Ala Asn Thr Thr Asp Ala Ile His Gln
       50                  55                  60
   Phe Ile Ile Leu Thr Asn Val Leu Ala Ser Arg Val Leu Ala Pro Ala
   65                  70                  75                  80
   Leu Thr Asn Ser Leu Thr Pro Ile Lys Asp Met Ser Leu Glu Asp Arg
                   85                  90                  95
   Glu Lys Leu Leu Ala Ser Trp Arg Asp Ser Pro Ile Ala Ala Lys Arg
               100                 105                 110
   Lys Leu Phe Arg Leu Val Ser Thr Leu Thr Leu Val Thr Phe Thr Arg
           115                 120                 125
   Leu Ala Asn Glu Leu His Leu Lys Ala Ile His Tyr Pro Gly Arg Glu
       130                 135                 140
   Asp Arg Glu Lys Ala Tyr Glu Thr Gln Glu Ile Asp Pro Phe Lys Tyr
   145                 150                 155                 160
   Gln Phe Met Glu Lys Pro Lys Phe Asp Gly Ala Glu Leu Tyr Leu Pro
                   165                 170                 175
   Asp Ile Asp Val Ile Ile Ile Gly Ser Gly Ala Gly Ala Gly Val Val
               180                 185                 190
   Ala His Thr Leu Ala Asn Asp Gly Phe Lys Ser Leu Val Leu Glu Lys
           195                 200                 205
   Gly Lys Tyr Phe Ser Asn Ser Glu Leu Asn Phe Asp Asp Lys Asp Gly
       210                 215                 220
   Val Gln Glu Leu Tyr Gln Ser Gly Gly Thr Leu Thr Thr Val Asn Gln
   225                 230                 235                 240
   Gln Leu Phe Val Leu Ala Gly Ser Thr Phe Gly Gly Gly Thr Thr Val
                   245                 250                 255
   Asn Trp Ser Ala Cys Leu Lys Thr Pro Phe Lys Val Arg Lys Glu Trp
               260                 265                 270
   Tyr Asp Glu Phe Gly Val Asp Phe Ala Ala Asp Glu Ala Tyr Asp Lys
   275 280 285
   Ala Gln Asp Tyr Val Trp Gln Gln Met Gly Ala Ser Thr Glu Gly Ile
   290 295 300
   Thr His Ser Leu Ala Asn Glu Ile Ile Ile Glu Gly Gly Lys Lys Leu
   305 310 315 320
   Gly Tyr Lys Ala Lys Val Leu Asp Gln Asn Ser Gly Gly His Pro Gln
   325 330 335
   His Arg Cys Gly Phe Cys Tyr Leu Gly Cys Lys His Gly Ile Lys Gln
   340 345 350
   Gly Ser Val Asn Asn Trp Phe Arg Asp Ala Ala Ala His Gly Ser Gln
   355 360 365
   Phe Met Gln Gln Val Arg Val Leu Gln Ile Leu Asn Lys Lys Gly Ile
   370 375 380
   Ala Tyr Gly Ile Leu Cys Glu Asp Val Val Thr Gly Ala Lys Phe Thr
   385 390 395 400
   Ile Thr Gly Pro Lys Lys Phe Val Val Ala Ala Gly Ala Leu Asn Thr
   405 410 415
   Pro Ser Val Leu Val Asn Ser Gly Phe Lys Asn Lys Asn Ile Gly Lys
   420 425 430
   Asn Leu Thr Leu His Pro Val Ser Val Val Phe Gly Asp Phe Gly Lys
   435 440 445
   Asp Val Gln Ala Asp His Phe His Asn Ser Ile Met Thr Ala Leu Cys
   450 455 460
   Ser Glu Ala Ala Asp Leu Asp Gly Lys Gly His Gly Cys Arg Ile Glu
   465 470 475 480
   Thr Ile Leu Asn Ala Pro Phe Ile Gln Ala Ser Phe Leu Pro Trp Arg
   485 490 495
   Gly Ser Asn Glu Ala Arg Arg Asp Leu Leu Arg Tyr Asn Asn Met Val
   500 505 510
   Ala Met Leu Leu Leu Ser Arg Asp Thr Thr Ser Gly Ser Val Ser Ala
   515 520 525
   His Pro Thr Lys Pro Glu Ala Leu Val Val Glu Tyr Asp Val Asn Lys
       530                 535                 540
   Phe Asp Arg Asn Ser Ile Leu Gln Ala Leu Leu Val Thr Ala Asp Leu
   545                 550                 555                 560
   Leu Tyr Ile Gln Gly Ala Lys Arg Ile Leu Ser Pro Gln Ala Trp Val
                   565                 570                 575
   Pro Ile Phe Glu Ser Asp Lys Pro Lys Asp Lys Arg Ser Ile Lys Asp
               580                 585                 590
   Glu Asp Tyr Val Glu Trp Arg Ala Lys Val Ala Lys Ile Pro Phe Asp
           595                 600                 605
   Thr Tyr Gly Ser Pro Tyr Gly Ser Ala His Gln Met Ser Ser Cys Arg
       610                 615                 620
   Met Ser Gly Lys Gly Pro Lys Tyr Gly Ala Val Asp Thr Asp Gly Arg
   625                 630                 635                 640
   Leu Phe Glu Cys Ser Asn Val Tyr Val Ala Asp Ala Ser Leu Leu Pro
                   645                 650                 655
   Thr Ala Ser Gly Ala Asn Pro Met
               660

## Beschreibung

### Gebiet der Erfindung

Gegenstand der Erfindung sind gentechnisch modifizierte *Candida tropicalis* Zellen, deren Verwendung sowie ein Verfahren zur Herstellung von ω-Hydroxy-Carbonsäuren und ω-Hydroxy-Carbonsäureestern.

### Stand der Technik

*Candida tropicalis* ist aufgrund seiner Fähigkeit, Dicarbonsäuren aus Alkanen zu bilden, ein gut charakterisierter Schlauchpilz.

WO91/006660 beschreibt durch Unterbrechung der POX4 und/oder POX5 Gene in der β-Oxidation vollständig inhibierte *Candida tropicalis* Zellen, welche erhöhte Ausbeuten an α,ω-Dicarbonsäuren erzielen.

WO00/020566 beschreibt Cytochrom P450 Monooxygenasen und NADPH Cytochrom P450 Oxidoreduktasen von *Candida tropicalis* und deren Verwendung zur Beeinflussung der ω-Hydroxylierung, dem ersten Schritt der ω-Oxidation.

WO03/089610 beschreibt Enzyme aus *Candida tropicalis,* die den zweiten Schritt der ω-Oxidation, die Umsetzung eines Fettalkohols zu einem Aldehyd, katalysieren, sowie deren Verwendung zur verbesserten Produktion von Dicarbonsäuren.

DATABASE EMBL, accession no. GU056287, Ness J.E., offenbart die Teilgensequenz von dem Candida tropicalis strain H5343 clone B11 alcohol dehydrogenase (ADH) Gen.

Die DE102007060705 beschreibt Zellen, welche gegenüber ihrem Wildtyp derart gentechnisch verändert worden ist, dass sie im Vergleich zu ihrem Wildtyp mehr [omega]-Aminocarbonsäuren oder mehr aus [omega]-Aminocarbonsäuren abgeleitete Lactame aus Carbonsäuren oder Carbonsäureestern herzustellen vermögen.

Fabritius et al., Identification and production of 3-hydroxy-DELTA-9-cis-1,18-octadecenedioic acid by mutants of Candida tropicalis, Appl Microbiol Biotechnol. 1996, 45, 432-438 offenbart omega-Hydroxycarbonsäuren und omega-Hydroxycarbonsäureester und deren Verwendung zur Herstellung von Polymeren.

Die bisher beschriebenen Zellen und Verfahren sind zur Herstellung von ω-Hydroxy-Carbonsäuren oder deren Ester ungeeignet, da die ω-Hydroxy-Carbonsäuren immer nur als kurzfristiges Intermediat vorliegen und sofort weiter verstoffwechselt werden.

ω-Hydroxy-Carbonsäuren und deren Ester sind als Vorläuferstoffe von Polymeren wirtschaftlich bedeutende Verbindungen, wodurch die gewerbliche Anwendbarkeit der vorliegenden Erfindung begründet ist.

Aufgabe der Erfindung war es, eine Möglichkeit zu finden, ω-Hydroxy-Carbonsäuren bzw. ω-Hydroxy-Carbonsäureestern fermentativ in ausreichenden Mengen, insbesondere in dem die Zellen umgebenden Medium, bereitzustellen.

### Beschreibung der Erfindung

Überraschenderweise wurde gefunden, dass die im Folgenden beschriebenen Zellen einen Beitrag zur Lösung der gestellten Aufgabe leisten.

Gegenstand der vorliegenden Erfindung ist daher eine Zelle wie in Anspruch 1 beschrieben.
Ein weiterer Gegenstand der Erfindung ist die Verwendung der erfindungsgemäßen Zelle sowie ein Verfahren zur Herstellung von ω-Hydroxy-Carbonsäuren und ω-Hydroxy-Carbonsäureestern unter Einsatz der erfindungsgemäßen Zellen.

Vorteile der Erfindung sind die schonende Umsetzung des eingesetzten Eduktes zu den ω-Hydroxy-Carbonsäuren und korrespondierenden Estern sowie eine hohe Spezifität des Verfahrens und somit einhergehender hohen Ausbeute bezogen auf das eingesetzte Edukt.

Ein Gegenstand der vorliegenden Erfindung ist eine *Candida tropicalis* Zelle, insbesondere eine vom Stamm ATCC 20336, welche dadurch gekennzeichnet ist, dass die Zelle im Vergleich zu Ihrem Wildtyp eine verminderte Aktivität mindestens eines der Enzyme aufweist, welche kodiert werden durch die intronfreien Nukleinsäuresequenzen ausgewählt aus den beiden Gruppe bestehend aus
A)Seq ID Nr. 63,
B) eine Sequenz, die zu mindestens 80 %, besonders bevorzugt zu mindestens 90 %, darüber hinaus bevorzugt zu mindestens 95 % und am meisten bevorzugt zu mindestens 99 % identisch zu ist, wobei unter der Aktivität der Enzyme die enzymatische Aktivität zu verstehen ist, die die Umsetzungen von 12-Hydroxy-Dodekansäure zu 1,12-Dodekandisäure durch die gesamte Zelle katalysiert,
   weiterhin dadurch gekennzeichnet, dass die Verminderung der enzymatischen Aktivität erreicht wird durch Modifikation eines Genes umfassend eine der vorgenannten Nukleinsäuresequenzen, wobei die Modifikation ausgewählt ist aus der Gruppe umfassend Insertion von Fremd-DNA in das Gen, Deletion mindestens von Teilen des Gens, Punktmutationen in der Gensequenz, Aussetzen des Gens unter den Einfluss von RNA-Interferenz und Austausch von Teilen des Gens mit Fremd-DNA, insbesondere des Promoterbereichs.

In diesem Zusammenhang erfindungsgemäß bevorzugte Nukleinsäuresequenzgruppe ist Gruppe A).

Unter einem "Wildtyp" einer Zelle wird im Zusammenhang mit der vorliegenden Erfindung bevorzugt der Ausgangsstamm verstanden, aus dem die erfindungsgemäße Zelle durch Manipulation an den Elementen (wie beispielsweise die Gene umfassend die genannten Nukleinsäuresequenzen kodierend für entsprechendes Enzym oder die im entsprechenden Gen enthaltenen Promotoren, die mit den genannten Nukleinsäuresequenzen funktional verknüpft sind), die die Aktivitäten der Enzyme kodiert durch die genannten Nukleinsäure Seq ID Nr. beeinflussen, hervorgegangen ist. Wird beispielsweise die Aktivität des Enzyms kodiert durch die Seq ID Nr. 1 in dem Stamm ATCC 20336 durch Unterbrechung des korrespondierenden Gens vermindert, so ist als "Wildtyp" der unveränderte und zur entsprechenden Manipulation eingesetzte Stamm ATCC 20336 zu betrachten.

Unter dem Begriff "Gen" wird im Zusammenhang mit der vorliegenden Erfindung nicht nur der kodierende bzw. in mRNA transkribierte DNA-Bereich, das "Strukturgen", verstanden, sondern außerdem Promotor-, mögliche Intron-, Enhancer- und andere Regulatorsequenz-, sowie Terminator-Bereiche, verstanden.

Unter dem Begriff "Aktivität eines Enzyms" im Zusammenhang mit der Erfindung ist immer die enzymatische Aktivität zu verstehen, die die Umsetzungen von 12-Hydroxy-Dodekansäure zu 1,12-Dodekandisäure durch die gesamte Zelle katalysiert.
Diese Aktivität wird bevorzugt bestimmt nach dem folgenden Verfahren:
Ausgehend von einer Einzelkolonie wird ein 100 ml Erlenmeyerkolben mit 10 ml YM Medium (0,3% Hefe Extrakt, 0,3% Malz Extrakt, 0,5% Pepton und 1,0% (w/w) Glucose) bei 30 °C und 90 rpm über 24 h kultiviert. Anschließend werden ausgehend von dieser Kultur 10 ml in einen 1 1 Erlenmeyerkolben mit 100 ml Produktionsmedium (für 1 1: 25 g Glucose, 7,6 g NH₄Cl, 1,5 g Na₂SO₄, 300 ml eines 1 mM Kaliumphosphatpuffers (pH 7,0), 20 mg ZnSO₄ x 7 H₂O, 20 mg MnSO₄ x 4 H₂O, 20 mg Nikotinsäure, 20 mg Pyridoxin, 8 mg Thiamin und 6 mg Pantothenat) angeimpft. Die Kultivierung erfolgt für 24 h bei 30 °C.
Nach 24 h wird der Zellsuspension 12-Hydroxy-Dodekansäure zudosiert, so dass die Konzentration nicht größer als 0,5 g/l beträgt. Zudem wird Glucose oder Glycerin als Co-Substrat zugegeben, so dass die Konzentration des Co-Substrates nicht unter 0,2 g/l sinkt. Nach 0h, 0,5 h, 1h, und dann bis zu 24 h Kultivierungsdauer stündlich werden Proben (1 ml) für die Messung von 12-Hydroxy-Dodekansäure, 12-Oxo-Dodekansäure und der 1, 12-Dodekandisäure und der korrespondierenden Methylester, sowie der Überprüfung der Zellzahl entnommen. Der pH Wert wird nach jeder Messung mit 6 N NaOH bzw. 4 N H₂SO₄ zwischen 5,0 und 6,5 gehalten. Während der Kultivierungen wird das Zellwachstum durch Überprüfung der "Colony Forming Units"(CFU) überprüft. Die Abnahme von 12-Hydroxy-Dodekansäure Und die Produktion von 1,12-Dodekandisäure bzw. der korrespondierenden Methylester werden durch LC-MS überprüft. Dafür werden 500 µl Kulturbrühe auf pH 1 eingestellt und anschließend mit dem gleichen Volumen Diethylether oder Ethylacetat extrahiert und mittels LC-MS analysiert.
Das Messsystem besteht aus einer HP1100 HPLC (Agilent Technologies, Waldbronn, D) mit Degasser, Autosampler und Säulenofen, gekoppelt an einen massenselektiven Quadrupol Detektor MSD (Agilent Technologies, Waldbronn, D). Die chromatographische Trennung wird auf einer Umkehrphase z.B. 125x2 mm Luna C18(2) Säule (Phenomenex, Aschaffenburg, D) bei 40°C erzielt. Die Gradientenelution erfolgt bei einem Fluss von 0,3 ml/min (A: 0.02% Ameisensäure in Wasser und B: 0.02% Ameisensäure in Acetonitril). Alternativ werden die organischen Extrakte mittels GC-FID (Perkin Elmer, Rodgau-Jügesheim, D) analysiert. Die chromatographische Trennung erfolgt auf einer Methylpolysiloxan (5% Phenyl) Phase z.B. Elite 5, 30 m, 0,25 mm ID, 0,25 µm FD (Perkin Elmer, Rodgau-Jügesheim, D). Freie Säuren werden vor der Messung mit einem Methylierungsreagenz z.B. Trimethylsulphoniumhydroxid "TMSH" (Macherey-Nagel GmbH & Co. KG, Düren, D) versetzt und bei der Injektion in die entsprechenden Methylester überführt.
Durch Berechnung der gemessenen Konzentration an 1,12-Dodekandisäure und der Zellzahl zu dem Probenzeitpunkt, kann die spezifische Produktionsrate der 1,12-Dodekandisäure aus 12-Hydroxy-Dodekansäure bestimmt und somit die oben definierte "Aktivität eines Enzyms" in einer Zelle bestimmt werden.
Unter der Formulierung "im Vergleich zu ihrem Wildtyp verminderte Aktivität" wird vorzugsweise eine um mindestens 50% verminderte, besonders bevorzugt um mindestens 90%, darüber hinaus bevorzugt um mindestens 99.9%, darüber hinaus noch mehr bevorzugt um mindestens 99,99% und am meisten bevorzugt um mindestens 99,999% verminderte Aktivität bezogen auf die Wildtyp-Aktivität verstanden.
Die Verminderung der Aktivität der erfindungsgemäßen Zelle im Vergleich zu ihrem Wildtyp wird bestimmt nach oben beschriebenen Verfahren zur Bestimmung der Aktivität unter Einsatz von möglichst gleichen Zellzahlen/-konzentrationen, wobei die Zellen unter gleichen Bedingungen wie beispielsweise Medium, Begasung, Agitation angezogen wurden.

Die "Nukleotid-Identität" relativ zu den angebenden Sequenzen, kann mit Hilfe bekannter Verfahren bestimmt werden. Generell werden besondere Computerprogramme mit Algorithmen unter Berücksichtigung spezieller Erfordernisse verwendet.
Bevorzugte Verfahren zur Bestimmung der Identität erzeugen zunächst die größte Übereinstimmung zwischen den zu vergleichenden Sequenzen. Computer-Programme zur Bestimmung der Identität umfassen, sind jedoch nicht beschränkt auf, das GCG-Programmpaket, einschließlich
- GAP (Deveroy, J. et al., Nucleic Acid Research 12 (1984), Seite 387, Genetics Computer Group University of Wisconsin, Medicine (Wi), und
- BLASTP, BLASTN und FASTA (Altschul, S. et al., Journal of Molecular Biology 215 (1990), Seiten 403-410. Das BLAST-Programm kann erhalten werden vom National Center For Biotechnology Information (NCBI) und aus weiteren Quellen (BLAST Handbuch, Altschul S. et al., NCBI NLM NIH Bethesda ND 22894; Altschul S. et al., vorstehend).
Der bekannte Smith-Waterman Algorithmus kann ebenso zur Bestimmung der Nukleotid-Identität verwendet werden.

Bevorzugte Parameter für die Bestimmung der "Nukleotid-Identität" sind bei Verwendung des BLASTN-Programms (Altschul, S. et al., Journal of Molecular Biology 215 (1990), Seiten 403-410:

| | |
|---|---|
| Expect Threshold: | 10 |
| Word size: | 28 |
| Match Score: | 1 |
| Mismatch Score: | -2 |
| Gap costs: | Linear |

Die vorstehenden Parameter sind die default-Parameter im Nukleotidsequenzvergleich.
Das GAP-Programm ist ebenso zur Verwendung mit den vorstehenden Parametern geeignet.
Eine Identität von 80 % gemäß dem vorstehenden Algorithmus bedeutet im Zusammenhang mit der vorliegenden Erfindung 80 % Identität. Das gleiche gilt für höhere Identitäten.

Durch den Begriff "welche kodiert werden durch die intronfreien Nukleinsäuresequenzen" ist klar herausgestellt, dass bei einem Sequenzvergleich mit den hier angegebenen Sequenzen die zu vergleichenden Nukleinsäuresequenzen zuvor um etwaige Introns bereinigt werden müssen.

Alle angegebenen Prozent (%) sind wenn nicht anders angegeben Massenprozent.

Verfahren zur Verminderung von enzymatischen Aktivitäten in Mikroorganismen sind dem Fachmann bekannt.
Insbesondere molekularbiologische Techniken bieten sich hier an. Anleitung zur Modifikation und Verminderung von Proteinexpressionen und damit einhergehender Enzymaktivitätsverringerung speziell für *Candida tropicalis,* insbesondere zum Unterbrechen spezieller Gene findet der Fachmann in der WO91/006660; WO03/100013; Picataggio et al. Mol Cell Biol. 1991 Sep;11(9):4333-9; Rohrer et al. Appl Microbiol Biotechnol. 1992 Feb;36(5):650-4; Picataggio et al. Biotechnology (N Y). 1992 Aug;10(8):894-8; Ueda et al. Biochim Biophys Acta. 2003 Mar 17;1631(2):160-8; Ko et al. Appl Environ Microbiol. 2006 Jun;72(6):4207-13; Hara et al. Arch Microbiol. 2001 Nov;176(5):364-9; Kanayama et al. J Bacteriol. 1998 Feb;180(3):690-8.

Erfindungsgemäße Zellen sind dadurch gekennzeichnet, dass die Verminderung der enzymatischen Aktivität erreicht wird durch Modifikation mindestens eines Genes umfassend eine der Sequenzen ausgewählt aus den zuvor genannten Nukleinsäuresequenzgruppen A) und B), wobei die Modifikation ausgewählt ist aus der Gruppe umfassend, bevorzugt bestehend aus, Insertion von Fremd-DNA in das Gen, Deletion mindestens von Teilen des Gens, Punktmutationen in der Gensequenz und Aussetzen des Gens unter den Einfluss von RNA-Interferenz oder Austausch von Teilen des Gens mit Fremd-DNA, insbesondere des Promoterbereichs.
Unter Fremd-DNA ist in diesem Zusammenhang jegliche DNA-Sequenz zu verstehe, die dem Gen (und nicht dem Organismus) "fremd" ist, d.h. auch *Candida tropicalis* endogene DNA-Sequenzen können in diesem Zusammenhang als "Fremd-DNA" fungieren.
In diesem Zusammenhang ist es insbesondere bevorzugt, dass das Gen durch Insertion eines Selektionsmarkergens unterbrochen wird, somit die Fremd-DNA ein Selektionsmarkergen ist, wobei bevorzugt die Insertion durch homologe Rekombination in den Genlocus erfolgte.
In diesem Zusammenhang kann es vorteilhaft sein, wenn das Selektionsmarkergen durch weitere Funktionalitäten erweitert wird, die wiederum eine anschließende Entfernung aus dem Gen ermöglichen, dies kann beispielsweise durch ein Cre/loxP-System, durch *Flippase Recognition Targets* (FRT) oder homologe Rekombination erreicht werden.

Erfindungsgemäß bevorzugte Zellen sind dadurch gekennzeichnet, dass sie in ihrer β-Oxidation mindestens teilweise, bevorzugt vollständig, blockiert sind, da dies den Abfluss von Substrat verhindert und somit höhere Titer ermöglicht werden.
Beispiele für teilweise in Ihrer β-Oxidation blockierten *Candida tropicalis* Zellen sind in der EP0499622 als Stämme H41, H41B, H51, H45, H43, H53, H534, H534B und H435 beschrieben, von denen eine erfindungsgemäß bevorzugte *Candida tropicalis* Zelle sich ableitent.
Weitere, in der β-Oxidation blockierte *Candida tropicalis* Zellen werden beispielsweise in der WO03/100013 beschrieben.
In diesem Zusammenhang sind Zellen bevorzugt, bei denen die β-Oxidation durch eine induzierte Missfunktion mindestens eines der Gene POX2, POX4 oder POX5 hervorgerufen wird.
Somit sind in diesem Zusammenhang Zellen bevorzugt, die dadurch gekennzeichnet sind, dass eine erfindungsgemäß bevorzugte *Candida tropicalis* Zelle sich ableitet von Stämmen ausgewählt aus der Gruppe bestehend aus
ATCC 20962 und dem in US2004/0014198 beschriebenen *Candida tropicalis* HDC100.

Ein weiteren Beitrag zur Lösung der der Erfindung gestellten Aufgabe leistet die Verwendung der erfindungsgemäßen Zellen zur Herstellung von ω-Hydroxy-Carbonsäuren oder ω-Hydroxy-Carbonsäureestern.

Insbesondere die Verwendung der erfindungsgemäßen Zellen zur Herstellung von ω-Hydroxy-Carbonsäuren oder ω-Hydroxy-Carbonsäureestern mit einer Kettenlänge der Carbonsäure von 6 bis 24, bevorzugt 8 bis 18 und besonders bevorzugt 10 bis 16 Kohlenstoffatomen, welche bevorzugt linear, gesättigt und unsubstituiert sind, und einer Kettenlänge der Alkoholkomponente des Esters von 1 bis 4, insbesondere von 1 oder 2 Kohlenstoffatomen, ist vorteilhaft. In diesem Zusammenhang ist es bevorzugt, dass es sich bei der ω-Hydroxy-Carbonsäuren um 12-Hydroxy-Dodekansäure und bei dem ω-Hydroxy-Carbonsäureester um 12-Hydroxy-Dodekansäuremethylester handelt.
Bevorzugte Verwendung ist erfindungsgemäß dadurch gekennzeichnet, dass erfindungsgemäß bevorzugte Zellen wie oben beschrieben verwendet werden.

Ein weiteren Beitrag zur Lösung der der Erfindung gestellten Aufgabe leistet ein Verfahren zur Herstellung der zuvor beschriebenen erfindungsgemäßen C. *tropicalis* Zelle umfassend die Verfahrensschritte:
I) Bereitstellen einer C. *tropicalis* Zelle, bevorzugt einer in ihrer β-Oxidation mindestens teilweise, bevorzugt vollständig, blockierten Zelle
II) Modifikation mindestens eines Genes umfassend eine der Intronfreien Nukleinsäuresequenzenausgewählt aus den zuvor genannten Nukleinsäuresequenzgruppen A) und B) durch Insertion von Fremd-DNA, insbesondere von DNA kodierend für ein Selektionsmarkergen, in das Gen, Deletion mindestens von Teilen des Gens, Punktmutationen in der Gensequenz und Aussetzen des Gens unter den Einfluss von RNA-Interferenz oder Austausch von Teilen des Gens mit Fremd-DNA, insbesondere des Promoterbereichs.

Ein weiteren Beitrag zur Lösung der der Erfindung gestellten Aufgabe leistet ein Verfahren zur Herstellung von ω-Hydroxy-Carbonsäuren oder ω-Hydroxy-Carbonsäureestern, insbesondere von ω-Hydroxy-Carbonsäuren oder ω-Hydroxy-Carbonsäureestern mit einer Kettenlänge der Carbonsäure von 6 bis 24, bevorzugt 8 bis 18 und besonders bevorzugt 10 bis 16 Kohlenstoffatomen, welche bevorzugt linear, gesättigt und unsubstituiert sind, und einer Kettenlänge der Alkoholkomponente des Esters von 1 bis 4, insbesondere von 1 oder 2 Kohlenstoffatomen, insbesondere von 12-Hydroxy-Dodekansäure oder 12-Hydroxy-Dodekansäuremethylester umfassend die Verfahrensschritte
A) In Kontakt bringen einer zuvor beschriebenen erfindungsgemäßen Zelle mit einem Medium beinhaltend eine Carbonsäure bzw. einen Carbonsäureester, insbesondere eine Carbonsäure oder einen Carbonsäureester mit einer Kettenlänge der Carbonsäure von 6 bis 24, bevorzugt 8 bis 18 und besonders bevorzugt 10 bis 16 Kohlenstoffatomen, welche bevorzugt linear, gesättigt und unsubstituiert sind, und einer Kettenlänge der Alkoholkomponente des Esters von 1 bis 4 Kohlenstoffatomen, insbesondere Dodekansäure oder Dodekansäuremethylester,
B) Kultivieren der Zelle unter Bedingungen, die es der Zelle ermöglichen aus der Carbonsäure bzw. dem Carbonsäureester die korrespondierende ω-Hydroxy-Carbonsäure oder ω-Hydroxy-Carbonsäureester zu bilden und
C) gegebenenfalls Isolierung der gebildeten ω-Hydroxy-Carbonsäure oder ω-Hydroxy-Carbonsäureester.

Erfindungsgemäß bevorzugte Verfahren setzen oben als erfindungsgemäß bevorzugt genannte Zellen ein.

Somit ist beispielsweise ein Verfahren zur Herstellung von 12-Hydroxy-Dodekansäure oder 12-Hydroxy-Dodekansäuremethylester umfassend die Verfahrensschritte
a) In Kontakt bringen einer in ihrer β-Oxidation mindestens teilweise blockierten *Candida tropicalis* Zelle vom Stamm ATTC 20336, die im Vergleich zu Ihrem Wildtyp eine verminderte Aktivität mindestens eines der Enzyme aufweist, welche kodiert werden durch die intronfreien Nukleinsäuresequenzen ausgewählt aus den zuvor genannten Nukleinsäuresequenzgruppen A) und B) wobei die Verminderung der enzymatischen Aktivität erreicht wird durch Modifikation eines Genes umfassend eine der Nukleinsäuresequenzen ausgewählt aus den zuvor genannten Nukleinsäuresequenzgruppen A) und B),
   wobei die Modifikation aus Insertion eines Selektionsmarkergens in das Gen besteht,
   mit einem Medium beinhaltend Dodekansäure oder
   Dodekansäuremethylester,
b) Kultivieren der Zelle unter Bedingungen, die es der Zelle ermöglichen aus der Carbonsäure bzw. dem Carbonsäureester die korrespondierende ω-Hydroxy-Carbonsäure oder ω-Hydroxy-Carbonsäureester zu bilden und
c) gegebenenfalls Isolierung der gebildeten ω-Hydroxy-Carbonsäure oder ω-Hydroxy-Carbonsäureester ganz besonders bevorzugt.

Geeignete Kultivierungsbedingungen für *Candida tropicalis* sind dem Fachmann bekannt. Insbesondere für Verfahrensschritt b) geeignete Bedingungen sind solche, die dem Fachmann aus Biokonversionsverfahren zur Herstellung von Dicarbonsäuren mit *Candida tropicalis* bekannt sind.

Solche Kultivierungsbedingungen sind beispielsweise in der WO00/017380 und WO00/015828 beschrieben.

Zur Isolierung der gebildeten ω-Hydroxy-Carbonsäure oder ω-Hydroxy-Carbonsäureester sind dem Fachmann Verfahren bekannt. Dies sind Standardverfahren zur Isolierung von langkettigen Carbonsäuren aus wässrigen Lösung, wie beispielsweise Destillation oder Extraktion und können beispielsweise auch der WO2009/077461 entnommen werden.

Es ist vorteilhaft, die durch erfindungsgemäßes Verfahren erhaltenen ω-Hydroxy-Carbonsäuren oder ω-Hydroxy-Carbonsäureester zur Herstellung von Polymeren, insbesondere Polyestern, zu verwenden. Des Weiteren können aus den ω-Hydroxy-Carbonsäuren auch Lactone hergestellt werden, die dann zum Beispiel wiederum zur Herstellung von Polyestern eingesetzt werden können.
Eine weitere vorteilhafte Verwendung ist es, die ω-Hydroxy-Carbonsäuren oder ω-Hydroxy-Carbonsäureester zu ω-Amino-Carbonsäuren oder ω-Amino-Carbonsäureestern umzuwandeln, um als Polymer Polyamide zu erhalten. Die ω-Amino-Carbonsäuren oder ω-Amino-Carbonsäureestern können auch erst zu den entsprechenden Lactamen umgesetzt werden, die dann wiederum anionisch, oder auch säurekatalysiert zu einem Polyamid umgesetzt werden können. Ganz besonders vorteilhaft ist es, in einem ersten Reaktionsschritt die ω-Hydroxy-Carbonsäuren oder entsprechenden Ester in die ω-oxo-Carbonsäuren oder den entsprechenden Ester umzuwandeln und anschließend die Aminierung der Oxo-Gruppe z.B. im Zuge einer reduktiven Aminierung durchzuführen.

In diesem Zusammenhang ist die Verwendung von 12-Hydroxy-Dodekansäure oder 12-Hydroxy-Dodekansäuremethylester zur Herstellung von Polymeren, insbesondere von Polyamid 12 besonders bevorzugt.

## Patentansprüche

1. *Candida tropicalis* Zelle, **dadurch gekennzeichnet, dass** die Zelle im Vergleich zu Ihrem Wildtyp eine verminderte Aktivität mindestens eines der Enzyme aufweist,
welche kodiert werden durch die intronfreien Nukleinsäuresequenzen ausgewählt aus den beiden Gruppen A) und B) bestehend aus
A) Seq ID Nr. 63,
B) eine Sequenz, die zu mindestens 80 % identisch zu Seq ID Nr. 63 ist,
wobei unter der Aktivität der Enzyme die enzymatische Aktivität zu verstehen ist, die die Umsetzungen von 12-Hydroxy-Dodekansäure zu 1,12-Dodekandisäure durch die gesamte Zelle katalysiert,
weiterhin **dadurch gekennzeichnet, dass** die Verminderung der enzymatischen Aktivität erreicht wird durch Modifikation eines Genes umfassend eine der vorgenannten Nukleinsäuresequenzen, wobei die Modifikation ausgewählt ist aus der Gruppe umfassend Insertion von Fremd-DNA in das Gen, Deletion mindestens von Teilen des Gens, Punktmutationen in der Gensequenz, Aussetzen des Gens unter den Einfluss von RNA-Interferenz und Austausch von Teilen des Gens mit Fremd-DNA, insbesondere des Promoterbereichs.

2. *Candida tropicalis* Zelle gemäß Anspruch 1, **dadurch gekennzeichnet, dass** die Fremd-DNA ein Selektionsmarkergen ist.

3. *Candida tropicalis* Zelle gemäß mindestens einem der vorherigen Ansprüche, **dadurch gekennzeichnet, dass** die Zelle in ihrer β-Oxidation mindestens teilweise blockiert ist.

4. *Candida tropicalis* Zelle gemäß mindestens einem der vorherigen Ansprüche, **dadurch gekennzeichnet, dass** die *Candida tropicalis* Zelle sich ableitet von Stämmen ausgewählt aus der Gruppe bestehend aus *Candida tropicalis* H41, *Candida tropicalis* H41B, *Candida tropicalis* H51, *Candida tropicalis* H45, *Candida tropicalis* H43, *Candida tropicalis* H53, *Candida tropicalis* H534, *Candida tropicalis* 534B, *Candida tropicalis* H435, *Candida tropicalis* ATCC20962 und *Candida tropicalis* HDC100, insbesondere bestehend aus *Candida tropicalis* ATCC20962 und *Candida tropicalis* HDC100.

5. Verwendung der Zellen gemäß mindestens einem der vorherigen Ansprüche zur Herstellung von ω-Hydroxy-Carbonsäuren oder ω-Hydroxy-Carbonsäureestern.

6. Verfahren zur Herstellung einer *C*. *tropicalis* Zelle nach mindestens einem der Ansprüche 1 bis 4 umfassend die Verfahrensschritte:
I) Bereitstellen einer *C*. *tropicalis* Zelle und
II) Modifikation mindestens eines Genes umfassend eine der Sequenzen ausgewählt aus den in Anspruch 1 genannten Nukleinsäuresequenzgruppen A) und B) durch Insertion von Fremd-DNA, insbesondere von DNA kodierend für ein Selektionsmarkergen, in das Gen, Deletion mindestens von Teilen des Gens, Punktmutationen in der Gensequenz, Aussetzen des Gens unter den Einfluss von RNA-Interferenz und Austausch von Teilen des Gens mit Fremd-DNA, insbesondere des Promoterbereichs.

7. Verfahren zur Herstellung von ω-Hydroxy-Carbonsäuren oder ω-Hydroxy-Carbonsäureestern, insbesondere von ω-Hydroxy-Carbonsäuren oder ω-Hydroxy-Carbonsäureestern mit einer Kettenlänge der Carbonsäure von 6 bis 24 Kohlenstoffatomen und einer Kettenlänge der Alkoholkomponente des Esters von 1 bis 4 Kohlenstoffatomen, insbesondere von 12-Hydroxy-Dodekansäure oder 12-Hydroxy-Dodekansäuremethylester umfassend die Verfahrensschritte
a) In Kontakt bringen der Zelle gemäß mindestens einem der Ansprüche 1 bis 4 mit einem Medium beinhaltend eine Carbonsäure bzw. einen Carbonsäureester,
b) Kultivieren der Zelle unter Bedingungen, die es der Zelle ermöglichen aus der Carbonsäure bzw. dem Carbonsäureester die korrespondierende ω-Hydroxy-Carbonsäure oder ω-Hydroxy-Carbonsäureester zu bilden und
c) gegebenenfalls Isolierung der gebildeten ω-Hydroxy-Carbonsäure oder ω-Hydroxy-Carbonsäureester

8. Verfahren zur Herstellung eines Polymers umfassend das Verfahren zur Herstellung von ω-Hydroxy-Carbonsäuren oder ω-Hydroxy-Carbonsäureestern gemäß Anspruch 7 und den Verfahrensschritt
Verwendung der ω-Hydroxy-Carbonsäuren oder der ω-Hydroxy-Carbonsäureester zur Herstellung eines Polymers.

9. Verfahren zur Herstellung von ω-Amino-Carbonsäuren oder ω-Amino-Carbonsäureestern umfassend die Verfahrensschritte
a) in Kontakt Bringen einer *Candida tropicalis* Zelle gemäß mindestens einem der Ansprüche 1 bis 4 mit einem Medium beinhaltend eine Carbonsäure bzw. einen Carbonsäureester,
b) Kultivieren der Zelle unter Bedingungen, die es der Zelle ermöglichen aus der Carbonsäure bzw. dem Carbonsäureester die korrespondierende ω-Hydroxy-Carbonsäure oder ω-Hydroxy-Carbonsäureester zu bilden,
c) gegebenenfalls Isolierung der gebildeten ω-Hydroxy-Carbonsäure oder ω-Hydroxy-Carbonsäureester und
einen Reaktionsschritt zur Umwandlung der ω-Hydroxy-Carbonsäuren oder entsprechenden Ester in die ω-oxo-Carbonsäuren oder den entsprechenden Ester und anschließend Durchführung der Aminierung der OxoGruppe.

10. Verfahren gemäß Anspruch 9, **dadurch gekennzeichnet, dass** die ω-Hydroxy-Carbonsäure oder der ω-Hydroxy-Carbonsäureester, eine Kettenlänge der Carbonsäure von 6 bis 24 Kohlenstoffatomen und eine Kettenlänge der Alkoholkomponente des Esters von 1 bis 4 Kohlenstoffatomen aufweisen.

11. Verfahren gemäß Anspruch 9 oder 10, **dadurch gekennzeichnet, dass** die ω-Hydroxy-Carbonsäure oder der ω-Hydroxy-Carbonsäureester 12-Hydroxy-Dodekansäure oder 12-Hydroxy-Dodekansäuremethylester darstellen.

## Claims

1. *Candida tropicalis* cell, **characterized in that** the cell has, compared with its wild type, a reduced activity of at least one of the enzymes that are encoded by the intron-free nucleic acid sequences selected from the two groups A) and B) consisting of
A) Seq ID No. 63,
B) a sequence that is identical to at least 80% to Seq ID No. 63,
where the activity of the enzymes is to be understood as meaning the enzymatic activity which catalyses the conversions of 12-hydroxydodecanoic acid into 1,12-dodecandioic acid by the entire cell,
furthermore **characterized in that** the decrease in enzymatic activity is achieved by modification of a gene comprising one of the abovementioned nucleic acid sequences, the modification being selected from the group comprising insertion of foreign DNA into the gene, deletion of at least parts of the gene, point mutations in the gene sequence, subjecting the gene to the influence of RNA interference and exchange of parts of the gene with foreign DNA, in particular of the promoter region.

2. *Candida tropicalis* cell according to Claim 1, **characterized in that** the foreign DNA is a selection marker gene.

3. *Candida tropicalis* cell according to at least one of the preceding claims, **characterized in that** the cell is blocked at least partially in its β-oxidation.

4. *Candida tropicalis* cell according to at least one of the preceding claims, **characterized in that** the *Candida tropicalis* cell is derived from strains selected from the group comprising *Candida tropicalis* H41, *Candida tropicalis* H41B, *Candida tropicalis* H51, *Candida tropicalis* H45, *Candida tropicalis* H43, *Candida tropicalis* H53, *Candida tropicalis* H534, *Candida tropicalis* 534B, *Candida tropicalis* H435, *Candida tropicalis* ATCC20962 and *Candida tropicalis* HDC100, in particular consisting of *Candida tropicalis* ATCC20962 and *Candida tropicalis* HDC100.

5. Use of the cells according to at least one of the preceding claims for the production of ω-hydroxycarboxylic acids or ω-hydroxycarboxylic acid esters.

6. A method of production of a *C*. *tropicalis* cell according to at least one of Claims 1 to 4 comprising the steps:
I) preparation of a *C*. *tropicalis* cell and
II) modification of at least one gene comprising one of the sequences selected from the nucleic acid sequence groups A) and B) stated in claim 1 by insertion of foreign DNA, in particular of DNA coding for a selection marker gene, into the gene, deletion at least of parts of the gene, point mutations in the gene sequence, subjecting the gene to the influence of RNA interference and exchange of parts of the gene with foreign DNA, in particular of the promoter region.

7. Method of production of ω-hydroxycarboxylic acids or ω-hydroxycarboxylic acid esters, in particular of ω-hydroxycarboxylic acids or ω-hydroxycarboxylic acid esters with a chain length of the carboxylic acid from 6 to 24 carbon atoms and a chain length of the alcohol component of the ester from 1 to 4 carbon atoms, in particular of 12-hydroxydodecanoic acid or 12-hydroxydodecanoic acid methyl ester comprising the steps
a) contacting the cell according to at least one of Claims 1 to 4 with a medium comprising a carboxylic acid or a carboxylic acid ester,
b) cultivating the cell under conditions that enable the cell to form the corresponding ω-hydroxycarboxylic acid or ω-hydroxycarboxylic acid esters from the carboxylic acid or the carboxylic acid ester and
c) optionally isolating the ω-hydroxycarboxylic acid or ω-hydroxycarboxylic acid esters that formed.

8. Method of production of a polymer, comprising the method of production of ω-hydroxycarboxylic acids or ω-hydroxycarboxylic acid esters according to Claim 7 and the method step
use of the ω-hydroxycarboxylic acids or of the ω-hydroxycarboxylic acid esters for the production of a polymer.

9. Method of production of ω-aminocarboxylic acids or ω-aminocarboxylic acid esters, comprising the method steps
a) contacting a *Candida tropicalis* cell according to at least one of Claims 1 to 4 with a medium comprising a carboxylic acid or a carboxylic acid ester,
b) cultivating the cell under conditions that enable the cell to form the corresponding ω-hydroxycarboxylic acid or ω-hydroxycarboxylic acid ester from the carboxylic acid or the carboxylic acid ester,
c) optionally isolating the ω-hydroxycarboxylic acid or ω-hydroxycarboxylic acid ester formed and
a reaction step for converting the ω-hydroxycarboxylic acids or corresponding esters into the ω-oxocarboxylic acids or the corresponding ester and subsequent amination of the oxo group.

10. Method according to Claim 9, **characterized in that** the ω-hydroxycarboxylic acid or the ω-hydroxycarboxylic acid ester has a carboxylic acid chain length of 6 to 24 carbon atoms and an alcohol component chain length of the ester of 1 to 4 carbon atoms.

11. Method according to Claim 9 or 10, **characterized in that** the ω-hydroxycarboxylic acid or the ω-hydroxycarboxylic acid ester is 12-hydroxydodecanoic acid or 12-hydroxydodecanoic acid methyl ester.

## Revendications

1. Cellule de Candida tropicalis, **caractérisée en ce que** la cellule présente, par rapport à son type sauvage, une activité diminuée d'au moins une des enzymes,
qui sont codées par les séquences d'acide nucléique exemptes d'introns, choisies parmi les deux groupes A) et B) constitués par
A) la séquence SEQ ID NO 63,
B) une séquence qui est identique à raison d'au moins 80% à la séquence SEQ ID NO 63,
l'activité des enzymes étant l'activité enzymatique qui catalyse les transformations de l'acide 12-hydroxy-docécanoïque en acide 1,12-dodécanedioïque à travers toute la cellule,
en outre **caractérisée en ce que** la diminution de l'activité enzymatique est obtenue par modification d'un gène comprenant une des séquences d'acide nucléique susmentionnées, la modification étant choisie dans le groupe comprenant une insertion d'ADN étranger dans le gène, une délétion d'au moins certaines parties du gène, des mutations ponctuelles dans la séquence génique, l'extinction du gène sous l'influence d'une interférence par ARN et le remplacement de certaines parties du gène par de l'ADN étranger, en particulier de la région de promoteur.

2. Cellule de Candida tropicalis selon la revendication 1, **caractérisée en ce que** l'ADN étranger est un gène d'un marqueur de sélection.

3. Cellule de Candida tropicalis selon au moins l'une quelconque des revendications précédentes, **caractérisée en ce que** la cellule est au moins partiellement bloquée en ce qui concerne sa ß-oxydation.

4. Cellule de Candida tropicalis selon au moins l'une quelconque des revendications précédentes, **caractérisée en ce que** la cellule de Candida tropicalis est dérivée de souches choisies dans le groupe constitué par Candida tropicalis H41, Candida tropicalis H41B, Candida tropicalis H51, Candida tropicalis H45, Candida tropicalis H43, Candida tropicalis H53, Candida tropicalis H534, Candida tropicalis 534B, Candida tropicalis H435, Candida tropicalis ATCC20962 et Candida tropicalis HDC100, en particulier constitué par Candida tropicalis ATCC20962 et Candida tropicalis HDC100.

5. Utilisation des cellules selon au moins l'une quelconque des revendications précédentes pour la préparation d'acides ω-hydroxy-carboxyliques ou d'esters d'acide ω-hydroxy-carboxylique.

6. Procédé pour la production d'une cellules de C. tropicalis selon au moins l'une quelconque des revendications 1 à 4, comprenant les étapes de procédé de :
I) de mise à disposition d'une cellule de C. tropicalis et
II) de modification d'au moins un gène comprenant une des séquences choisies parmi les groupes de séquences d'acide nucléique A) et B) mentionnés dans la revendication 1, par insertion d'ADN étranger, en particulier d'ADN codant pour un gène de marqueur de sélection, dans le gène, par délétion d'au moins certaines parties du gène, par des mutations ponctuelles dans la séquence génique, par l'extinction du gène sous l'influence d'une interférence par ARN et par remplacement de certaines parties du gène par de l'ADN étranger, en particulier de la région de promoteur.

7. Procédé pour la préparation d'acides ω-hydroxy-carboxyliques ou d'esters d'acide ω-hydroxy-carboxylique, en particulier d'acides ω-hydroxy-carboxyliques ou d'esters d'acide ω-hydroxy-carboxylique présentant une longueur de chaîne de l'acide carboxylique de 6 à 24 atomes de carbone et présentant une longueur de chaîne du composant alcool de l'ester de 1 à 4 atomes de carbone, en particulier d'acide 12-hydroxy-docécanoïque ou d'ester méthylique de l'acide 12-hydroxy-dodécanoïque, comprenant les étapes de procédé
a) de mise en contact de la cellule selon au moins l'une quelconque des revendications 1 à 4 avec un milieu contenant un acide carboxylique ou un ester d'acide carboxylique,
b) de culture de la cellule dans des conditions qui permettent à la cellule de former, à partir de l'acide carboxylique ou à partir de l'ester d'acide carboxylique, l'acide ω-hydroxy-carboxylique ou l'ester d'acide ω-hydroxy-carboxylique correspondant et
c) le cas échéant d'isolement de l'acide ω-hydroxy-carboxylique formé ou de l'ester d'acide ω-hydroxy-carboxylique formé.

8. Procédé pour la préparation d'un polymère comprenant le procédé pour la préparation d'acides ω-hydroxy-carboxyliques ou d'esters d'acide ω-hydroxy-carboxylique selon la revendication 7 et l'étape de procédé
d'utilisation des acides ω-hydroxy-carboxyliques ou des esters d'acide ω-hydroxy-carboxylique pour la préparation d'un polymère.

9. Procédé pour la préparation d'acides ω-amino-carboxyliques ou d'esters d'acide ω-amino-carboxylique, comprenant les étapes de procédé
a) de mise en contact d'une cellule de Candida tropicalis selon au moins l'une quelconque des revendications 1 à 4 avec un milieu contenant un acide carboxylique ou un ester d'acide carboxylique,
b) de culture de la cellule dans des conditions qui permettent à la cellule de former, à partir de l'acide carboxylique ou à partir de l'ester d'acide carboxylique, l'acide ω-hydroxy-carboxylique ou l'ester d'acide ω-hydroxy-carboxylique correspondant,
c) le cas échéant d'isolement de l'acide ω-hydroxy-carboxylique formé ou de l'ester d'acide ω-hydroxy-carboxylique formé et
une étape de réaction pour la transformation des acides ω-hydroxy-carboxyliques ou des esters correspondants en acides ω-oxo-carboxyliques ou en esters correspondants et la réalisation consécutive de l'amination du groupe oxo.

10. Procédé selon la revendication 9, **caractérisé en ce que** l'acide ω-hydroxy-carboxylique ou l'ester d'acide ω-hydroxy-carboxylique présentent une longueur de chaîne de l'acide carboxylique de 6 à 24 atomes de carbone et une longueur de chaîne du composant alcool de l'ester de 1 à 4 atomes de carbone.

11. Procédé selon la revendication 9 ou 10, **caractérisé en ce que** l'acide ω-hydroxy-carboxylique ou l'ester d'acide ω-hydroxy-carboxylique représentent respectivement l'acide 12-hydroxydodécanoïque ou l'ester méthylique de l'acide 12-hydroxydodécanoïque.
